# EUROPEAN PATENT SPECIFICATION

(11) **EP 0 419 355 B1**
(45) Date of publication and mention of the grant of the patent: **09.02.2000**
(21) Application number: 90402590.5
(22) Date of filing: 19.09.1990
(51) Int. Cl.: C07K 14/00, A61K 39/04, C12N 15/31, G01N 33/569, C12Q 1/68

(54) **Recombinant polypeptides and peptides, nucleic acids coding for the same and use of these polypeptides and peptides in the diagnostic of tuberculosis**
Rekombinante Polypeptide und Peptide, dafür kodierende Nukleinsäuren und ihre Verwendung in der Tuberkulose-Diagnose
Polypeptides et peptides recombinantes, acides nucléiques lesencodantes, et l'utilisation de celles-ci pour le diagnostique de la tuberculose

(30) Priority: 19.09.1989 EP 89402571
(43) Date of publication of application: 27.03.1991
(73) Proprietor: N.V. INNOGENETICS S.A., 9052 Gent (BE)
(72) Inventor: Content,Jean, B-1640 Rhode St-Genèse (BE); De Wit,Lucas, B-2670 Puurs (BE); De Bruyn,Jacqueline, B-1640 Beersel (BE); Van Vooren,Jean Paul, B-1600 St-Pieters Leeuw (BE)

(56) References cited:
- EP-A- 0 288 306
- BE-A- 905 582
- CHEMICAL ABSTRACTS, vol. 99, no. 11, 12th September 1983, page 413, abstract no. 86251m, Columbus, Ohio, US; H. TASAKA et al.: "Purification and antigenic specificity of alpha protein (Yoneda and Fukui) from Mycobacterium tuberculosis and Mycobacterium intracellulare", & HIROSHIMA J. MED. SCI. 1983, 32(1), 1-8
- JOURNAL OF CLINICAL MICROBIOLOGY, vol. 25, no. 7, July 1987, pages 1176-1180, American Society for Microbiology; M.L. COHEN et al.: "Expression of proteins of Mycobacterium tuberculosis in Eschericha coli and potential of recombinant genes and proteins for development of diagnostic reagents"
- JOURNAL OF BACTERIOLOGY, vol. 170, no. 9. September 1988, pages 3847-3854, American Society for Microbiology; K. MATSUO et al.: "Cloning and expression of the Mycobacterium bovis BCG gene for extracellular alpha antigen"
- INT. ARCHS ALLERGY APPL. IMMUN., vol. 81, 1986, pages 307-314, S. Karger AG, Basel, CH; H.G. WIKER et al.: "MPB59, a widely cross-reacting protein of Mycobacterium bovis BCG"
- MICROBIAL PATHOGENESIS, vol. 2, 1987, pages 351-366, Academic Press Inc., London, GB; J. DE BRUYN et al.: "Purification, characterization and identification of a 32 kDa protein antigen of Mycobacterium bovis BCG"
- INFECTION AND IMMUNITY, vol. 57, no. 10, October 1989, pages 3123-3130, American Society for Microbiology; M. BORREMANS et al.: "Cloning, sequence determination, and expression of a 32-kilodalton-protein gene Mycobacterium tuberculosis"
- INFECTION AND IMMUNITY, vol. 59, 1991, page 3205
- INFECTION AND IMMUNITY, vol. 58, 1990, page 550
- JOURNAL OF CLINICAL MICROBIOLOGY, vol. 29, 1991, page 2348
- SCAND. J. IMMUNOL., vol. 36, 1992, page 307
- INT. ARCHS ALLERGY APPL. IMMUN., vol. 81, 1986, page 298
- RES. MICROBIOL., vol. 141, 1990, page 407

## Description

The invention relates to recombinant polypeptides and peptides, which can be used for the diagnosis of tuberculosis. The invention also relates to a process for preparing the above-said polypeptides and peptides, which are in a state of biological purity such that they can be used as part of the active principle in the preparation of vaccines against tuberculosis.

It also relates to nucleic acids coding for said polypeptides and peptides.

Furthermore, the invention relates to the in vitro diagnostic methods and kits using the above-said polypeptides and peptides and to the vaccines containing the above-said polypeptides and peptides as active principle against tuberculosis.

By "recombinant polypeptides or peptides" it is to be understood that it relates to any molecule having a polypeptidic chain liable to be produced by genetic engineering, through transcription and translation, of a corresponding DNA sequence under the control of appropriate regulation elements within an efficient cellular host. Consequently, the expression "recombinant polypeptides" such as is used herein does not exclude the possibility for the polypeptides to comprise other groups, such as glycosylated groups.

The term "recombinant" indeed involves the fact that the polypeptide has been produced by genetic engineering, particularly because it results from the expression in a cellular host of the corresponding nucleic acid sequences which have previously been introduced into the expression vector used in said host.

Nevertheless, it must be understood that this expression does not exclude the possibility for the polypeptide to be produced by a different process, for instance by classical chemical synthesis according to methods used in the protein synthesis or by proteolytic cleavage of larger molecules.

The expression "biologically pure" or "biological purity" means on the one hand a grade of purity such that the recombinant polypeptide can be used for the production of vaccinating compositions and on the other hand the absence of contaminants, more particularly of natural contaminants.

Tuberculosis remains a major disease in developing countries. The situation is dramatic in some countries, particularly where high incidence of tuberculosis among AIDS patients represents a new source of dissemination of the disease.

Tuberculosis is a chronic infectious disease in which cell-mediated immune mechanisms play an essential role both for protection against and control of the disease.

Despite BCG vaccination, and some effective drugs, tuberculosis remains a major global problem. Skin testing with tuberculin PPD (protein-purified derivative) largely used for screening of the disease is poorly specific, due to cross reactivity with other pathogenic or environmental saprophytic mycobacteria.

Moreover, tuberculin PPD when used in serological tests (ELISA) does not allow to discriminate between patients who have been vaccinated by BCG, or those who have been primo-infected, from those who are developing evolutive tuberculosis and for whom an early and rapid diagnosis would be necessary.

A protein with a molecular weight of 32-kDa has been purified (9) from zinc deficient Mycobacterium bovis BCG culture filtrate (8). This 32-kDa protein of M. bovis BCG has been purified from Sauton zinc deficient culture filtrate of M. bovis BCG using successively hydrophobic chromatography on Phenyl-Sepharose, ion exchange on DEAE-Sephacel and molecular sieving on Sephadex G-100. The final preparation has been found to be homogeneous as based on several analyses. This P₃₂ protein is a constituent of BCG cells grown in normal conditions. It represents about 3% of the soluble fraction of a cellular extract, and appears as the major protein released in normal Sauton culture filtrate. This protein has been found to have a molecular weight of 32 000 by SDS-polyacrylamide gel electrophoresis and by molecular sieving.

The NH₂-terminal amino acid sequence of the 32-kDa protein of M. bovis BCG (Phe-Ser-Arg-Pro-Gly-Leu) is identical to that reported for the MPB 59 protein purified from M. bovis BCG substrain Tokyo (34).

Purified P₃₂ of M. bovis BCG has been tested by various cross immunoelectrophoresis techniques, and has been shown to belong to the antigen 85 complex in the reference system for BCG antigens. It has been more precisely identified as antigen 85A in the closs reference system for BCG antigens (7).

Increased levels of immunoglobulin G antibodies towards the 32-kDa protein of M. bovis BCG could be detected in 70% of tuberculous patients (30).

Furthermore, the 32-kDa protein of M. bovis BCG induces specific lymphoproliferation and interferon(IFN-γ) production in peripheral blood leucocytes from patients with active tuberculosis (12) and PPD-positive healthy subjects. Recent findings indicate that the amount of 32-kDa protein of M. bovis BCG-induced IFN-γ in BCG-sensitized mouse spleen cells is under probable H-2 control (13). Finally, the high affinity of mycobacteria for fibronectin is related to proteins of the BCG 85 antigen complex (1).

Matsuo et al. (17) recently cloned the gene encoding the antigen α, a major protein secreted by BCG (substrain Tokyo) and highly homologous to MPB 59 antigen in its NH₂-terminal amino acid sequence, and even identical for its first 6 amino acids : Phe-Ser-Arg-Pro-Gly-Leu.

This gene was cloned by using a nucleotide probe homologous to the N-terminal amino acid sequence of antigen α, purified from M. tuberculosis as described in Tasaka, H. et al., 1983. "Purification and antigenic specificity of alpha protein (Yoneda and Fukui) from Mycobacterium tuberculosis and Mycobacterium intracellulare. Hiroshima J. Med. Sci. 32, 1-8.

The presence of antigens of around 30-32-kDa, named antigen 85 complex, has been revealed from electrophoretic patterns of proteins originating from culture media of mycobacteria, such as Mycobacterium tuberculosis. By immunoblotting techniques, it has been shown that these antigens cross-react with rabbit sera raised against the 32-kDa protein of BCG (8).

A recent study reported on the preferential humoral response to a 30-kDa and 31-kDa antigen in lepromatous leprosy patients, and to a 32-kDa antigen in tuberculoid leprosy patients (24).

It has also been found that fibronectin (FN)-binding antigens are prominent components of short-term culture supernatants of Mycobacterium tuberculosis. In 3-day-old supernatants, a 30-kilodalton (kDa) protein was identified as the major (FN)-binding molecule. In 21-day-old supernatants, FN was bound to a double protein band of around 30 to 32-kDa, as well as to a group of antigens of larger molecular mass (57 to 60 kDa)(1).

In other experiments, recombinant plasmids containing DNA from Mycobacterium tuberculosis were transformed into Escherichia coli, and three colonies were selected by their reactivity with polyclonal antisera to M. tuberculosis. Each recombinant produced 35- and 53-kilodalton proteins (35K and 53K proteins, respectively) ("Expression of Proteins of Mycobacterium tuberculosis in Escherichia coli and Potential of Recombinant Genes and Proteins for Development of Diagnostic Reagents", Mitchell L Cohen et al., Journal of Clinical Microbiology, July 1987, p.1176-1180).

Concerning the various results known to date, the physico-chemical characteristics of the antigen P₃₂ of Mycobacterium tuberculosis are not precise and, furthermore, insufficient to enable its unambiguous identifiability, as well as the characterization of its structural and functional elements.

Moreover, the pathogenicity and the potentially infectious property of M. tuberculosis has hampered research enabling to identify, purify and characterize the constituents as well as the secretion products of these bacteria.

An aspect of the invention is to provide recombinant polypeptides which can be used as purified antigens for the detection and control of tuberculosis.

Another aspect of the invention is to provide nucleic acids coding for the peptidic chains of biologically pure recombinant polypeptides which enable their preparation on a large scale.

Another aspect of the invention is to provide antigens which can be used in serological tests as an in vitro rapid diagnostic of tuberculosis.

Another aspect of the invention is to provide a rapid in vitro diagnostic means for tuberculosis, enabling it to discriminate between patients suffering from an evolutive tuberculosis from those who have been vaccinated against BCG or who have been primo-infected.

Another aspect of the invention is to provide nucleic acid probes which can be used as an in vitro diagnostic reagent for tuberculosis, as well as in vitro diagnostic reagent for identifying M. tuberculosis from other strains of mycobacteria.

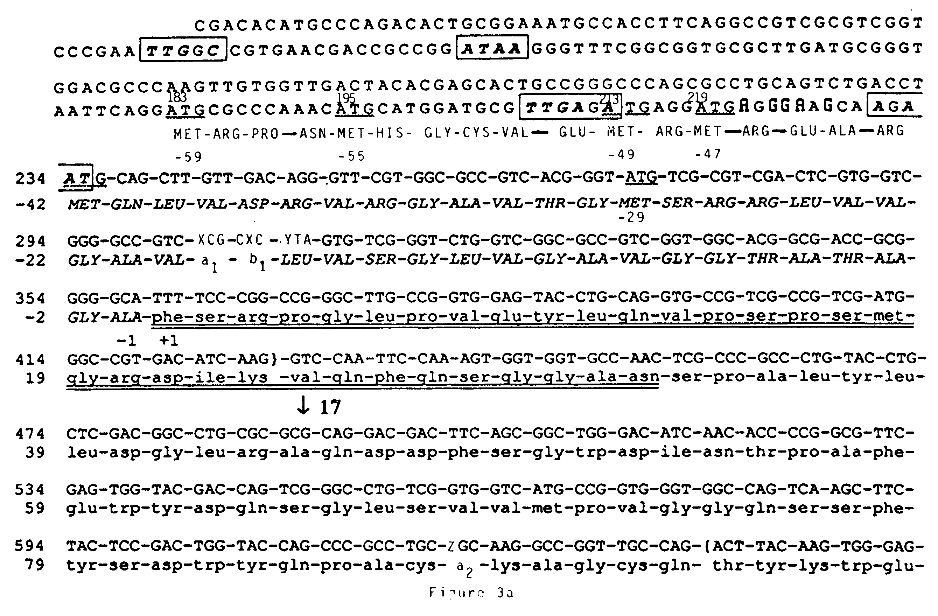

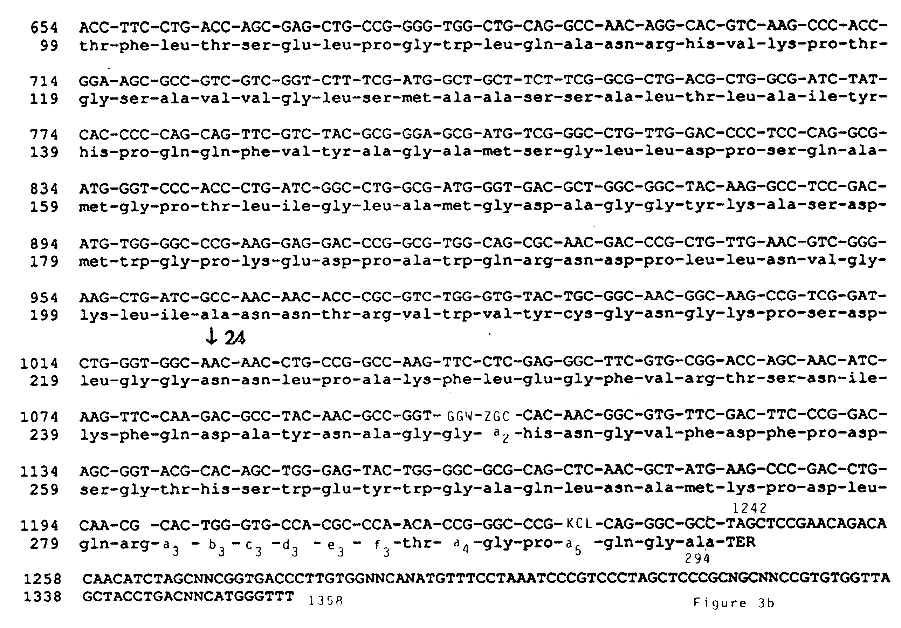

The biologically pure recombinant polypeptides of the invention contain in their polypeptidic chain one at least of the following amino acid sequences:
- the one extending from the extremity constituted by amino acid at position (-29) to the extremity constituted by amino acid at position (-1) represented on fig. 3a and fig. 3b, or
- the one extending from the extremity constituted by amino acid at position (12) to the extremity constituted by amino acid at position (31) represented on fig. 3a and fig. 3b, or
- the one extending from the extremity constituted by amino acid at position (36) to the extremity constituted by amino acid at position (55) represented on fig. 3a and fig. 3b, or
- the one extending from the extremity constituted by amino acid at position (77) to the extremity constituted by amino acid at position (96) represented on fig. 3a and fig. 3b, or
- the one extending from the extremity constituted by amino acid at position (101) to the extremity constituted by amino acid at position (120) represented on fig. 3a and fig. 3b, or
- the one extending from the extremity constituted by amino acid at position (175) to the extremity constituted by amino acid at position (194) represented on fig. 3a and fig. 3b, or
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b, or
- the one extending from the extremity constituted by amino acid at position (1) to the extremity constituted by amino acid at position (294) represented on fig. 3a and fig. 3b,
- the one extending from the extremity constituted by amino acid at position (-42) to the extremity constituted by amino acid at position (-1) represented on fig. 3a and fig. 3b,
- the one extending from the extremity constituted by amino acid at position (-47) to the extremity constituted by amino acid at position (-1) represented on fig. 3a and fig. 3b,
- the one extending from the extremity constituted by amino acid at position (-49) to the extremity constituted by amino acid at position (-1) represented on fig. 3a and fig. 3b,
- the one extending from the extremity constituted by amino acid at position (-55) to the extremity constituted by amino acid at position (-1) represented on fig. 3a and fig. 3b,
- the one extending from the extremity constituted by amino acid at position (-59) to the extremity constituted by amino acid at position (-1) represented on fig. 3a and fig. 3b,
- the one extending from the extremity constituted by amino acid at position (-29) to the extremity constituted by amino acid at position (294) represented on fig. 3a and fig. 3b,
- the one extending from the extremity constituted by amino acid at position (-42) to the extremity constituted by amino acid at position (294) represented on fig. 3a and fig. 3b,
- the one extending from the extremity constituted by amino acid at position (-47) to the extremity constituted by amino acid at position (294) represented on fig. 3a and fig. 3b,
- the one extending from the extremity constituted by amino acid at position (-49) to the extremity constituted by amino acid at position (294) represented on fig. 3a and fig. 3b,
- the one extending from the extremity constituted by amino acid at position (-55) to the extremity constituted by amino acid at position (294) represented on fig. 3a and fig. 3b,
- the one extending from the extremity constituted by amino acid at position (-59) to the extremity constituted by amino acid at position (294) represented on fig. 3a and fig. 3b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

On figures 3a and 3b :
- X represents G or GG,
- Y represents C or CC,
- Z represents C or G,
- W represents C or G and is different from Z,
- K represents C or CG,
- L represents G or CC,
- a₁-b₁ represents ALA-ARG or GLY-ALA-ALA,
- a₂ represents arg or gly,
- a₃-b₃-c₃-d₃-e₃-f₃- represents his-trp-val-pro-arg-pro or
   ala-leu-gly-ala,
- a₄ represents pro or pro-asn-thr,
- a₅ represents pro or ala-pro.

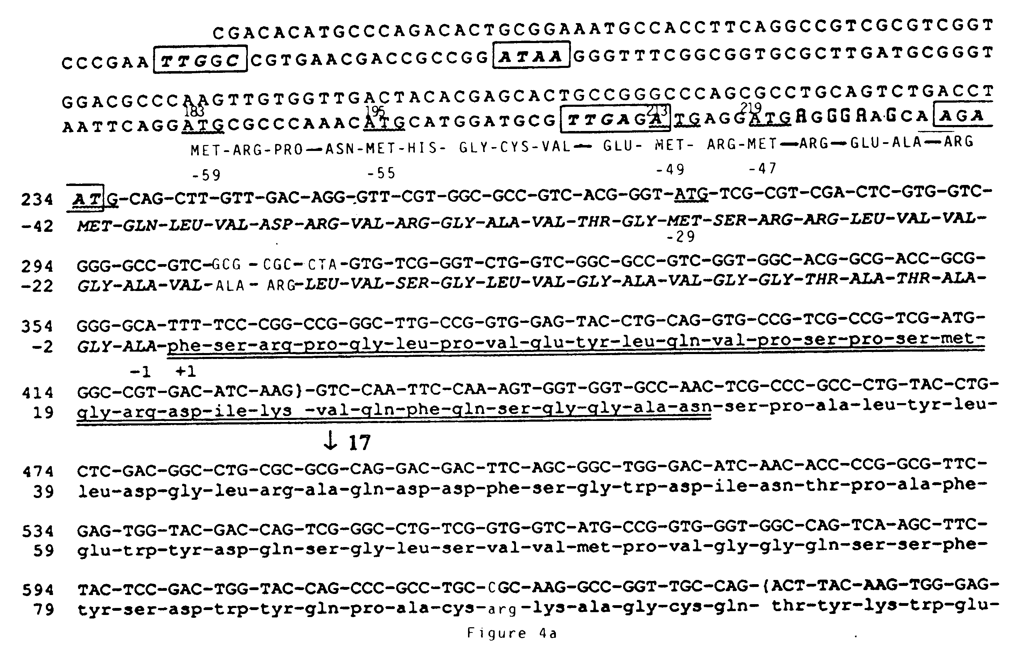

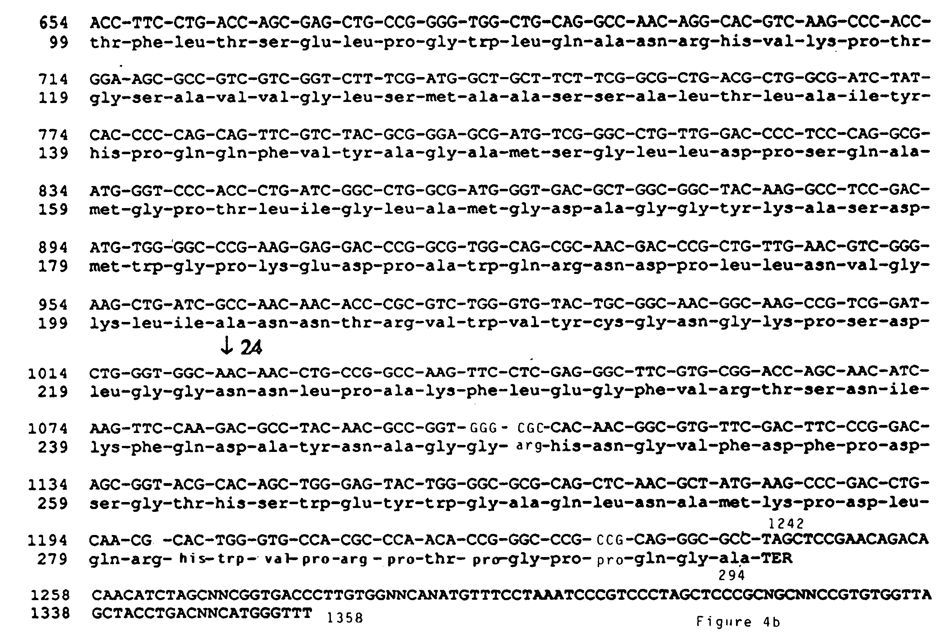

The biologically pure recombinant polypeptides of the invention contain in their polypeptidic chain one at least of the following amino acid sequences:
- the one extending from the extremity constituted by amino acid at position (-29) to the extremity constituted by amino acid at position (-1) represented on fig. 4a and fig. 4b, or
- the one extending from the extremity constituted by amino aid at position (12) to the extremity constituted by amino acid at position (31) represented on fig. 4a and fig. 4b, or
- the one extending from the extremity constituted by amino acid at position (36) to the extremity constituted by amino acid at position (55) represented on fig. 4a and fig, 4b, or
- the one extending from the extremity constituted by amino acid at position (77) to the extremity constituted by amino acid at position (96) represented on fig. 4a and fig. 4b, or
- the one extending from the extremity constituted by amino acid at position (101) to the extremity constituted by amino acid at position (120) represented on fig. 4a and fig. 4b, or
- the one extending from the extremity constituted by amino acid at position (175) to the extremity constituted by amino acid at position (194) represented on fig. 4a and fig. 4b, or
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b,
- the one extending from the extremity constituted by amino acid at position (1) to the extremity constituted by amino acid at position (294) represented on fig. 4a and fig. 4b,
- the one extending from the extremity constituted by amino acid at position (-42) to the extremity constituted by amino acid at position (-1) represented on fig. 4a and fig. 4b,
- the one extending from the extremity constituted by amino acid at position (-47) to the extremity constituted by amino acid at position (-1) represented on fig. 4a and fig. 4b,
- the one extending from the extremity constituted by amino acid at position (-49) to the extremity constituted by amino acid at position (-1) represented on fig. 4a and fig. 4b,
- the one extending from the extremity constituted by amino acid at position (-55) to the extremity constituted by amino acid at position (-1) represented on fig. 4a and fig. 4b,
- the one extending from the extremity constituted by amino acid at position (-59) to the extremity constituted by amino acid at position (-1) represented on fig. 4a and fig. 4b,
- the one extending from the extremity constituted by amino acid at position (-29) to the extremity constituted by amino acid at position (294) represented on fig. 4a and fig. 4b,
- the one extending from the extremity constituted by amino acid at position (-42) to the extremity constituted by amino acid at position (294) represented on fig. 4a and fig. 4b,
- the one extending from the extremity constituted by amino acid at position (-47) to the extremity constituted by amino acid at position (294) represented on fig. 4a and fig. 4b,
- the one extending from the extremity constituted by amino acid at position (-49) to the extremity constituted by amino acid at position (294) represented on fig. 4a and fig. 4b,
- the one extending from the extremity constituted by amino acid at position (-55) to the extremity constituted by amino acid at position (294) represented on fig. 4a and fig. 4b,
- the one extending from the extremity constituted by amino acid at position (-59) to the extremity constituted by amino acid at position (294) represented on fig. 4a and fig. 4b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

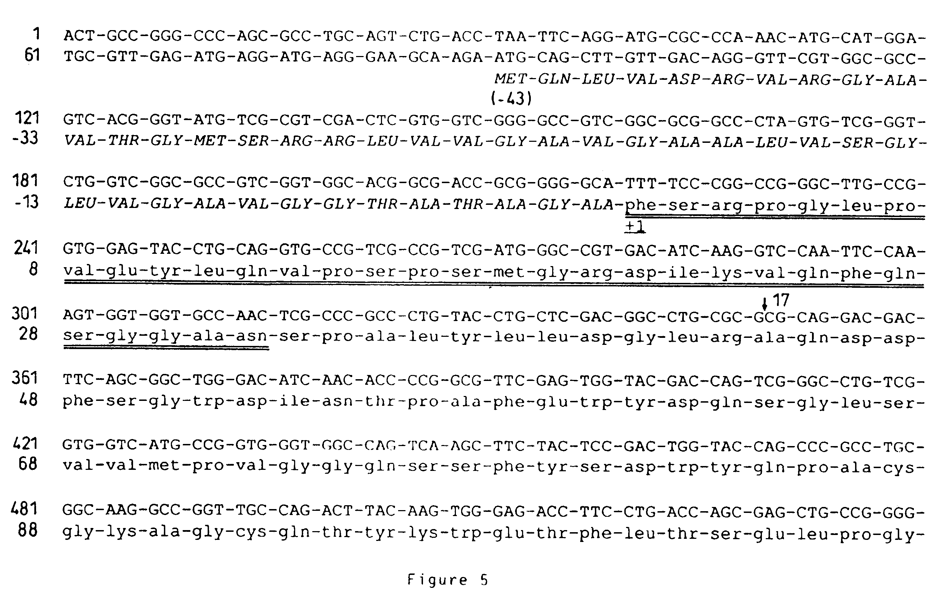

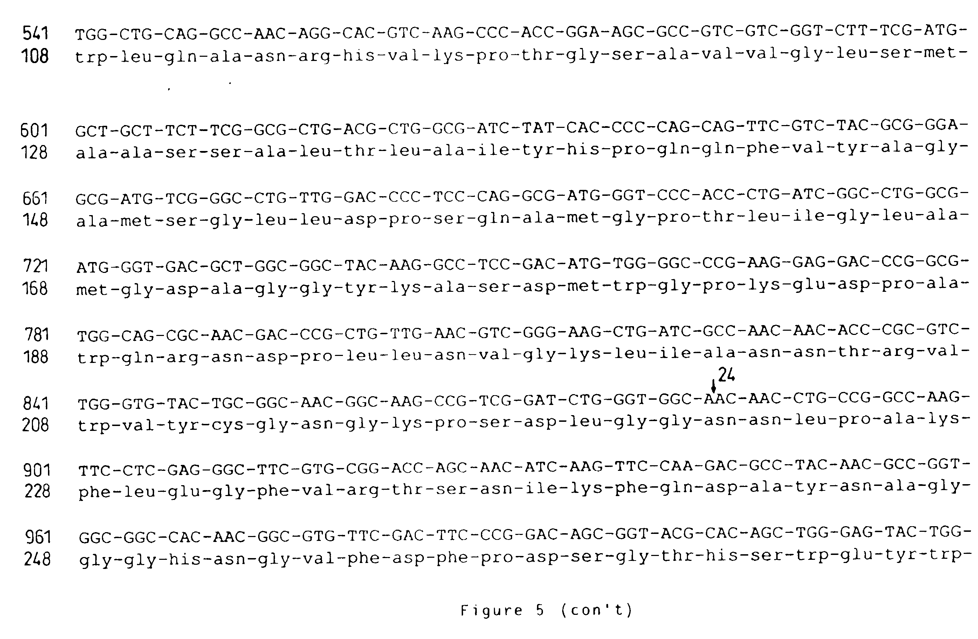

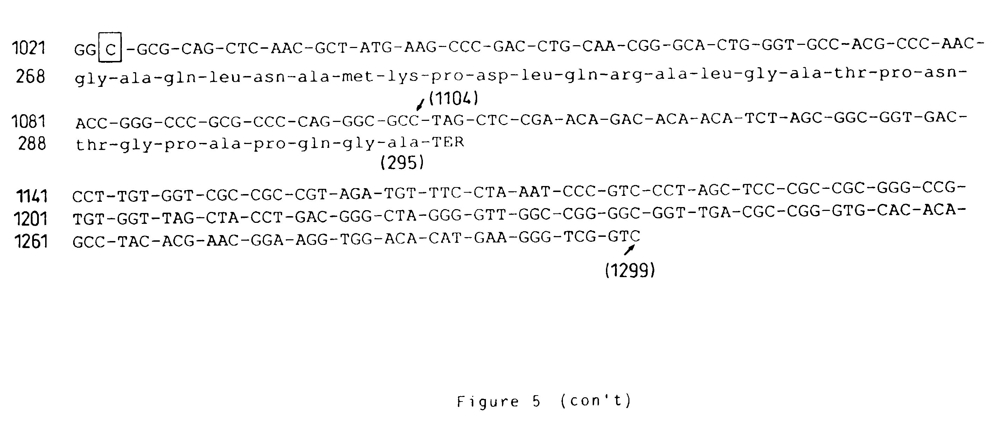

Preferably the biologically pure recombinant polypeptides of the invention contain in their polypeptide chain one at least of the following amino acid sequences :
- the one extending from the extremity constituted by amino acid at position (-30) to the extremity constituted by amino acid at position (-1) represented on fig. 5, or
- the one extending from the extremity constituted by amino acid at position (12) to the extremity constituted by amino acid at position (31) represented on fig. 5, or
- the one extending from the extremity constituted by amino acid at position (36) to the extremity constituted by amino acid at position (55) represented on fig. 5, or
- the one extending from the extremity constituted by amino acid at position (77) to the extremity constituted by amino acid at position (96) represented on fig. 5, or
- the one extending from the extremity constituted by amino acid at position (101) to the extremity constituted by amino acid at position (120) represented on fig. 5, or
- the one extending from the extremity constituted by amino acid at position (175) to the extremity constituted by amino acid at position (194) represented on fig. 5, or
- the one extending from the extremity constituted by amino acid at position (211) to the extremity constituted by amino acid at position (230) represented on fig. 5, or
- the one extending from the extremity constituted by amino acid at position (275) to the extremity constituted by amino acid at position (295) represented on fig. 5, or
- the one extending from the extremity constituted by amino acid at position (1) to the extremity constituted by amino acid at position (295) represented on fig. 5,
- the one extending from the extremity constituted by amino acid at position (-43) to the extremity constituted by amino acid at position (-1) represented on fig. 5.
- the one extending from the extremity constituted by amino acid at position (-30) to the extremity constituted by amino acid at position (295) represented on fig. 5,
- the one extending from the extremity constituted by amino acid at position (-43) to the extremity constituted by amino acid at position (295) represented on fig. 5, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

Advantageous polypeptides of the invention are characterized by the fact that they react with rabbit polyclonal antiserum raised against the protein of 32-kDa of M. bovis BCG culture filtrate, hereafter designated by "P₃₂ protein of BCG".

Advantageous polypeptides of the invention are characterized by the fact that they selectively react with human sera from tuberculous patients and particularly patients developing an evolutive tuberculosis at an early stage.

Hereafter is given, in a non limitative way a process for preparing rabbit polyclonal antiserum raised against the P₃₂ protein of BCG and a test for giving evidence of the reaction between the polypeptides of the invention and said rabbit polyclonal antiserum raised against the P₃₂ protein of BCG.

### 1) process for preparing rabbit polyclonal antiserum raised against the P₃₂ protein of BCG:

Purified P₃₂ protein of BCG from culture filtrate is used.

### a) Purification of protein P₃₂ of BCG

P₃₂ protein can be purified as follows :
The bacterial strains used are M. bovis BCG substrains 1173P2 (Pasteur Institute, Paris) and GL2 (Pasteur Institute, Brussels).

The culture of bacteria is obtained as follows :
Mycobacterium bovis BCG is grown as a pellicle on Sauton medium, at 37.5°C for 14 days. As the medium is prepared with distilled water, zinc sulfate is added to the final concentration of 5 µM (normal Sauton medium) (De Bruyn J., Weckx M., Beumer-Jochmans M.-P. Effect of zinc deficiency on Mycobacterium tuberculosis var. bovis (BCG). J. Gen. Microbiol. 1981; 124:353-7). When zinc deficient medium was needed, zinc sulfate is omitted.

The filtrates from zinc deficient cultures are obtained as follows :
The culture medium is clarified by decantation. The remaining bacteria are removed by filtration through Millipak 100 filter unit (Millipore Corp., Bedford, Mass.). When used for purification, the filtrate is adjusted to 20 mM in phosphate, 450 mM in NaCl, 1 mM in EDTA, and the pH is brought to 7.3 with 5 M HCl before sterile filtration.

The protein analysis is carried out by polyacrylamide gel electrophoresis. Sodium dodecyl sulfate polyacrylamide gel electrophoresis (SDS-PAGE) was done on 13% (w/v) acrylamide-containing gels as described by Laemmli UK. (Cleavage of structural proteins during the assembly of the head of bacteriophage T4. Nature 1970; 227:680-5). The gels are stained with Coomassie Brilliant Blue R-250 and for quantitative analysis, scanned at 595 nm with a DU8 Beckman spectrophotometer. For control of purity the gel is revealed with silver stain (Biorad Laboratories, Richmond, Calif.).

The purification step of P₃₂ is carried out as follows:
Except for hydrophobic chromatography on Phenyl-Sepharose, all buffers contain Tween 80 (0.005% final concentration). The pH is adjusted to 7.3 before sterilization. All purification steps are carried out at +4°C. Elutions are followed by recording the absorbance at 280 nm. The fractions containing proteins are analysed by SDS-PAGE.
(i) The treated filtrate from a 4 liters zinc-deficient culture, usually containing 125 to 150 mg protein per liter, is applied to a column (5.0 by 5.0 cm) of Phenyl-Sepharose CL-4B (Pharmacia Fine Chemicals, Uppsala, Sweden), which is previously equilibrated with 20 mM phosphate buffer (PB) containing 0.45 M NaCl and 1 mM EDTA, at a flow rate of 800 ml per hour. The gel is then washed with one column volume of the same buffer to remove unfixed material and successively with 300 ml of 20 mM and 4 mM PB and 10% ethanol (v/v). The P₃₂ appears in the fraction eluted with 10% ethanol.
(ii) After the phosphate concentration of this fraction has been brought to 4 mM, it is applied to a column (2.6 by 10 cm) of DEAE-Sephacel (Pharmacia Fine Chemicals), which is equilibrated with 4 mM PB. After washing with the equilibrating buffer the sample is eluted with 25 mM phosphate at a flow rate of 50 ml per hour. The eluate is concentrated in a 202 Amicon stirred cell equipped with a PM 10 membrane (Amicon Corp., Lexington, Mass.).
(iii) The concentrated material is submitted to 4 mg of P₃₂ protein of BCG (soluble extract) or molecular sieving on a Sephadex G-100 (Pharmacia) column (2.6 by 45 cm) equilibrated with 50 mM PB, at a flow rate of 12 ml per hour. The fractions of the peak giving one band in SDS-PAGE are pooled. The purity of the final preparation obtained is controlled by SDS-PAGE followed by silverstaining and by molecular sieving on a Superose 12 (Pharmacia) column (12.0 by 30 cm) equilibrated with 50 mM PB containing 0.005% Tween 80 at a flow rate of 0.2 ml/min. in the Fast Protein Liquid Chromatography system (Pharmacia). Elution is followed by recording the absorbance at 280 nm and 214 nm.

### b) Preparation of rabbit polyclonal antiserum raised against the P₃₂ protein of BCG :

400 µg of purified P₃₂ protein of BCG per ml physiological saline are mixed with one volume of incomplete Freund's adjuvant. The material is homogenized and injected intradermally in 50 µl doses delivered at 10 sites in the back of the rabbits, at 0, 4, 7 and 8 weeks (adjuvant is replaced by the diluent for the last injection). One week later, the rabbits are bled and the sera tested for antibody level before being distributed in aliquots and stored at -80°C;

### 2) test for giving evidence of the reaction between the polypeptides of the invention and said rabbit polyclonal antiserum raised against the P₃₂ protein of BCG:

the test used was an ELISA test; the ELISA for antibody determination is based on the method of Engvall and Perlmann (Engvall, E., and P. Perlmann. 1971. Enzyme-linked immunosorbent assay (ELISA). Quantitative assay of immunoglobulin G. Immunochemistry 8:871-874)

Immulon Microelisa plates (Dynatech, Kloten, Switzerland) are coated by adding to each well 1 µg of one of the polypeptides of the invention in 100 µl Tris hydrochloride buffer 50 mM (pH 8.2). After incubation for 2 h at 27°C in a moist chamber, the plates are kept overnight at 4°C. They are washed four times with
0.01 M phosphate-buffered saline (pH 7.2) containing 0.05% Tween 20 by using a Titertek microplate washer (Flow Laboratories. Brussels. Belgium). Blocking is done with 0.5% gelatin in 0.06 M carbonate buffer (pH 9.6) for 1 h. Wells are then washed as before, and
100 µl of above mentioned serum diluted in phosphate-buffered saline containing 0.05% Tween 20 and 0.5% gelatin is added. According to the results obtained in preliminary experiments, the working dilutions are set at 1:200 for IgG, 1:20 for IgA and 1:80 for IgM determinations. Each dilution is run in duplicate. After 2 h of incubation and after the wells are washed, they are filled with 100 µl of peroxidase-conjugated rabbit immunoglobulins directed against human IgG, IgA or IgM (Dakopatts, Copenhagen, Denmark), diluted 1:400, 1:400 and 1:1.200, respectively in phosphate-buffered saline containing 0.05% Tween 20 and 0.5% gelatin and incubated for
90 min. After the wash, the amount of peroxidase bound to the wells is quantified by using a freshly prepared solution of o-phenylenediamine (10 mg/100 ml) and hydrogen peroxide (8µl of 30% H₂O₂ per 100 ml) in
0.15 M citrate buffer (pH 5.0) as a substrate. The enzymatic reaction is stopped with 8 N H₂SO₄ after 15 min. of incubation. The optical density is read at 492 nm with a Titertek Multiskan photometer (Flow Laboratories).

Wells without sera are used as controls for the conjugates. Each experiment is done by including on each plate one negative and two positive reference sera with medium and low antibody levels to correct for plate-to-plate and day-to-day variations. The antibody concentrations are expressed as the optical density values obtained after correction of the readings according to the mean variations of the reference sera.

Hereafter is also given in a non limitative way, a test for giving evidence of the fact that polypeptides of the invention are recognized selectively by human sera from tuberculous patients.

This test is an immunoblotting (Western blotting) analysis, in the case where the polypeptides of the invention are obtained by recombinant techniques. This test can also be used for polypeptides of the invention obtained by a different preparation process. After sodium dodecyl sulfate-polyacrylamide gel electrophoresis, polypeptides of the invention are blotted onto nitrocellulose membranes (Hybond C. (Amersham)) as described by Towbin et al. (29). The expression of polypeptides of the invention fused to β-galactosidase in E. coli Y1089, is visualized by the binding of a polyclonal rabbit anti-32-kDa BCG protein serum (1:1,000) or by using a monoclonal anti-β-galactosidase antibody (Promega). The secondary antibody (alkaline phosphatase anti-rabbit immunoglobulin G and anti-mouse alkaline phosphatase immunoglobulin G conjugates, respectively) is diluted as recommended by the supplier (Promega).

In order to identify selective recognition of polypeptides of the invention and of fusion proteins of the invention by human tuberculous sera, nitrocellulose sheets are incubated overnight with these sera (1:50) (after blocking aspecific protein-binding sites). The human tuberculous sera are selected for their reactivity (high or low) against the purified 32-kDa antigen of BCG tested in a dot blot assay as described in document (31) of the bibliography hereafter. Reactive areas on the nitrocellulose sheets are revealed by incubation with peroxidase conjugated goat anti-human immunoglobulin G antibody (Dakopatts, Copenhagen, Denmark) (1:200) for 4h, and after repeated washings, color reaction is developed by adding peroxidase substrate (α-chloronaphtol) (Bio-Rad Laboratories, Richmond, Calif.) in the presence of peroxidase and hydrogen peroxide.

It goes without saying that the free reactive functions which are present in some of the amino acids, which are part of the constitution of the polypeptides of the invention, particularly the free carboxyl groups which are carried by the groups Glu or by the C-terminal amino acid on the one hand and/or the free NH₂ groups carried by the N-terminal amino acid or by amino acid inside the peptidic chain, for instance Lys, on the other hand, can be modified in so far as this modification does not alter the above mentioned properties of the polypeptide.

The molecules which are thus modified are naturally part of the invention. The above mentioned carboxyl groups can be acylated or esterified.

Other modifications are also part of the invention. Particularly, the amine or ester functions or both of terminal amino acids can be themselves involved in the bond with other amino acids. For instance, the N-terminal amino acid can be linked to a sequence comprising from 1 to several amino acids corresponding to a part of the C-terminal region of another peptide.

The polypeptides according to the invention can be glycosylated or not, particularly in some of their glycosylation sites of the type Asn-X-Ser or Asn-X-Thr, X representing any amino acid.

Advantageous recombinant polypeptides of the invention consist of one of the following amino acid sequences:
- the one extending from the extremity constituted by amino acid at position (-59) to the extremity constituted by amino acid at position (294) represented on fig. 3a and fig. 3b,
- the one extending from the extremity constituted by amino acid at position (-59) to the extremity constituted by amino acid at position (-1) represented on fig. 3a and fig. 3b,
- the one extending from the extremity constituted by amino acid at position (-55) to the extremity constituted by amino acid at position (294) represented on fig. 3a and fig. 3b,
- the one extending from the extremity constituted by amino acid at position (-55) to the extremity constituted by amino acid at position (-1) represented on fig. 3a and fig. 3b,
- the one extending from the extremity constituted by amino acid at position (-49) to the extremity constituted by amino acid at position (294) represented on fig. 3a and fig. 3b,
- the one extending from the extremity constituted by amino acid at position (-49) to the extremity constituted by amino acid at position (-1) represented on fig. 3a and fig. 3b,
- the one extending from the extremity constituted by amino acid at position (-47) to the extremity constituted by amino acid at position (294) represented on fig. 3a and fig. 3b,
- the one extending from the extremity constituted by amino acid at position (-47) to the extremity constituted by amino acid at position (-1) represented on fig. 3a and fig. 3b,
- the one extending from the extremity constituted by amino acid at position (-42) to the extremity constituted by amino acid at position (294) represented on fig. 3a and fig. 3b,
- the one extending from the extremity constituted by amino acid at position (-42) to the extremity constituted by amino acid at position (-1) represented on fig. 3a and fig. 3b,
- the one extending from the extremity constituted by amino acid at position (-29) to the extremity constituted by amino acid at position (294) represented on fig. 3a and fig. 3b,
- the one extending from the extremity constituted by amino acid at position (-29) to the extremity constituted by amino acid at position (-1) represented on fig. 3a and fig. 3b,
- the one extending from the extremity constituted by amino acid at position (1) to the extremity constituted by amino acid at position (294) represented on fig. 3a and fig. 3b,
- the one extending from the extremity constituted by amino acid at position (12) to the extremity constituted by amino acid at position (31) represented on fig. 3a and fig. 3b,
- the one extending from the extremity constituted by amino acid at position (36) to the extremity constituted by amino acid at position (55) represented on fig. 3a and fig. 3b,
- the one extending from the extremity constituted by amino acid at position (77) to the extremity constituted by amino acid at position (96) represented on fig. 3a and fig. 3b,
- the one extending from the extremity constituted by amino acid at position (101) to the extremity constituted by amino acid at position (120) represented on fig. 3a and fig. 3b,
- the one extending from the extremity constituted by amino acid at position (175) to the extremity constituted by amino acid at position (194) represented on fig. 3a and fig. 3b,
- the one extending from the extremity constituted by amino acid at position (211) to the extremity constituted by amino acid at position (230) represented on fig. 3a and fig. 3b,
- the one extending from the extremity constituted by amino acid at position (275) to the extremity constituted by amino acid at position (294) represented on fig. 3a and fig. 3b,

Other advantageous recombinant polypetides of the invention, consist of one of the following amino acid sequences :
- the one extending from the extremity constituted by amino acid at position (-59) to the extremity constituted by amino acid at position (294) represented on fig. 4a and fig. 4b,
- the one extending from the extremity constituted by amino acid at position (-59) to the extremity constituted by amino acid at position (-1) represented on fig. 4a and fig. 4b,
- the one extending from the extremity constituted by amino acid at position (-55) to the extremity constituted by amino acid at position (294) represented on fig. 4a and fig. 4b,
- the one extending from the extremity' constituted by amino acid at position (-55) to the extremity constituted by amino acid at position (-1) represented on fig. 4a and fig. 4b,
- the one extending from the extremity constituted by amino acid at position (-49) to the extremity constituted by amino acid at position (294) represented on fig. 4a and fig. 4b,
- the one extending from the extremity constituted by amino acid at position (-49) to the extremity constituted by amino acid at position (-1) represented on fig. 4a and fig. 4b,
- the one extending from the extremity constituted by amino acid at position (-47) to the extremity constituted by amino acid at position (294) represented on fig. 4a and fig. 4b,
- the one extending from the extremity constituted by amino acid at position (-47) to the extremity constituted by amino acid at position (-1) represented on fig. 4a and fig. 4b,
- the one extending from the extremity constituted by amino acid at position (-42) to the extremity constituted by amino acid at position (294) represented on fig. 4a and fig. 4b,
- the one extending from the extremity constituted by amino acid at position (-42) to the extremity constituted by amino acid at position (-1) represented on fig. 4a and fig. 4b,
- the one extending from the extremity constituted by amino acid at position (-29) to the extremity constituted by amino acid at position (294) represented on fig. 4a and fig. 4b,
- the one extending from the extremity constituted by amino acid at position (-29) to the extremity constituted by amino acid at position (-1) represented on fig. 4a and fig. 4b,
- the one extending from the extremity constituted by amino acid at position (1) to the extremity constituted by amino acid at position (294) represented on fig. 4a and fig. 4b,
- the one extending from the extremity constituted by amino acid at position (12) to the extremity constituted by amino acid at position (31) represented on fig. 4a and fig. 4b,
- the one extending from the extremity constituted by amino acid at position (36) to the extremity constituted by amino acid at position (55) represented on fig. 4a and fig. 4b,
- the one extending from the extremity constituted by amino acid at position (77) to the extremity constituted by amino acid at position (96) represented on fig. 4a and fig. 4b,
- the one extending from the extremity constituted by amino acid at position (101) to the extremity constituted by amino acid at position (120) represented on fig. 4a and fig. 4b,
- the one extending from the extremity constituted by amino acid at position (175) to the extremity constituted by amino acid at position (194) represented on fig. 4a and fig. 4b,
- the one extending from the extremity constituted by amino acid at position (211) to the extremity constituted by amino acid at position (230) represented on fig. 4a and fig. 4b,
- the one extending from the extremity constituted by amino acid at position (275) to the extremity constituted by amino acid at position (294) represented on fig. 4a and fig. 4b,

Other advantageous recombinant polypeptides of the invention, consist of one of the following amino acid sequences :
- the one extending from the extremity constituted by amino acid at position (-43) to the extremity constituted by amino acid at position (295) represented on fig. 5,
- the one extending from the extremity constituted by amino acid at position (-43) to the extremity constituted by amino acid at position (-1) represented on fig. 5,
- the one extending from the extremity constituted by amino acid at position (-30) to the extremity constituted by amino acid at position (295) represented on fig. 5,
- the one extending from the extremity constituted by amino acid at position (-30) to the extremity constituted by amino acid at position (-1) represented on fig. 5,
- the one extending from the extremity constituted by amino acid at position (1) to the extremity constituted by amino acid at position (295) represented on fig. 5,
- the one extending from the extremity constituted by amino acid at position (12) to the extremity constituted by amino acid at position (31) represented on fig. 5,
- the one extending from the extremity constituted by amino acid at position (36) to the extremity constituted by amino acid at position (55) represented on fig. 5,
- the one extending from the extremity constituted by amino acid at position (77) to the extremity constituted by amino acid at position (96) represented on fig. 5,
- the one extending from the extremity constituted by amino acid at position (101) to the extremity constituted by amino acid at position (120) represented on fig. 5,
- the one extending from the extremity constituted by amino acid at position (175) to the extremity constituted by amino acid at position (194) represented on fig. 5,
- the one extending from the extremity constituted by amino acid at position (211) to the extremity constituted by amino acid at position (230) represented on fig. 5,
- the one extending from the extremity constituted by amino acid at position (275) to the extremity constituted by amino acid at position (295) represented on fig. 5.

It is to be noted that the above mentioned polypeptides are derived from the expression products of a DNA derived from the nucleotide sequence coding for a protein of 32-kDa secreted by Mycobacterium tuberculosis as explained hereafter in the examples.

The invention also relates to biologically pure recombinant polypeptides constituted by the above mentioned polypeptides and a protein having a heterologous sequence with respect to said polypeptide, said heterologous sequence comprising for instance from about 1 to about 1000 amino acids. These recombinant polypeptides will be called fusion proteins.

In an advantageous fusion protein of the invention, the heterologous protein is β-galactosidase.

Other advantageous fusion proteins of the invention are the ones containing an heterologous protein resulting from the expression of one of the following plasmids:
pEX1
pEX2
pEX3
pUEX1 pmTNF MPH
pUEX2
pUEX3

The invention also relates to any nucleotide sequence coding for a polypeptide of the invention.

The invention also relates to nucleic acids comprising nucleotide sequences which are complementary to the nucleotide sequences coding for any of the above mentioned polypeptides.

It is to be noted that in the above defined nucleic acids, as well as in the hereafter defined nucleic acids, the nucleotide sequences which are brought into play are such that T can be replaced by U.

A group of preferred nucleic acids of the invention comprises one at least of the following nucleotide sequences:
- the one extending from the extremity constituted by nucleotide at position (1) to the extremity constituted by nucleotide at position (182) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (1242) to the extremity constituted by nucleotide at position (1358), wherein N represents one of the five A, T, C, G or I nucleotides, represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (195) to the extremity constituted at position (1358) represented in fig. 3a and fig. 3b,
- the one extending from the extremity constituted by nucleotide at position (195) to the extremity constituted at position (1241) represented in fig. 3a and fig. 3b,
- the one extending from the extremity constituted by nucleotide at position (213) to the extremity constituted at position (1358) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1358) represented in fig. 3a and fig. 3b, or the complementary sequences thereof,
   or the above said nucleotide sequences wherein T is replaced by U.

Other preferred nucleic acids of the invention comprise one at least of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (1242) to the extremity constituted by nucleotide at position (1358), wherein N represents one of the five A, T, C, G or I nucleotides, represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (183) to the extremity constituted by nucleotide at position (1358) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1358) represented in fig. 4a and fig. 4b, or the complementary sequences thereof,
   or the above said nucleotide sequences wherein T is replaced by U.

Other preferred nucleic acids of the invention comprise one at least of the following nucleotide sequences :
- the one extending from the extremity constituted by nucleotide at position (130) to the extremity constituted by nucleotide at position (219) represented in fig. 5,
- the one extending from the extremity constituted by nucleotide at position (220) to the extremity constituted by nucleotide at position (1104) represented in fig. 5,
- the one extending from the extremity constituted by nucleotide at position (1105) to the extremity constituted by nucleotide at position (1299),
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1299) represented in fig. 5,
- the one extending from the extremity constituted by nucleotide at position (90) to the extremity constituted by nucleotide at position (1104) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity constituted by nucleotide at position (1299) represented in fig. 5,
- the one extending from the extremity constituted by nucleotide at position (220) to the extremity constituted by nucleotide at position (1299) represented in fig. 5, or the complementary sequences thereof,
   or the above said nucleotide sequences wherein T is replaced by U.

According to a preferred embodiment, nucleic acids of the current invention consist of one of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (360) to the extremity constituted by nucleotide at position (1358) represented in fig. 3a and fig. 3b,
- the one extending from the extremity constituted by nucleotide at position (1242) to the extremity constituted by nucleotide at position (1358) represented in fig. 3a and fig. 3b, or the complementary sequences thereof,
   or the above said nucleotide sequences wherein T is replaced by U.

According to another embodiment, preferred nucleic acids of the invention consist of one of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity constituted by nucleotide at position (183) to the extremity constituted by nucleotide at position(1358) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (360) to the extremity constituted by nucleotide at position (1358) represented in fig. 4a and fig. 4b,
- the one extending from the extremity constituted by nucleotide at position (1242) to the extremity constituted by nucleotide at position (1358) represented in fig. 4a and fig. 4b, or the complementary sequences thereof,
   or the above said nucleotide sequences wherein T is replaced by U.

According to another embodiment, preferred nucleic acids of the invention consist of one of the following nucleotide sequences :
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1104) represented in fig. 5,
- the one extending from the extremity constituted by nucleotide at position (90) to the extremity constituted by nucleotide at position (1299) represented in fig. 5,
- the one extending from the extremity constituted by nucleotide at position (130) to the extremity constituted by nucleotide at position (219) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity constituted by nucleotide at position (1299) represented in fig. 5,
- the one extending from the extremity constituted by nucleotide at position (220) to the extremity constituted by nucleotide at position (1104) represented in fig. 5,
- the one extending from the extremity constituted by nucleotide at position (220) to the extremity constituted by nucleotide at position (1299) represented in fig. 5,
- the one extending from the extremity constituted by nucleotide at position (1105) to the extremity constituted by nucleotide at position (1299) represented in fig. 5, or the complementary sequences thereof,
   or the above said nucleotide sequences wherein T is replaced by U.

The invention also relates to any recombinant nucleic acids containing at least a nucleic acid of the invention inserted in an heterologous nucleic acid.

The invention relates more particularly to recombinant nucleic acid such as defined, in which the nucleotide sequence of the invention is preceded by a promoter (particularly an inducible promoter) under the control of which the transcription of said sequence is liable to be processed and possibly followed by a sequence coding for transcription termination signals.

The invention also relates to the recombinant nucleic acids in which the nucleic acid sequences coding for the polypeptide of the invention and possibly the signal peptide, are recombined with control elements which are heterologous with respect to the ones to which they are normally associated within the bacteria gene and, more particularly, the regulation elements adapted to control their expression in the cellular host which has been chosen for their production.

The invention also relates to recombinant vectors, particularly for cloning and/or expression, comprising a vector sequence, notably of the type plasmid, cosmid or phage, and a recombinant nucleic acid of the invention, in one of the non essential sites for its replication.

Appropriate vectors for expression of the recombinant antigen are the following ones:
pEX1, pmTNF MPH,
pEX2, PIGRI,
pEX3,
pUEX1,
pUEX2,
pUEX3,

The pEX1, pEX2 and pEX3 vectors are commercially available and can be obtained from Boehringer Mannheim.

The pUEX1, pUEX2 and pUEX3 vectors are also commercially available and can be obtained from Amersham.

According to an advantageous embodiment of the invention, the recombinant vector contains, in one of its non essential sites for its replication, necessary elements to promote the expression of polypeptides according to the invention in a cellular host and possibly a promoter recognized by the polymerase of the cellular host, particularly an inducible promoter and possibly a signal sequence and/or an anchor sequence.

According to another additional embodiment of the invention, the recombinant vector contains the elements enabling the expression by E. coli of a nucleic acid according to the invention inserted in the vector, and particularly the elements enabling the expression of the gene or part thereof of β-galactosidase.

The invention also relates to a cellular host which is transformed by a recombinant vector according to the invention, and comprising the regulation elements enabling the expression of the nucleotide sequence coding for the polypeptide according to the invention in this host.

The invention also relates to a cellular host chosen from among bacteria such as E. coli, transformed by a vector as above defined, and defined hereafter in the examples, or chosen from among eukaryotic organism, such as CHO cells, insect cells, Sf9 cells [Spodoptera frugiperda] infected by the virus Ac NPV (Autographa californica nuclear polyhydrosis virus) containing suitable vectors such as pAc 373 pYM1 or pVC3, BmN [Bombyx mori] infected by the virus BmNPV containing suitable vectors such as pBE520 or p89B310.

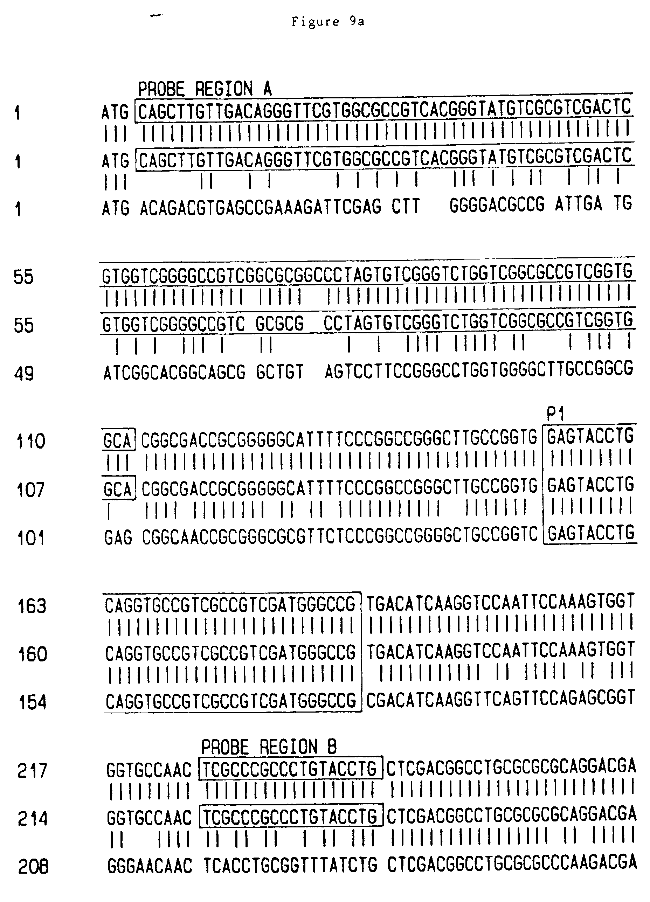

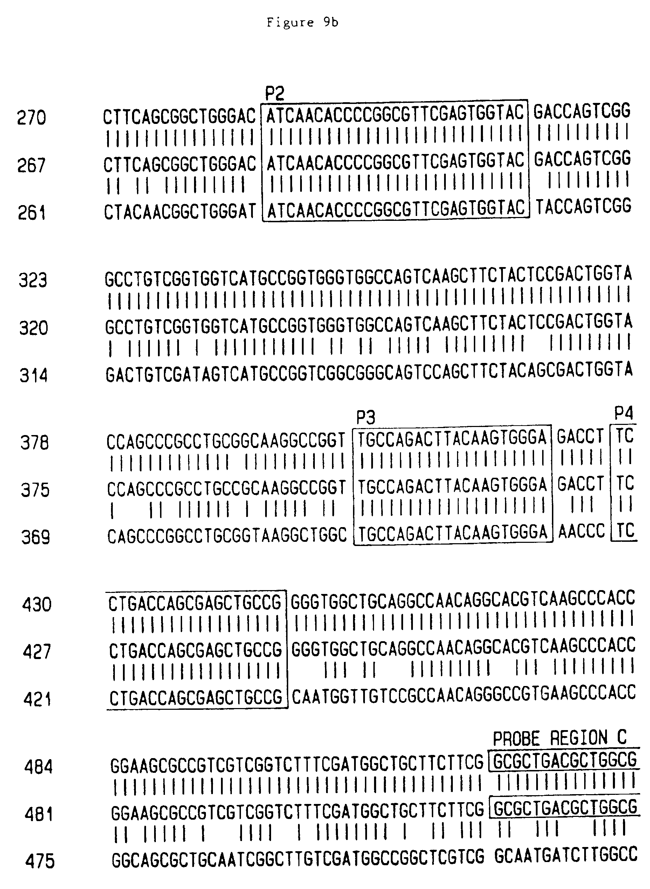

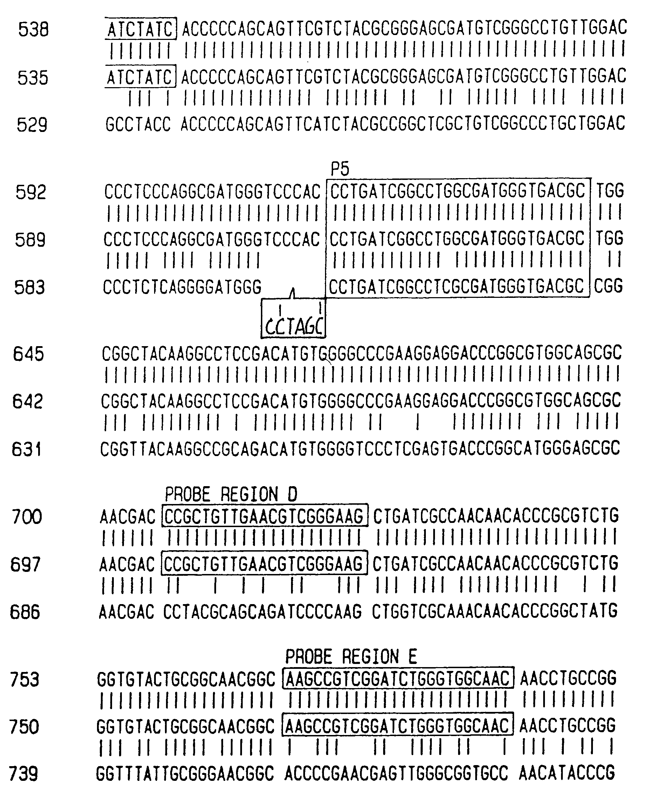

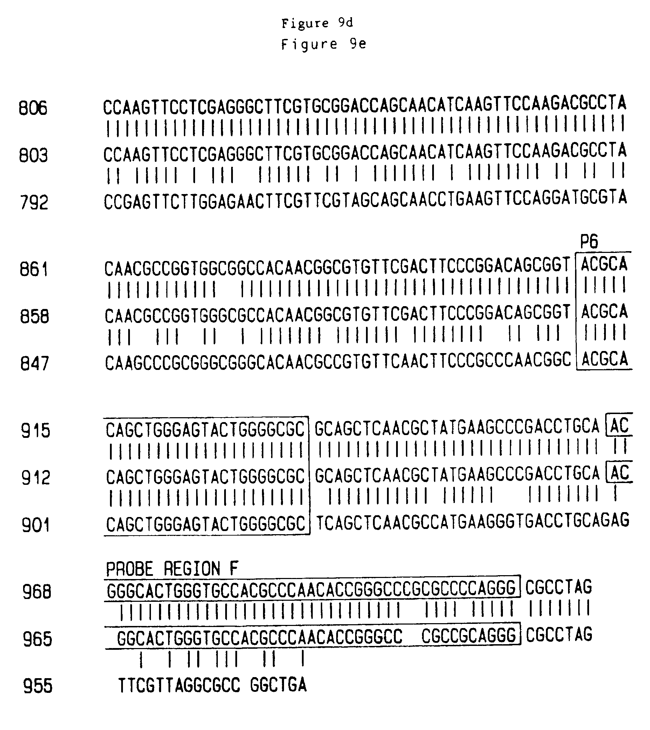

The invention also relates to nucleotidic probes, hybridizing with anyone of the nucleic acids or with their complementary sequences, and particularly the probes chosen among the following nucleotidic sequences gathered in Table 1, and represented in fig. 9.

**TABLE 1**

| Probes A(i), A(ii), A(iii), A(iv) and A(v) | |
|---|---|
| A(i) | CAGCTTGTTGACAGGGTTCGTGGC |
| A(ii) | GGTTCGTGGCGCCGTCACG |
| A(iii) | CGTCGCGCGCCTAGTGTCGG |
| A(iv) | CGGCGCCGTCGGTGGCACGGCGA |
| A(v) | CGTCGGCGCGGCCCTAGTGTCGG |

| Probe B | |
|---|---|
| TCGCCCGCCCTGTACCTG | |

| Probe C | |
|---|---|
| GCGCTGACGCTGGCGATCTATC | |

| Probe D | |
|---|---|
| CCGCTGTTGAACGTCGGGAAG | |

| Probe E | |
|---|---|
| AAGCCGTCGGATCTGGGTGGCAAC | |

| Probes F(i), F(ii), F(iii) and F(iv) | |
|---|---|
| F (i) | ACGGCACTGGGTGCCACGCCCAAC |
| F(ii) | ACGCCCAACACCGGGCCCGCCGCA |
| F(iii) | ACGGGCACTGGGTGCCACGCCCAAC |
| F(iv) | ACGCCCCAACACCGGGCCCGCGCCCCA |
| or their complementary nucleotidic sequences. | |

The hybridization conditions can be the following ones:
- hybridization and wash medium: 3 X SSC, 20% formamide (1 X SSC is 0,15 M NaCl, 0.015 M sodium citrate, pH 7.0),
- hybridization temperature (HT) and wash temperature (WT):

| (WT) °C: | HT and WT (°C) |
|---|---|
| A(i) | 50 |
| A(ii) | 50 |
| A(iii) | 52 |
| A(iv) | 60 |
| A(v) | 52 |
| B | 48 |
| C | 50 |
| D | 45 |
| E | 52 |
| F(i) | 55 |
| F(ii) | 59 |
| F(iii) | 55 |
| F(iv) | 59 |

These probes might enable to differentiate M. tuberculosis from other bacterial strains and in particular from the following mycobacteria species:
- Mycobacterium marinum, Mycobacterium scrofulaceum, Mycobacterium gordonae, Mycobacterium szulgai, Mycobacterium intracellulare, Mycobacterium xenopi, Mycobacterium gastri, Mycobacterium nonchromogenicum, Mycobacterium terrae and Mycobacterium triviale, and more particularly from M. bovis, Mycobacterium kansasii, Mycobacterium avium, Mycobacterium phlei and Mycobacterium fortuitum.

The invention also relates to DNA or RNA primers which can be used for the synthesis of nucleotidic sequences according to the invention by PCR (polymerase chain reaction technique), such as described in US Patents n° 4,683,202 and n° 4,683,195 and European Patent n° 200362.

The invention also relates to any DNA or RNA primer constituted by about 15 to about 25 nucleotides of a nucleotide sequence coding for a polypeptide according to the invention, and specifically amplifying said nucleotide sequence.

The invention also relates to any DNA or RNA primer constituted by about 15 to about 25 nucleotides complementary to a nucleotide sequence coding for a polypeptide according to the invention, and specifically amplifying said nucleotide sequence.

The sequences which can be used as primers are given in Table 2 hereafter (sequences P1 to P6 or their complement) and illustrated in fig. 9 :

**TABLE 2**

| | |
|---|---|
| P1 | GAGTACCTGCAGGTGCCGTCGCCGTCGATGGGCCG |
| P2 | ATCAACACCCCGGCGTTCGAGTGGTAC |
| P2 compl. | GTACCACTCGAACGCCGGGGTGTTGAT |
| P3 | TGCCAGACTTACAAGTGGGA |
| P3 compl. | TCCCACTTGTAAGTCTGGCA |
| P4 | TCCTGACCAGCGAGCTGCCG |
| P4 compl. | CGGCAGCTCGCTGGTCAGGA |
| P5 | CCTGATCGGCCTGGCGATGGGTGACGC |
| P5 compl. | GCGTCACCCATCGCCAGGCCGATCAGG |
| P6 | ACGCACAGCTGGGAGTACTGGGGCGC |
| P6 compl. | GCGCCCCAGTACTCCCAGCTGTGCGT |
| compl. = complementary sequence | |

The sequences can be combined in twelve different primer-sets (given in Table 3) which allow enzymatical amplification by the polymerase chain reaction (PCR) technique of any of the nucleotide sequences of the invention, and more particularly the one extending from the extremity constituted by nucleotide at position 1 to the extremity constituted by nucleotide at position 1358, as well as the nucleotide sequence of antigen α of BCG (17).

The detection of the PCR amplified product can be achieved by a hybridization reaction with an oligonucleotide sequence of at least 10 nucleotides which is located between PCR primers which have been used to amplify the DNA.

The PCR products of the nucleotide sequences of the invention can be distinguished from the α-antigen gene of BCG or part thereof by hybridization techniques (dot-spot, Southern blotting, etc.) with the probes indicated in Table 3. The sequences of these probes can be found in Table 1 hereabove.

**TABLE 3**

| Primer set | | Detection with probe |
|---|---|---|
| 1. | P1 and the complementary sequence of P2 | B |
| 2. | P1 and the complementary sequence of P3 | B |
| 3. | P1 and the complementary sequence of P4 | B |
| 4. | P1 and the complementary sequence of P5 | B or C |
| 5. | P1 and the complementary sequence of P6 | B, C, D or E |
| 6. | P2 and the complementary sequence of P5 | C |
| 7. | P2 and the complementary sequence of P6 | C, D or E |
| 8. | P3 and the complementary sequence of P5 | C |
| 9. | P3 and the complementary sequence of P6 | C, D or E |
| 10. | P4 and the complementary sequence of P5 | C |
| 11. | P4 and the complementary sequence of P6 | C, D or E |
| 12. | P5 and the complementary sequence of P6 | D or E |

It is to be noted that enzymatic amplification can also be achieved with all oligonucleotides with sequences of about 15 consecutive bases of the primers given in Table 2. Primers with elongation at the 5'-end or with a small degree of mismatch may not considerably affect the outcome of the enzymatic amplification if the mismatches do not interfere with the base-pairing at the 3'-end of the primers.

Specific enzymatic amplification of the nucleotide sequences of the invention and not of the BCG gene can be achieved when the probes (given in Table 1) or their complements are used as amplification primers.

When the above mentioned probes of Table 1 are used as primers, the primer sets are constituted by any of the nucleotide sequences (A, B, C, D, E, F) of Table 1 in association with the complementary sequence of any other nucleotide sequence, chosen from A, B, C, D, E or F, it being understood that sequence A means any of the sequences A(i), A(ii), A(iii), A(iv), A(v) and sequence F, any of the sequences F(i), F(ii), F(iii) and F(iv).

Advantageous primer sets for enzymatic amplification of the nucleotide sequence of the invention can be one of the following primer sets given in Table 3bis hereafter:

**TABLE 3BIS**

| | |
|---|---|
| A(i) | and the complementary sequence of B |
| or A(ii) | |
| or A(iii) | |
| or A(iv) | |
| or A(v) | |
| A(i) | and the complementary sequence of C |
| or A(ii) | |
| or A(iii) | |
| or A(iv) | |
| or A(v) | |
| B | and the complementary sequence of C |
| A(i) | and the complementary sequence of F |
| or A(ii) | |
| or A(iii) | |
| or A(iv) | |
| or A(v) | |
| A(i) | and the complementary sequence of D |
| or A(ii) | |
| or A(iii) | |
| or A(iv) | |
| or A(v) | |
| A(i) | and the complementary sequence of E |
| or A(ii) | |
| or A(iii) | |
| or A(iv) | |
| or A(v) | |
| B | and the complementary sequence of D |
| B | and the complementary sequence of E |
| B | and the complementary sequence of F |
| C | and the complementary sequence of D |
| C | and the complementary sequence of E |
| C | and the complementary sequence of F |
| D | and the complementary sequence of E |
| D | and the complementary sequence of F |
| E | and the complementary sequence of F |
| A(i), A(ii), A(iii), A(iv), A(v), B, C, D, E and F having the nucleotide sequence indicated in Table 1. | |

In the case of amplification of a nucleotide sequence of the invention with any of the above mentioned primer sets defined in Table 3bis hereabove, the detection of the amplified nucleotide sequence can be achieved by a hybridisation reaction with an oligonucleotide sequence of at least 10 nucleotides, said sequence being located between the PCR primers which have been used to amplify the nucleotide sequence. An oligonucleotide sequence located between said two primers can be determined from figure 9 where the primers A, B, C, D, E and F are represented by the boxed sequences respectively named probe region A, probe region B, probe region C, probe region D, probe region E and probe region F.

The invention also relates to a kit for enzymatic amplification of a nucleotide seguence by PCR technique and detection of the amplified nucleotide sequence containing
- one of the PCR primer sets defined in Table 3 and one of the detection probes of the invention, advantageously the probes defined in Table 1,
   or one of the PCR primer sets defined in Table 3bis, and a detection sequence consisting for instance in an oligonucleotide sequence of at least 10 nucleotides, said sequence being located (fig. 9) between the two PCR primers constituting the primer set which has been used for amplifying said nucleotide sequence.

The invention also relates to a process for preparing a polypeptide according to the invention comprising the following steps:
- the culture in an appropriate medium of a cellular host which has previously been transformed by an appropriate vector containing a nucleic acid according to the invention,
- the recovery of the polypeptide produced by the above said transformed cellular host from the above said culture medium, and
- the purification of the polypeptide produced, eventually by means of immobilized metal ion affinity chromatography (IMAC).

The polypeptides of the invention can be prepared according to the classical techniques in the field of peptide synthesis.

The synthesis can be carried out in homogeneous solution or in solid phase.

For instance, the synthesis technique in homogeneous solution which can be used is the one described by Houbenweyl in the book titled "Methode der organischen chemie" (Method of organic chemistry) edited by E. Wunsh, vol. 15-I et II. THIEME, Stuttgart 1974.

The polypeptides of the invention can also be prepared according to the method described by R.D. MERRIFIELD in the article titled "Solid phase peptide synthesis" (J.P. Ham.Socks. , 45, 2149-2154).

The invention also relates to a process for preparing the nucleic acids according to the invention.

A suitable method for chemically preparing the single-stranded nucleic acids (containing at most 100 nucleotides of the invention) comprises the following steps :
- DNA synthesis using the automatic β-cyanoethyl phosphoramidite method described in Bioorganic Chemistry 4; 274-325, 1986.

In the case of single-stranded DNA, the material which is obtained at the end of the DNA synthesis can be used as such.

A suitable method for chemically preparing the double-stranded nucleic acids (containing at most 100 bp of the invention) comprises the following steps:
- DNA synthesis of one sense oligonucleotide using the automatic β-cyanoethyl phosphoramidite method described in Bioorganic Chemistry 4; 274-325, 1986, and DNA synthesis of one anti-sense oligonucleotide using said above-mentioned automatic β-cyanoethyl phosphoramidite method,
- combining the sense and anti-sense oligonucleotides by hybridization in order to form a DNA duplex,
- cloning the DNA duplex obtained into a suitable plasmid vector and recovery of the DNA according to classical methods, such as restriction enzyme digestion and agarose gel electrophoresis.

A method for the chemical preparation of nucleic acids of length greater than 100 nucleotides - or bp, in the case of double-stranded nucleic acids - comprises the following steps :
- assembling of chemically synthesized oligonucleotides, provided at their ends with different restriction sites, the sequences of which are compatible with the succession of amino acids in the natural peptide, according to the principle described in Proc. Nat. Acad. Sci. USA 80; 7461-7465, 1983,
- cloning the DNA thereby obtained into a suitable plasmid vector and recovery of the desired nucleic acid according to classical methods, such as restriction enzyme digestion and agarose gel electrophoresis.

The invention also relates to monoclonal antibodies themselves formed against the polypeptides according to the invention.

The monoclonal antibodies of the invention may be produced by any hybridoma liable to be formed according to classical methods from splenic cells of an animal, particularly of a mouse or rat, immunized against the purified polypeptide of the invention on the one hand, and of cells of a myeloma cell line on the other hand, and to be selected by its ability to produce the monoclonal antibodies recognizing the polypeptide which has been initially used for the immunization of the animals.

The invention also relates to any monoclonal antibody of the invention labeled by an appropriate label of the enzymatic, fluorescent or radioactive type.

The peptides which are advantageously used to produce monoclonal antibodies, are the following ones gathered in Table 4:

**TABLE 4a**

| (see fig. 4a and 4b) | | |
|---|---|---|
| Amino acid position (NH₂-terminal) | | Amino acid position (COOH-terminal) |
| 12 | QVPSPSMGRDIKVQFQSGGA | 31 |
| 36 | LYLLDGLRAQDDFSGWDINT | 55 |
| 77 | SFYSDWYQPACRKAGCQTYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |

**TABLE 4b**

| (see fig. 5) | | |
|---|---|---|
| Amino acid position (NH₂-terminal) | | Amino acid position (COOH-terminal) |
| 77 | SFYSDWYQPACGKAGCQTYK | 96 |
| 276 | PDLQRALGATPNTGPAPQGA | 295 |

The amino acid sequences are given in the 1-letter code.

Variations of the peptides listed in Table 4 are also possible depending on their intended use. For example, if the peptides are to be used to raise antisera, the peptides may be synthesized with an extra cysteine residue added. This extra cysteine residue is preferably added to the amino terminus and facilitates the coupling of the peptide to a carrier protein which is necessary to render the small peptide immunogenic. If the peptide is to be labeled for use in radioimmune assays, it may be advantageous to synthesize the protein with a tyrosine attached to either the amino or carboxyl terminus to facilitate iodination. These peptides possess therefore the primary sequence of the peptides listed in Table 4 but with additional amino acids which do not appear in the primary sequence of the protein and whose sole function is to confer the desired chemical properties to the peptides.

The invention also relates to a process for detecting in vitro antibodies related to tuberculosis in a human biological sample liable to contain them, this process comprising
- contacting the biological sample with a polypeptide or a peptide according to the invention under conditions enabling an in vitro immunological reaction between said polypeptide and the antibodies which are possibly present in the biological sample and
- the in vitro detection of the antigen/antibody complex which may be formed.

Preferably, the biological medium is constituted by a human serum.

The detection can be carried out according to any classical process.

By way of example a preferred method brings into play an immunoenzymatic process according to ELISA technique or immunofluorescent or radioimmunological (RIA) or the equivalent ones.

Thus the invention also relates to any polypeptide according to the invention labeled by an appropriate label of the enzymatic, fluorescent, radioactive... type.

Such a method for detecting in vitro antibodies related to tuberculosis comprises for instance the following steps:
- deposit of determined amounts of a polypeptidic composition according to the invention in the wells of a titration microplate,
- introduction into said wells of increasing dilutions of the serum to be diagnosed,
- incubation of the microplate,
- repeated rinsing of the microplate,
- introduction into the wells of the microplate of labeled antibodies against the blood immunoglobulins,
- the labeling of these antibodies being carried out by means of an enzyme which is selected from among the ones which are able to hydrolyze a substrate by modifying the absorption of the radiation of this latter at least at a given wave length,
- detection by comparing with a control standard of the amount of hydrolyzed substrate.

The invention also relates to a process for detecting and identifying in vitro antigens of M. tuberculosis in a human biological sample liable to contain them, this process comprising:
- contacting the biological sample with an appropriate antibody of the invention under conditions enabling an in vitro immunological reaction between said antibody and the antigens of M. tuberculosis which are possibly present in the biological sample and the in vitro detection of the antigen/antibody complex which may be formed.

Preferably, the biological medium is constituted by sputum, pleural effusion liquid, broncho-alveolar washing liquid, urine, biopsy or autopsy material.

Appropriate antibodies are advantageously monoclonal antibodies directed against the peptides which have been mentioned in Table 4.

The invention also relates to an additional method for the in vitro diagnostic of tuberculosis in a patient liable to be infected by Mycobacterium tuberculosis comprising the following steps:
- the possible previous amplification of the amount of the nucleotide sequences according to the invention, liable to be contained in a biological sample taken from said patient by means of a DNA primer set as above defined,
- contacting the above mentioned biological sample with a nucleotide probe of the invention, under conditions enabling the production of an hybridization complex formed between said probe and said nucleotide sequence,
- detecting the above said hybridization complex which has possibly been formed.

To carry out the in vitro diagnostic method for tuberculosis in a patient liable to be infected by Mycobacterium tuberculosis as above defined, the following necessary or kit can be used, said necessary or kit comprising:
- a determined amount of a nucleotide probe of the invention,
- advantageously the appropriate medium for creating an hybridization reaction between the sequence to be detected and the above mentioned probe,
- advantageously, reagents enabling the detection of the hybridization complex which has been formed between the nucleotide sequence and the probe during the hybridization reaction.

The invention also relates to an additional method for the in vitro diagnostic of tuberculosis in a patient liable to be infected by Mycobacterium tuberculosis comprising :
- contacting a biological sample taken from a patient with a polypeptide or a peptide of the invention, under conditions enabling an in vitro immunological reaction between said polypeptide or peptide and the antibodies which are possibly present in the biological sample and
- the in vitro detection of the antigen/antibody complex which has possibly been formed.

To carry out the in vitro diagnostic method for tuberculosis in a patient liable to be infected by Mycobacterium tuberculosis, the following necessary or kit can be used, said necessary or kit comprising:
- a polypeptide or a peptide according to the invention,
- reagents for making a medium appropriate for the immunological reaction to occur,
- reagents enabling to detect the antigen/antibody complex which has been produced by the immunological reaction, said reagents possibly having a label, or being liable to be recognized by a labeled reagent, more particularly in the case where the above mentioned polypeptide or peptide is not labeled.

The invention also relates to an additional method for the in vitro diagnostic of tuberculosis in a patient liable to be infected by M. tuberculosis, comprising the following steps:
- contacting the biological sample with an appropriate antibody of the invention under conditions enabling an in vitro immunological reaction between said antibody and the antigens of M. tuberculosis which are possibly present in the biological sample and - the in vitro detection of the antigen/antibody complex which may be formed.

Appropriate antibodies are advantageously monoclonal antibodies directed against the peptides which have been mentioned in Table 4.

To carry out the in vitro diagnostic method for tuberculosis in a patient liable to be infected by Mycobacterium tuberculosis, the following necessary or kit can be used, said necessary or kit comprising:
- an antibody of the invention,
- reagents for making a medium appropriate for the immunological reaction to occur,
- reagents enabling to detect the antigen/antibody complexes which have been produced by the immunological reaction, said reagent possibly having a label or being liable to be recognized by a label reagent, more particularly in the case where the above mentioned antibody is not labeled.

An advantageous kit for the diagnostic in vitro of tuberculosis comprises:
- at least a suitable solid phase system, e.g. a microtiter-plate for deposition thereon of the biological sample to be diagnosed in vitro,
- a preparation containing one of the monoclonal antibodies of the invention,
- a specific detection system for said monoclonal antibody,
- appropriate buffer solutions for carrying out the immunological reaction between a test sample and said monoclonal antibody on the one hand, and the bonded monoclonal antibodies and the detection system on the other hand.

The invention also relates to a kit, as described above, also containing a preparation of one of the polypeptides or peptides of the invention, said antigen of the invention being either a standard (for quantitative determination of the antigen of M. tuberculosis which is sought) or a competitor, with respect to the antigen which is sought, for the kit to be used in a competition dosage process.

The invention also relates to a biologically pure recombinant polypeptide according to the invention, for use in an immunogenic composition, in association with a pharmaceutically acceptable vehicle.

The invention also relates to a biologically pure recombinant polypeptide of the invention for use in a vaccine composition, possibly coupled to a natural protein or to a synthetic polypeptide having a sufficient molecular weight so that the conjugate is able to induce in vivo the production of antibodies neutralizing Mycobacterium tuberculosis, or induce in vivo a cellular immune response by activating M. tuberculosis antigen-responsive T cells.

The peptides of the invention which are advantageously used as immunogenic principle have one of the following sequences:

**TABLE 4a**

| (see fig. 4a and 4b) | | |
|---|---|---|
| Amino acid position (NH₂-terminal) | | Amino acid position (COOH-terminal) |
| 12 | QVPSPSMGRDIKVQFQSGGA | 31 |
| 36 | LYLLDGLRAQDDFSGWDINT | 55 |
| 77 | SFYSDWYQPACRKAGCQTYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |

**TABLE 4b**

| (see fig. 5) | | |
|---|---|---|
| Amino acid position (NH₂-terminal) | | Amino acid position (COOH-terminal) |
| 77 | SFYSDWYQPACGKAGCQTYK | 96 |
| 276 | PDLQRALGATPNTGPAPQGA | 295 |

The amino acid sequences are given in the 1-letter code.

Other characteristics and advantages of the invention will appear in the following examples and the figures illustrating the invention.

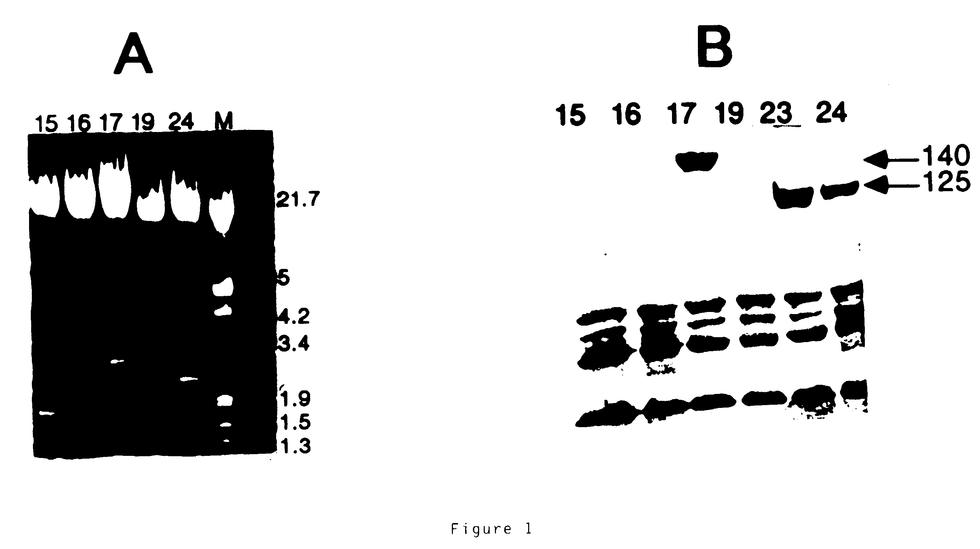

Figures 1(A) and 1(B) correspond to the identification of six purified λgt11 M. tuberculosis recombinant clones. Figure 1(A) corresponds to the EcoRI restriction analysis of clone 15, clone 16, clone 17, clone 19, clone 24 and EcoRI-HindIII digested lambda DNA-molecular weight marker lane (in kilobase pairs) (M) (Boehringer).

Figure 1(B) corresponds to the immunoblotting analysis of crude lysates of E. coli lysogenized with clone 15, clone 16, clone 17, clone 19, clone 23 and clone 24.

Arrow (<―) indicates fusion protein produced by recombinant λgt11-M-tuberculosis clones. Expression and immunoblotting were as described above. Molecular weight (indicated in kDa) were estimated by comparison with molecular weight marker (High molecular weight-SDS calibration kit, Pharmacia).

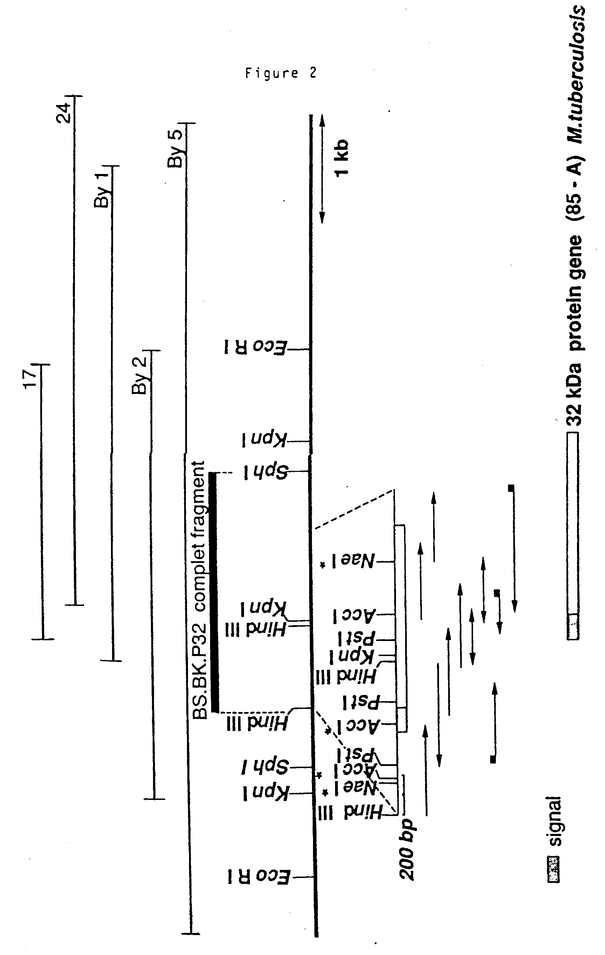

Figure 2 corresponds to the restriction map of the DNA inserts in the λgt11 M. tuberculosis recombinant clones 17 and 24 identified with polyclonal anti-32-kDa (BCG) antiserum as above defined and of clones By1, By2 and By5 selected by hybridization with a 120 bp EcoRI-Kpn I restriction fragment of clone 17.

DNA was isolated from λgt11 phage stocks by using the Lambda Sorb Phage Immunoadsorbent, as described by the manufacturer (Promega). Restriction sites were located as described above. Some restriction sites (*) were deduced from a computer analysis of the nucleotide sequence.

The short vertical bars 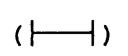 represent linker derived EcoRI sites surrounding the DNA inserts of recombinant clones. The lower part represents a magnification of the DNA region containing the antigen of molecular weight of 32-kDa, that has been sequenced. Arrows indicate strategies and direction of dideoxy-sequencing. (―>) fragment subcloned in Bluescribe M13; (<―>) fragment subcloned in mp10 and mp11 M13 vectors; (■―>) sequence determined with the use of a synthetic oligonucleotide.

Figures 3a and 3b correspond to the nucleotide and amino acid sequences of the general formula of the antigens of the invention.

Figures 4a and 4b correspond to the nucleotide and amino acid sequences of one of the antigens of the invention.

Two groups of sequences resembling the E. coli consensus promoter sequences are boxed and the homology to the consensus is indicated by italic bold letters. Roman bold letters represent a putative Shine-Dalgarno motif.

The NH₂-terminal amino acid sequence of the mature protein which is underlined with a double line happens to be very homologous - 29/32 amino acids - with the one of MPB 59 antigen (34). Five additional ATG codons, upstream of the ATG at position 273 are shown (dotted underlined). Vertical arrows (↓) indicate the presumed NH₂ end of clone 17 and clone 24. The option taken here arbitrarily represents the 59 amino acid signal peptide corresponding to ATG₁₈₃.

Figure 5 corresponds to the nucleotide and amino acid sequences of the antigen of 32-kDa of the invention.

The NH₂-terminal amino acid sequence of the mature protein which is underlined with a double line happens to be very homologous - 29/32 amino acids - with the one of MPB 59 antigen (34). Vertical arrows (↓) indicate the presumed NH₂ end of clone 17 and clone 24.

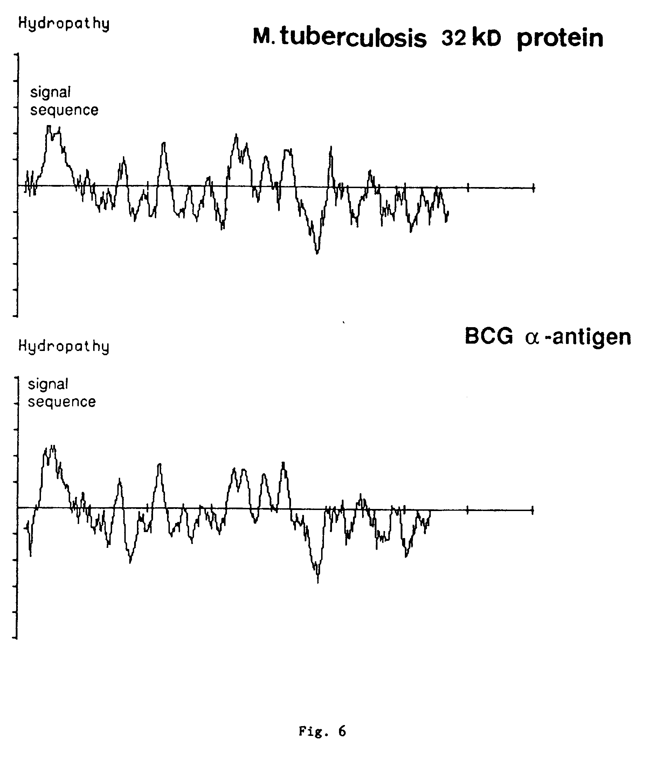

Figure 6 is the hydropathy pattern of the antigen of the invention of a molecular weight of 32-kDa and of the antigen α of BCG (17).

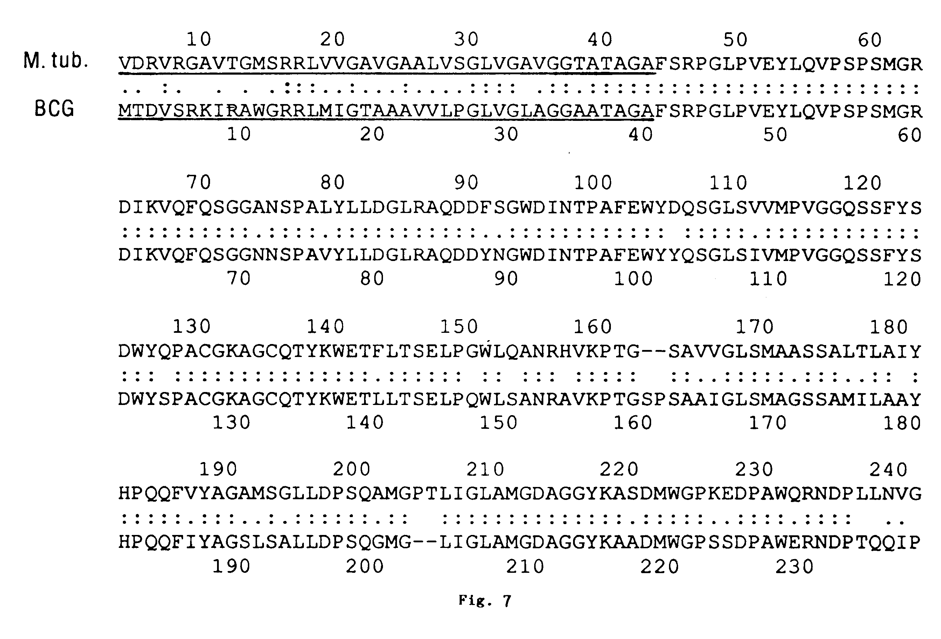

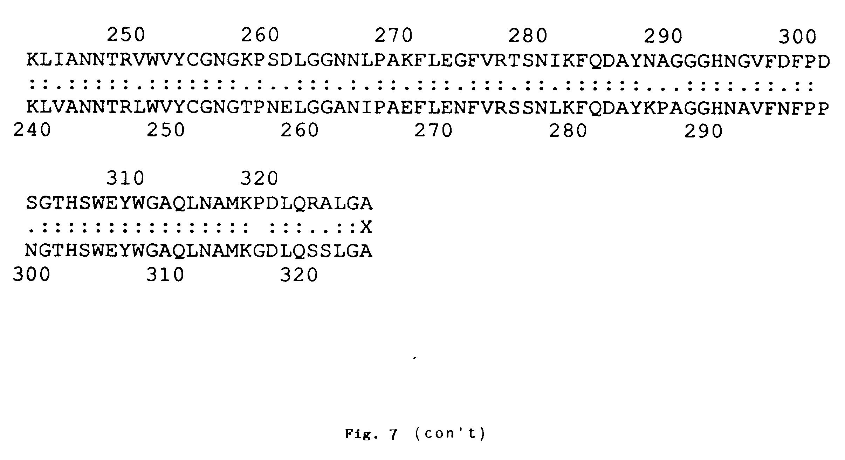

Figure 7 represents the homology between the amino acid sequences of the antigen of 32-kDa of the invention and of antigen α of BCG (revised version).

Identical amino acids; (:) evolutionarily conserved replacement of an amino acid (.), and absence of homology () are indicated. The Underlined sequence represents the signal peptide, the option taken here arbitrarily representing the 43-amino acid signal peptide corresponding to ATG₉₁ on fig. 5. Dashes in the sequences indicate breaks necessary for obtaining the optimal alignment.

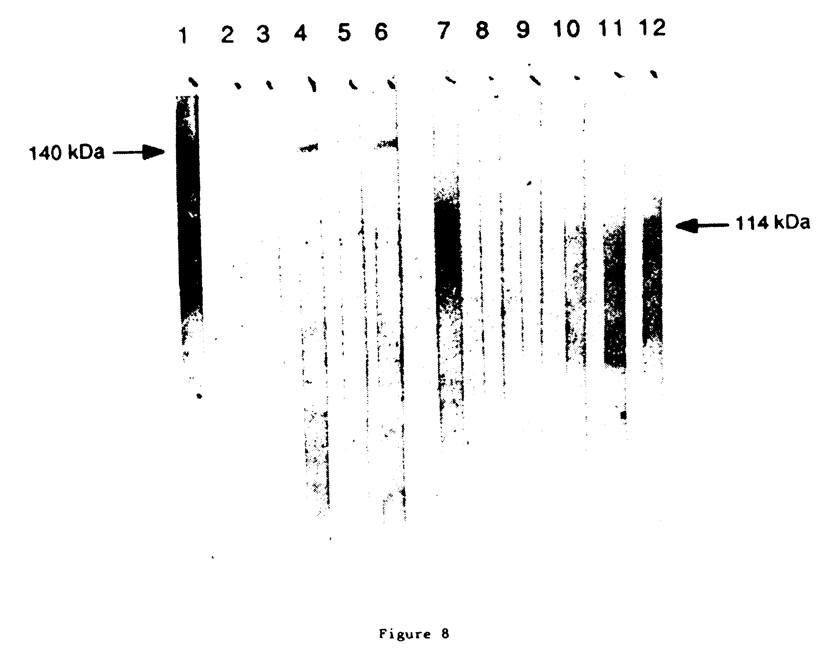

Figure 8 illustrates the fact that the protein of 32-kDa of the invention is selectively recognized by human tuberculous sera.

Figure 8 represents the immunoblotting with human tuberculous sera, and anti-β-galactosidase monoclonal antibody. Lanes 1 to 6: E. coli lysate expressing fusion protein (140 kDa); lanes 7 to 12:unfused β-galactosidase (114 kDa). The DNA insert of clone 17 (2.7 kb) was subcloned into pUEX₂ and expression of fusion protein was induced as described by Bresson and Stanley (4). Lanes 1 and 7 were probed with the anti-β-galactosidase monoclonal antibody: lanes 4, 5, 6 and 10, 11, 12 with 3 different human tuberculous sera highly responding towards purified protein of the invention of 32-kDa; lanes 2 and 3 and 8 and 9 were probed with 2 different low responding sera.

Figure 9 represents the nucleic acid sequence alignment of the 32-kDa protein gene of M. tuberculosis of the invention (upper line), corresponding to the sequence in fig. 5, of the gene of fig. 4a and 4b of the invention (middle line), and of the gene for antigen α of BCG (lower line).

Dashes in the sequence indicate breaks necessary for obtaining optimal alignment of the nucleic acid sequence.

The primer regions for enzymatical amplification are boxed (P1 to P6).

The specific probe regions are boxed and respectively defined by probe region A, probe region B, probe region C, probe region D, probe region E and probe region F.

It is to be noted that the numbering of nucleotides is different from the numbering of figures 3a and figure 3b, and of figure 5, because nucleotide at position 1 (on figure 9) corresponds to nucleotide 234 on Figure 3a, and corresponds to nucleotide 91 on figure 5.

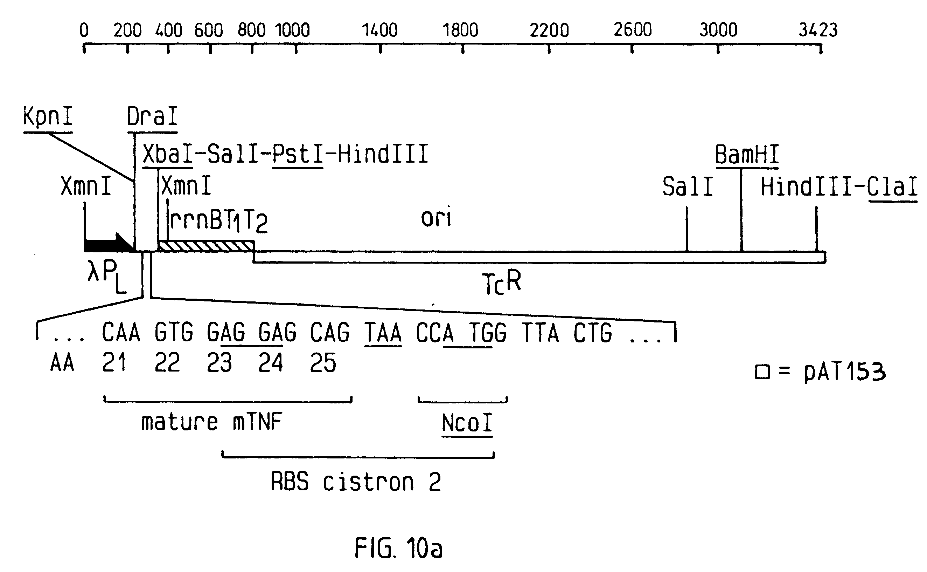

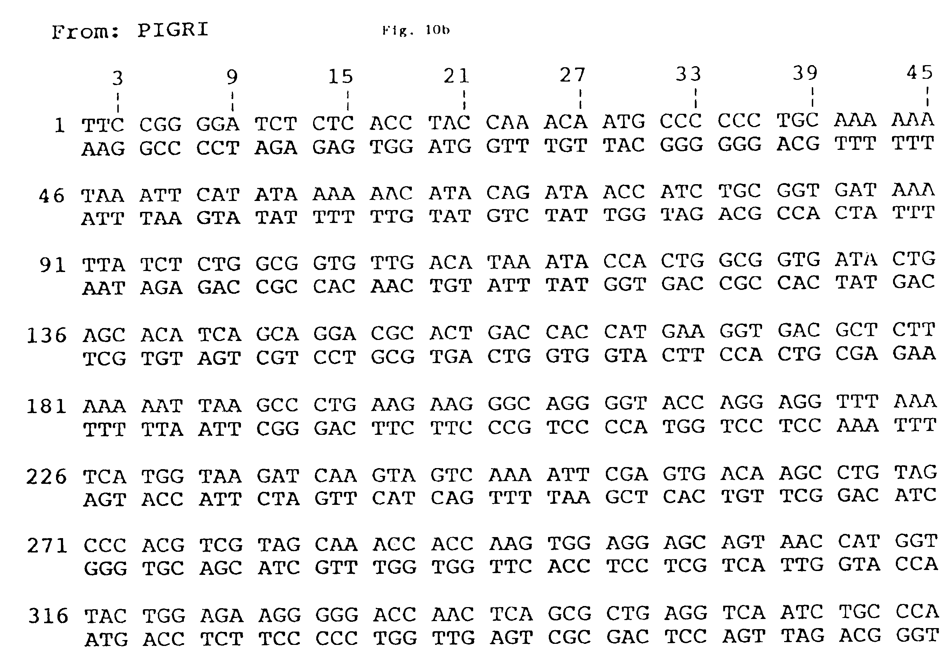

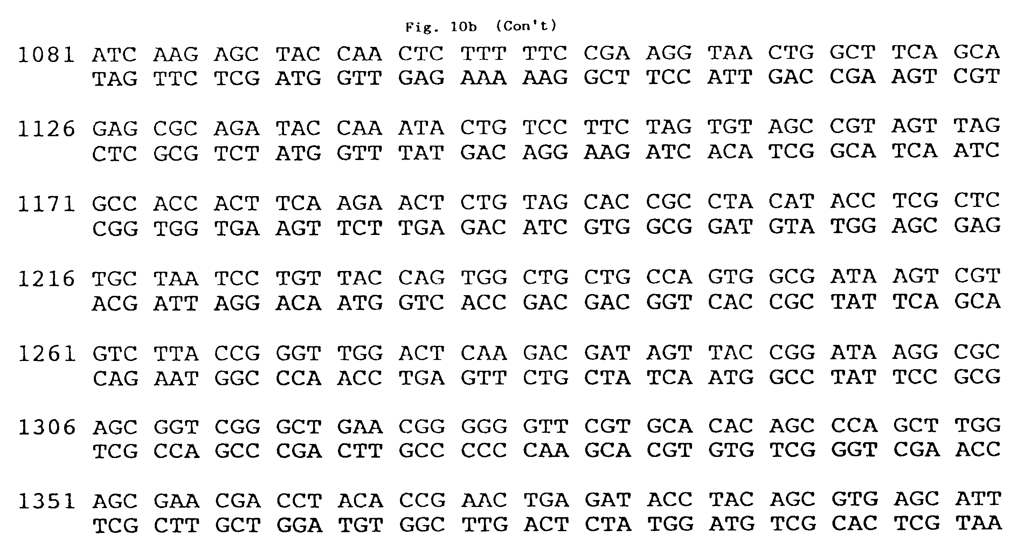

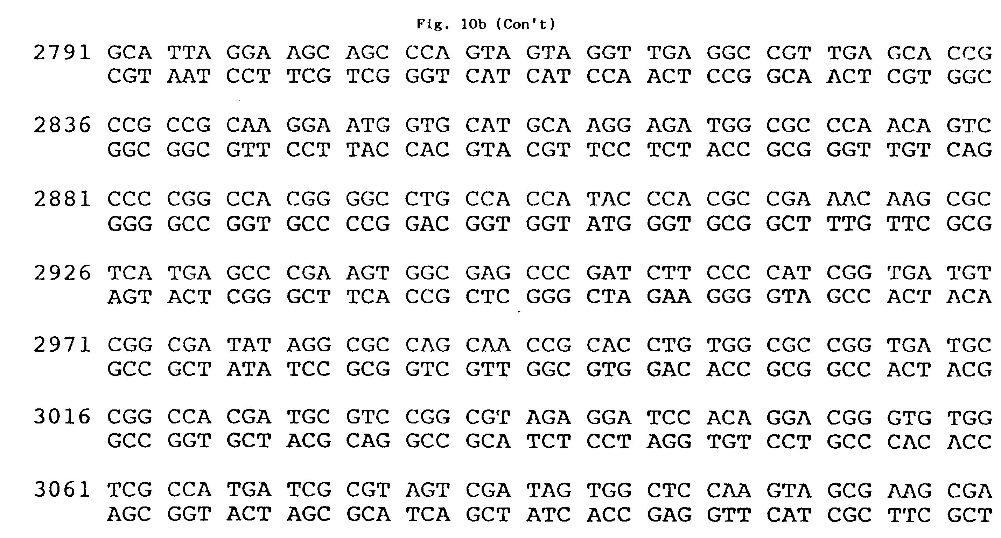

Figure 10a corresponds to the restriction and genetic map of the pIGRI plasmid used in Example IV for the expression of the P₃₂ antigen of the invention in E. coli.

On this figure, underlined restriction sites are unique.

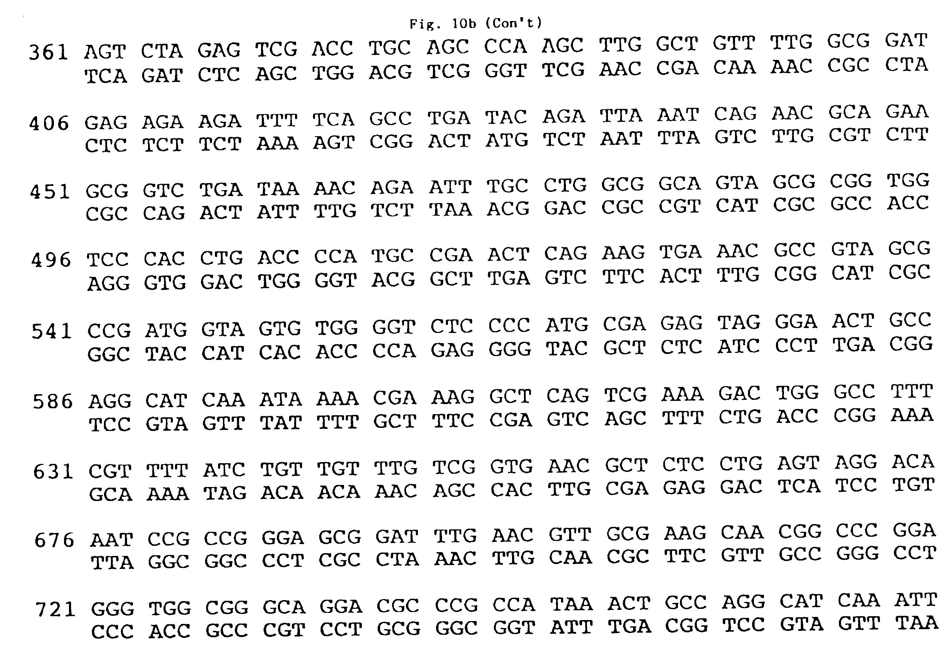

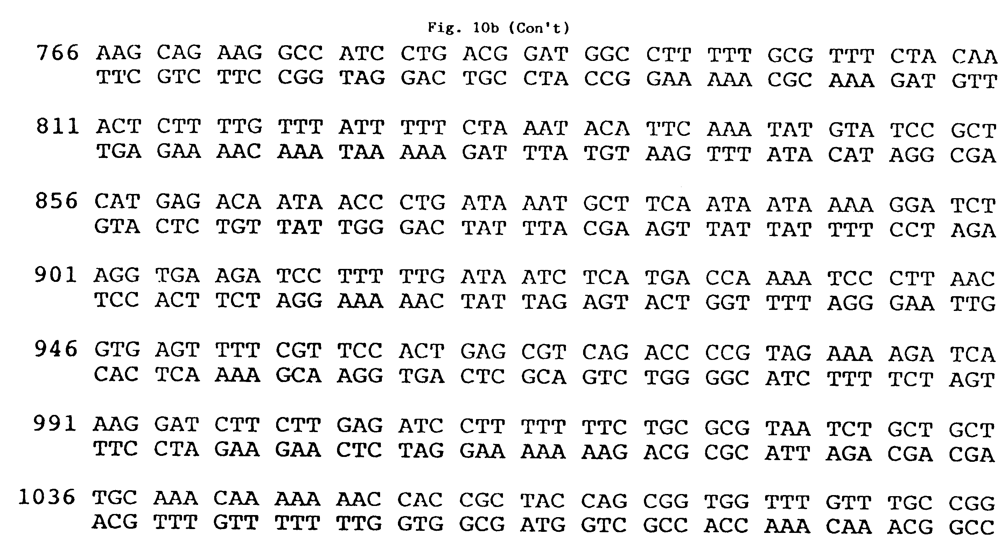

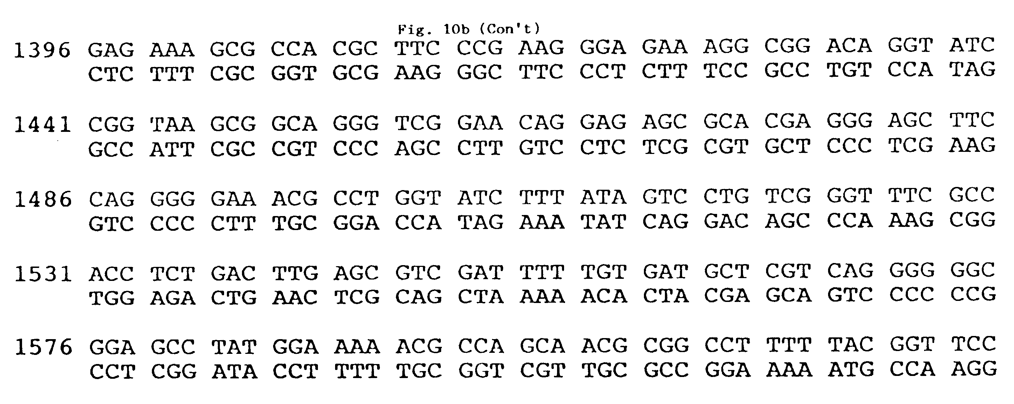

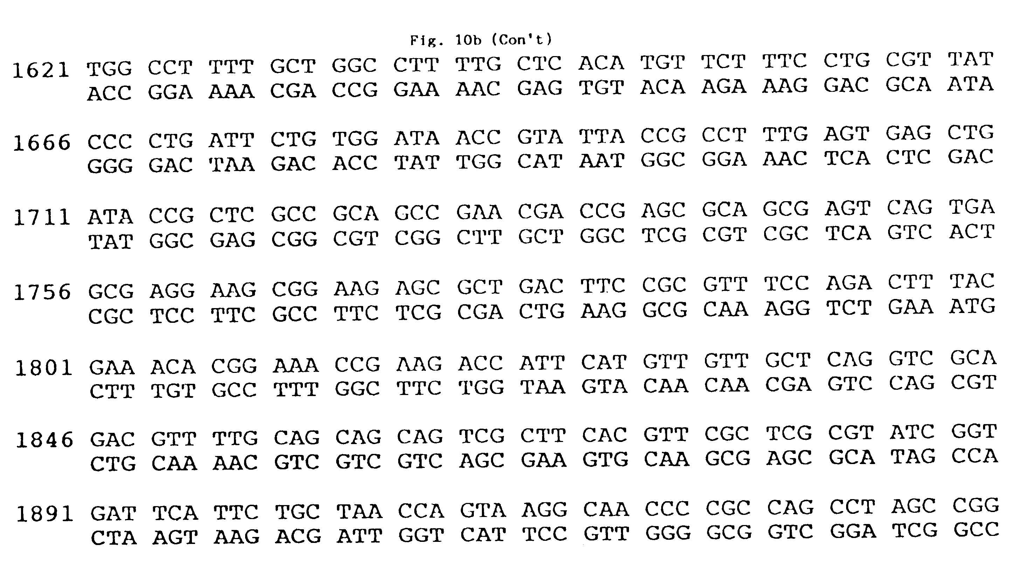

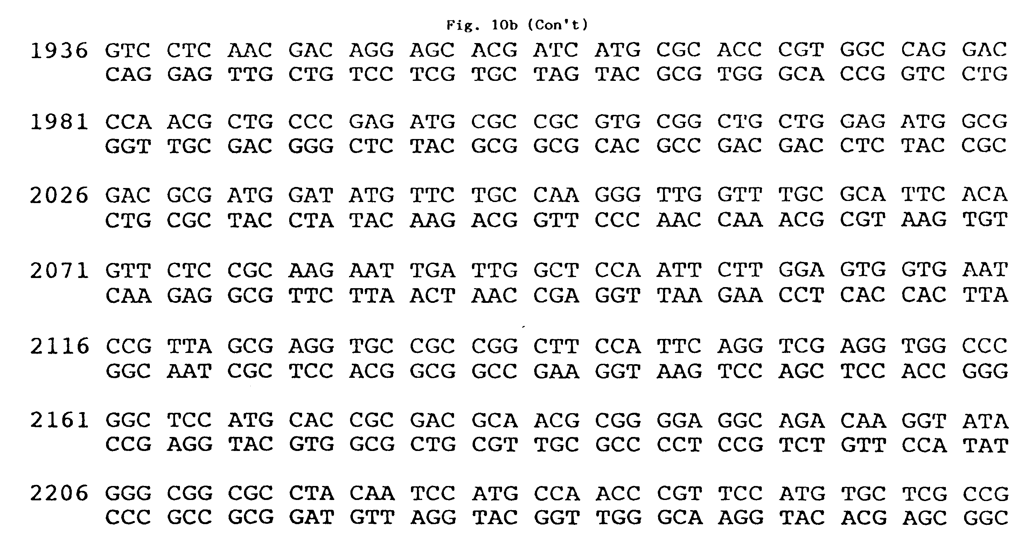

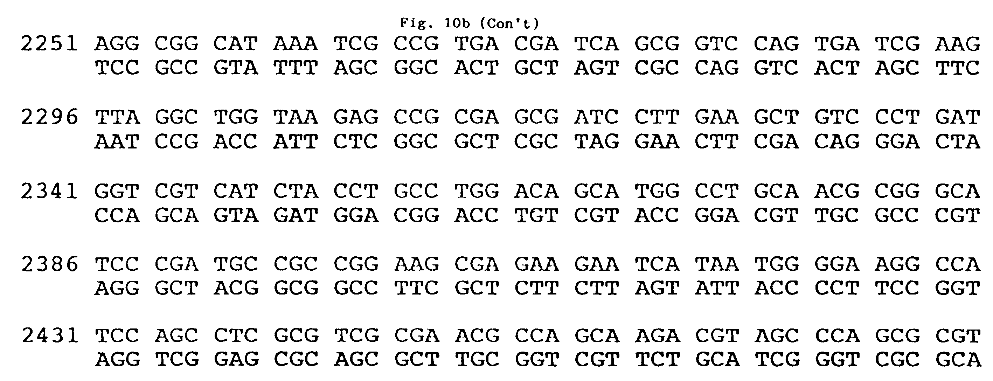

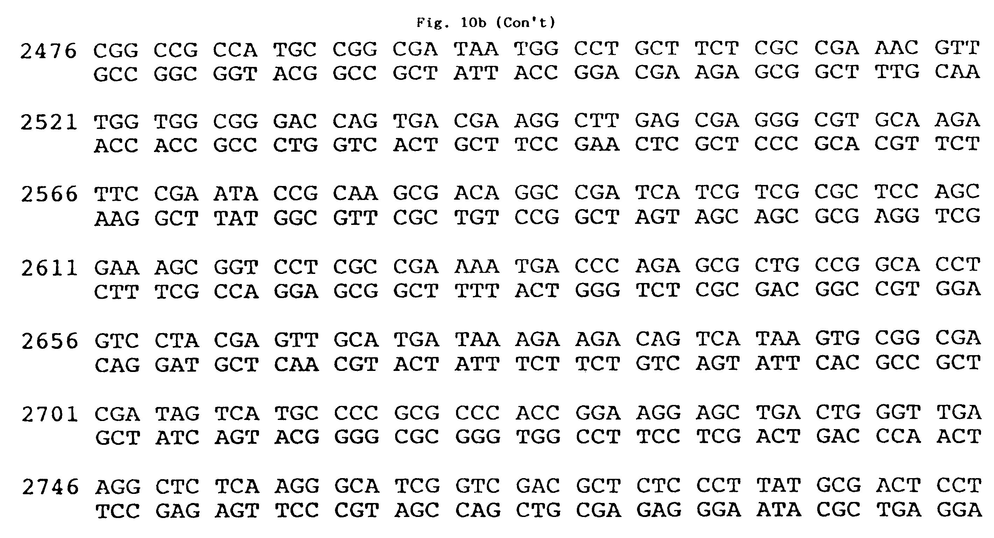

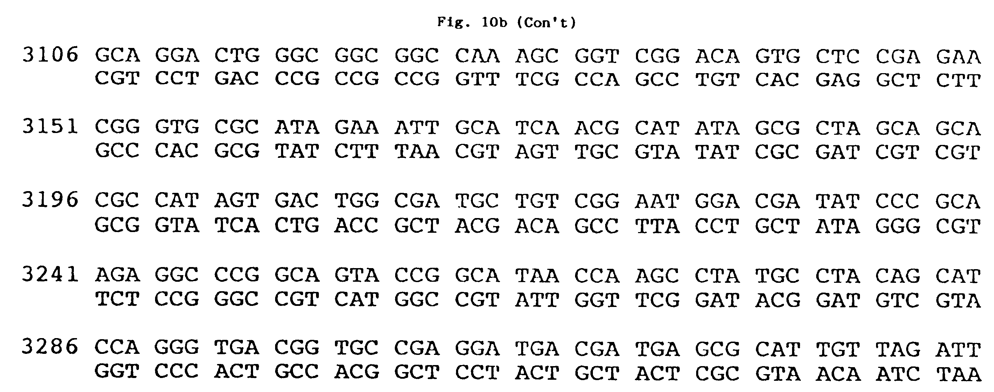

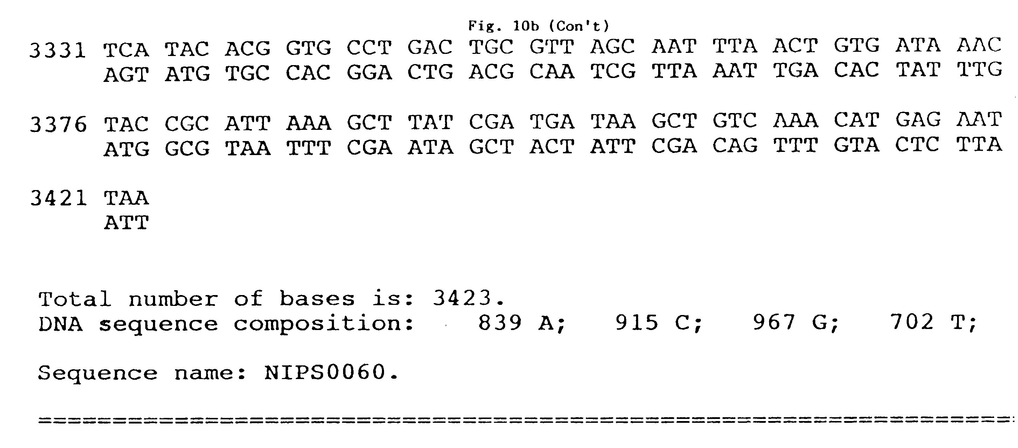

Figure 10b corresponds to the pIGRI nucleic acid sequence.

On this figure, the origin of nucleotide stretches used to construct plasmid pIGRI are specified hereafter.

### Position

- 3422-206 :: lambda PL containing EcoRI blunt-MboII blunt fragment of pPL(λ) (Pharmacia)
- 207-384 :: synthetic DNA sequence
- 228-230 :: initiation codon ATG of first cistron
- 234-305 :: DNA encoding amino acids 2 to 25 of mature mouse TNF
- 306-308 :: stop codon (TAA) first cistron
- 311-312 :: initiation codon (ATG) second cistron
- 385-890 :: rrnBT₁T₂ containing HindIII-SspI fragment from pKK223 (Pharmacia)
- 891-3421 :: DraI-EcoRI blunt fragment of pAT₁₅₃ (Bioexcellence) containing the tetracycline resistance gene and the origin of replication.

Table 5 hereafter corresponds to the complete restriction site analysis of pIGRI.

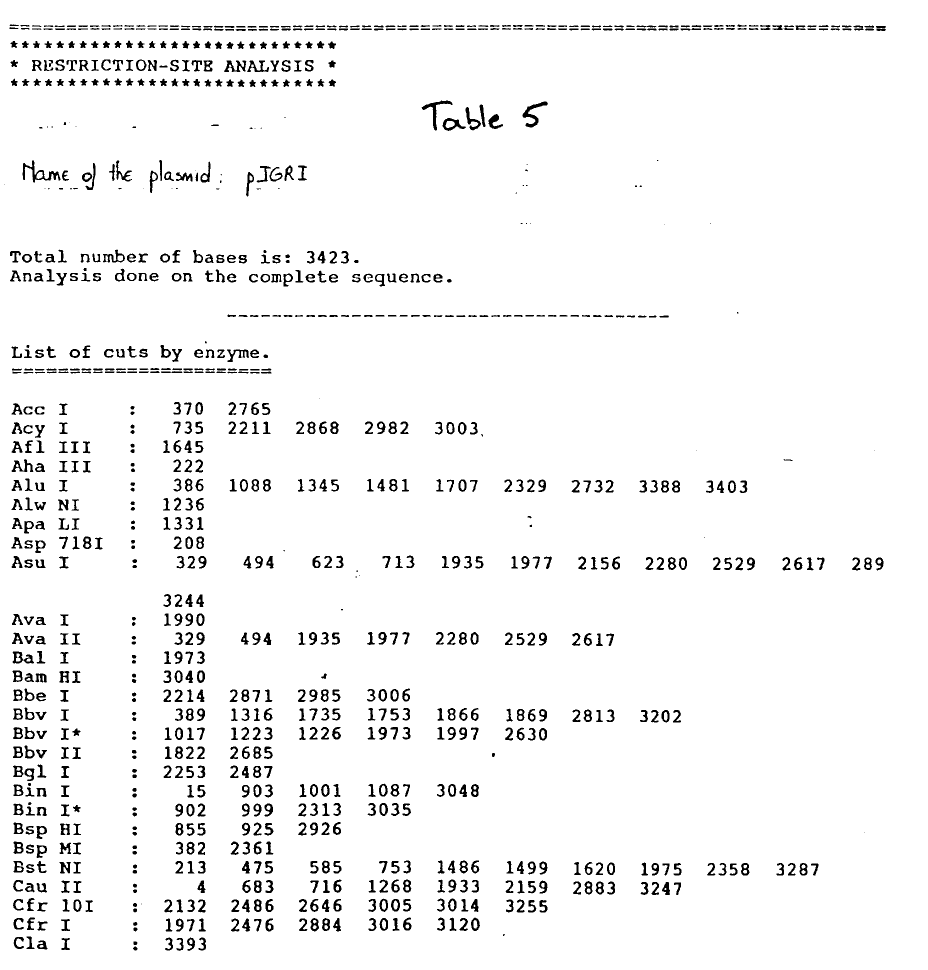

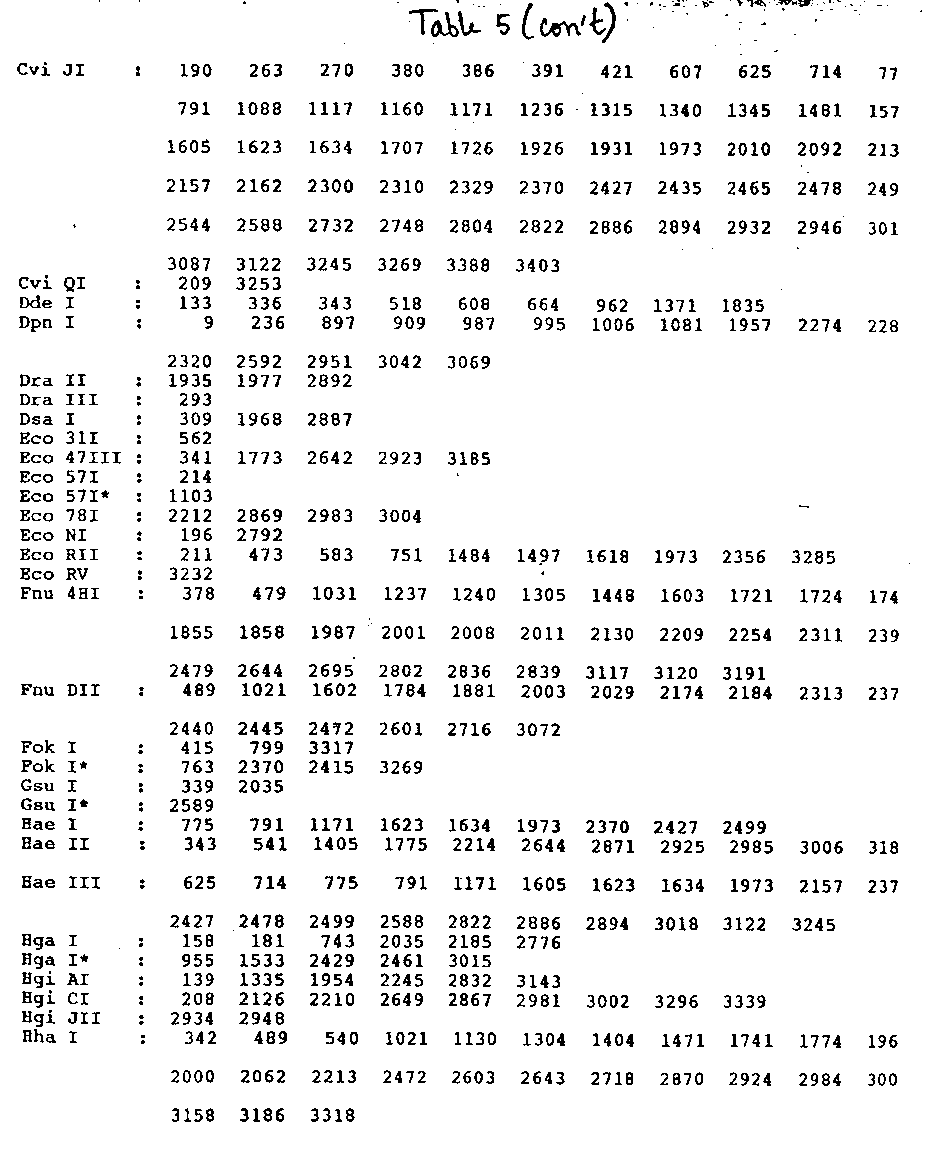

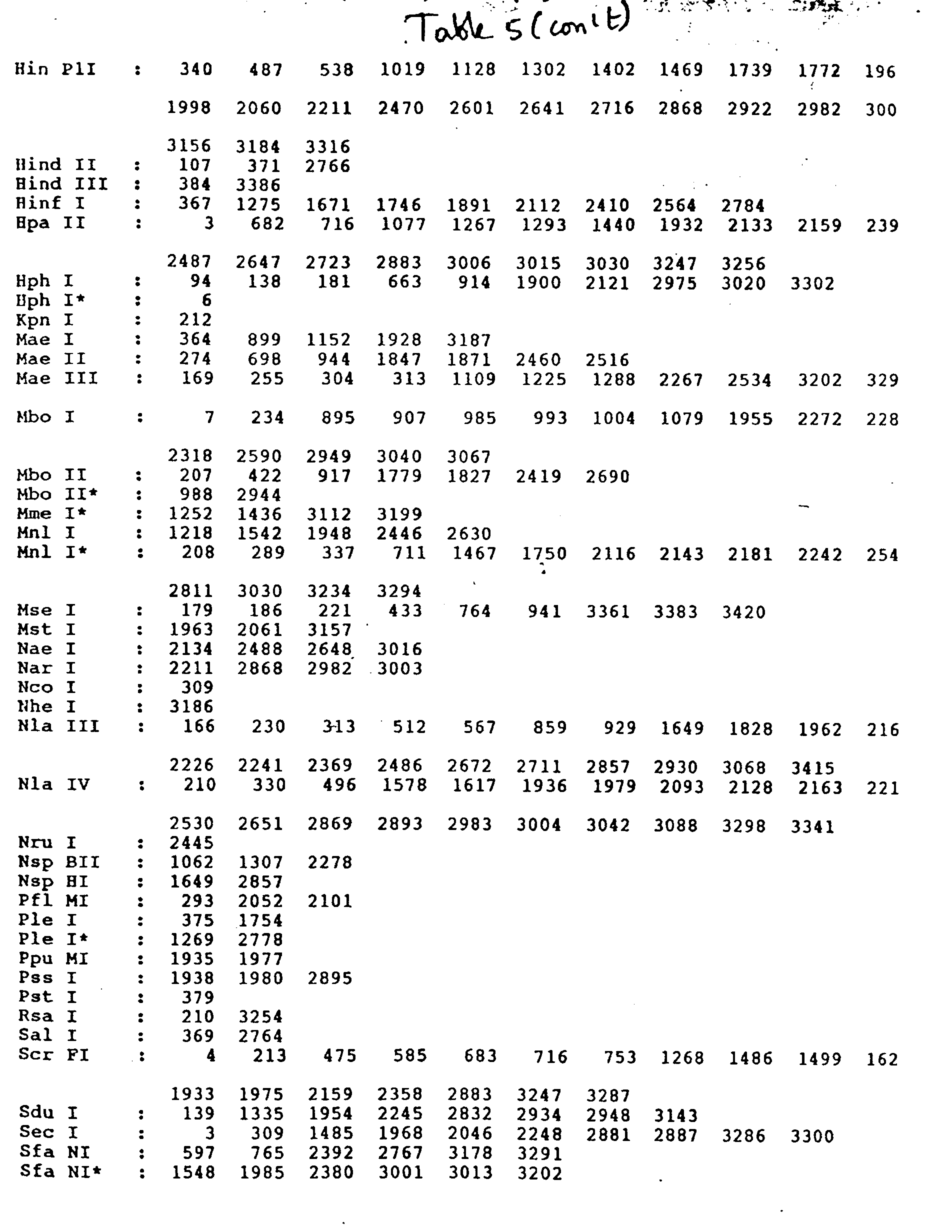

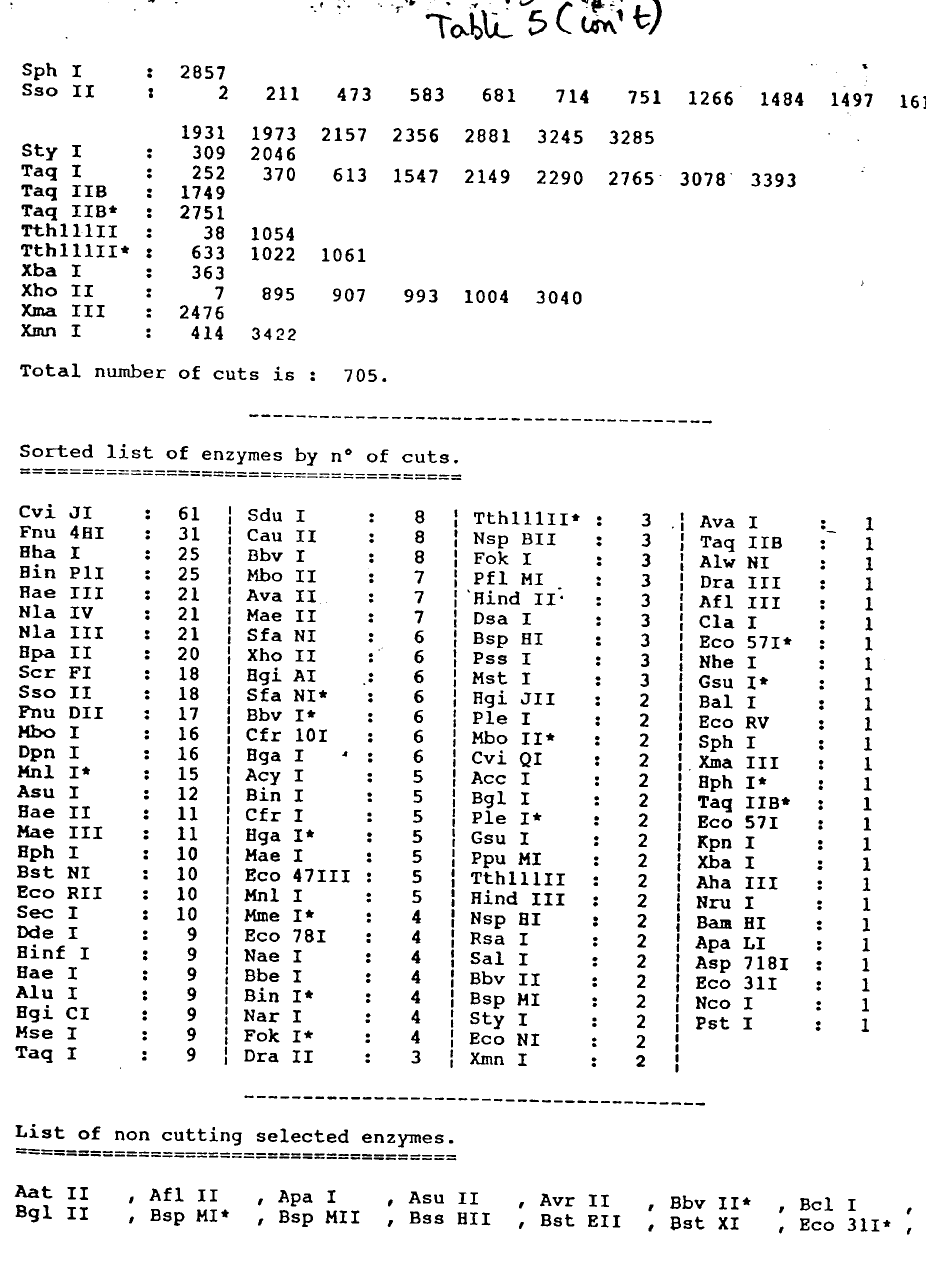

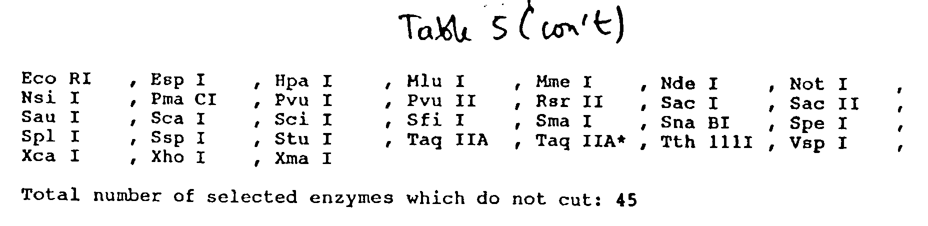

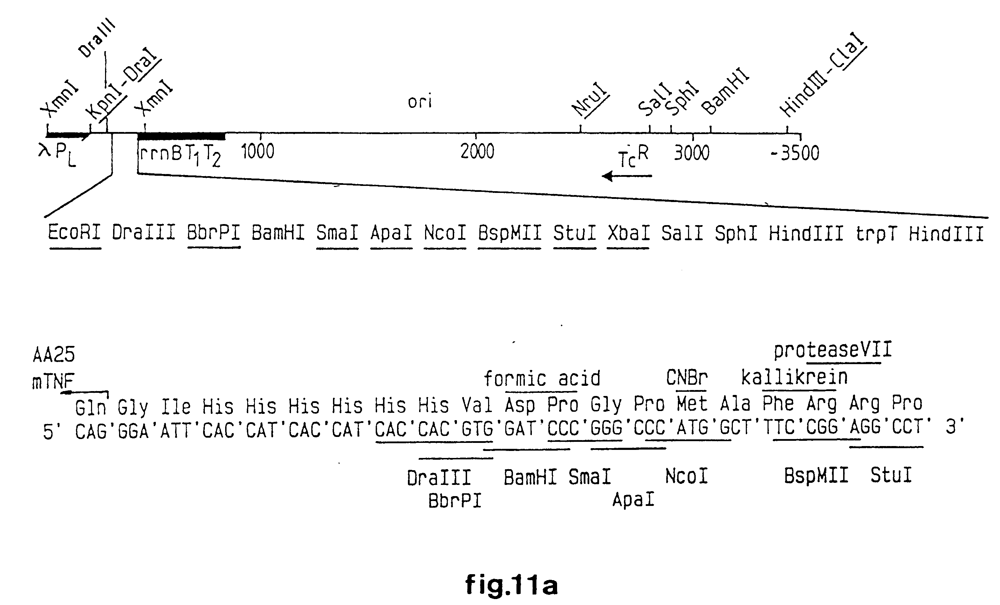

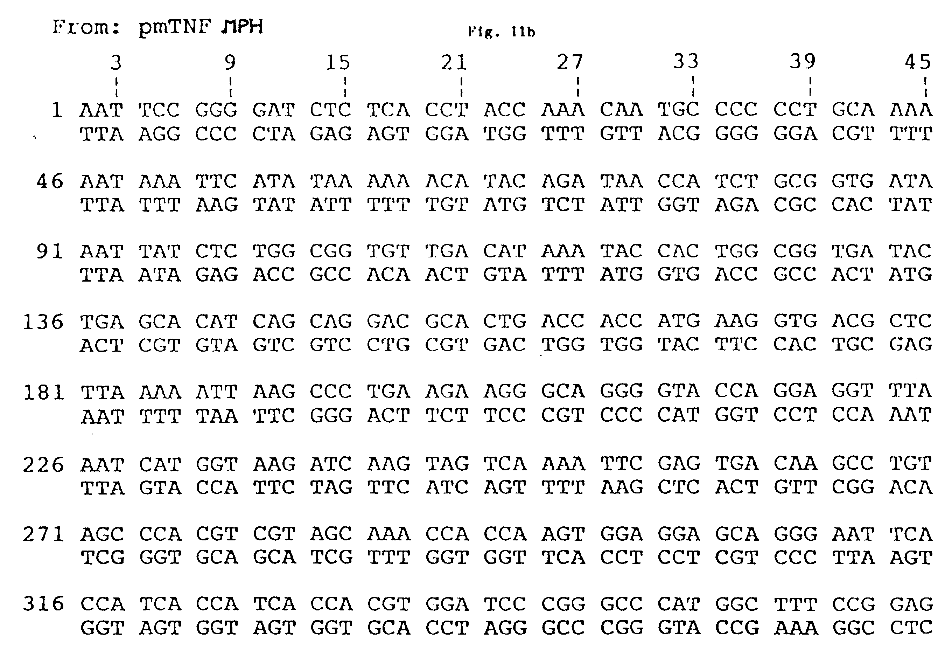

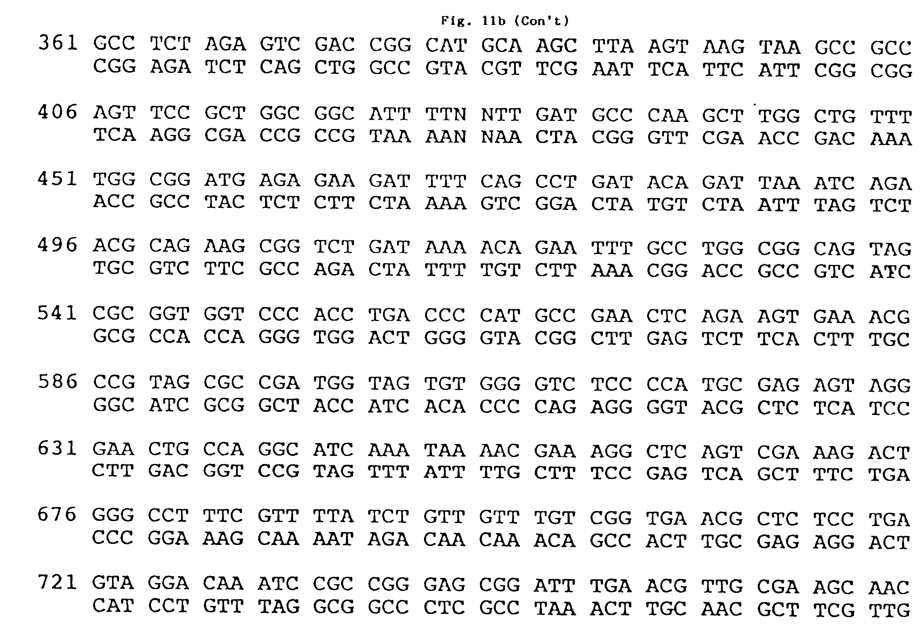

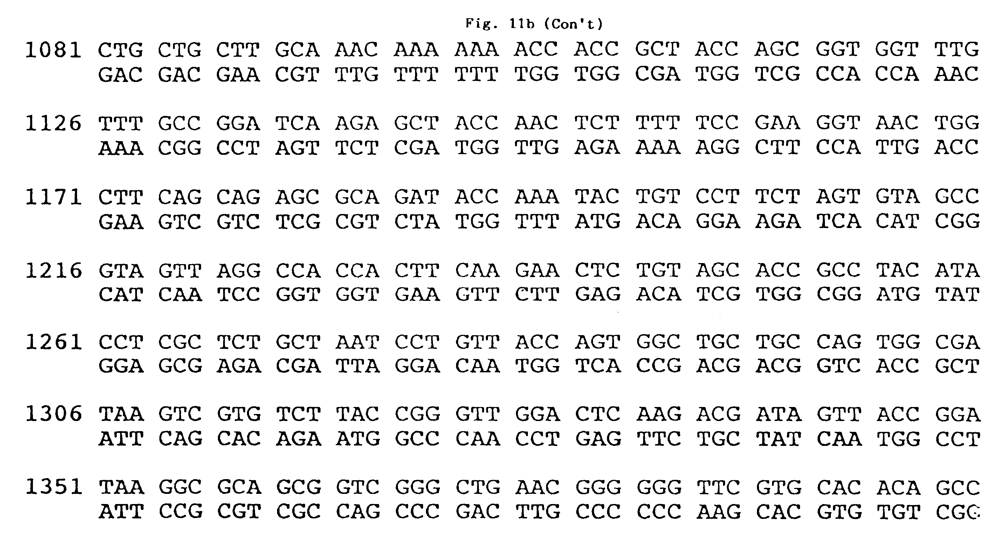

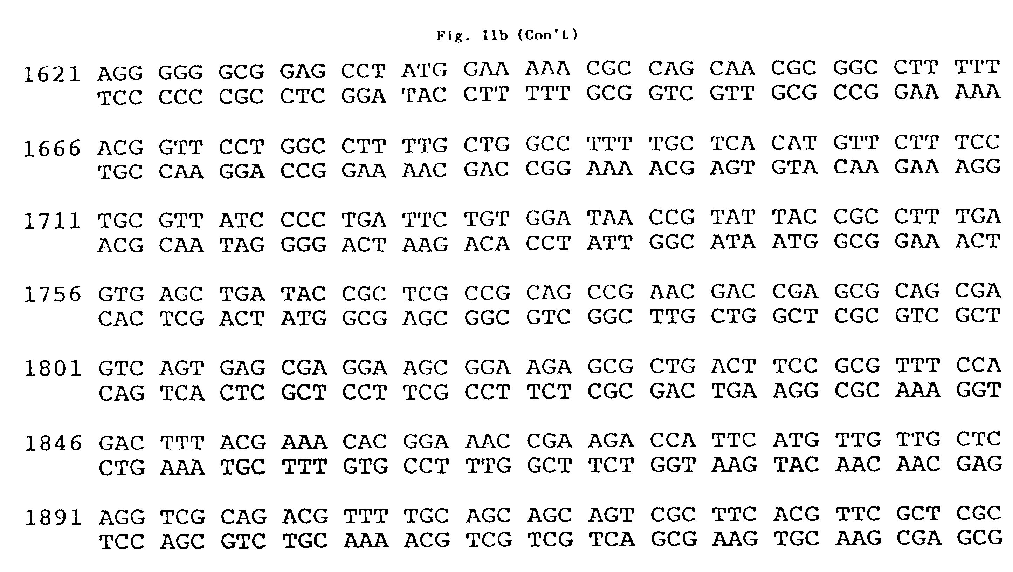

Figure 11a corresponds to the restriction and genetic map of the pmTNF MPH plasmid used in Example V for the expression of the P₃₂ antigen of the invention in E. coli.

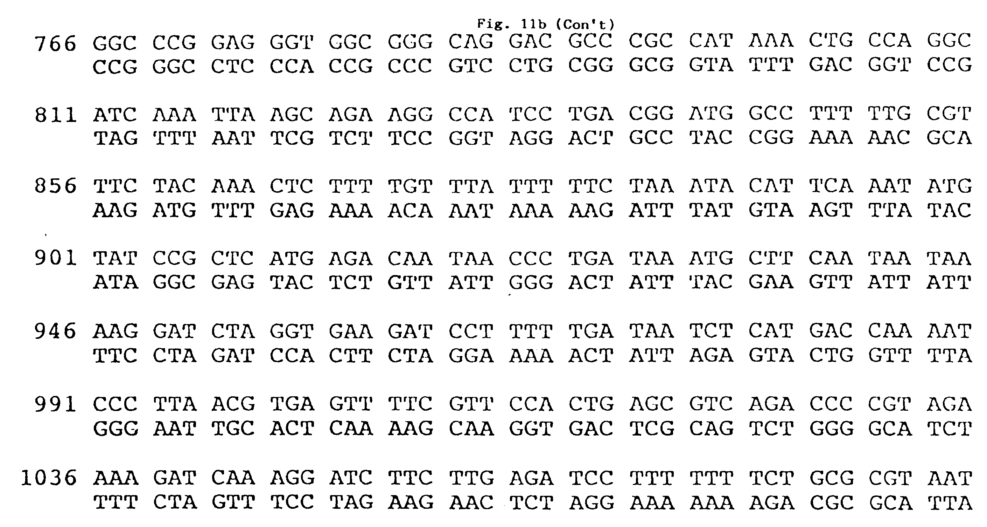

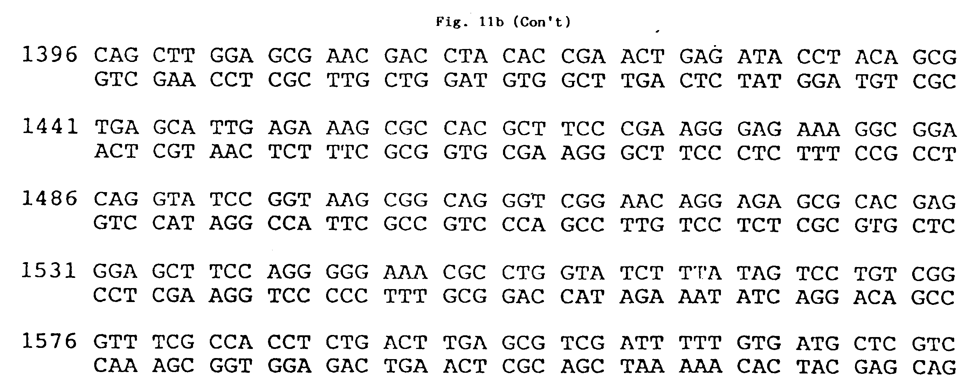

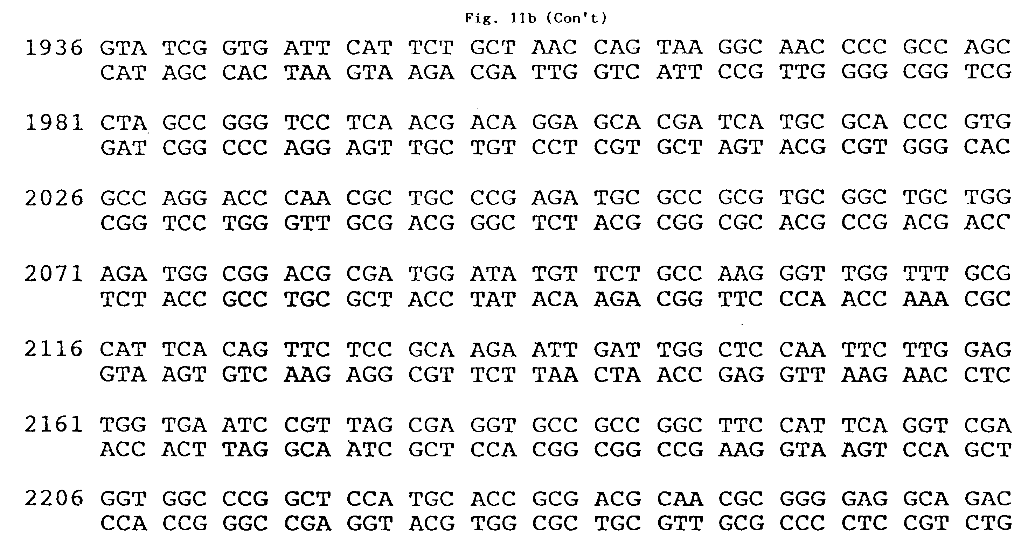

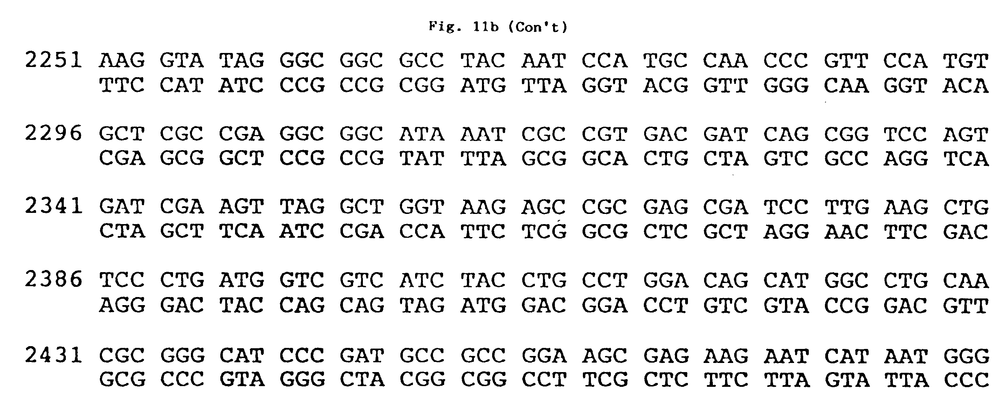

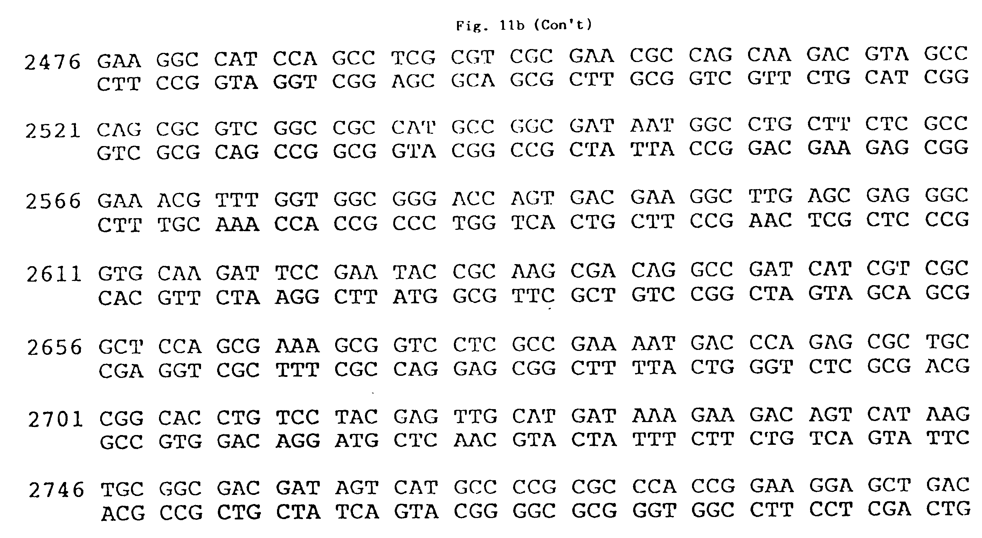

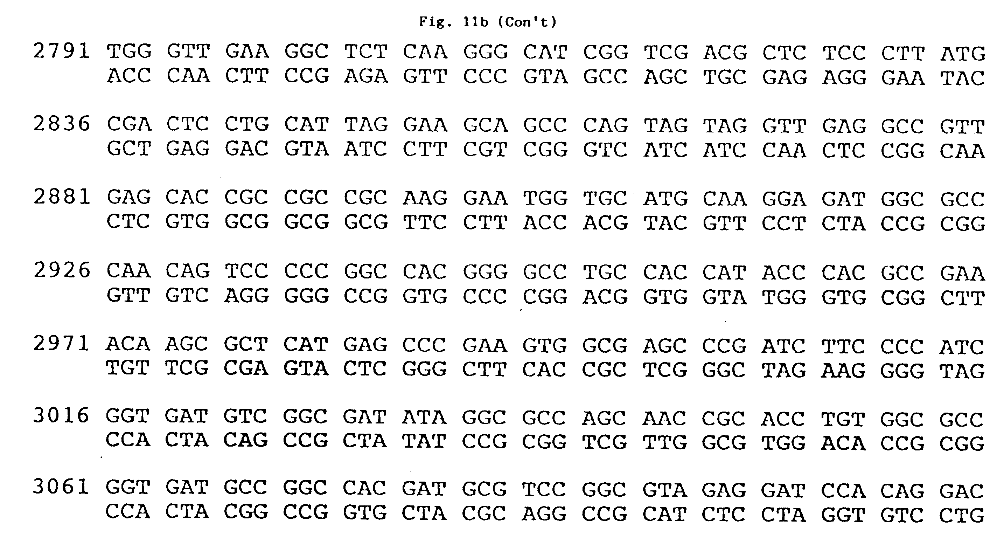

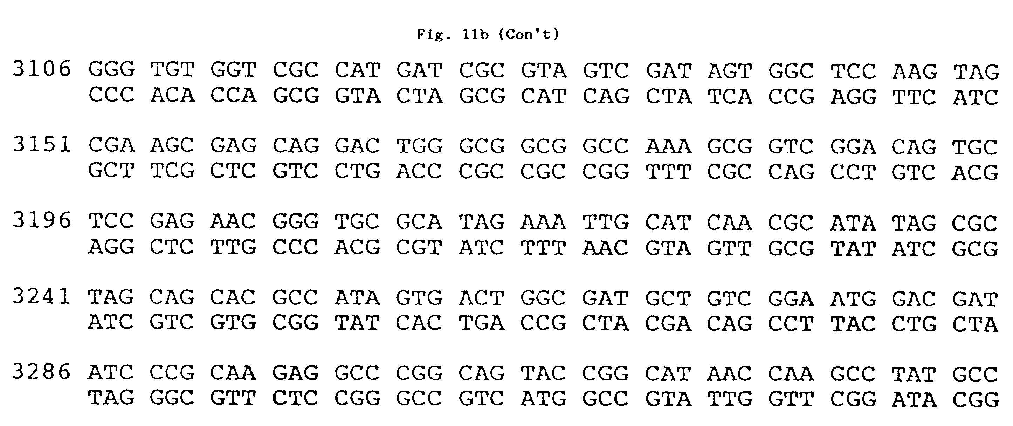

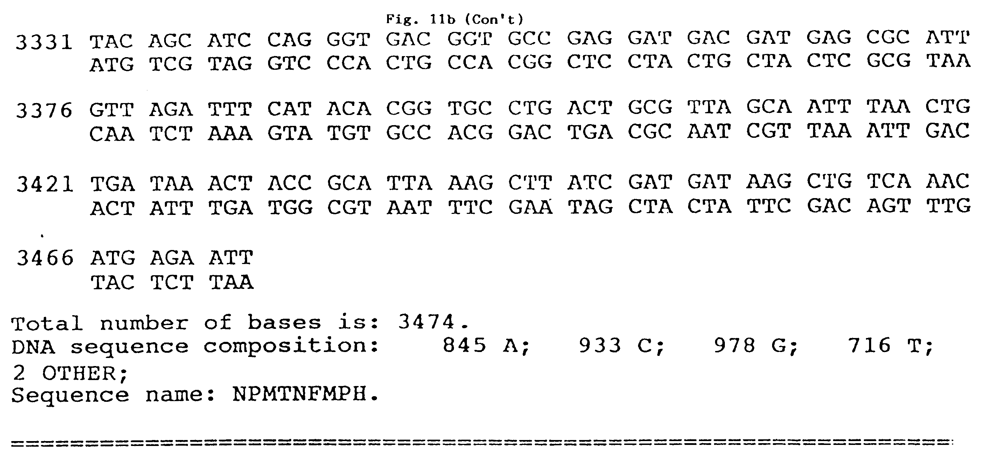

Figure 11b corresponds to the pmTNF-MPH nucleic acid sequence.

On this figure, the origin of nucleotide stretches used to construct plasmid pmTNF-MPH is specified hereafter.

### Position

- 1-208 :: lambda PL containing EcoRI blunt-MboII blunt fragment of pPL(λ) (Pharmacia)
- 209-436 :: synthetic DNA fragment
- 230-232 :: initiation codon (ATG) of mTNF fusion protein
- 236-307 :: sequence encoding AA 2 to 25 of mature mouse TNF
- 308-384 :: multiple cloning site containing His₆ encoding sequence at position 315-332
- 385-436 :: HindIII fragment containing E. coli trp terminator
- 437-943 :: rrnBT₁T₂ containing HindIII-SspI fragment from pKK223 (Pharmacia)
- 944-3474 :: DraI-EcoRI blunt fragment of pAT₁₅₃ (Bioexcellence) containing the tetracycline resistance gene and the origin of replication.

Table 6 hereafter corresponds to the complete restriction site analysis of pmTNF-MPH.

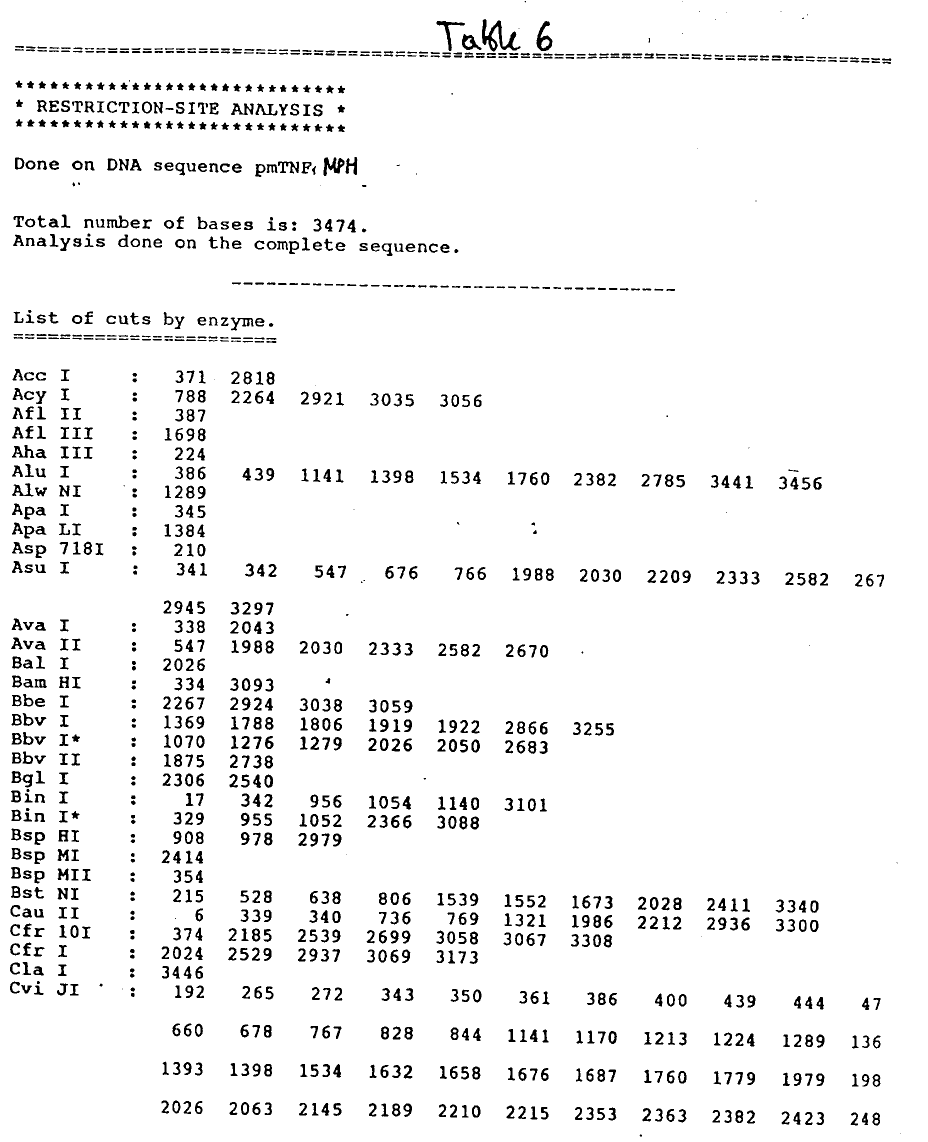

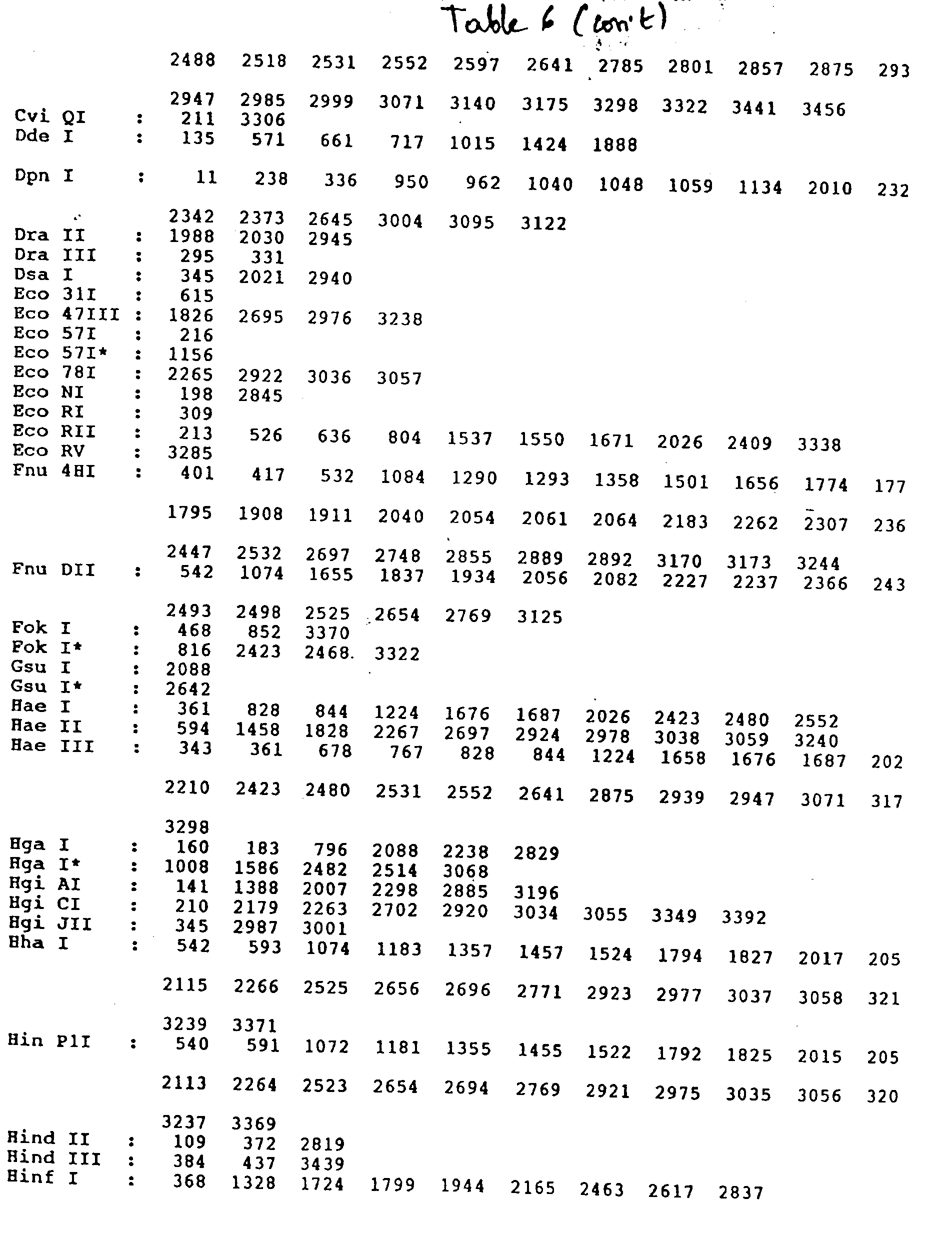

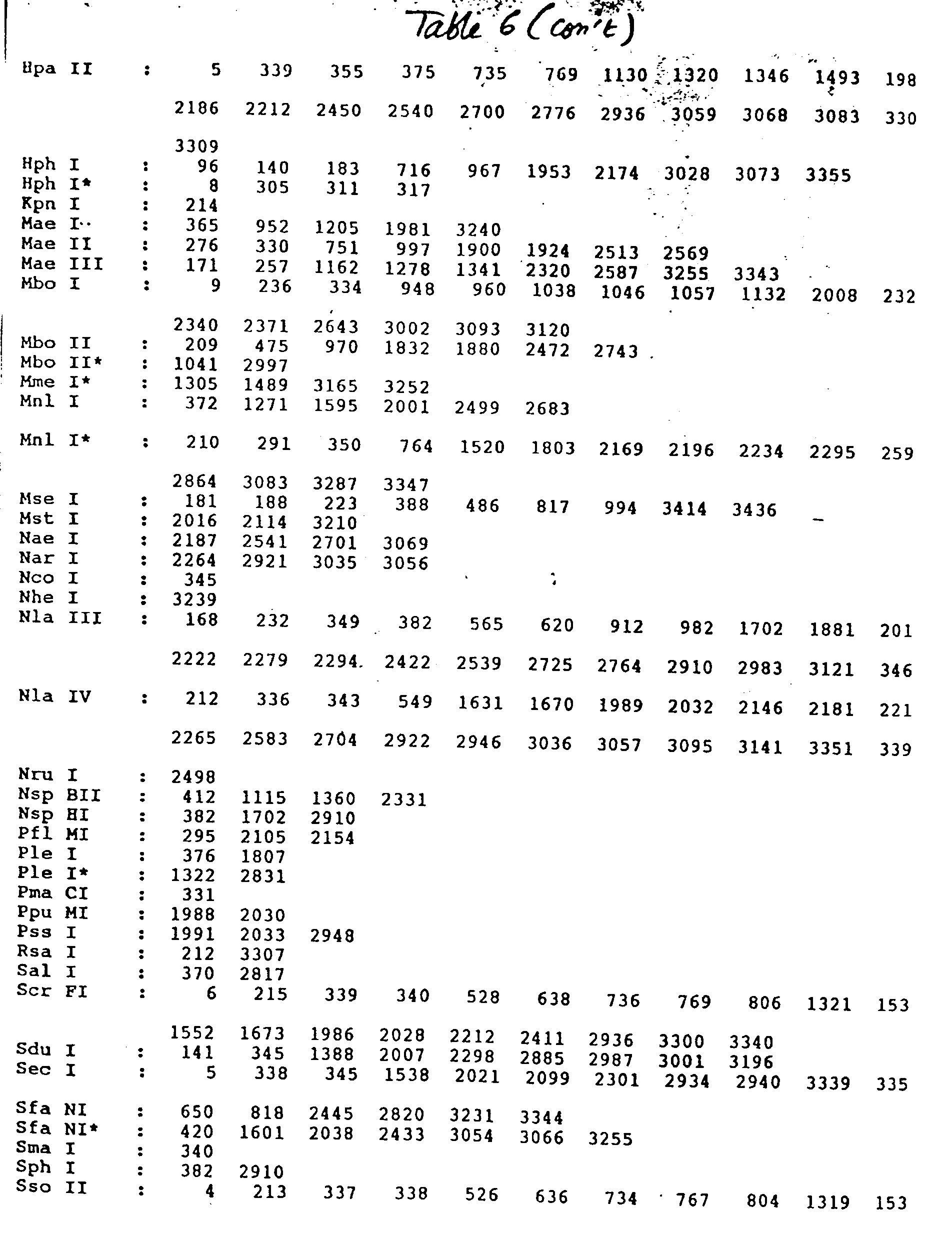

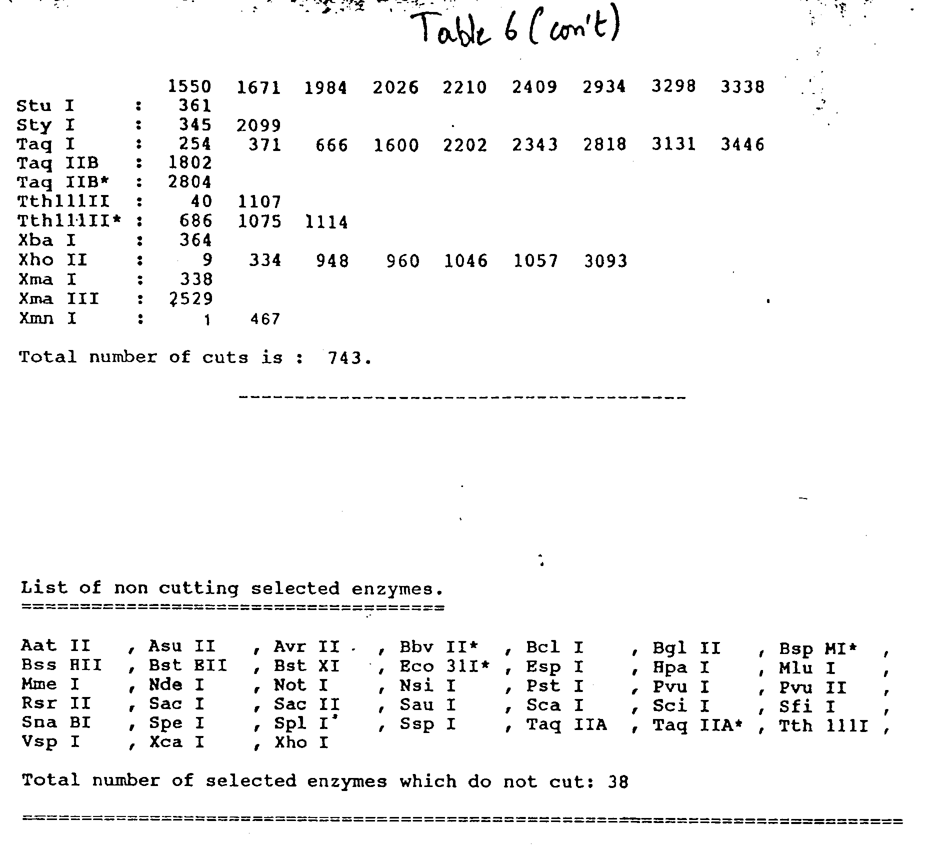

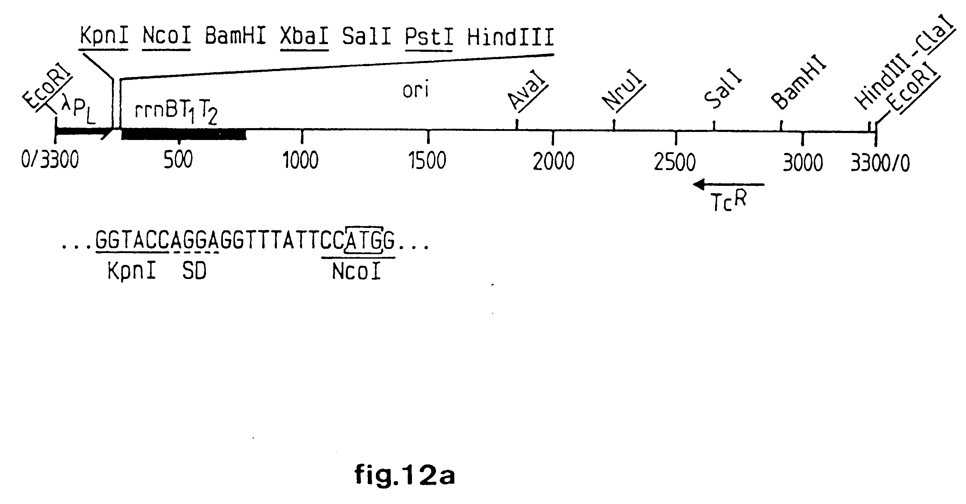

Figure 12a corresponds to the restriction and genetic map of the plasmid pIG2 used to make the intermediary construct pIG2 Mt32 as described in Example IV for the subcloning of the P₃₂ antigen in plasmid pIGRI.

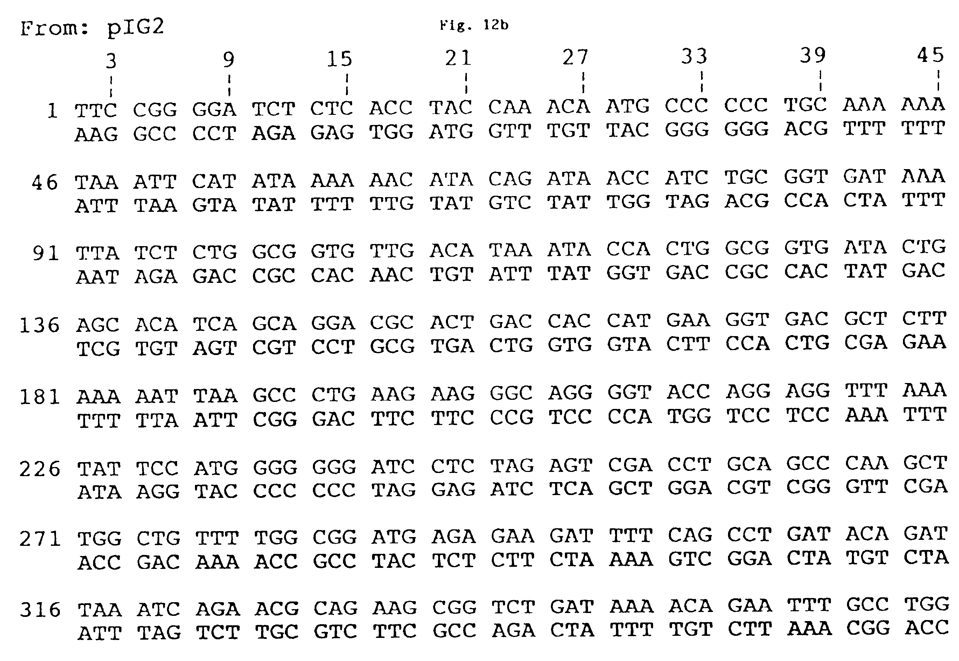

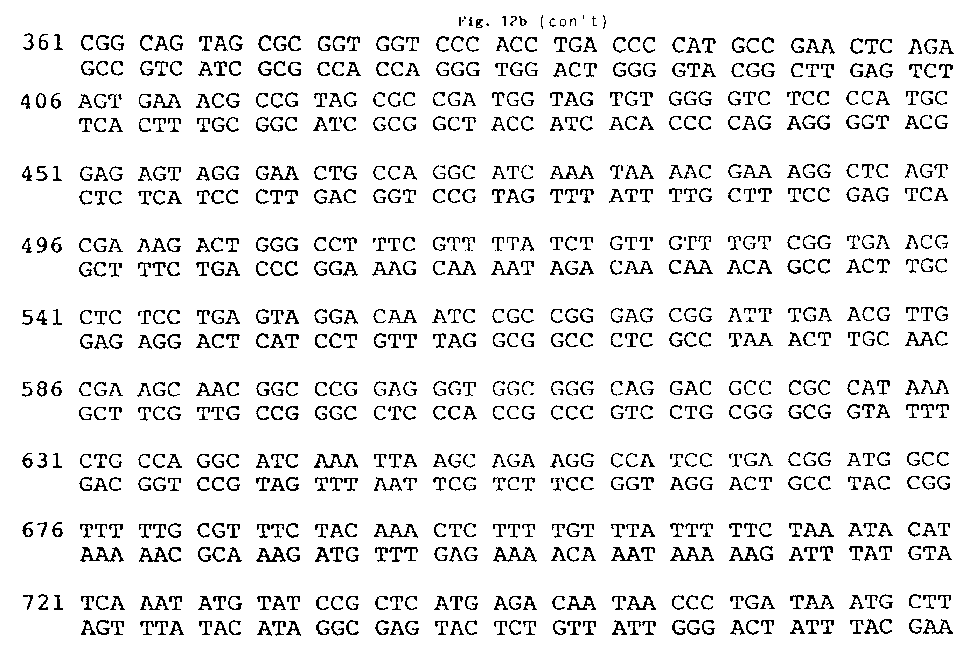

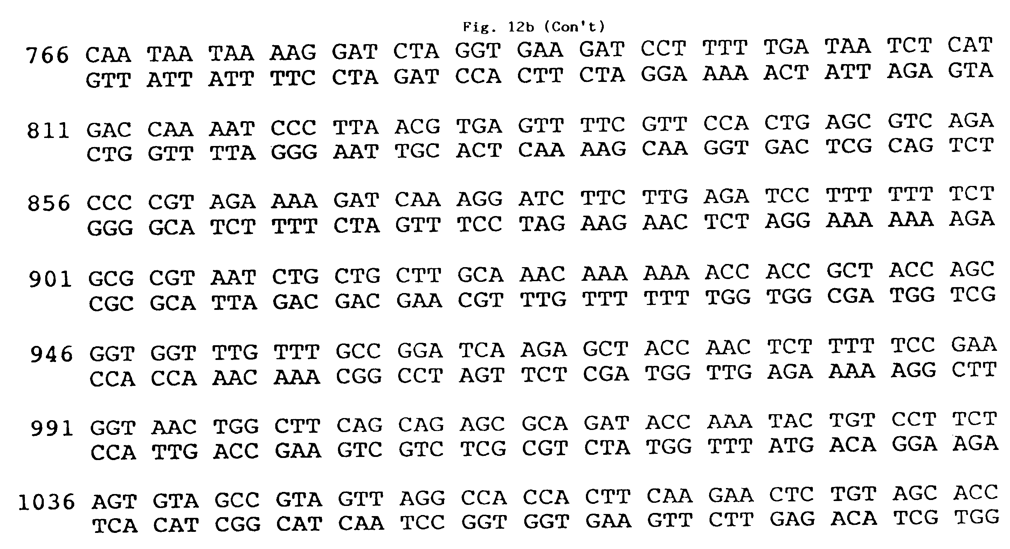

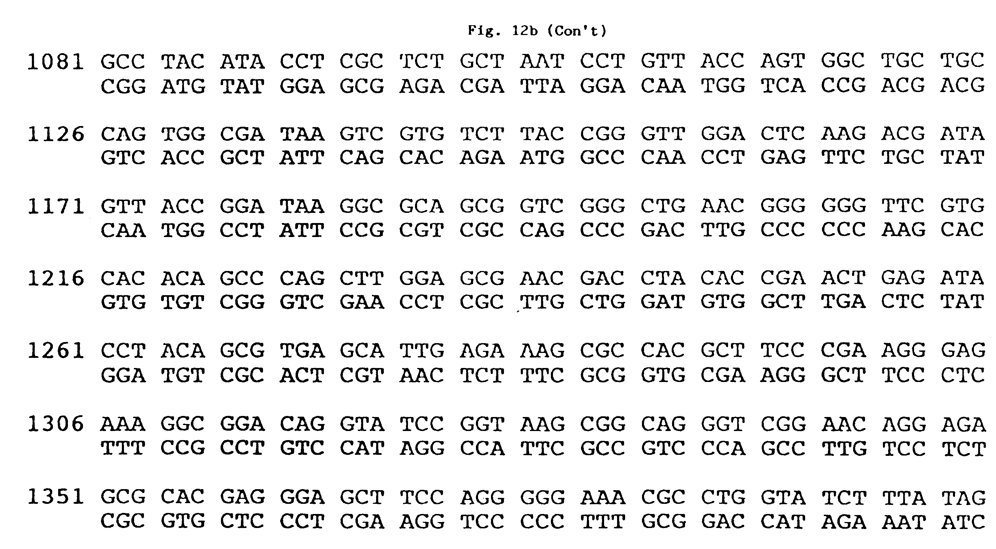

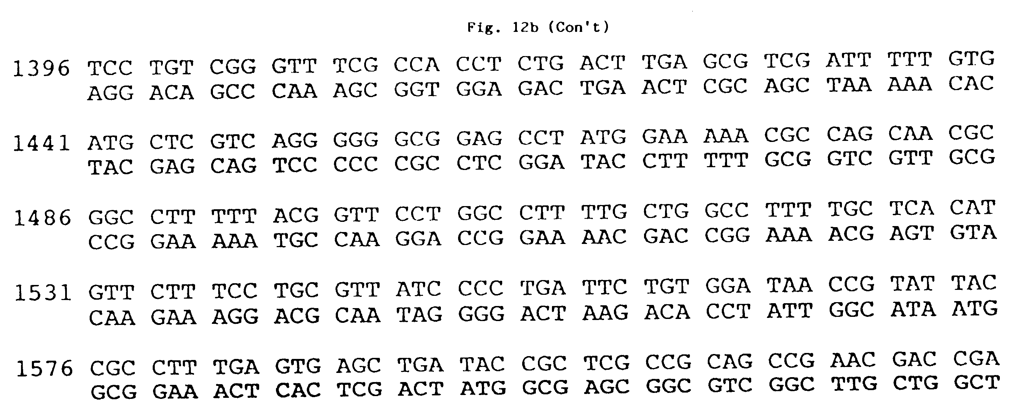

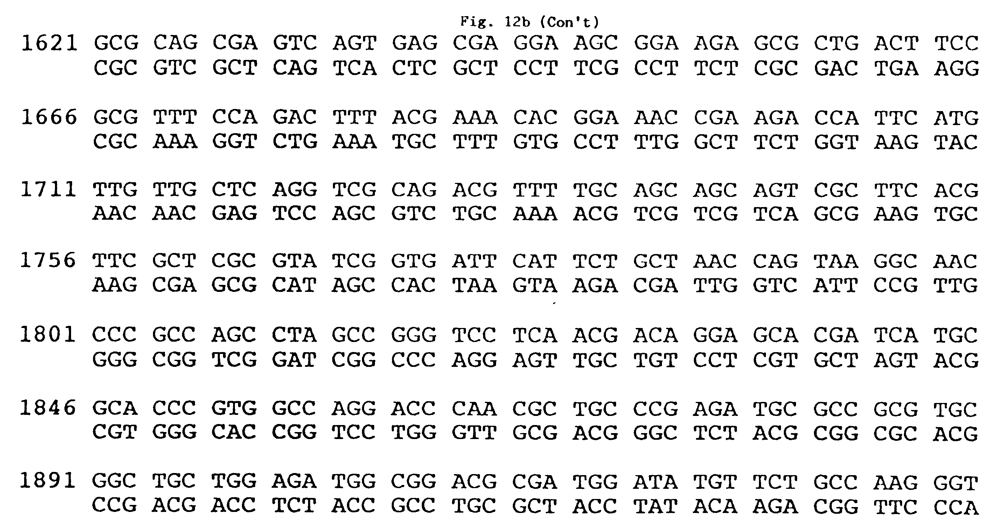

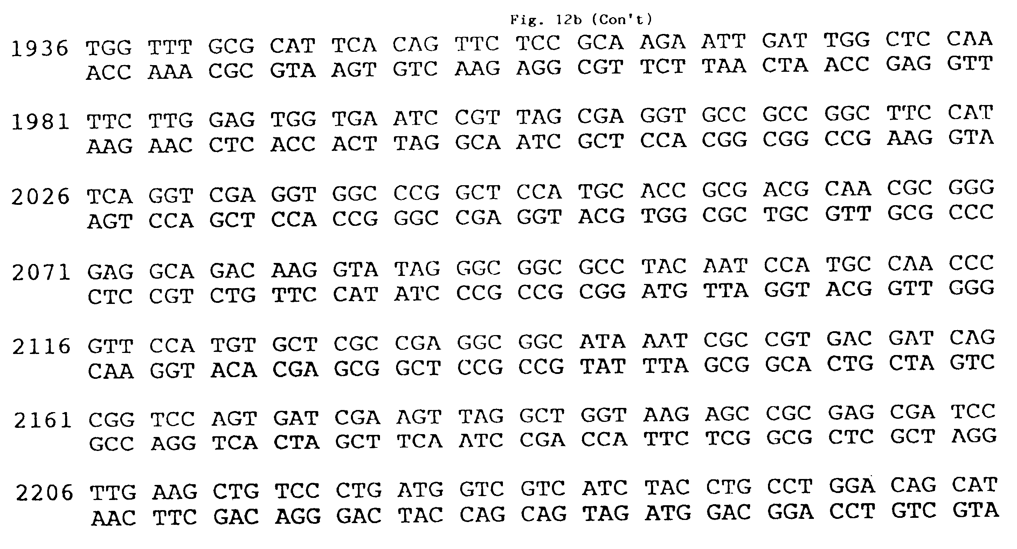

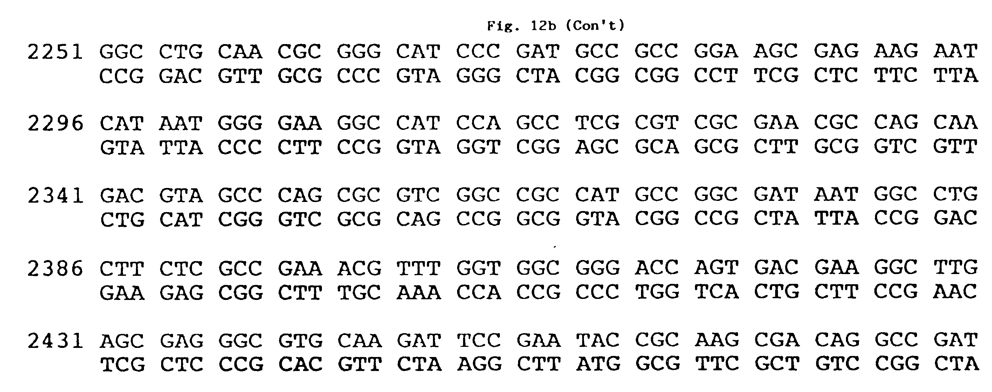

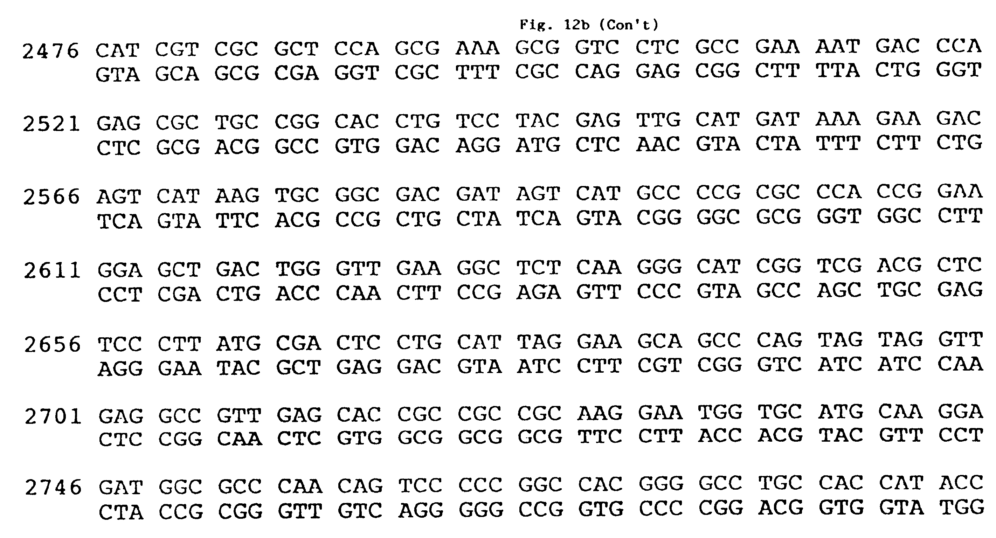

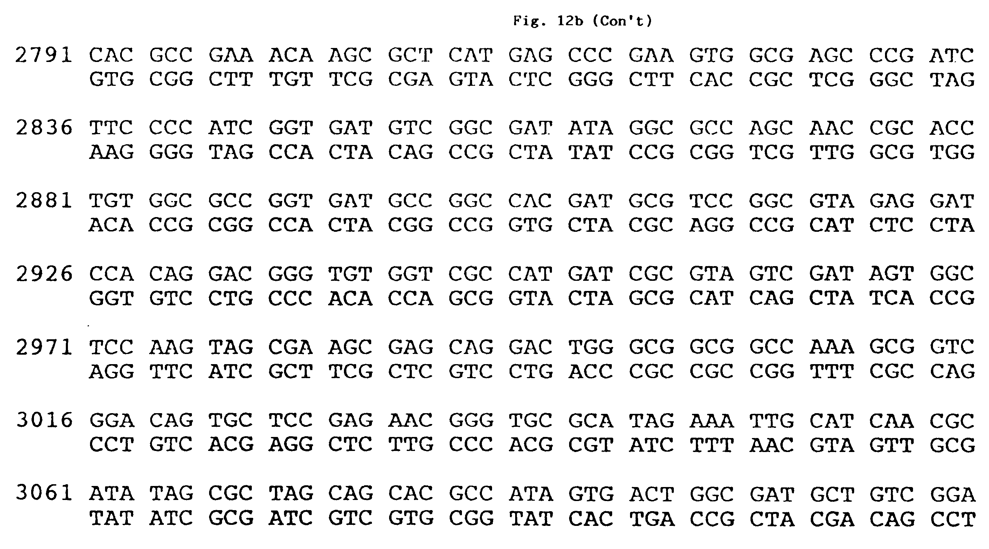

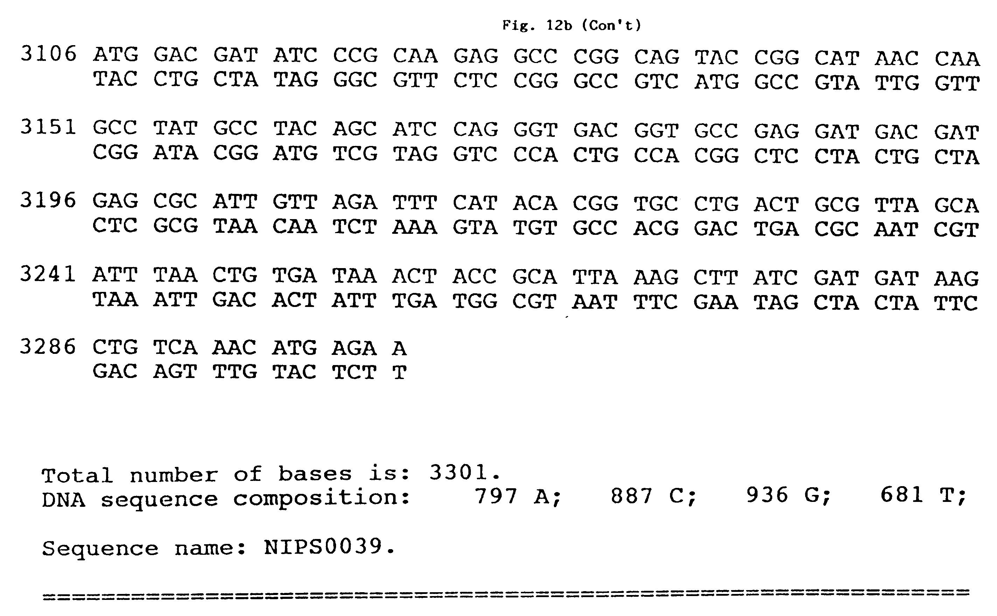

Figure 12b corresponds to the pIG2 nucleic acid sequence.

On this figure, the origin of nucleotide stretches used to construct plasmid pIG2 is specified hereafter.

### Position

- 3300-206 :: lambda PL containing EcoRI-MboII blunt fragment of pPL(λ) (Pharmacia)
- 207-266 :: synthetic sequence containing multiple cloning site and ribosome binding site of which the ATG initiation codon is located at position 232-234
- 267-772 :: rrnBT₁T₂ containing HindIII-SspI fragment from pKK223 (Pharmacia)
- 773-3300 :: tetracycline resistance gene and origin of replication containing EcoRI-DraI fragment of pAT 153 (Bioexcellence)

Table 7 corresponds to the complete restriction site analysis of pIG2.

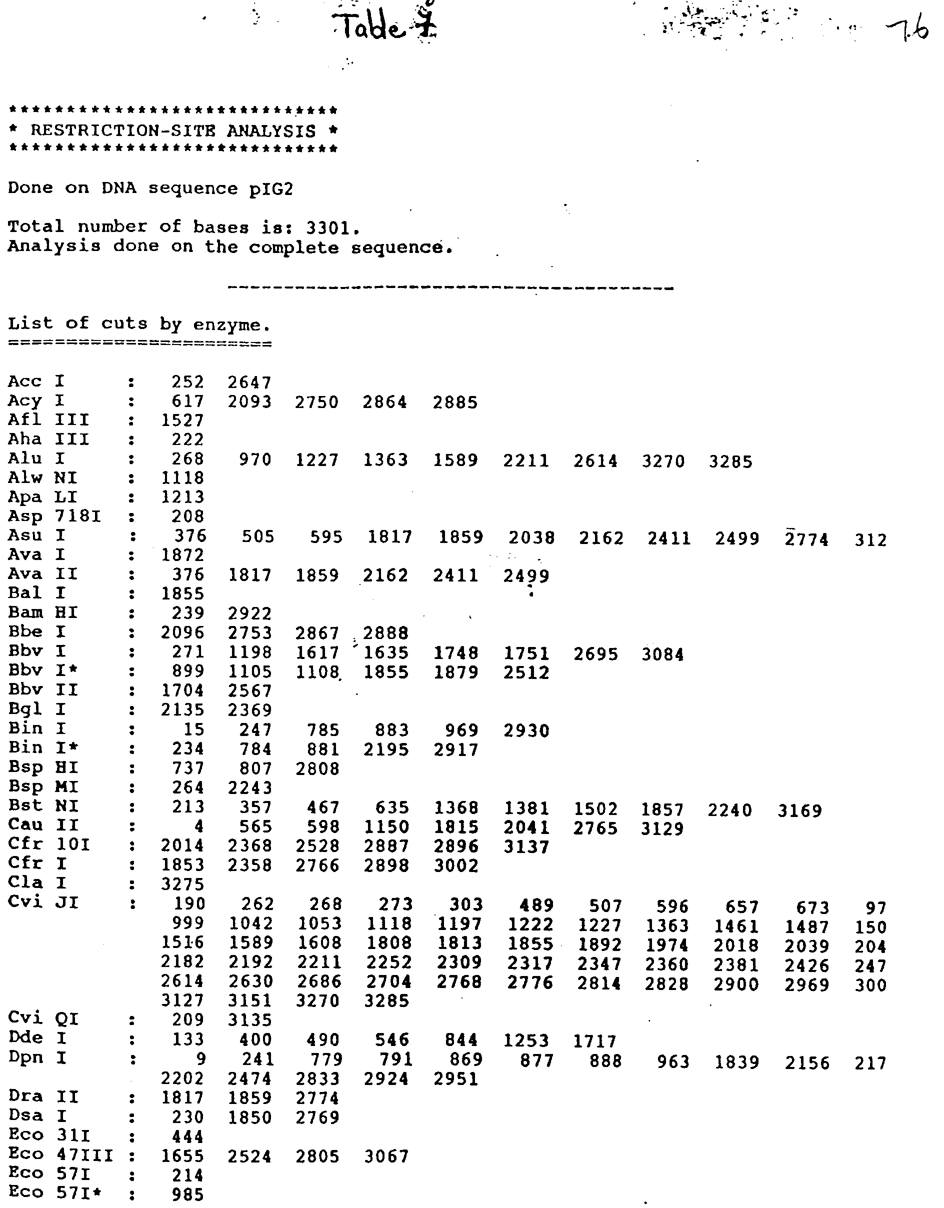

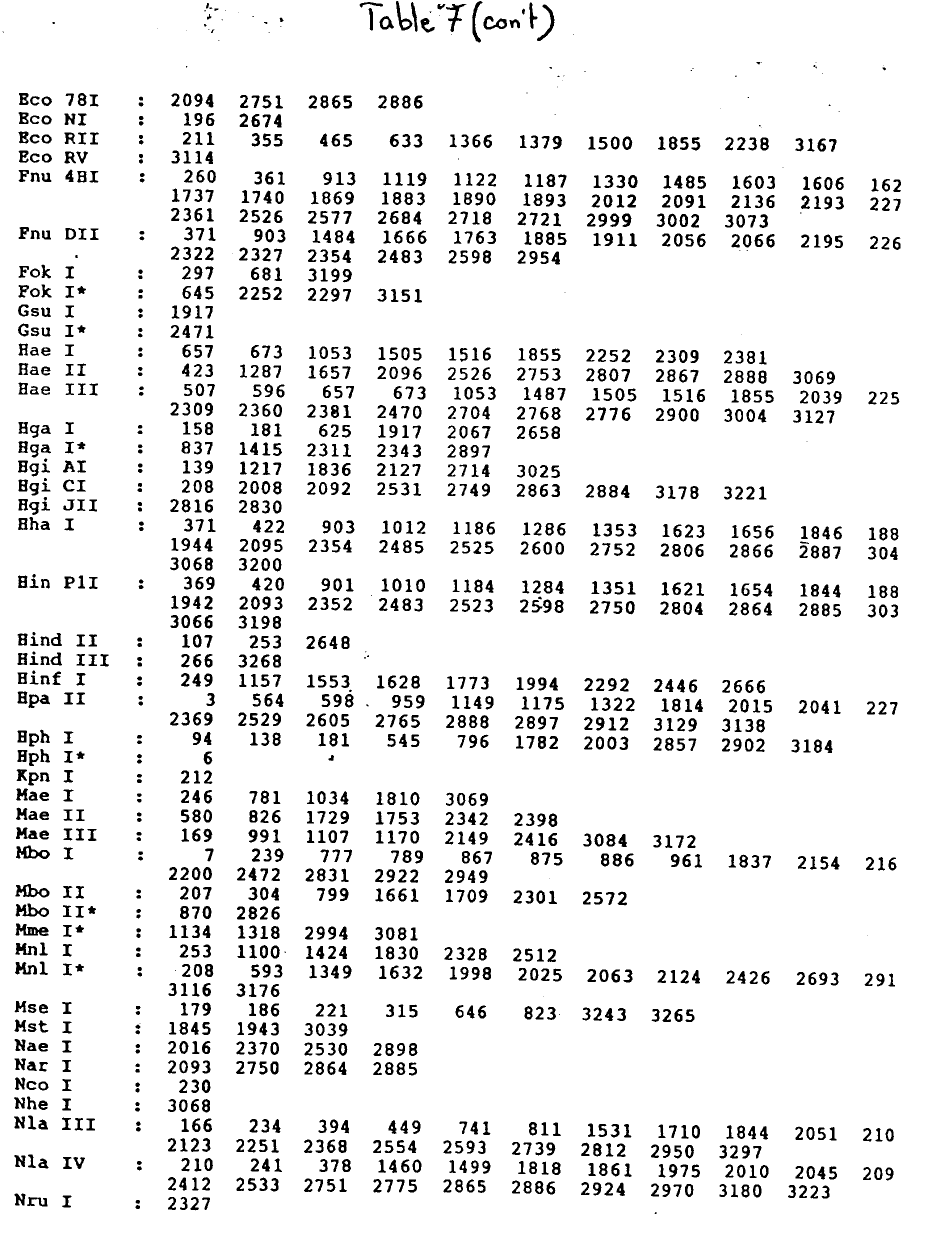

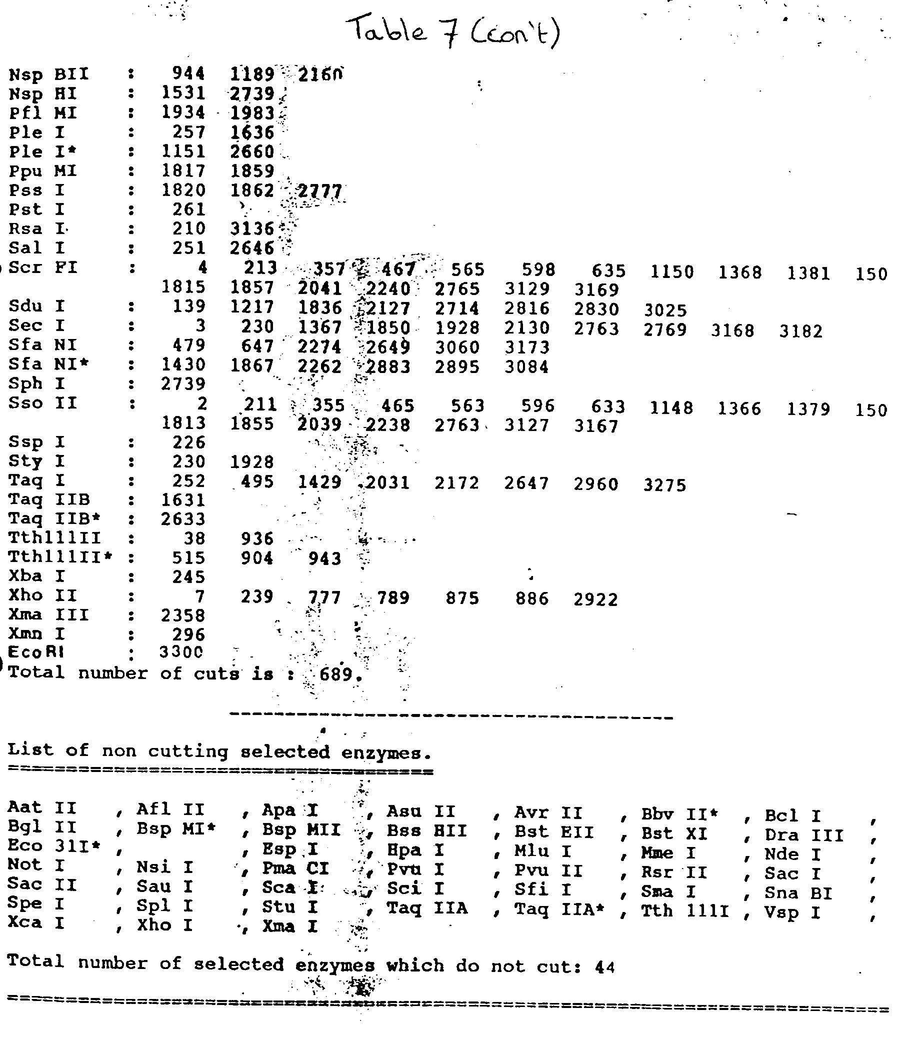

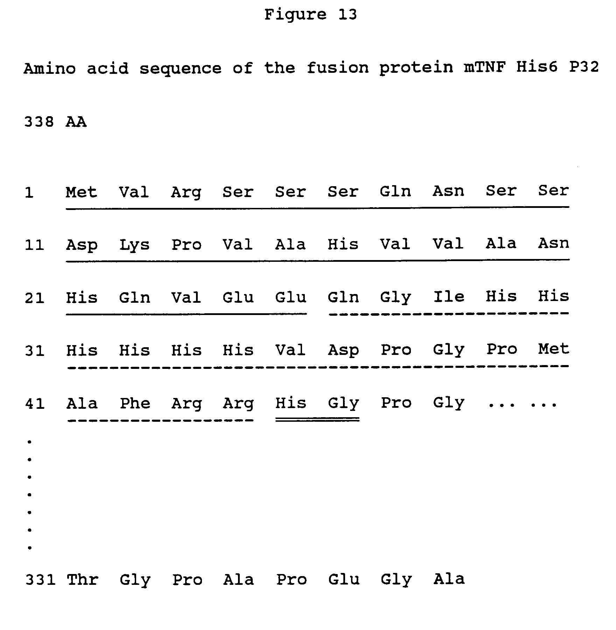

Figure 13 corresponds to the amino acid sequence of the total fusion protein mTNF-His₆-P₃₂.

On this figure :
- the continuous underlined sequence ( ) represents the mTNF sequence (first 25 amino acids),
- the dotted underlined sequence (-----) represents the polylinker sequence,
- the double underlined sequence 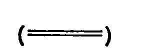 represents the extra amino acids created at cloning site, and
- the amino acid marked with nothing is the antigen sequence starting from the amino acid at position 4 of figure 5.

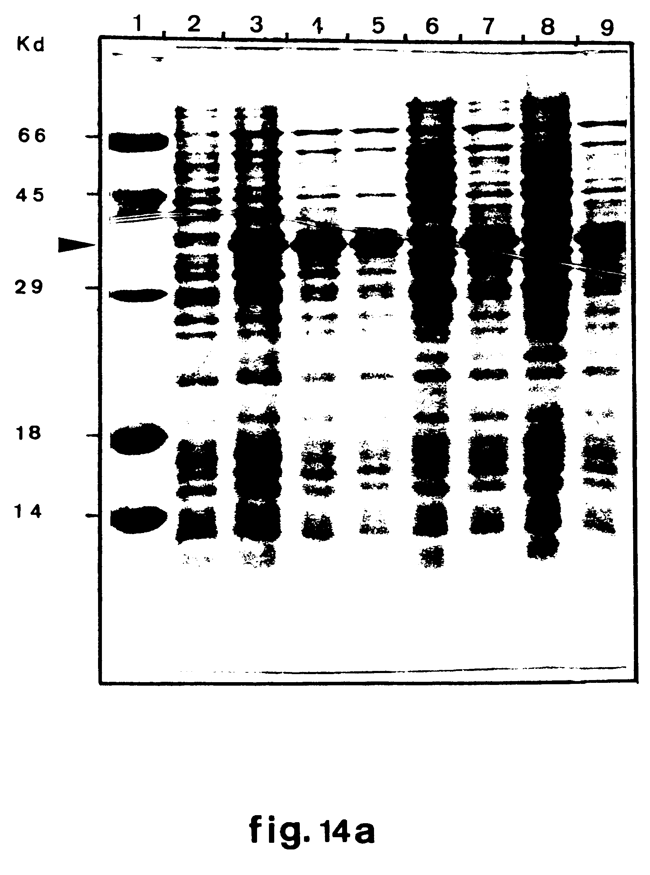

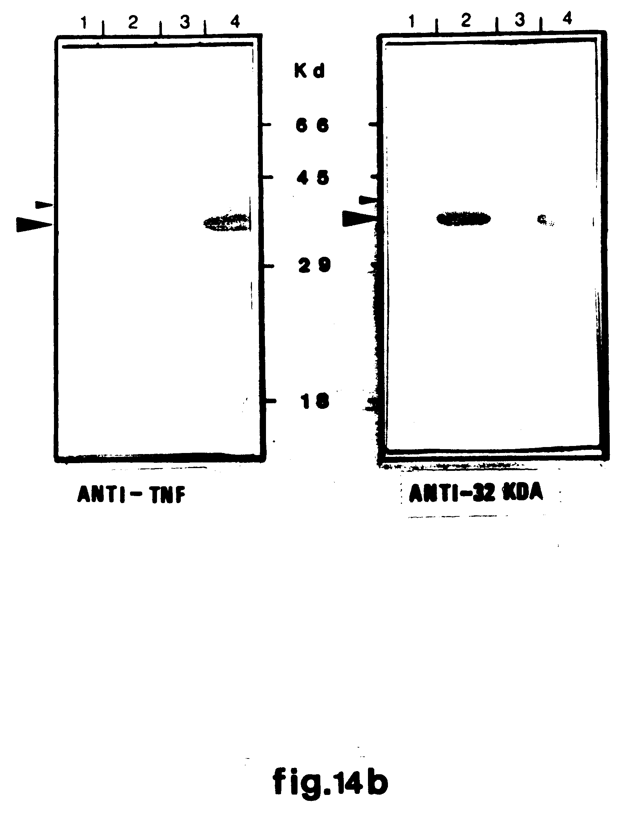

Figure 14a and 14b correspond to the expression of the mTNF-His₆-P₃₂ fusion protein in K12ΔH, given in Example VI, with Fig. 14a representing the Coomassie Brilliant Blue stained SDS-PAGE and 14b representing immunoblots of the gel with anti-32-kDa and anti-mTNF-antibody.

On fig. 14a, the lanes correspond to the following:

| Lanes | | | |
|---|---|---|---|
| 1. | protein molecular weight markers | | |
| 2. | pmTNF-MPH-Mt32 | 28°C | 1 h induction |
| 3. | pmTNF-MPH-Mt32 | 42°C | 1 h induction |
| 4. | pmTNF-MPH-Mt32 | 42°C | 2 h induction |
| 5. | pmTNF-MPH-Mt32 | 42°C | 3 h induction |
| 6. | pmTNF-MPH-Mt32 | 28°C | 4 h induction |
| 7. | pmTNF-MPH-Mt32 | 42°C | 4 h induction |
| 8. | pmTNF-MPH-Mt32 | 28°C | 5 h induction |
| 9. | pmTNF-MPH-Mt32 | 42°C | 5 h induction |

On fig. 14b, the lanes correspond to the following:

| Lanes | | | |
|---|---|---|---|
| 1. | pmTNF-MPH-Mt32 | 28°C | 1 h induction |
| 2. | pmTNF-MPH-Mt32 | 42°C | 1 h induction |
| 3. | pmTNF-MPH-Mt32 | 28°C | 4 h induction |
| 4. | pmTNF-MPH-Mt32 | 42°C | 4 h induction |

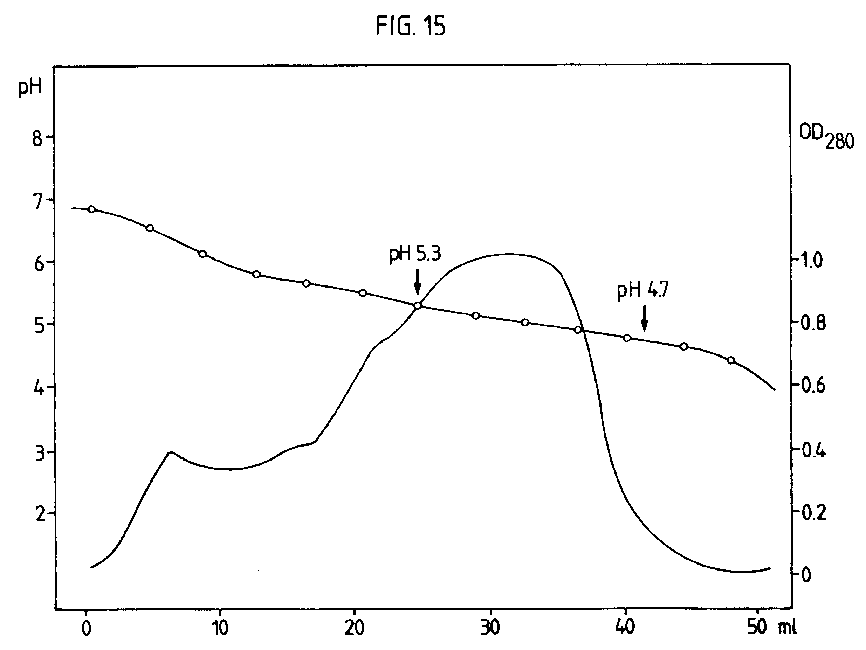

Figure 15 corresponds to the IMAC elution profile of the recombinant antigen with decreasing pH as presented in Example VII.

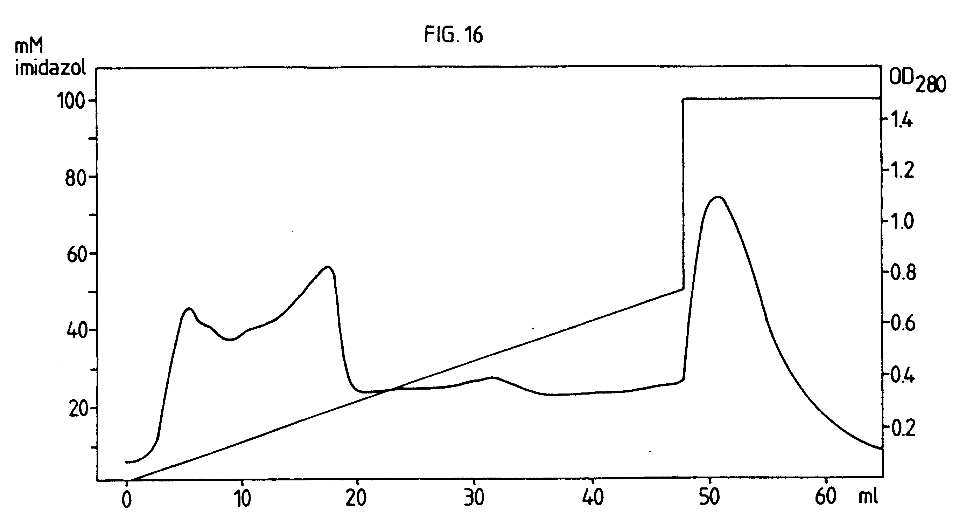

Figure 16 corresponds to the IMAC elution profile of the recombinant antigen with increasing imidazole concentrations as presented in Example VII.

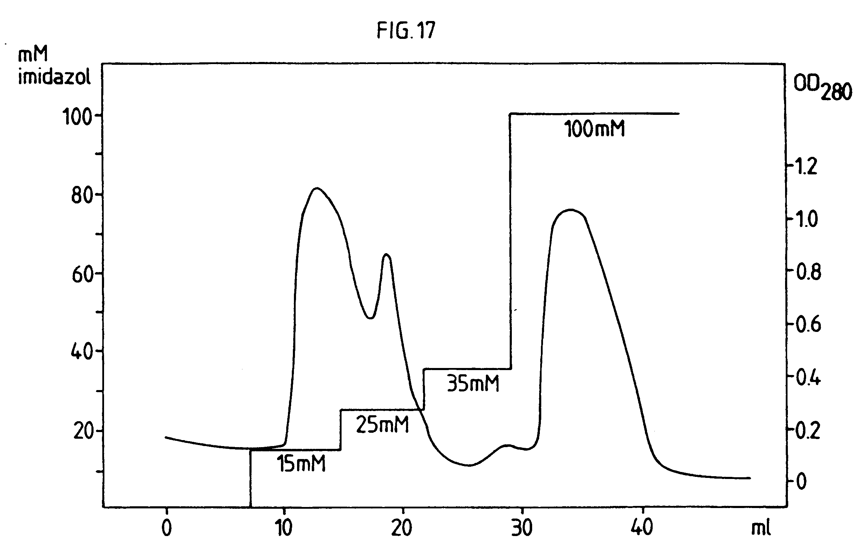

Figure 17 corresponds to the IMAC elution profile of the recombinant antigen with a step gradient of increasing imidazole concentrations as presented in Example VII.

### EXAMPLE I:

### MATERIAL AND METHODS

### Screening of the λgt11 M. tuberculosis recombinant DNA library with anti-32-kDa antiserum

A λgt11 recombinant library constructed from genomic DNA of M. tuberculosis (Erdman strain), was obtained from R. Young (35). Screening was performed as described (14,35) with some modifications hereafter mentioned. λgt11 infected E. coli Y1090 (10⁵ pfu per 150 mm plate) were seeded on NZYM plates (Gibco)(16) and incubated at 42°C for 24 hrs. To induce expression of the β-galactosidase-fusion proteins the plates were overlaid with isopropyl β-D-thiogalactoside (IPTG)-saturated filters (Hybond C extra, Amersham), and incubated for 2 hrs at 37°C. Screening was done with a polyclonal rabbit anti-32-kDa antiserum. Said polyclonal antiserum rabbit anti-32-kDa antiserum was obtained by raising antiserum against the P₃₂ M. bovis BCG (strain 1173P2 - Institut Pasteur Paris) as follows: 400 µg (purified P₃₂ protein of M. bovis BCG) per ml physiological saline were mixed with one volume of incomplete Freund's adjuvant. The material was homogenized and injected intradermally in 50 µl doses, delivered at 10 sites in the back of the rabbits, at 0, 4, 7 and 8 weeks (adjuvant was replaced by the diluent for the last injection). One week later, the rabbits were bled and the sera tested for antibody level before being distributed in aliquots and stored at -80°C.

The polyclonal rabbit anti-32-kDa antiserum was pre-absorbed on E. coli lysate (14) and used at a final dilution of 1:300. A secondary alkaline-phosphatase anti-rabbit IgG conjugate (Promega), diluted at 1:5000 was used to detect the β-galactosidase fusion proteins. For color development nitro blue tetrazolium (NBT) and 5-bromo-4-chloro-3-indolyl phosphate (BCIP) were used. Reactive areas on the filter turned deep purple within 30 min. Usually three consecutive purification steps were performed to obtain pure clones. IPTG, BCIP and NBT were from Promega corp. (Madison WI.).

### Plaque screening by hybridization for obtaining the secondary clones BY1, By2 and By5 hereafter defined

The procedure used was as described by Maniatis et al. (14).

### Preparation of crude lysates from λgt11 recombinant lysogens

Colonies of E. coli Y1089 were lysogenized with appropriate λgt11 recombinants as described by Hyunh et al. (14). Single colonies of lysogenized E. coli Y1089 were inoculated into LB medium and grown to an optical density of 0.5 at 600nm at 30°C. After a heat shock at 45°C for 20 min., the production of β-galactosidase-fusion protein was induced by the addition of IPTG to a final concentration of 10 mM. Incubation was continued for 60 min. at 37°C and cells were quickly harvested by centrifugation. Cells were concentrated 50 times in buffer (10 mM Tris pH 8.0, 2 mM EDTA) and rapidly frozen into liquid nitrogen. The samples were lysed by thawing and treated with 100 µg/ml DNase I in EcoRI restriction buffer, for 5-10 minutes at 37°C.

### Immunoblotting (Western blotting) analysis:

After SDS-PAGE electrophoresis, recombinant lysogen proteins were blotted onto nitrocellulose membranes (Hybond C, Amersham) as described by Towbin et al. (29). The expression of mycobacterial antigens, fused to β-galactosidase in E. coli Y1089 was visualized by the binding of a polyclonal rabbit anti-32-kDa antiserum (1:1000) obtained as described in the above paragraph "Screening of the λgt11 M. tuberculosis recombinant DNA library with anti-32-kDa antiserum" and using a monoclonal anti-β-galactosidase antibody (Promega). A secondary alkaline-phosphatase anti-rabbit IgG conjugate (Promega) diluted at 1:5000, was used to detect the fusion proteins.

The use of these various antibodies enables to detect the β-galactosidase fusion protein. This reaction is due to the M. tuberculosis protein because of the fact that non fused-β-galactosidase is also present on the same gel and is not recognized by the serum from tuberculous patients.

In order to identify selective recognition of recombinant fusion proteins by human tuberculous sera, nitrocellulose sheets were incubated overnight with these sera (1:50) (after blocking aspecific protein binding sites). The human tuberculous sera were selected for their reactivity (high or low) against the purified 32-kDa antigen of M. bovis BCG tested in a Dot blot assay as previously described (31). Reactive areas on the nitrocellulose sheets were revealed by incubation with peroxidase conjugated goat anti-human IgG antibody (Dakopatts, Copenhagen, Denmark) (1:200) for 4 hrs and after repeated washings color reaction was developed by adding peroxidase substrate (α-chloronaphtol) (Bio-Rad) in the presence of peroxidase and hydrogen peroxide.

### Recombinant DNA analysis

Initial identification of M. tuberculosis DNA inserts in purified λgt11 clones was performed by EcoRI restriction. After digestion, the excised inserts were run on agarose gels and submitted to Southern hybridization. Probes were labeled with α³²P-dCTP by random priming (10). Other restriction sites were located by single and double digestions of recombinant λgt11 phage DNA or their subcloned EcoRI fragments by HindIII, pstI, KpnI, AccI and SphI.

### Sequencing

Sequence analysis was done by the primer extension dideoxy termination method of Sanger et al. (25) after subcloning of specific fragments in Bluescribe-M13 (6) or in mp10 and mp11 M13 vectors (Methods in Enzymology, vol. 101, 1983, p.20-89, Joachim Messing, New M13 vectors for cloning, Academic Press). Sequence analysis was greatly hampered by the high GC content of the M. tuberculosis DNA (65%). Sequencing reactions were therefore performed with several DNA polymerases: T7 DNA polymerase ("Sequenase" USB), Klenow fragment of DNA polymerase I (Amersham) and in some cases with AMV reverse transcriptase (Super RT, Anglian Biotechnology Ltd.) and sometimes with dITP instead of dGTP. Several oligodeoxynucleotides were synthesized and used to focus ambiguous regions of the sequence. The sequencing strategy is summarized in Fig. 2 In order to trace possible artefactual frameshifts in some ambiguous regions, a special program was used to define the most probable open reading frame in sequences containing a high proportion of GC (3). Several regions particularly prone to sequencing artefacts were confirmed or corrected by chemical sequencing (18). For this purpose, fragments were subcloned in the chemical sequencing vector pGV462 (21) and analysed as described previously. Selected restriction fragments of about 250-350bp were isolated, made blunt-ended by treatment with either Klenow polymerase or Mung bean nuclease, and subcloned in the SmaI or HincII site of pGV462. Both strands of the inserted DNA were sequenced by single-end labeling at Tth 111I or BstEII (32) and a modified chemical degradation strategy (33).

Routine computer aided analysis of the nucleic acid and deduced amino acid sequences were performed with the LGBC program from Bellon (2). Homology searches used the FASTA programs from Pearson and Lipman (23) and the Protein Identification Resource (PIR) from the National Biomedical Research Fundation - Washington (NBRF) (NBRF/PIR data bank), release 16 (march 1988).

### RESULTS

### - Screening of the λgt11M, M. tuberculosis recombinant DNA library with polyclonal anti-32-kDa antiserum :

Ten filters representing 1.5x10⁶ plaques were probed with a polyclonal rabbit anti-32-kDa antiserum (8). Following purification, six independent positive clones were obtained.

### Analysis of recombinant clones

EcoRI restriction analysis of these 6 purified λgt11 recombinant clones DNA, (Fig. 1A) revealed 4 different types of insert. Clone 15 had an insert with a total length of 3.8 kb with two additional internal EcoRI sites resulting in three DNA fragments of 1.8 kb, 1.5 kb and 0.5 kb. The DNA Insert of clone 16 was 1.7 kb long. Clones 17 and 19 had a DNA insert of almost identical length being 2.7 kb and 2.8 kb respectively.

Finally, clone 23 (not shown) and clone 24 both contained an insert of 4 kb with one additional EcoRI restriction site giving two fragments of 2.3 kb and 1.7 kb. Southern analysis (data not shown) showed that the DNA inserts of clones 15, 16, 19 and the small fragment (1.7 kb) of clone 24 only hybridized with themselves whereas clone 17 (2.7 kb) hybridized with itself but also equally well with the 2.3 kb DNA fragment of clone 24. Clones 15, 16 and 19 are thus distinct and unrelated to the 17, 23, 24 group. This interpretation was further confirmed by analysis of crude lysates of E. coli Y1089 lysogenized with the appropriate λgt11 recombinants and induced with IPTG. Western blot analysis (Fig. 1B), showed no fusion protein, either mature or incomplete, reactive with the polyclonal anti-32-kDa antiserum in cells expressing clones 15, 16 and 19. Clones 15, 16 and 19, were thus considered as false positives and were not further studied. On the contrary, cells lysogenized with clone 23 and 24 produced an immunoreactive fusion protein containing about 10 kDa of the 32-kDa protein. Clone 17 finally expressed a fusion protein of which the foreign polypeptide part is about 25 kDa long. The restriction endonuclease maps of the 2.3 kb insert of clone 24 and of the 2.7 kb fragment of clone 17 (Fig. 2) allowed us to align and orient the two inserts suggesting that the latter corresponds to a ±0.5 kb 5′ extension of the first.

As clone 17 was incomplete, the same λgt11 recombinant M. tuberculosis DNA library was screened by hybridization with a 120 bp EcoRI-Kpn1 restriction fragment corresponding to the very 5′ end of the DNA insert of clone 17 (previously subcloned in a Blue Scribe vector commercialized by Vector cloning Systems (Stratagene Cloning System) (Fig. 2). Three 5′-extended clones By1, By2 and By5 were isolated, analyzed by restriction and aligned. The largest insert, By5 contained the information for the entire coding region (see below) flanked by 3.1 kb upstream and 1.1 kb downstream (Fig. 2).

### DNA sequencing

The 1358 base pairs nucleotide sequence derived from the various λgt11 overlapping clones is represented in Fig. 3a and Fig. 3b. The DNA sequence contains a 1059 base pair open reading frame starting at position 183 and ending with a TAG codon at position 1242. It occurs that the NH₂-terminal amino-acid sequence, (phe-ser-arg-pro-gly-leu-pro-val-glu-tyr-leu-gln-val-pro-ser-pro-ser-met-gly-arg-asp-ile-lys-val-gln-phe-gln-ser-gly-gly-ala-asn) which can be located within this open reading frame from the nucleotide sequence beginning with a TTT codon at position 360 corresponds to the same NH₂-terminal amino acid sequence of the MPB 59 antigen except for the amino acids at position 20, 21, 31, which are respectively gly, cys and asn in the MPB 59 (34). Therefore, the DNA region upstream of this sequence is expected to encode a signal peptide required for the secretion of a protein of 32-kDa. The mature protein thus presumably consists of 295 amino acid residues from the N-terminal Phe (TTT codon) to the C-terminal Ala (GCC codon) (Fig. 5).

Six ATG codons were found to precede the TTT at position 360 in the same reading frame. Usage of any of these ATGs in the same reading frame would lead to the synthesis of signal peptides of 29,42,47,49,55 and 59 residues.

### Hydropathy pattern

The hydropathy pattern coding sequence of the protein of 32-kDa of the invention and that of the antigen α of BCG (17) were determined by the method of Kyte and Doolittle (15). The nonapeptide profiles are shown in Fig. 6. Besides the initial hydrophobic signal peptide region, several hydrophilic domains could be identified. It is interesting to note that the overall hydrophilicity pattern of the protein of 32-kDa of the invention is comparable to that of the BCG antigen α. For both proteins, a domain of highest hydrophilicity could be identified between amino acid residues 200 and 250.

### Homology

Matsuo et al. (17) recently published the sequence of a 1095 nucleotide cloned DNA corresponding to the gene coding for the antigen α of BCG. The 978 bp coding region of M. bovis antigen α as revised in Infection and Immunity, vol. 58, p. 550-556, 1990, and 1017 bp coding regions of the protein of 32-kDa of the invention show a 77.5% homology, in an aligned region of 942 bp. At the amino acid level both precursor protein sequences share 75.6% identical residues. In addition, 17.6% of the amino acids correspond to evolutionary conserved replacements as defined in the algorithm used for the comparison (PAM250 matrix, ref 23). Figure 7 shows sequence divergences in the N-terminal of the signal peptide. The amino terminal sequence - 32 amino acids - of both mature proteins is identical except for position 31.

### Human sera recognize the recombinant 32-kDa protein

Fig. 8 shows that serum samples from tuberculous patients when immunoblotted with a crude E. coli extract expressing clone 17 distinctly react with the 140 kDa fusion protein (lanes 4 to 6) contain the protein of 32-kDa of the invention, but not with unfused β-galactosidase expressed in a parallel extract (lanes 10 to 12). Serum samples from two negative controls selected as responding very little to the purified protein of 32-kDa of the invention does neither recognize the 140 kDa fused protein containing the protein of 32-kDa of the invention, nor the unfused β-galactosidase (lanes 2, 3 and 8 and 9). The 140 k-Da fused protein and the unfused β-galactosidase were easily localized reacting with the anti-β-galactosidase monoclonal antibody (lanes 1 to 7).

The invention has enabled to prepare a DNA region coding particularly for a protein of 32-kDa (cf. fig.5); said DNA region containing an open reading frame of 338 codons (stop codon non included). At position 220 a TTT encoding the first amino acid of the mature protein is followed by the 295 triplets coding for the mature protein of 32-kDa. The size of this open reading frame, the immunoreactivity of the derived fusion proteins, the presence of a signal peptide and, especially, the identification within this gene of a NH₂-terminal region highly homologous to that found in the MPB 59 antigen (31/32 amino acids homology) and in the BCG antigen α (31/32 amino acids homology) (see Fig. 7), strongly suggest that the DNA fragment described contains the complete cistron encoding the protein of 32-kDa secreted by M. tuberculosis, which had never been so far identified in a non-ambiguous way.

Six ATG codons were found to precede this TTT at position 220 in the same reading frame. Usage of any of these ATGs in the same reading frame would lead to the synthesis of signal peptides of 43, 48, 50, 56 or 60 residues. Among these various possibilities, initiation is more likely to take place either at ATG₉₁ or ATG₅₂ because both are preceded by a plausible E. coli-like promoter and a Shine-Dalgarno motif.

If initiation takes place at ATG₉₁, the corresponding signal peptide would code for a rather long peptide signal of 43 residues. This length however is not uncommon among secreted proteins from Gram positive bacteria (5). It would be preceded by a typical E. coli Shine-Dalgarno motif (4/6 residues homologous to AGGAGG) at a suitable distance.

If initiation takes place at ATG₅₂, the corresponding signal peptide would code for a peptide signal of 56 residues but would have a less stringent Shine-Dalgarno ribosome binding site sequence.

The region encompassing the translation termination triplet was particularly sensitive to secondary structure effects which lead to so-called compressions on the sequencing gels. In front of the TAG termination codon at position 1105, 22 out of 23 residues are G-C base pairs, of which 9 are G's.

Upstream ATG₁₃₀, a sequence resembling an E. coli promoter (11) comprising an hexanucleotide (TTGAGA) (homology 5/6 to TTGACA) and a AAGAAT box (homology 4/6 to TATAAT) separated by 16 nucleotides was observed. Upstream the potential initiating codon ATG₉₁, one could detect several sequences homologous to the E. coli "-35 hexanucleotide box", followed by a sequence resembling a TATAAT box. Among these, the most suggestive is illustrated on Fig. 3a and 3b. It comprises a TTGGCC at position 59 (fig. 3a and 3b) (homology 4/6 to TTGACA) separated by 14 nucleotides from a GATAAG (homology 4/6 to TATAAT). Interestingly this putative promoter region shares no extensive sequence homology with the promoter region described for the BCG protein α-gene (17) nor with that described for the 65 kDa protein gene (26, 28).

Searching the NBRF data bank (issue 16.0) any significant homology between the protein of 32-kDa of the invention and any other completely known protein sequence could not be detected. In particular no significant homology was observed between the 32-kDa protein and α and β subunits of the human fibronectin receptor (1). The NH₂-terminal sequence of the 32-kDa protein of the invention is highly homologous - 29/32 amino acids - to that previously published for BCG MPB 59 antigen (34) and to that of BCG α-antigen - 31/32 amino acids - (Matsuo, 17) and is identical in its first 6 amino acids with the 32-kDa protein of M. bovis BCG (9). However, the presumed initiating methionine precedes an additional 29 or 42 amino acid hydrophobic sequence which differs from the one of α-antigen (cf. Fig. 7), but displaying all the characteristics attributed to signal sequences of secreted polypeptides in prokaryotes (22).

Interestingly, no significant homology between the nucleic acid (1-1358) of the invention (cf. fig. 3a and 3b) and the DNA of the antigen α of Matsuo exists within their putative promoter regions.

### EXAMPLE II: CONSTRUCTION OF A BACTERIAL PLASMID CONTAINING THE ENTIRE CODING SEQUENCE OF THE 32-kDa PROTEIN OF M. TUBERCULOSIS

In the previous example, in figure 2, the various overlapping λgt11 isolates covering the 32-kDa protein gene region from M. tuberculosis were described. Several DNA fragments were subcloned from these λgt11 phages in the Blue Scribe M13+ plasmid (Stratagene). Since none of these plasmids contained the entire coding sequence of the 32-kDa protein gene, a plasmid containing this sequence was reconstructed.

### Step 1 : Preparation of the DNA fragments :

1) The plasmid BS-By5-800 obtained by subcloning HindIII fragments of By5 (cf. fig. 2) into the Blue Scribe M13⁺ plasmid (Stratagene), was digested with HindIII and a fragment of 800 bp was obtained and isolated from a 1% agarose gel by electroelution.
2) The plasmid BS-4.1 obtained by subcloning the 2,7 kb EcoRI insert from λgt11-17) into the Blue Scribe M13⁺ plasmid (Stratagene) (see fig.2 of patent application) was digested with HindIII and SphI and a fragment of 1500 bp was obtained and isolated from a 1% agarose gel by electroelution.
3) Blue Scribe M13⁺ was digested with HindIII and SphI, and treated with calf intestine alkaline phosphatase (special quality for molecular biology, Boehringer Mannheim) as indicated by the manufacturer.

### Step 2 : ligation :

The ligation reaction contained :
125 ng of the 800 bp HindIII fragment (1)
125 ng of the 1500 bp SphI-HindIII insert (2)
50 ng of the HindIII-SphI digested BSM13⁺ vector (3)
2 µl of 10 ligation buffer (Maniatis et al., 1982)
1 µl of (= 2,5 U) of T4 DNA ligase (Amersham)
4 µl PEG 6000, 25% (w/v)
8 µl H₂O

The incubation was for 4 hours at 16°C.

### Step 3 : Transformation :

100 µl of DH5α E. coli (Gibco BRL) were transformed with 10 µl of the ligation reaction (step 2) and plated on IPTG, X-Gal ampicillin plates, as indicated by the manufacturer. About 70 white colonies were obtained.

### step 4 :

As the 800 bp fragment could have been inserted in both orientations, plasmidic DNA from several clones were analyzed by digestion with PstI in order to select one clone (different from clone 11), characterized by the presence of 2 small fragments of 229 and 294 bp. This construction contains the HindIII-HindIII-SphI complex in the correct orientation. The plasmid containing this new construction was called : "BS.BK.P₃₂.complet".

### EXAMPLE III: EXPRESSION OF A POLYPEPTIDE OF THE INVENTION IN E. COLI:

The DNA sequence coding for a polypeptide, or part of it, can be linked to a ribosome binding site which is part of the expression vector, or can be fused to the information of another protein or peptide already present on the expression vector.

In the former case the information is expressed as such and hence devoid of any foreign sequences (except maybe for the aminoterminal methionine which is not always removed by E. coli).

In the latter case the expressed protein is a hybrid or a fusion protein.

The gene, coding for the polypeptide, and the expression vector are treated with the appropriate restriction enzyme(s) or manipulated otherwise as to create termini allowing ligation. The resulting recombinant vector is used to transform a host. The transformants are analyzed for the presence and proper orientation of the inserted gene. In addition, the cloning vector may be used to transform other strains of a chosen host. Various methods and materials for preparing recombinant vectors, transforming them to host cells and expressing polypeptides and proteins are described by Panayatatos, N., in "Plasmids, a practical approach (ed. K.G. Hardy, IRL Press) pp.163-176, by Old and Primrose, principals of gene manipulation (2d Ed, 1981) and are well known by those skilled in the art.

Various cloning vectors may be utilized for expression. Although a plasmid is preferable, the vector may be a bacteriophage or cosmid. The vector chosen should be compatible with the host cell chosen.

Moreover, the plasmid should have a phenotypic property that will enable the transformed host cells to be readily identified and separated from those which are not transformed. Such selection genes can be a gene providing resistance to an antibiotic like for instance, tetracyclin, carbenicillin, kanamycin, chloramphenicol, streptomycin, etc.

In order to express the coding sequence of a gene in E. coli the expression vector should also contain the necessary signals for transcription and translation.

Hence it should contain a promoter, synthetic or derived from a natural source, which is functional in E. coli. Preferably, although usually not absolutely necessary, the promoter should be controllable by the manipulator. Examples of widely used controllable promoters for expression in E. coli are the lac, the trp, the tac and the lambda PL and PR promoter.

Preferably, the expression vector should also contain a terminator of transcription functional in E. coli. Examples of used terminators of transcription are the trp and the rrnB terminators.

Furthermore, the expression vector should contain a ribosome binding site, synthetic or from a natural source, allowing translation and hence expression of a downstream coding sequence. Moreover, when expression devoid of foreign sequences is desired, a unique restriction site, positioned in such a way that it allows ligation of the sequence directly to the initiation codon of the ribosome binding site, should be present.

A suitable plasmid for performing this type of expression is pKK233-2 (Pharmacia). This plasmid contains the trc promoter, the lac Z ribosome binding site and the rrnB transcription terminator.

Also suitable is plasmid pIGRI (Innogenetics, Ghent, Belgium). This plasmid contains the tetracycline resistance gene and the origin of replication of pAT₁₅₃ (available from Bioexcellence, Biores B.V., Woerden, The Netherlands), the lambda PL promoter up to the MboII site in the 5′ untranslated region of the lambda N gene (originating from pPL(λ)); Pharmacia).

Downstream from the PL promoter, a synthetic sequence was introduced which encodes a "two cistron" translation casette whereby the stop codon of the first cistron (being the first 25 amino acids of TNF, except for Leu at position 1 which is converted to Val) is situated between the Shine-Dalgarno sequence and the initiation codon of the second ribosome binding site. The restriction and genetic map of pIGRI iS represented in Fig. 10a.

Fig. 10b and Table 5 represenx respectively the nucleic acid sequence and complete restriction site analysis of pIGRI.

However, when expression as a hybrid protein is desired, then the expression vector should also contain the coding sequence of a peptide or polypeptide which is (preferably highly) expressed by this vector in the appropriate host.

In this case the expression vector should contain a unique cleavage site for one or more restriction endonucleases downstream of the coding sequence.

Plasmids pEX1, 2 and 3 (Boehringer, Mannheim) and pUEX1, 2 and 2 (Amersham) are useful for this purpose.

They contain an ampicillin resistance gene and the origin of replication of pBR322 (Bolivar at al. (1977) Gene 2, 95-113), the lac Z gene fused at its 5' end to the lambda PR promoter together with the coding seguence for the 9 first amino acids of its natural gene cro, and a multiple cloning site at the 3' end of the lac Z coding sequence allowing production of a beta galactosidase fused polypeptide.

The pUEX vectors also contain the CI857 allele of the bacteriophage lambda CI repressor gene.

Also useful is plasmid pmTNF MPH (Innogenetics). It contains the tetracycline resistance gene and the origin of replication of pAT₁₅₃ (obtainable from Bioexcellence, Biores B.V., Woerden. The Netherlands), the lambda PL promoter up to the MboII site in the N gene 5′ untranslated region (originating from pPL(λ); Pharmacia), followed by a synthetic ribosome binding site (see sequence data), and the information encoding the first 25 AA of mTNF (except for the initial Leu which is converted to Val). This sequence is, in turn, followed by a synthetic polylinker sequence which encodes six consecutive histidines followed by several proteolytic sites (a formic acid, CNBr, kallikrein, and E. coli protease VII sensitive site, respectively), each accessible via a different restriction enzyme which is unique for the plasmid (SmaI, NcoI, BspMII and StuI, respectively; see restriction and genetic map, Fig. 11a). Downstream from the polylinker, several transcription terminators are present including the E. coli trp terminator (synthetic) and the rrnBT₁T₂ (originating from pKK223-3; Pharmacia). The total nucleic acid sequence of this plasmid is represented in Fig. 11b.

Table 6 gives a complete restriction site analysis of pmTNF MPH.

The presence of 6 successive histidines allows purification of the fusion protein by Immobilized Metal Ion Affinity Chromatography (IMAC).

After purification, the foreign part of the hybrid protein can be removed by a suitable protein cleavage method and the cleaved product can then be separated from the uncleaved molecules using the same IMAC based purification procedure.

In all the above-mentioned plasmids where the lambda PL or PR promoter is used, the promoter is temperature-control led by means of the expression of the lambda cI ts 857 allele which is either present on a defective prophage incorporated in the chromosome of the host (K12ΔH, ATCC n° 33767) or on a second compatible plasmid (pACYC derivative). Only in the pUEX vectors is this cI allele present on the vector itself.

It is to be understood that the plasmids presented above are exemplary and other plasmids or types of expression vectors maybe employed without departing from the spirit or scope of the present invention.

If a bacteriophage or phagemid is used, instead of plasmid, it should have substantially the same characteristics used to select a plasmid as described above.

### EXAMPLE IV : SUBCLONING OF THE P32 ANTIGEN IN PLASMID pIGRI :

Fifteen µg of plasmid "BS-BK-P₃₂ complet" (see Example II) was digested with EclXI and BstEII (Boehringer, Mannheim) according to the conditions recommended by the supplier except that at least 3 units of enzyme were used per µg of DNA. EclXI cuts at position 226 (Fig. 5) and BstEII at position 1136, thus approaching very closely the start and stop codon of the mature P₃₂ antigen. This DNA is hereafter called DNA coding for the "P₃₂ antigen fragment".

The DNA coding for the "P₃₂ antigen fragment" (as defined above) is subcloned in pIGRI (see fig. 10a) for expression of a polypeptide devoid of any foreign sequences. To bring the ATG codon of the expression vector in frame with the P₃₂ reading frame, an intermediary construct is made in pIG2 (for restriction and genetic map, see fig. 12a; DNA sequences, see fig. 12b; complete restriction site analysis, see Table 7).

Five µg of plasmid pIG2 is digested with NcoI. Its 5' sticky ends are filled in prior to dephosphorylation.

Therefore, the DNA was incubated in 40 µl NB buffer (0.05 M Tris-Cl pH 7.4; 10 mM MgCl₂; 0.05% β-mercaptoethanol) containing 0.5 mM of all four dXTP (X = A,T,C,G) and 2 µl of Klenow fragment of E. coli DNA polymerase I (5 U/µl, Boehringer, Mannheim) for at least 3 h at 15°C.

After blunting, the DNA was once extracted with one volume of phenol equilibrated against 200 mM Tris-Cl pH 8, twice with at least two volumes of diethylether and finally collected using the "gene clean kit^{T.M.}" (Bio101) as recommended by the supplier. The DNA was then dephosphorylated at the 5' ends in 30 µl of CIP buffer (50 mM TrisCl pH 8, 1 mM ZnCl₂) and 20 to 25 units of calf intestine phosphatase (high concentration, Boehringer, Mannheim). The mixture was incubated at 37°C for 30 min, then EGTA (ethyleneglycol bis (β-aminoethylether)-N,N,N',N' tetraacetic acid) pH 8 is added to a final concentration of 10 mM. The mixture was then extracted with phenol followed by diethylether as described above, and the DNA was precipitated by addition of 1/10 volume of 3 M KAc (Ac = CH₃COO) pH 4.8 and 2 volumes of ethanol followed by storage at -20°C for at least one hour.

After centrifugation at 13000 rpm in a Biofuge A (Hereaus) for 5 min the pelleted DNA was dissolved in H₂O to a final concentration of 0.2 µg/µl.

The EclXI-BstEII fragment, coding for the "P₃₂ antigen fragment" (see above) was electrophoresed on a 1% agarose gel (BRL) to separate it from the rest of the plasmid and was isolated from the gel by centrifugation over a Millipore HVLP filter (⌀ 2cm) (2 min,, 13000 rpm, Biofuge at room temperature) and extracted with Tris equilibrated phenol followed by diethylether as described above.

The DNA was subsequently collected using the "Gene clean kit^{T.M.}" (Bio101) as recommended by the supplier.

After that, the 5' sticky ends were blunted by treatment with the Klenow fragment of E. coli DNA polymerase I as described above and the DNA was then again collected using the "Gene clean kit^{T.M.}" in order to dissolve it in 7 µl of H₂O.

One µl of vector DNA is added together with one µl of 10 x ligase buffer (0.5 M TrisCl pH 7.4, 100 mM MgCl₂, 5 mM ATP, 50 mM DTT (dithiothreitol)) and 1 µl of T4 DNA ligase (1 unit/µl, Boehringer, Mannheim). Ligation was performed for 6 h at 13°C and 5 µl of the mixture is then used to transform strain DH1 (lambda) [strain DH1 - ATCC N° 33849 - lysogenized with wild type bacteriophage λ] using standard transformation techniques as described for instance by Maniatis et al. in "Molecular cloning, a laboratory manual", Cold Spring Harbor Laboratory (1982).

Individual transformants are grown and lysed for plasmid DNA preparation using standard procedures (Experiments with gene fusions, Cold Spring Harbor Laboratory (1984) (T.J. Silhavy, H.L. Berman and L.W. Enquist, eds) and the DNA preparations are checked for the correct orientation of the gene within the plasmid by restriction enzyme analysis.

A check for correct blunting is done by verifying the restoration of the NcoI site at the 5′ and 3′ end of the antigen coding sequence. One of the clones containing the P₃₂ antigen fragment in the correct orientation is kept for further work and designated pIG₂-Mt32. In this intermediary construct, the DNA encoding the antigen is not in frame with the ATG codon. However, it can now be moved as a NcoI fragment to another expression vector.

15 µg of pIG₂-Mt32 is digested with NcoI. The NcoI fragment encoding the P₃₂ antigen is gel purified and blunted as described above. After purification, using "gene clear kit TM" it is dissolved in 7 µl of H₂O.

5 µg of plasmid pIGRI is digested with NcoI, blunted and dephosphorylated as described above. After phenol extraction, followed by diethylether and ethanolprecipitation, the pellet is dissolved in H₂O to a final concentration of 0.2 µg/µl.

Ligation of vector and "antigen fragment" DNA is carried out as described above. The ligation mixture is then transformed into strain DH1 (lambda) and individual transformants are analysed for the correct orientation of the gene within the plasmid by restriction enzyme analysis. A check for correct blunting is done by verifying the creation of a new NsiI site at the 5' and 3' ends of the antigen coding sequence. One of the clones containing the P₃₂ antigen fragment in the correct orientation is kept for further work and designated pIGRI.Mt32.

### EXAMPLE V: SUBCLONING OF THE P32 ANTIGEN IN pmTNF MPH:

Fifteen µg of the plasmid pIG2 Mt32 (see example IV) was digested with the restriction enzyme NcoI (Boehringer, Mannheim), according to the conditions recommended by the supplier except that at least 3 units of enzyme were used per µg of DNA.

After digestion, the reaction mixture is extracted with phenol equilibrated against 200mM TrisCl pH 8, (one volume), twice with diethylether (2 volumes) and precipitated by addition of 1/10 volume of 3 M KAc (Ac=CH₃COO) pH 4.8 and 2 volumes of ethanol followed by storage at -20°C for at least one hour.

After centrifugation for 5 minutes at 13000 rpm in a Biofuge A (Hereaus) the DNA is electrophoresed on a 1% agarose gel (BRL).

The DNA coding for the "P₃₂ antigen fragment" as described above, is isolated by centrifugation over a Millipore HVLP filter (⌀ 2cm) (2 minutes, 1300 rpm, Biofuge at room temperature) and extracted one with trisCl equilibrated phenol and twice with diethylether. The DNA is subsequently collected using "Gene clean kit ^{T.M.}" (Bio 101) and dissolved in 7µl of H₂O.

The 5' overhanging ends of the DNA fragment generated by digestion with NcoI were filled in by incubating the DNA in 40 µl NB buffer (0.05 M Tris-HCl, pH 7.4; 10 mM MgCl₂; 0.05% β-mercapteothanol) containing 0.5 mM of all four dXTPS (X = A, T, C, G) and 2µl of Klenow fragment of E. coli DNA polymerase I (5 units/µl Boehringer Mannheim) for at least 3 h at 15°C. After blunting, the DNA was extracted with phenol, followed by diethylether, and collected using a "gene clean kit ^{T.M.}" as described above.

Five µg of plasmid pmTNF MPH is digested with StuI, subsequently extracted with phenol, followed by diethylether, and precipitated as described above. The restriction digest is verified by electrophoresis of a 0.5 µg sample on an analytical 1,2% agarose gel.

The plasmid DNA is then desphosphorylated at the 5' ends to prevent self-ligation in 30µl of CIP buffer (50 mM TrisCl pH 8, 1mM ZnC12) and 20 to 25 units of calf intestine phosphatase (high concentration, Boehringer Mannheim). The mixture is incubated at 37°C for 30 minutes, then EGTA (ethyleneglycol bis (β-aminoethylether)-N,N,N',N' tetraacetic acid) pH8 is added to a final concentration of 10mM. The mixture is extracted with phenol followed by diethylether and the DNA is precipitated as described above. The precipitate is pelleted by centrifugation in a Biofuge A (Hereaus) at 13000 rpm for 10 min at 4°C and the pellet is dissolved in H₂O to a final DNA concentration of 0.2 µg/µl.

One µl of this vector DNA is mixed with the 7 µl solution containing the DNA fragment coding for the "P32antigen fragment" (as defined above) and 1 µl 10 x ligase buffer (0.5 M TrisCl pH7.4, 100 mM MgC12, 5 mM ATP, 50 mM DTT (dithiothreitol)) plus 1 µl T₄ DNA ligase (1 unit/µl, Boehringer Mannheim) is added. The mixture is incubated at 13°C for 6 hours and 5 µl of the mixture is then used for transformation into strain DH1(lambda, using standard transformation techniques are described by for instance Maniatis et al. in "Molecular cloning, a laboratory manual", Cold Spring Harbor Laboratory (1982).

Individual transformants are grown and then lysed for plasmid DNA preparation using standard procedures (Experiments with gene fusions, Cold Spring Harbor Laboratory 1984 (T.J. Silhavy, M.L. Berman and L.W. Enquist eds.)) and are checked for the correct orientation of the gene within the plasmid by restriction enzyme analysis.

One of the clones containing the DNA sequence encoding the antigen fragment in the correct orientation was retained for further work and designated pmTNF-MPH-Mt32. It encodes all information of the P₃₂ antigen starting from position +4 in the amino acid sequence (see fig. 5). The amino acid sequence of the total fusion protein is represented in fig. 13.

### EXAMPLE VI: iNDUCTION OF ANTIGEN EXPRESSION FROM pmTNF MPH Mt32 :

### A- MATERIAL AND METHODS

DNA of pmTNF-MPH-Mt32 is transformed into E. coli strain K12ΔH (ATCC 33767) using standard transformation procedures except that the growth temperature of the cultures is reduced to 28°C and the heat shock temperature to 34°C.

A culture of K12ΔH harboring pmTNF-MPH-Mt32, grown overnight in Luria broth at 28°C with vigorous shaking in the presence of 10 µg/ml tetracycline, is inoculated into fresh Luria broth containing tetracyclin (10 µg/ml) and grown to an optical density at 600 nanometers of 0.2 in the same conditions as for the overnight culture.

When the optical density at 600 nanometers has reached 0.2 half of the culture is shifted to 42°C to induce expression while the other half remains at 28°C as a control. At several time intervals aliquots are taken which are extracted with one volume of phenol equilibrated against M9 salts (0.1% ammonium chloride, 0.3% potassium dihydrogenium phosphate, 1.5% disodium hydrogenium phosphate, 12 molecules of water) and 1% SDS. At the same time, the optical density (600 nm) of the culture is checked. The proteins are precipitated from the phenol phase by addition of two volumes of acetone and storage overnight at -20°C. The precipitate is pelleted (Biofuge A, 5 min., 13000 rpm, room temperature) dried at the air, dissolved in a volume of Laemmli (Nature (1970) 227:680) sample buffer (+ β mercapto ethanol) according to the optical density and boiled for 3 min.

Samples are then run on a SDS polyacrylamide gel (15%) according to Laemmli (1970). Temperature induction of mTNF-His₆-P₃₂ is monitored by both Coomassie Brilliant Blue (CBB) staining and immunoblotting. CBB staining is performed by immersing the gel in a 1/10 diluted CBB staining solution (0.5 g CBB-R250 (Serva) in 90 ml methanol : H₂O (1:1 v/v) and 10 ml glacial acetic acid) and left for about one hour on a gently rotating platform. After destaining for a few hours in destaining solution (30% methanol, 7% glacial acetic acid) protein bands are visualised and can be scanned with a densitometer (Ultroscan XL Enhanced Laser Densitometer, LKB).

For immunoblotting the proteins are blotted onto Hybond C membranes (Amersham) as described by Townbin et al (1979). After blotting, proteins on the membrane are temporarily visualised with Ponceau S (Serva) and the position of the molecular weight markers is indicated. The stain is then removed by washing in H₂O. Aspecific protein binding sites are blocked by incubating the blots in 10% non-fat dried milk for about 1 hour on a gently rotating platform. After washing twice with NT buffer (25 mM Tris-HCl, pH 8.0; 150 mM NaCl) blots are incubated with polyclonal rabbit anti-32-kDa antiserum (1:1000), obtained as described in example I ("screening of the λgt11 M. tuberculosis recombinant DNA library with anti-32-kDa antiserum") in the presence of E. coli lysate or with monoclonal anti-hTNF-antibody which crossreacts with mTNF (Innogenetics, n° 17F5D10) for at least 2 hours on a rotating platform. After washing twice with NT buffer + 0.02% Triton.X.100, blots are incubated for at least 1 hour with the secondary antiserum : alkaline phosphatase-conjugated swine anti-rabbit immunoglobulins (1/500; Prosan) in the first case, and alkaline phosphatase conjugated rabbit anti-mouse immunoglobulins (1/500; Sigma) in the second case.

Blots are washed again twice with NT buffer + 0.02% Triton X100 and visualisation is then performed with nitro blue tetrazolium (NBT) and 5-bromo-4-chloro-3-indolyl-phosphate (BCIP) from Promega using conditions recommended by the supplier.

### B. RESULTS

Upon induction of K12ΔH cells containing pmTNF-MPH-Mt32, a clearly visible band of about 35-kDa appears on CBB stained gels, already one hour after start of induction (Fig. 14a). This band, corresponding to roughly 25% of total protein contents of the cell, reacts strongly with anti-32-kDa and anti-mTNF antisera on immunoblots (Fig. 14b). However, this band represents a cleavage product of the original fusion protein, since a minor band, around 37 kDa, is also visible on immunoblots, reacting specifically with both antisera as well. This suggests that extensive cleavage of the recombinant mTNF-His₆-P₃₂ takes place about 2-3 kDa from its carboxyterminal end.

### EXAMPLE VII : PURIFICATION OF RECOMBINANT ANTIGEN ON IMMOBILIZED METAL ION AFFINITY CHROMATOGRAPHY (IMAC) :

The hybrid protein mTNF-His₆-P₃₂ (amino acid sequence, see fig. 13) expressed by K12ΔH cells containing pmTNF.MPH.Mt32, is especially designed to facilitate purification by IMAC, since the 6 successive histidines in the polylinker sequence bring about a strong affinity for metal ions (HOCHULI et al, 1988).

### a. Preparation of the crude cell extract :

12 l of E. coli cells K12ΔH containing plasmid pmTNF-MPH-Mt32 were grown in Luria Broth containing tetracycline (10 µg/ml) at 28°C to an optical density (600 nm) of 0.2 and then induced by shifting the temperature to 42°C. After 3 hours of induction, cells were harvested by centrifugation (Beckman, JA 10 rotor, 7.500 rpm, 10 min). The cell paste was resuspended in lysis buffer (10 mM KCl, 10 mM Tris-HCl pH 6.8, 5 mM EDTA) to a final concentration of 50% (w/v) cells.

ε-NH₂-capronic acid and dithiotreitol (DTT) were added to a final concentration of resp. 20 mM and 1 mM, to prevent proteolytic degradation. This concentrated cell suspension was stored overnight at -70°C.

Cells were lysed by passing them three times through a French press (SLM-Aminco) at a working pressure of 800-1000 psi. During and after lysis, cells were kept systematically on ice.

The cell lysate was cleared by centrifugation (Beckman, JA 20, 18.000 rpm, 20 min, 4°C). The supernatant (SN) was carefully taken off and the pellet, containing membranes and inclusion bodies, was kept for further work since preliminary experiments had shown that the protein was mainly localised in the membrane fraction.

7 M guanidinium hydrochloride (GuHCl, marketed by ICN) in 100 mM phosphate buffer pH 7.2 was added to the pellet volume to a final concentration of 6 M GuHCl. The pellet was resuspended and extracted in a bounce tissue homogenizer (10 cycles).

After clearing (Beckman, JA 20, 18.000 rpm, 20 min, 4°C), about 100 ml of supernatant was collected (= extract 1) and the removing pellet was extracted again as described above (= extract 2, 40 ml).

The different fractions (SN,EX1,EX2) were analysed on SDS-PAGE (Laemmli, Nature 1970; 227:680) together with control samples of the induced culture. Scanning of the gel revealed that the recombinant protein makes up roughly 25% of the total protein content of the induced cell culture. After fractionation most of the protein was found back in the extracts. No difference was noticed between reducing and non-reducing conditions (plus and minus β-mercaptoethanol).

### b. Preparation of the Ni⁺⁺ IDA (Imino diacetic acid) column :

5 ml of the chelating gel, Chelating Sepharose 6B (Pharmacia) is washed extensively with water to remove the ethanol in which it is stored and then packed in a "Econo-column" (1 x 10 cm, Biorad). The top of the column is connected with the incoming fluid (sample, buffer, etc) while the end goes to the UV₂₈₀ detector via a peristaltic jump. Fractions are collected using a fraction collector and, when appropriate, pH of the fractions is measured manually.

The column is loaded with Ni⁺⁺ (6 ml NiCl₂.6H₂O; 5 µg/µl) and equilibrated with starting buffer (6 M guanidinium hydrochloride, 100 mM phosphate buffer, pH 7.2).

After having applied the sample, the column is washed extensively with starting buffer to remove unbound material.

To elute the bound material, 2 different elution procedures are feasible :
1) elution by decreasing pH,
2) elution by increasing imidazol concentration.

Both will be discussed here.

To regenerate the column, which has to be done after every 2-3 runs, 20 ml (about 5 column volumes) of the following solutions are pumped successively through the column :
- 0.05 M EDTA - 0.5 M NaCl
- 0.1 M NaOH
- H₂O
- 6 ml NiCl₂.6H₂O (5 mg/ml).

After equilibrating with starting buffer the column is ready to use again.

### c. Chromatography :

All buffers contained 6 M guanidinium hydrochloride throughout the chromatography. The column was developed at a flow rate of 0.5 ml/min at ambient temperature. Fractions of 2 ml were collected and, when appropriate, further analysed by SDS-PAGE and immunoblotting. Gels were stained with Coomassie Brilliant Blue R250 and silver stain, as described by ANSORGE (1985). Immunoblotting was carried out as described in example I. The primary antiserum used was either polyclonal anti-32kDa-antiserum (1/1000) obtained as described in example I ("screening of the λgt11 M. tuberculosis recombinant DNA library with anti-32kDa-antiserum") or anti-E. coli-immunoglobulins (1/500; PROSAN), or monoclonal anti-hTNF-antibody which cross-reacts with mTNF (Innogenetics, N° 17F5D10). The secondary antiserum was alkaline phosphatase conjugated swine anti-rabbit immunoglobulins (1/500, PROSAN), or alkaline phosphatase conjugated rabbit-anti-mouse immunoglobulins (1/500, Sigma).

### C1. Elution with decreasing pH :

Solutions used :
A : 6 M GuHCl 100 mM phosphate pH 7.2
B : 6 M GuHCl 25 mM phosphate pH 7.2
C : 6 M GuHCl 50 mM phosphate pH 4.2

After applying 3 ml of extract 1 (OD₂₈₀ = 32.0) and extensively washing with solution A, the column is equilibrated with solution B and then developed with a linear pH gradient from 7.2 to 4.2 (25 ml of solution B and 25 ml of solution C were mixed in a gradient former). The elution profile is shown in figure 15.

From SDS-PAGE analysis (Coomassie and silverstain) it was clear that most of the originally bound recombinant protein was eluted in the fractions between pH 5.3 and 4.7.

Screening of these fractions on immunoblot with anti-32-kDa and the 17F5D10 monoclonal antibody showed that, together with the intact recombinant protein, also some degradation products and higher aggregation forms of the protein were present, although in much lower amount. Blotting with anti-E. coli antibody revealed that these fractions (pH 5.3-4.7) still contained immunodetectable contaminating E. coli proteins (75, 65, 43, 35 and 31 kDa bands) and lipopolysaccharides..

### C2. Elution vith increasing imidazol concentration :

Solutions used :
A : 6 M GuHCl 100 mM phosphate pH 7.2
B : 6 M GuHCl 50 mM imidazol pH 7.2
C : 6 M GuHCl 100 mM imidazol pH 7.2
D : 6 M GuHCl 15 mM imidazol pH 7.2
E : 6 M GuHCl 25 mM imidazol pH 7.2
F : 6 M GuHCl 35 mM imidazol pH 7.2

Sample application and washing was carried out as in Cl, except that after washing, no equilibration was necessary with 6 M GuHCl 25 mM phosphate. The column was first developed with a linear gradient of imidazol going from 0 to 50 mM (25 ml of solution A and 25 ml of solution B were mixed in a gradient former) followed by a step elution to 100 mM imidazol (solution C). During the linear gradient, proteins were gradually eluted in a broad smear, while the step to 100 mM gave rise to a clear peak (fig. 16).

SDS-PAGE analysis of the fractions revealed that in the first part of the linear gradient (fr 1-24) most contaminating E. coli proteins were washed out, while the latter part of the gradient (fr 25-50) and the 100 mM peak contained more than 90% of the recombinant protein.

As in Cl, these fractions showed, besides a major band of intact recombinant protein, some minor bands of degradation and aggregation products. However, in this case, the region below 24-kDa seemed nearly devoid of protein bands, which suggests that less degradation products co-elute with the intact protein. Also, the same contaminating E. coli proteins were detected by immunoblotting, as in C1, although the 31-kDa band seems less intense and even absent in some fractions.

In a second stage, we developed the column with a step gradient of increasing imidazol concentrations. After having applied the sample and washed the column, 2 column volumes (about 8 ml) of the following solutions were brought successively onto the column : solution D, E, F and finally 4 column volumes of solution C. The stepgradient resulted in a more concentrated elution profile (fig. 17) which makes it more suitable for scaling up purposes.

In conclusion, the mTNF-His₆-P₃₂ protein has been purified to at least 90% by IMAC. Further purification can be achieved through a combination of the following purification steps :
- IMAC on chelating superose (Pharmacia)
- ion exchange chromatography (anion or cation)
- reversed phase chromatography
- gel filtration chromatography
- immunoaffinity chromatography
- elution from polyacrylamide gel.

These chromatographic methods are commonly used for protein purification.

The plasmids of figures 10b, 11b and 12b are new.

### BIBLIOGRAPHY

**1.** Abou-Zeid, C., T.L. Ratliff, H.G. Wiker, M. Harboe, J. Bennedsen and G.A.W. Rook, 1988. Characterization of fibronectin-biding antigens released by Mycobacterium tuberculosis and Mycobacterium bovis BCG. Infect. Imm. 56, 3046-3051.
**2.** Bellon, B. 1988. Apple Macintosh programs for nucleic and protein sequence analysis. Nucleic Acid Res. 16:1837-1846.
**3.** Bibb, M.J., P.R. Findlay and M.W. Jonhson. 1984. The relationship between base composition and codon usage in bacterial genes and its use for the simple and reliable identification of protein-coding sequences. Gene. 30: 157-166.
**4.** Bresson, G.M. and K.K. Stanley. 1987. pUEX, a bacterial expression vector related to pEX with universal host specificity. Nucl. Aci. Res. 15:10056.
**5.** Chang, S. Engineering for protein secretion in Gram positive bacteria. Methods Enzymol., 153:507-516.
**6.** Chen, E.J. and P.H. Seeburg. 1985. Supercoil sequencing: a fast simple method for sequencing plasmid DNA.DNA 4:165-170.
**7.** Closs, O., M. Harboe, N.H. Axelsen-Christensen and M. Magnussen. 1980. The antigens of Mycobacterium bovis, strain BCG, studied by cross-immunoelectrophoresis: a reference system. Scand. J. Immunol. S12N:249-263.
**8.** De Bruyn, J.R. Bosmans, J. Nyabenda and J.P. Van Vooren. 1989. Effect of zinc deficiency of the appearance of two immunodominant protein antigens (32-kDa and 65-kDa) in culture filtrates of Mycobacteria. J. Gen. Micriob. 135: 79-84.
**9.** De Bruyn, J., K. Huygen, R. Bosmans, M. Fauville, R. Lippens, J.P. Van Vooren, P. Falmagne, M. Weckx, H.G. Wiker, M. Harboe and M. Turneer. 1987. Purification, partial characterization and identification of a 32-kDa protein antigen of Mycobacterium bovis BCG. Microb. Pathogen. 2:351-366.
**10.** Felnberg, A.P. and R. Vogelstein. 1983. A technique for radiolabelling DNA restriction endonuclease fragments to high specific activity. Anal. Biochem. 132:6-13.
**11.** Hawley, D.K. and W.R. Mc Clure. 1983. Compilation and analysis of E. coli promoter DNA sequences. Nucleic Acids Res. 11:2237-2255.
**12.** Huygen, K., J.P. Van Vooren, M. Turneer, R. Bosmans, P. Dierckx and J. De Bruyn. 1988. Specific lymphoproliferation -interferon production and serum immunoglobulin G directed against a purified 32-kDa Mycobacterial antigen (P32) in patient with active tuberculosis. Scand. J. Immunol. 27:187-194.
**13.** Huygen, K., K. Palfliet, F. Jurton, J. Hilgers, R. ten Berg, J.P. Van Vooren and J. De Bruyn. 1989. H-2-linked control of in vitro interferon production in response to 32-kilodalton (P32) of Mycobacterium bovis bacillus Calmette-Guérin. Infect. Imm. 56:3196-3200.
**14.** Huynh, T.V., R.A. Young and R.W. Davis. 1985. Constructing and screening libraries in gt10 and gt11 p.49-78. in: DNA cloning. Vol.I, A practical approach. Ed. D.M. Glover. IRL Press, Oxford-Washington, D.C.
**15.** Kyte, J. and R.F. Doolittle. 1982. Simple method for displaying the hydropathy character of a protein. J. Mol. Biol. 157:105-132.
**16.** Maniatis, T., E.F. Fritsch and J. Sambrook. 1982. Molecular cloning: a laboratory manual. Cold Spring Harbor Laboratory, Cold Spring Harbor, N.Y.
**17.** Matsuo, K., R. Yamaguchi, A. Yamazaki, H. Tasaka and T. Yamada. 1988. Cloning and expression of the Mycobacterium bovis BCG gene for extracellular α-antigen. J. Bacteriol. 170:3847-3854.
**18.** Mawam, A.M. and W. Gilbert. 1977. A new method for sequencing DNA. Proc. Natl. Acad. Sci. USA. 74:560-564.
**19.** Mehra, V., D. sweetser and R.A. Young. 1986. Efficient mapping of protein antigenic determinants. Proc. Natl. Acad. Sci. USA. 83:7013-7017.
**20.** Mustafa, A.B., H.K. Gill, A. Nerland, W.J. Britton, V. Mehra, B.R. Bloom, R.A. Young and T. Godal. 1986. Human T-cell clones recognize a major M.Leprae protein antigen expressed in E. coli. Nature (London). 319:63-38.
**21.** Neesen, K. and G. Volckaert. 1989. Construction and shuttling of novel bifunctional vectors for Streptomyces spp. and Escherichia coli. J. Bacteriol. 171:1569-1573.
**22.** Oliver, D. 1985. Protein secretion in Escherichia coli. Ann. Rev. Microbiol. 39:615-648.
**23.** Pearson, W.R. and D.J. Lipman. 1988. Improved tools for biological sequence comparison. Proc. Natl. Acad. Sci. USA. 85:2444-2448.
**24.** Rumschlag, H.S., T.S. Shinnick and M.L. Cohen. 1988. Serological response of patients with lepromatous and tuberculous leprosy to 30-, 31- and 32-kilodalton antigens of Mycobacterium tuberculosis. J. Clin. Microbiol. 26:2200-2202.
**25.** Sanger, F., S. Niklon and A.R. Coulson. 1977. DNA sequencing with chain termination inhibitors. Proc. Natl. Acad. Sci. USA. 74:5463-5487.
**26.** Shinnick, T.M. 1987. The 65-kilodalton antigen of Mycobacterium tuberculosis. J. Bacteriol. 169:1080-1088.
**27.** Thole, J.E.R., W.C.A. Van Shooten, W.J. Keulen, P.W.M. Hermans, A.A., M. Janson, R.A.P. De Vries, A.H.J. Kolk and J.D.A. Van Embden. 1988. Use of recombinant antigens expressed in Escherichia coli K-12 to map B-cell and T-cell epitopes on the immunodominant 65-kilodalton protein of Mycobacterium bovis BCG. Infect. Immun 56:1633-1640.
**28.** Thole. J.E.R., W.J. Keulen, J. De Bruyn, A.H.J. Kolk, D.G. Groothuis, L.G. Berwald, R.H. Tiesjema and J.D.A. Van Embden. 1987. Characterization, sequence determination and immunogenicity of a 64-kilodalton protein of Mycobacterium bovis BCG expressed in Escherichia coli K-12. Infect. Imm. 55:1466-1475.
**29.** Towbin, H., T. Staehelin and J. Gordon. 1979. Electrophoretic transfer of proteins from polyacrylamide gels to nitrocellulose sheets: procedure and some applications. Proc. Natl. Acad. Sci. USA 76:4350-4354.
**30.** Turneer, M., J.P. Van Vooren, J. De Bruyn, E. Serruys, P. Dierckx and J.C. Yernault. 1988. Humoral immune response in human tuberculosis: immunoglobulins G, A and M directed against the purified P32 protein antigen of Mycobacterium bovis bacillus Calmette-Guérin. J. Clin. Microbiol. 26:1714-1719.
**31.** Van Vooren, J.P., C.M. Farber, E. Noël, N. Mavroudakis, M. Turneer, J. De Bruyn, F. Legros and J.C. Yernault. 1989 Local anti-P32 humoral response in tuberculous meningitis. Tubercle. 70:123-126.
**32.** Volckaert, G. 1987. A systematic approach to chemical sequencing by subcloning in pGV451 and derived vectors. Methods Enzymol. 155:231-250.
**33.** Volckaert, G., El. De Vieeschouwer, R. Frank and H. Bloecker. 1984. A novel type of cloning vectors for ultrarapid chemical degradation sequencing of DNA. Gene Anal. Techn. 1:52-59.
**34.** Wiker, H.G., M. Harboe, S. Nagal, M.E. Patarroyo, C. Ramirez and N. Cruz. 1986. MPB59, a widely cross-reacting protein of Mycobacterium bovis BCG. Int. Arch. Alllergy Appl. Immunol. 81:307-314.
**35.** Young, R.A., B.R. Bloom, C.M. Grosskinsky, J. Ivanji, D. Thomas and R.W. Davis. 1985. Dissection of Mycobacterium tuberculosis antigens using recombinant DNA. Proc. Natl. Acad; Sci. USA, 82:2583-2587.
**36.** HOCHULI, E., BANNWARTH, W., DÖBELI, H., GENTZ, R. and STÜBER, D. (1988). Genetic Approach to facilitate purification of recombinant proteins vith a novel metal chelate adsorbent. Biotechnology, nov. 1988, p. 1321-1325.
**37.** ANSORGE, W. (1985), Fast and sensitive detection of protein and DNA bands by treatment with potassium permanganate. J. Biochem. Biophys. Meth., 11:13-20.

## Claims (Claims for the following Contracting State(s): AT, BE, CH, LI, DE, DK, FR, GB, IT, LU, NL, SE)

1. Biologically pure recombinant polypeptide containing in its polypeptide chain one at least of the following amino acid sequences :
- the one extending from the extremity constituted by amino acid at position (-29) to the extremity constituted by amino acid at position (-1) represented on fig. 3a and fig. 3b, or
- the one extending from the extremity constituted by amino acid at position (12) to the extremity constituted by amino acid at position (31) represented on fig. 3a and fig. 3b, or
- the one extending from the extremity constituted by amino acid at position (36) to the extremity constituted by amino acid at position (55) represented on fig. 3a and fig. 3b, or
- the one extending from the extremity constituted by amino acid at position (77) to the extremity constituted by amino acid at position (96) represented on fig. 3a and fig. 3b, or
- the one extending from the extremity constituted by amino acid at position (101) to the extremity constituted by amino acid at position (120) represented on fig. 3a and fig. 3b, or
- the one extending from the extremity constituted by amino acid at position (175) to the extremity constituted by amino acid at position (194) represented on fig. 3a and fig. 3b, or
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b, or
- the one extending from the extremity constituted by amino acid at position (1) to the extremity constituted by amino acid at position (294) represented on fig. 3a and fig. 3b, or
- the one extending from the extremity constituted by amino acid at position (-42) to the extremity constituted by amino acid at position (-1) represented on fig. 3a and fig. 3b, or
- the one extending from the extremity constituted by amino acid at position (-47) to the extremity constituted by amino acid at position (-1) represented on fig. 3a and fig. 3b, or
- the one extending from the extremity constituted by amino acid at position (-49) to the extremity constituted by amino acid at position (-1) represented on fig. 3a and fig. 3b, or
- the one extending from the extremity constituted by amino acid at position (-55) to the extremity constituted by amino acid at position (-1) represented on fig. 3a and fig. 3b, or
- the one extending from the extremity constituted by amino acid at position (-59) to the extremity constituted by amino acid at position (-1) represented on fig. 3a and fig. 3b, or
- the one extending from the extremity constituted by amino acid at position (-29) to the extremity constituted by amino acid at position (294) represented on fig. 3a and fig. 3b, or
- the one extending from the extremity constituted by amino acid at position (-42) to the extremity constituted by amino acid at position (294) represented on fig. 3a and fig. 3b, or
- the one extending from the extremity constituted by amino acid at position (-47) to the extremity constituted by amino acid at position (294) represented on fig. 3a and fig. 3b, or
- the one extending from the extremity constituted by amino acid at position (-49) to the extremity constituted by amino acid at position (294) represented on fig. 3a and fig. 3b, or
- the one extending from the extremity constituted by amino acid at position (-55) to the extremity constituted by amino acid at position (294) represented on fig. 3a and fig. 3b, or
- the one extending from the extremity constituted by amino acid at position (-59) to the extremity constituted by amino acid at position (294) represented on fig. 3a and fig. 3b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

2. Biologically pure recombinant polypeptide according to claim 1 containing in its polypeptide chain one at least of the following amino acid sequences :
- the one extending from the extremity constituted by amino acid at position (-29) to the extremity constituted by amino acid at position (-1) represented on fig. 4a and fig. 4b, or
- the one extending from the extremity constituted by amino acid at position (12) to the extremity constituted by amino acid at position (31) represented on fig. 4a and fig. 4b, or
- the one extending from the extremity constituted by amino acid at position (36) to the extremity constituted by amino acid at position (55) represented on fig. 4a and fig. 4b, or
- the one extending from the extremity constituted by amino acid at position (77) to the extremity constituted by amino acid at position (96) represented on fig. 4a and fig. 4b, or
- the one extending from the extremity constituted by amino acid at position (101) to the extremity constituted by amino acid at position (120) represented on fig. 4a and fig. 4b, or
- the one extending from the extremity constituted by amino acid at position (175) to the extremity constituted by amino acid at position (194) represented on fig. 4a and fig. 4b, or
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b, or
- the one extending from the extremity constituted by amino acid at position (1) to the extremity constituted by amino acid at position (294) represented on fig. 4a and fig. 4b, or
- the one extending from the extremity constituted by amino acid at position (-42) to the extremity constituted by amino acid at position (-1) represented on fig. 4a and fig. 4b, or
- the one extending from the extremity constituted by amino acid at position (-47) to the extremity constituted by amino acid at position (-1) represented on fig. 4a and fig. 4b, or
- the one extending from the extremity constituted by amino acid at position (-49) to the extremity constituted by amino acid at position (-1) represented on fig. 4a and fig. 4b, or
- the one extending from the extremity constituted by amino acid at position (-55) to the extremity constituted by amino acid at position (-1) represented on fig. 4a and fig. 4b, or
- the one extending from the extremity constituted by amino acid at position (-59) to the extremity constituted by amino acid at position (-1) represented on fig. 4a and fig. 4b, or
- the one extending from the extremity constituted by amino acid at position (-29) to the extremity constituted by amino acid at position (294) represented on fig. 4a and fig. 4b, or
- the one extending from the extremity constituted by amino acid at position (-42) to the extremity constituted by amino acid at position (294) represented on fig. 4a and fig. 4b, or
- the one extending from the extremity' constituted by amino acid at position (-47) to the extremity constituted by amino acid at position (294) represented on fig. 4a and fig. 4b, or
- the one extending from the extremity constituted by amino acid at position (-49) to the extremity constituted by amino acid at position (294) represented on fig. 4a and fig. 4b, or
- the one extending from the extremity constituted by amino acid at position (-55) to the extremity constituted by amino acid at position (294) represented on fig. 4a and fig. 4b, or
- the one extending from the extremity constituted by amino acid at position (-59) to the extremity' constituted by amino acid at position (294) represented on fig. 4a and fig. 4b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

3. Biologically pure recombinant polypeptide according to claim 1 containing in its polypeptide chain one at least of the following amino acid sequences :
- the one extending from the extremity constituted by amino acid at position (-30) to the extremity constituted by amino acid at position (-1) represented on fig. 5, or
- the one extending from the extremity constituted by amino acid at position (12) to the extremity constituted by amino acid at position (31) represented on fig. 5, or
- the one extending from the extremity constituted by amino acid at position (36) to the extremity constituted by amino acid at position (55) represented on fig. 5, or
- the one extending from the extremity constituted by amino acid at position (77) to the extremity constituted by amino acid at position (96) represented on fig. 5, or
- the one extending from the extremity constituted by amino acid at position (101) to the extremity constituted by amino acid at position (120) represented on fig. 5, or
- the one extending from the extremity constituted by amino acid at position (175) to the extremity constituted by amino acid at position (194) represented on fig. 5, or
- the one extending from the extremity' constituted by amino acid at position (211) to the extremity constituted by amino acid at position (230) represented on fig. 5, or
- the one extending from the extremity constituted by amino acid at position (275) to the extremity constituted by amino acid at position (295) represented on fig. 5, or
- the one extending from the extremity constituted by amino acid at position (1) to the extremity constituted by amino acid at position (295) represented on fig. 5, or
- the one extending from the extremity constituted by amino acid at position (-43) to the extremity constituted by amino acid at position (-1) represented on fig. 5, or
- the one extending from the extremity' constituted by amino acid at position (-30) to the extremity constituted by amino acid at position (295) represented on fig. 5, or
- the one extending from the extremity constituted by amino acid at position (-43) to the extremity constituted by amino acid at position (295) represented on fig. 5, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

4. Recombinant polypeptide according to claim 1, consisting of one of the following amino acid sequences :
- the one extending from the extremity constituted by amino acid at position (-59) to the extremity constituted by amino acid at position (294) represented on fig. 3a and fig. 3b,
- the one extending from the extremity constituted by amino acid at position (-59) to the extremity constituted by amino acid at position (-1) represented on fig. 3a and fig. 3b,
- the one extending from the extremity constituted by amino acid at position (-55) to the extremity constituted by amino acid at position (294) represented on fig. 3a and fig. 3b,
- the one extending from the extremity constituted by amino acid at position (-55) to the extremity constituted by amino acid at position (-1) represented on fig. 3a and fig. 3b,
- the one extending from the extremity constituted by amino acid at position (-49) to the extremity constituted by amino acid at position (294) represented on fig. 3a and fig. 3b,
- the one extending from the extremity constituted by amino acid at position (-49) to the extremity constituted by amino acid at position (-1) represented on fig. 3a and fig. 3b,
- the one extending from the extremity constituted by amino acid at position (-47) to the extremity constituted by amino acid at position (294) represented on fig. 3a and fig. 3b
- the one extending from the extremity constituted by amino acid at position (-47) to the extremity constituted by amino acid at position (-1) represented on fig. 3a and fig. 3b,
- the one extending from the extremity constituted by amino acid at position (-42) to the extremity constituted by amino acid at position (294) represented on fig. 3a and fig. 3b,
- the one extending from the extremity constituted by amino acid at position (-42) to the extremity constituted by amino acid at position (-1) represented on fig. 3a and fig. 3b,
- the one extending from the extremity constituted by amino acid at position (-29) to the extremity constituted by amino acid at position (294) represented on fig. 3a and fig. 3b,
- the one extending from the extremity constituted by amino acid at position (-29) to the extremity constituted by amino acid at position (-1) represented on fig. 3a and fig. 3b,
- the one extending from the extremity constituted by amino acid at position (1) to the extremity constituted by amino acid at position (294) represented on fig. 3a and fig. 3b,
- the one extending from the extremity constituted by amino acid at position (12) to the extremity constituted by amino acid at position (31) represented on fig. 3a and fig. 3b,
- the one extending from the extremity constituted by amino acid at position (36) to the extremity constituted by amino acid at position (55) represented on fig. 3a and fig. 3b,
- the one extending from the extremity constituted by amino acid at position (77) to the extremity constituted by amino acid at position (96) represented on fig. 3a and fig. 3b,
- the one extending from the extremity constituted by amino acid at position (101) to the extremity constituted by amino acid at position (120) represented on fig. 3a and fig. 3b,
- the one extending from the extremity constituted by amino acid at position (175) to the extremity constituted by amino acid at position (194) represented on fig. 3a and fig. 3b,
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b.

5. Recombinant polypeptide according to claim 2, consisting of one of the following amino acid sequences :
- the one extending from the extremity constituted by amino acid at position (-59) to the extremity constituted by amino acid at position (294) represented on fig. 4a and fig. 4b,
- the one extending from the extremity constituted by amino acid at position (-59) to the extremity constituted by amino acid at position (-1) represented on fig. 4a and fig. 4b,
- the one extending from the extremity constituted by amino acid at position (-55) to the extremity constituted by amino acid at position (294) represented on fig. 4a and fig. 4b,
- the one extending from the extremity constituted by amino acid at position (-55) to the extremity constituted by amino acid at position (-1) represented on fig. 4a and fig. 4b,
- the one extending from the extremity constituted by amino acid at position (-49) to the extremity constituted by amino acid at position (294) represented on fig. 4a and fig. 4b,
- the one extending from the extremity constituted by amino acid at position (-49) to the extremity constituted by amino acid at position (-1) represented on fig. 4a and fig. 4b,
- the one extending from the extremity constituted by amino acid at position (-47) to the extremity constituted by amino acid at position (294) represented on fig. 4a and fig. 4b,
- the one extending from the extremity constituted by amino acid at position (-47) to the extremity constituted by amino acid at position (-1) represented on fig. 4a and fig. 4b,
- the one extending from the extremity constituted by amino acid at position (-42) to the extremity constituted by amino acid at position (294) represented on fig. 4a and fig. 4b,
- the one extending from the extremity constituted by amino acid at position (-42) to the extremity constituted by amino acid at position (-1) represented on fig. 4a and fig. 4b,
- the one extending from the extremity constituted by amino acid at position (-29) to the extremity constituted by amino acid at position (294) represented on fig. 4a and fig. 4b,
- the one extending from the extremity constituted by amino acid at position (-29) to the extremity constituted by amino acid at position (-1) represented on fig. 4a and fig. 4b,
- the one extending from the extremity constituted by amino acid at position (1) to the extremity constituted by amino acid at position (294) represented on fig. 4a and fig. 4b,
- the one extending from the extremity constituted by amino acid at position (12) to the extremity constituted by amino acid at position (31) represented on fig. 4a and fig. 4b,
- the one extending from the extremity constituted by amino acid at position (36) to the extremity constituted by amino acid at position (55) represented on fig. 4a and fig. 4b,
- the one extending from the extremity constituted by amino acid at position (77) to the extremity constituted by amino acid at position (96) represented on fig. 4a and fig. 4b,
- the one extending from the extremity constituted by amino acid at position (101) to the extremity constituted by amino acid at position (120) represented on fig. 4a and fig. 4b,
- the one extending from the extremity constituted by amino acid at position (175) to the extremity constituted by amino acid at position (194) represented on fig. 4a and fig. 4b,
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b.

6. Recombinant polypeptide according to claim 3, consisting of one of the following amino acid sequences :
- the one extending from the extremity constituted by amino acid at position (-43) to the extremity constituted by amino acid at position (295) represented on fig. 5,
- the one extending from the extremity constituted by amino acid at position (-43) to the extremity constituted by amino acid at position (-1) represented on fig. 5,
- the one extending from the extremity constituted by amino acid at position (-30) to the extremity constituted by amino acid at position (295) represented on fig. 5,
- the one extending from the extremity' constituted by amino acid at position (-30) to the extremity constituted by amino acid at position (-1) represented on fig. 5,
- the one extending from the extremity constituted by amino acid at position (1) to the extremity constituted by amino acid at position (295) represented on fig. 5,
- the one extending from the extremity constituted by amino acid at position (12) to the extremity constituted by amino acid at position (31) represented on fig. 5,
- the one extending from the extremity constituted by amino acid at position (36) to the extremity constituted by amino acid at position (55) represented on fig. 5,
- the one extending from the extremity constituted by amino acid at position (77) to the extremity constituted by amino acid at position (96) represented on fig. 5,
- the one extending from the extremity constituted by amino acid at position (101) to the extremity constituted by amino acid at position (120) represented on fig. 5,
- the one extending from the extremity constituted by amino acid at position (175) to the extremity constituted by amino acid at position (194) represented on fig. 5,
- the one extending from the extremity constituted by amino acid at position (211) to the extremity constituted by amino acid at position (230) represented on fig. 5, or
- the one extending from the extremity constituted by amino acid at position (275) to the extremity constituted by amino acid at position (295) represented on fig. 5.

7. Biologically pure recombinant polypeptide constituted by a polypeptide according to any of claims 1 to 6, and a protein having a heterologous sequence with respect to said polypeptide, said heterologous sequence comprising from about 1 to about 1000 amino acids.

8. Recombinant polypeptide according to claim 7, wherein the heterologous protein is β-galactosidase.

9. Nucleic acid comprising
(a) a nucleotide sequence coding for a polypeptide according to any of claims 1 to 6, or
(b) a nucleotide sequence which is complementary to (a), or
(c) the nucleotide sequences of (a) or (b) wherein T can be replaced by U.

10. Nucleic acid comprising one at least of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (1242) to the extremity constituted by nucleotide at position (1358), wherein N represents one of the five A, T, C, G or I nucleotides, represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1358) represented in fig. 3a and fig. 3b, or the complementary sequences thereof,
or the above said nucleotide sequences wherein T is replaced by U.

11. Nucleic acid according to claim 10 comprising one at least of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (1242) to the extremity constituted by nucleotide at position (1358), wherein N represents one of the five A, T, C, G or I nucleotides, represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (183) to the extremity constituted by nucleotide at position (1358) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1358) represented in fig. 4a and fig. 4b, or the complementary sequences thereof,
or the above said nucleotide sequences wherein T is replaced by U.

12. Nucleic acid according to claim 10 comprising one at least of the following nucleotide sequences :
- the one extending from the extremity constituted by nucleotide at position (130) to the extremity constituted by nucleotide at position (219) represented in fig. 5,
- the one extending from the extremity constituted by nucleotide at position (220) to the extremity constituted by nucleotide at position (1104) represented in fig. 5,
- the one extending from the extremity constituted by nucleotide at position (1105) to the extremity constituted by nucleotide at position (1299),
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1299) represented in fig. 5,
- the one extending from the extremity constituted by nucleotide at position (90) to the extremity constituted by nucleotide at position (1104) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity constituted by nucleotide at position (1299) represented in fig. 5,
- the one extending from the extremity constituted by nucleotide at position (220) to the extremity constituted by nucleotide at position (1299) represented in fig. 5, or the complementary sequences thereof,
or the above said nucleotide sequences wherein T is replaced by U.

13. Nucleic acid according to claim 10 consisting of one of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (360) to the extremity constituted by nucleotide at position (1358) represented in fig. 3a and fig. 3b,
- the one extending from the extremity constituted by nucleotide at position (1242) to the extremity constituted by nucleotide at position (1358) represented in fig. 3a and fig. 3b, or the complementary sequences thereof,
or the above said nucleotide sequences wherein T is replaced by U.

14. Nucleic acid according to claim 11 consisting of one of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity constituted by nucleotide at position (183) to the extremity constituted by nucleotide at position(1358) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (360) to the extremity constituted by nucleotide at position (1358) represented in fig. 4a and fig. 4b,
- the one extending from the extremity constituted by nucleotide at position (1242) to the extremity constituted by nucleotide at position (1358) represented in fig. 4a and fig. 4b, or the complementary sequences thereof,
or the above said nucleotide sequences wherein T is replaced by U.

15. Nucleic acid according to claim 12 consisting of one of the following nucleotide sequences :
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1104) represented in fig. 5,
- the one extending from the extremity constituted by nucleotide at position (90) to the extremity constituted by nucleotide at position (1299) represented in fig. 5,
- the one extending from the extremity constituted by nucleotide at position (130) to the extremity constituted by nucleotide at position (219) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity constituted by nucleotide at position (1299) represented in fig. 5,
- the one extending from the extremity constituted by nucleotide at position (220) to the extremity constituted by nucleotide at position (1104) represented in fig. 5,
- the one extending from the extremity constituted by nucleotide at position (220) to the extremity constituted by nucleotide at position (1299) represented in fig. 5,
- the one extending from the extremity constituted by nucleotide at position (1105) to the extremity constituted by nucleotide at position (1299) represented in fig. 5, or the complementary sequences thereof,
or the above said nucleotide sequences wherein T is replaced by U.

16. Recombinant nucleic acid containing at least one of the nucleotide sequences according to claims 9 to 15, inserted in a heterologous nucleic acid.

17. A DNA or RNA primer constituted by 15 to 25 nucleotides of a nucleotide sequence according to any one of claims 13 to 15, which can be used as a primer to specifically amplify any of said nucleotide sequences.

18. A DNA or RNA primer according to claim 17, with said primer being chosen from the following group of nucleotide sequences:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
or the complementary sequences thereof or the corresponding ribonucleotide sequences.

19. A DNA or RNA primer set for the enzymatic amplification of a nucleotide sequence according to any one of claims 13 to 15, with said primer set comprising
- a A nucleotide sequence combined with the complementary nucleotide sequence of either B, or C, or D, or E, or F; or
- the B nucleotide sequence combined with the complementary nucleotide sequence of C, or D, or E, or F; or
- the C nucleotide sequence combined with the complementary nucleotide sequence of D, or E, or F; or
- the D nucleotide sequence combined with the complementary nucleotide sequence of E, or F; or
- the E nucleotide sequence combined with a complementary nucleotide sequence of F, with the nucleotide sequences A, B, C, D, E and F being specified in claim 18, and with A meaning any of the sequences A(i), A(ii), A(iii), A(iv), and A(v) and F meaning any of the sequences F(i), F(ii), F(iii), and F(iv).

20. Recombinant vector, particularly for cloning and/or expression, comprising a vector sequence of the type plasmid, cosmid or phage, and a nucleic acid according to anyone of claims 9 to 15, in one of the non essential sites for its replication.

21. Recombinant vector according to claim 20, containing in one of the non essential sites for its replication the necessary elements to promote the expression of a polypeptide according to anyone of claims 1 to 6 in a cellular host.

22. Recombinant vector according to claim 21, containing the elements enabling the expression by E. coli of a polypeptide according to anyone of claims 1 to 6.

23. Cellular host comaining a recombinant vector according to anyone of claims 20 to 22, and comprising the regulation elements enabling the expression of a polypeptide according to anyone of claims 1 to 6 in this host.

24. Cellular host according to claim 23, chosen from among bacteria such as E. coli, transformed by a vector according to claim 20, or chosen from among eukaryotic organisms, transformed by a vector according to claim 20.

25. Monoclonal antibody specifically directed against a recombinant polypeptide according to anyone of claims 1 to 6.

26. A nucleotide probe hybridizing with a nucleic acid according to any of claims 13 to 15, chosen from the following group of nucleotide sequences:
| Probes A(i), A(ii), A(iii), A(iv) and A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Probe B | |
|---|---|
| TCGCCCGCCCTGTACCTG, | |
| Probe C | |
|---|---|
| GCGCTGACGCTGGCGATCTATC, | |
| Probe D | |
|---|---|
| CCGCTGTTGAACGTCGGGAAG, | |
| Probe E | |
|---|---|
| AAGCCGTCGGATCTGGGTGGCAAC, | |
| Probe F(i) and F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| or their complementary nucleotide sequences. | |

27. Process for preparing a recombinant polypeptide according to anyone of claims 1 to 8 comprising the following steps :
- the culture in an appropriate medium of a cellular host which has previously been transformed by an appropriate vector containing a nucleic acid according to anyone of claims 9 to 15, and
- the recovery of the polypeptide produced by the above said transformed cellular host from the above said culture medium.

28. Method for the in vitro diagnosis of tuberculosis in a patient liable to be infected by Mycobacterium tuberculosis comprising the following steps :
- the amplification of a nucleic acid according to anyone of claims 9 to 15, liable to be contained in a biological sample taken from said patient, by means of a primer according to any of claims 17 to 18, and/or
- contacting the above mentioned biological sample with a nucleotide probe according to claim 26, under conditions enabling the formation of a hybridization complex between said probe and said nucleic acid of the sample, and
- detecting the above said hybridization complex.

29. Method for the in vitro diagnosis of tuberculosis in a patient liable to be infected by Mycobacterium tuberculosis comprising
- contacting a biological sample taken from a patient with a polypeptide according to anyone of claims 1 to 8, under conditions enabling an in vitro immunological reaction between said polypeptide and the antibodies which my be present in the biological sample and
- the in vitro detection of the antigen/antibody complex which may be formed.

30. Method for the in vitro diagnosis of tuberculosis in a patient liable to be infected by M. tuberculosis, comprising the following steps :
- contacting the biological sample with an appropriate antibody according to claim 25, under conditions enabling an in vitro immunological reaction between said antibody and the antigens of M. tuberculosis which are possibly present in the biological sample and
- the in vitro detection of the antigen/antibody complex which may be formed.

31. Kit for an in vitro diagnostic method of tuberculosis in a patient liable to be infected by Mycobacterium tuberculosis according to claim 28, said kit comprising a determined amount of a nucleotide probe according to claim 26.

32. Kit for an in vitro diagnostic method of tuberculosis in a patient liable to be infected by Mycobacterium tuberculosis according to claim 29, comprising
- a polypeptide according to anyone of claims 1 to 8,
- reagents for making a medium appropriate for the immunological reaction to occur,
- reagents enabling to detect the antigen/antibody complex which has been produced by the immunological reaction, said reagents possibly having a label, or being liable to be recognized by a labelled reagent, more particularly in the case where the above mentioned polypeptide is not labelled.

33. Kit for an in vitro diagnostic method of tuberculosis in a patient liable to be infected by Mycobacterium tuberculosis according to claim 30, comprising
- an antibody according to claim 25,
- reagents for making a medium appropriate for the immunological reaction to occur,
- reagents enabling to detect the antigen/antibody complexes which have been produced by the immunological reaction, said reagents possibly having a label or being liable to be recognized by a labelled reagent, more particularly in the case where the above mentioned antibody is not labelled.

34. Biologically pure recombinant polypeptide according to anyone of claims 1 to 8 for use in an immunogenic composition in association with a pharmaceutically acceptable vehicle.

35. Biologically pure recombinant polypeptide according to anyone of claims 1 to 8, for use in a vaccine compositon

36. Process for the enzymatical amplification of a nucleotide sequence according to any of claims 13 to 15, and detection of the amplified nucleotide sequence, wherein the amplification is achieved by the PCR technique by means of a primer set and the detection of the PCR amplified product is achieved by a hybridization reaction with a detection probe, with said primer set and detection probe being chosen from among the following elements : 
Primer set
P1 GAGTACCTGCAGGTGCCGTCGCCGTCGATGGGCCG
 
P2 compl.     GTACCACTCGAACGCCGGGGTGTTGAT
Probe B
TCGCCCGCCCTGTACCTG

Primer set
P1          GAGTACCTGCAGGTGCCGTCGCCGTCGATGGGCCG
P3 compl.     TCCCACTTGTAAGTCTGGCA
Probe B
TCGCCCGCCCTGTACCTG

Primer set
P1          GAGTACCTGCAGGTGCCGTCGCCGTCGATGGGCCG
P4 compl.     CGGCAGCTCGCTGGTCAGGA
Probe B
TCGCCCGCCCTGTACCTG

Primer set
P1          GAGTACCTGCAGGTGCCGTCGCCGTCGATGGGCCG
P5 compl.     GCGTCACCCATCGCCAGGCCGATCAGG
Probe B
TCGCCCGCCCTGTACCTG or
Probe C
GCGCTGACGCTGGCGATCTATC

Primer set
P1          GAGTACCTGCAGGTGCCGTCGCCGTCGATGGGCCG
P6 compl.     GCGCCCCAGTACTCCCAGCTGTGCGT
Probe B
TCGCCCGCCCTGTACCTG or
Probe C
GCGCTGACGCTGGCGATCTATC or
Probe D
 
CCGCTGTTGAACGTCGGGAAG or
Probe E
AAGCCGTCGGATCTGGGTGGCAAC

Primer set
P2 ATCAACACCCCGGCGTTCGAGTGGTAC
P5 compl. GCGTCACCCATCGCCAGGCCGATCAGG
Probe C
CGCGTGACGCTGGCGATCTATC

Primer set
P2 ATCAACACCCCGGCGTTCGAGTGGTAC
P6 compl. GCGCCCAGTACTCCCAGCTGTGCGT
Probe C
GCGCTGACGCTGGCGATCTATC or
Probe D
CCGCTGTTGAACGTCGGGAAG or
Probe E
AAGCCGTCGGATCTGGGTGGCAAC

Primer set
P3 TGCCAGACTTACAAGTGGGA
P5 compl. GCGTCACCCATCGCCAGGCCGATCAGG
Probe C
GCGCTGACGCTGGCGATCTATC

Primer set
P3 TGCCAGACTTACAAGTGGGA
P6 compl. GCGCCCAGTACTCCCAGCTGTGCGT
Probe C
GCGCTGACGCTGGCGATCTATC or
Probe D
 
CCGCTGTTGAACGTCGGGAAG or
Probe E
AAGCCGTCGGATCTGGGTGGCAAC

Primer set
P4 TCCTGACCAGCGAGCTGCCG
P5 compl. GCGTCACCCATCGCCAGGCCGATCAGG
Probe C
GCGCTGACGCTGGCGATCTATC

Primer set
P4 TCCTGACCAGCGAGCTGCCG
P6 compl. GCGCCCCAGTACTCCCAGCTGTGCGT
Probe C
GCGCTGACGCTGGCGATCTATC or
Probe D
CCGCTGTTGAACGTCGGGAAG or
Probe E
AAGCCGTCGGATCTGGGTGGCAAC

Primer set
P5 CCTGATCGGCCTGGCGATGGGTGACGC
P6 compl. GCGCCCCAGTACTCCCAGCTGTGCGT
Probe D
CCGCTGTTGAACGTCGGGAAG or
Probe E
AAGCCGTCGGATCTGGGTGGCAAC
 or with said primer set being chosen from among the primer sets according to claim 19, and the detection probe being constituted by any oligonucleotide sequence of at least 10 nucleotides, said sequence being located between the two PCR primers constituting the primer set which has been used for amplifying said nucleotide sequence.

37. A recombinant vector according to claim 20, with the vector sequence having either the nucleic acid sequence represented in fig. 10b, or the nucleic acid sequence represented in fig. 11b, or the nucleic acid sequence represented in fig. 12b.

38. Peptides according to claim 1, advantageously used to produce antibodies, particularly monoclonal antibodies and which have the following amino acid sequences :
| Amino acid position (NH₂- terminal) | | Amino acid position (COOH-terminal) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |

## Claims (Claims for the following Contracting State(s): ES, GR)

1. Biologically pure recombinant polypeptide containing in its polypeptide chain one at least of the following amino acid sequences :
- the one extending from the extremity constituted by amino acid at position (-29) to the extremity constituted by amino acid at position (-1) represented on fig. 3a and fig. 3b, or
- the one extending from the extremity constituted by amino acid at position (12) to the extremity constituted by amino acid at position (31) represented on fig. 3a and fig. 3b, or
- the one extending from the extremity constituted by amino acid at position (36) to the extremity constituted by amino acid at position (55) represented on fig. 3a and fig. 3b, or
- the one extending from the extremity constituted by amino acid at position (77) to the extremity constituted by amino acid at position (96) represented on fig. 3a and fig. 3b, or
- the one extending from the extremity constituted by amino acid at position (101) to the extremity constituted by amino acid at position (120) represented on fig. 3a and fig. 3b, or
- the one extending from the extremity constituted by amino acid at position (175) to the extremity constituted by amino acid at position (194) represented on fig. 3a and fig. 3b, or
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b, or
- the one extending from the extremity constituted by amino acid at position (1) to the extremity constituted by amino acid at position (294) represented on fig. 3a and fig. 3b, or
- the one extending from the extremity constituted by amino acid at position (-42) to the extremity constituted by amino acid at position (-1) represented on fig. 3a and fig. 3b, or
- the one extending from the extremity constituted by amino acid at position (-47) to the extremity constituted by amino acid at position (-1) represented on fig. 3a and fig. 3b, or
- the one extending from the extremity constituted by amino acid at position (-49) to the extremity constituted by amino acid at position (-1) represented on fig. 3a and fig. 3b, or
- the one extending from the extremity constituted by amino acid at position (-55) to the extremity constituted by amino acid at position (-1) represented on fig. 3a and fig. 3b, or
- the one extending from the extremity constituted by amino acid at position (-59) to the extremity constituted by amino acid at position (-1) represented on fig. 3a and fig. 3b, or
- the one extending from the extremity constituted by amino acid at position (-29) to the extremity constituted by amino acid at position (294) represented on fig. 3a and fig. 3b, or
- the one extending from the extremity constituted by amino acid at position (-42) to the extremity constituted by amino acid at position (294) represented on fig. 3a and fig. 3b, or
- the one extending from the extremity constituted by amino acid at position (-47) to the extremity constituted by amino acid at position (294) represented on fig. 3a and fig. 3b, or
- the one extending from the extremity constituted by amino acid at position (-49) to the extremity constituted by amino acid at position (294) represented on fig. 3a and fig. 3b, or
- the one extending from the extremity constituted by amino acid at position (-55) to the extremity constituted by amino acid at position (294) represented on fig. 3a and fig. 3b, or
- the one extending from the extremity constituted by amino acid at position (-59) to the extremity constituted by amino acid at position (294) represented on fig. 3a and fig. 3b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

2. Biologically pure recombinant polypeptide according to claim 1 containing in its polypeptide chain one at least of the following amino acid sequences :
- the one extending from the extremity constituted by amino acid at position (-29) to the extremity constituted by amino acid at position (-1) represented on fig. 4a and fig. 4b, or
- the one extending from the extremity constituted by amino acid at position (12) to the extremity constituted by amino acid at position (31) represented on fig. 4a and fig. 4b, or
- the one extending from the extremity constituted by amino acid at position (36) to the extremity constituted by amino acid at position (55) represented on fig. 4a and fig. 4b, or
- the one extending from the extremity constituted by amino acid at position (77) to the extremity constituted by amino acid at position (96) represented on fig. 4a and fig. 4b, or
- the one extending from the extremity constituted by amino acid at position (101) to the extremity constituted by amino acid at position (120) represented on fig. 4a and fig. 4b, or
- the one extending from the extremity constituted by amino acid at position (175) to the extremity constituted by amino acid at position (194) represented on fig. 4a and fig. 4b, or
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b, or
- the one extending from the extremity constituted by amino acid at position (1) to the extremity constituted by amino acid at position (294) represented on fig. 4a and fig. 4b, or
- the one extending from the extremity constituted by amino acid at position (-42) to the extremity constituted by amino acid at position (-1) represented on fig. 4a and fig. 4b, or
- the one extending from the extremity constituted by amino acid at position (-47) to the extremity constituted by amino acid at position (-1) represented on fig. 4a and fig. 4b, or
- the one extending from the extremity constituted by amino acid at position (-49) to the extremity constituted by amino acid at position (-1) represented on fig. 4a and fig. 4b, or
- the one extending from the extremity constituted by amino acid at position (-55) to the extremity constituted by amino acid at position (-1) represented on fig. 4a and fig. 4b, or
- the one extending from the extremity constituted by amino acid at position (-59) to the extremity constituted by amino acid at position (-1) represented on fig. 4a and fig. 4b, or
- the one extending from the extremity constituted by amino acid at position (-29) to the extremity constituted by amino acid at position (294) represented on fig. 4a and fig. 4b, or
- the one extending from the extremity constituted by amino acid at position (-42) to the extremity constituted by amino acid at position (294) represented on fig. 4a and fig. 4b, or
- the one extending from the extremity constituted by amino acid at position (-47) to the extremity constituted by amino acid at position (294) represented on fig. 4a and fig. 4b, or
- the one extending from the extremity constituted by amino acid at position (-49) to the extremity constituted by amino acid at position (294) represented on fig. 4a and fig. 4b, or
- the one extending from the extremity constituted by amino acid at position (-55) to the extremity constituted by amino acid at position (294) represented on fig. 4a and fig. 4b, or
- the one extending from the extremity constituted by amino acid at position (-59) to the extremity constituted by amino acid at position (294) represented on fig. 4a and fig. 4b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

3. Biologically pure recombinant polypeptide according to claim 1 containing in its polypeptide chain one at least of the following amino acid sequences :
- the one extending from the extremity constituted by amino acid at position (-30) to the extremity constituted by amino acid at position (-1) represented on fig. 5, or
- the one extending from the extremity constituted by amino acid at position (12) to the extremity constituted by amino acid at position (31) represented on fig. 5, or
- the one extending from the extremity constituted by amino acid at position (36) to the extremity constituted by amino acid at position (55) represented on fig. 5, or
- the one extending from the extremity constituted by amino acid at position (77) to the extremity constituted by amino acid at position (96) represented on fig. 5, or
- the one extending from the extremity constituted by amino acid at position (101) to the extremity constituted by amino acid at position (120) represented on fig. 5, or
- the one extending from the extremity constituted by amino acid at position (175) to the extremity constituted by amino acid at position (194) represented on fig. 5, or
- the one extending from the extremity constituted by amino acid at position (211) to the extremity constituted by amino acid at position (230) represented on fig. 5, or
- the one extending from the extremity constituted by amino acid at position (275) to the extremity constituted by amino acid at position (295) represented on fig. 5, or
- the one extending from the extremity constituted by amino acid at position (1) to the extremity constituted by amino acid at position (295) represented on fig. 5, or
- the one extending from the extremity constituted by amino acid at position (-43) to the extremity constituted by amino acid at position (-1) represented on fig. 5, or
- the one extending from the extremity constituted by amino acid at position (-30) to the extremity constituted by amino acid at position (295) represented on fig. 5, or
- the one extending from the extremity constituted by amino acid at position (-43) to the extremity constituted by amino acid at position (295) represented on fig. 5, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage and/or
- a conjugate of said polypeptides is able to induce in vivo the production of antibodies neutralizing M. tuberculosis or induce in vivo a cellular immune response by activating M. tuberculosis responsive T-cells.

4. Recombinant polypeptide according to claim 1, consisting of one of the following amino acid sequences :
- the one extending from the extremity constituted by amino acid at position (-59) to the extremity constituted by amino acid at position (294) represented on fig. 3a and fig. 3b,
- the one extending from the extremity constituted by amino acid at position (-59) to the extremity constituted by amino acid at position (-1) represented on fig. 3a and fig. 3b,
- the one extending from the extremity constituted by amino acid at position (-55) to the extremity constituted by amino acid at position (294) represented on fig. 3a and fig. 3b,
- the one extending from the extremity constituted by amino acid at position (-55) to the extremity constituted by amino acid at position (-1) represented on fig. 3a and fig. 3b,
- the one extending from the extremity constituted by amino acid at position (-49) to the extremity constituted by amino acid at position (294) represented on fig. 3a and fig. 3b,
- the one extending from the extremity constituted by amino acid at position (-49) to the extremity constituted by amino acid at position (-1) represented on fig. 3a and fig. 3b,
- the one extending from the extremity constituted by amino acid at position (-47) to the extremity constituted by amino acid at position (294) represented on fig. 3a and fig. 3b,
- the one extending from the extremity constituted by amino acid at position (-47) to the extremity constituted by amino acid at position (-1) represented on fig. 3a and fig. 3b,
- the one extending from the extremity constituted by amino acid at position (-42) to the extremity constituted by amino acid at position (294) represented on fig. 3a and fig. 3b,
- the one extending from the extremity constituted by amino acid at position (-42) to the extremity constituted by amino acid at position (-1) represented on fig. 3a and fig. 3b,
- the one extending from the extremity constituted by amino acid at position (-29) to the extremity constituted by amino acid at position (294) represented on fig. 3a and fig. 3b,
- the one extending from the extremity constituted by amino acid at position (-29) to the extremity constituted by amino acid at position (-1) represented on fig. 3a and fig. 3b,
- the one extending from the extremity constituted by amino acid at position (1) to the extremity constituted by amino acid at position (294) represented on fig. 3a and fig. 3b,
- the one extending from the extremity constituted by amino acid at position (12) to the extremity constituted by amino acid at position (31) represented on fig. 3a and fig. 3b,
- the one extending from the extremity constituted by amino acid at position (36) to the extremity constituted by amino acid at position (55) represented on fig. 3a and fig. 3b,
- the one extending from the extremity constituted by amino acid at position (77) to the extremity constituted by amino acid at position (96) represented on fig. 3a and fig. 3b,
- the one extending from the extremity constituted by amino acid at position (101) to the extremity constituted by amino acid at position (120) represented on fig. 3a and fig. 3b,
- the one extending from the extremity constituted by amino acid at position (175) to the extremity constituted by amino acid at position (194) represented on fig. 3a and fig. 3b,
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b.

5. Recombinant polypeptide according to claim 2, consisting of one of the following amino acid sequences :
- the one extending from the extremity constituted by amino acid at position (-59) to the extremity constituted by amino acid at position (294) represented on fig. 4a and fig. 4b,
- the one extending from the extremity constituted by amino acid at position (-59) to the extremity constituted by amino acid at position (-1) represented on fig. 4a and fig. 4b,
- the one extending from the extremity constituted by amino acid at position (-55) to the extremity constituted by amino acid at position (294) represented on fig. 4a and fig. 4b,
- the one extending from the extremity constituted by amino acid at position (-55) to the extremity constituted by amino acid at position (-1) represented on fig. 4a and fig. 4b,
- the one extending from the extremity constituted by amino acid at position (-49) to the extremity constituted by amino acid at position (294) represented on fig. 4a and fig. 4b,
- the one extending from the extremity constituted by amino acid at position (-49) to the extremity constituted by amino acid at position (-1) represented on fig. 4a and fig. 4b,
- the one extending from the extremity constituted by amino acid at position (-47) to the extremity constituted by amino acid at position (294) represented on fig. 4a and fig. 4b,
- the one extending from the extremity constituted by amino acid at position (-47) to the extremity constituted by amino acid at position (-1) represented on fig. 4a and fig. 4b,
- the one extending from the extremity constituted by amino acid at position (-42) to the extremity constituted by amino acid at position (294) represented on fig. 4a and fig. 4b,
- the one extending from the extremity constituted by amino acid at position (-42) to the extremity constituted by amino acid at position (-1) represented on fig. 4a and fig. 4b,
- the one extending from the extremity constituted by amino acid at position (-29) to the extremity constituted by amino acid at position (294) represented on fig. 4a and fig. 4b,
- the one extending from the extremity constituted by amino acid at position (-29) to the extremity constituted by amino acid at position (-1) represented on fig. 4a and fig. 4b,
- the one extending from the extremity constituted by amino acid at position (1) to the extremity constituted by amino acid at position (294) represented on fig. 4a and fig. 4b,
- the one extending from the extremity constituted by amino acid at position (12) to the extremity constituted by amino acid at position (31) represented on fig. 4a and fig. 4b,
- the one extending from the extremity constituted by amino acid at position (36) to the extremity constituted by amino acid at position (55) represented on fig. 4a and fig. 4b,
- the one extending from the extremity constituted by amino acid at position (77) to the extremity constituted by amino acid at position (96) represented on fig. 4a and fig. 4b,
- the one extending from the extremity constituted by amino acid at position (101) to the extremity constituted by amino acid at position (120) represented on fig. 4a and fig. 4b,
- the one extending from the extremity constituted by amino acid at position (175) to the extremity constituted by amino acid at position (194) represented on fig. 4a and fig. 4b,
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b.

6. Recombinant polypeptide according to claim 3, consisting of one of the following amino acid sequences :
- the one extending from the extremity constituted by amino acid at position (-43) to the extremity constituted by amino acid at position (295) represented on fig. 5,
- the one extending from the extremity constituted by amino acid at position (-43) to the extremity constituted by amino acid at position (-1) represented on fig. 5,
- the one extending from the extremity constituted by amino acid at position (-30) to the extremity constituted by amino acid at position (295) represented on fig. 5,
- the one extending from the extremity constituted by amino acid at position (-30) to the extremity constituted by amino acid at position (-1) represented on fig. 5,
- the one extending from the extremity constituted by amino acid at position (1) to the extremity constituted by amino acid at position (295) represented on fig. 5,
- the one extending from the extremity constituted by amino acid at position (12) to the extremity constituted by amino acid at position (31) represented on fig. 5,
- the one extending from the extremity constituted by amino acid at position (36) to the extremity constituted by amino acid at position (55) represented on fig. 5,
- the one extending from the extremity constituted by amino acid at position (77) to the extremity constituted by amino acid at position (96) represented on fig. 5,
- the one extending from the extremity constituted by amino acid at position (101) to the extremity constituted by amino acid at position (120) represented on fig. 5,
- the one extending from the extremity constituted by amino acid at position (175) to the extremity constituted by amino acid at position (194) represented on fig. 5,
- the one extending from the extremity constituted by amino acid at position (211) to the extremity constituted by amino acid at position (230) represented on fig. 5, or
- the one extending from the extremity constituted by amino acid at position (275) to the extremity constituted by amino acid at position (295) represented on fig. 5.

7. Biologically pure recombinant polypeptide constituted by a polypeptide according to any of claims 1 to 6, and a protein having a heterologous sequence with respect to said polypeptide, said heterologous sequence comprising from about 1 to about 1000 amino acids.

8. Recombinant polypeptide according to claim 7, wherein the heterologous protern is β-galactosidase.

9. Nucleic acid comprising
(a) a nucleotide sequence coding for a polypeptide according to any of claims 1 to 6, or
(b) a nucleotide sequence which is complementary to (a), or
(c) the nucleotide sequences of (a) or (b) wherein T can be replaced by U.

10. Nucleic acid comprising one at least of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (1242) to the extremity constituted by nucleotide at position (1358), wherein N represents one of the five A, T, C, G or I nucleotides, represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1358) represented in fig. 3a and fig. 3b, or the complementary sequences thereof,
or the above said nucleotide sequences wherein T is replaced by U.

11. Nucleic acid according to claim 10 comprising one at least of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (1242) to the extremity constituted by nucleotide at position (1358), wherein N represents one of the five A, T, C, G or I nucleotides, represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (183) to the extremity constituted by nucleotide at position (1358) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1358) represented in fig. 4a and fig. 4b, or the complementary sequences thereof,
or the above said nucleotide sequences wherein T is replaced by U.

12. Nucleic acid according to claim 10 comprising one at least of the following nucleotide sequences :
- the one extending from the extremity constituted by nucleotide at position (130) to the extremity constituted by nucleotide at position (219) represented in fig. 5,
- the one extending from the extremity constituted by nucleotide at position (220) to the extremity constituted by nucleotide at position (1104) represented in fig. 5,
- the one extending from the extremity constituted by nucleotide at position (1105) to the extremity constituted by nucleotide at position (1299),
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1299) represented in fig. 5,
- the one extending from the extremity constituted by nucleotide at position (90) to the extremity constituted by nucleotide at position (1104) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity-constituted by nucleotide at position (1299) represented in fig. 5,
- the one extending from the extremity constituted by nucleotide at position (220) to the extremity constituted by nucleotide at position (1299) represented in fig. 5,
or the complementary sequences thereof,
or the above said nucleotide sequences wherein T is replaced by U.

13. Nucleic acid according to claim 10 consisting of one of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (360) to the extremity constituted by nucleotide at position (1358) represented in fig. 3a and fig. 3b,
- the one extending from the extremity constituted by nucleotide at position (1242) to the extremity constituted by nucleotide at position (1358) represented in fig. 3a and fig. 3b, or the complementary sequences thereof,
or the above said nucleotide sequences wherein T is replaced by U.

14. Nucleic acid according to claim 11 consisting of one of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (183) to the extremity constituted by nucleotide at position (1241) represented in fig. 4a and fig 4b,
- the one extending from the extremity constituted by nucleotide at position (183) to the extremity constituted by nucleotide at position(1358) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (360) to the extremity constituted by nucleotide at position (1358) represented in fig. 4a and fig. 4b,
- the one extending from the extremity constituted by nucleotide at position (1242) to the extremity constituted by nucleotide at position (1358) represented in fig. 4a and fig. 4b, or the complementary sequences thereof,
or the above said nucleotide sequences wherein T is replaced by U.

15. Nucleic acid according to claim 12 consisting of one of the following nucleotide sequences :
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1104) represented in fig. 5,
- the one extending from the extremity constituted by nucleotide at position (90) to the extremity constituted by nucleotide at position (1299) represented in fig. 5,
- the one extending from the extremity constituted by nucleotide at position (130) to the extremity constituted by nucleotide at position (219) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity constituted by nucleotide at position (1299) represented in fig. 5,
- the one extending from the extremity constituted by nucleotide at position (220) to the extremity constituted by nucleotide at position (1104) represented in fig. 5,
- the one extending from the extremity constituted by nucleotide at position (220) to the extremity constituted by nucleotide at position (1299) represented in fig. 5,
- the one extending from the extremity constituted by nucleotide at position (1105) to the extremity constituted by nucleotide at position (1299) represented in fig. 5, or the complementary sequences thereof,
or the above said nucleotide sequences wherein T is replaced by U.

16. Recombinant nucleic acid containing at least one of the nucleotide sequences according to claims 9 to 15, inserted in a heterologous nucleic acid.

17. A DNA or RNA primer constituted by 15 to 25 nucleotides of a nucleotide sequence according to any one of claims 13 to 15, which can be used as a primer to specifically amplify any of said nucleotide sequences.

18. A DNA or RNA primer according to claim 17, with said primer being chosen from the following group of nucleotide sequences:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
or the complementary sequences thereof or the corresponding ribonucleotide sequences.

19. A DNA or RNA primer set for the enzymatic amplification of a nucleotide sequence according to any one of claims 13 to 15, with said primer set comprising
- a A nucleotide sequence combined with the complementary nucleotide sequence of either B, or C, or D, or E, or F; or
- the B nucleotide sequence combined with the complementary nucleotide sequence of C, or D, or E, or F; or
- the C nucleotide sequence combined with the complementary nucleotide sequence of D, or E, or F; or
- the D nucleotide sequence combined with the complementary nucleotide sequence of E, or F; or
- the E nucleotide sequence combined with a complementary nucleotide sequence of F, with the nucleotide sequences A, B, C, D, E and F being specified in claim 18, and with A meaning any of the sequences A(i), A(ii), A(iii), A(iv), and A(v) and F meaning any of the sequences F(i), F(ii), F(iii), and F(iv).

20. Recombinant vector, particularly for cloning and/or expression, comprising a vector sequence of the type plasmid, cosmid or phage, and a nucleic acid according to anyone of claims 9 to 15, in one of the non essential sites for its replication.

21. Recombinant vector according to claim 20, containing in one of the non essential sites for its replication the necessary elements to promote the expression of a polypeptide according to anyone of claims 1 to 6 in a cellular host.

22. Recombinant vector according to claim 21, containing the elements enabling the expression by E. coli of a polypeptide according to anyone of claims 1 to 6.

23. Cellular host containing a recombinant vector according to anyone of claims 20 to 22, and comprising the regulation elements enabling the expression of a polypeptide according to anyone of claims 1 to 6 in this host.

24. Cellular host according to claim 23, chosen from among bacteria such as E. coli transformed by a vector according to claim 20, or chosen from among eukaryotic organisms, transformed by a vector according to claim 20.

25. Monoclonal antibody specifically directed against a recombinant polypeptide according to anyone of claims 1 to 6.

26. A nucleotide probe hybridizing with a nucleic acid according to any of claims 13 to 15, chosen from the following group of nucleotide sequences:
| Probes A(i), A(ii), A(iii), A(iv) and A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Probe B | |
|---|---|
| TCGCCCGCCCTGTACCTG, | |
| Probe C | |
|---|---|
| GCGCTGACGCTGGCGATCTATC, | |
| Probe D | |
|---|---|
| CCGCTGTTGAACGTCGGGAAG, | |
| Probe E | |
|---|---|
| AAGCCGTCGGATCTGGGTGGCAAC, | |
| Probe F(i) and F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| or their complementary nucleotide sequences. | |

27. Process for preparing a recombinant polypeptide according to anyone of claims 1 to 8 comprising the following steps :
- the culture in an appropriate medium of a cellular host which has previously been transformed by an appropriate vector containing a nucleic acid according to anyone of claims 9 to 15, and
- the recovery of the polypeptide produced by the above said transformed cellular host from the above said culture medium.

28. Method for the in vitro diagnosis of tuberculosis in a patient liable to be infected by Mycobacterium tuberculosis comprising the following steps :
- the amplification of a nucleic acid according to anyone of claims 9 to 15, liable to be contained in a biological sample taken from said patient, by means of a primer according to any of claims 17 to 18, and/or
- contacting the above mentioned biological sample with a nucleotide probe according to claim 26, under conditions enabling the formation of a hybridization complex between said probe and said nucleic acid of the sample, and
- detecting the above said hybridization complex.

29. Method for the in vitro diagnosis of tuberculosis in a patient liable to be infected by Mycobacterium tuberculosis comprising
- contacting a biological sample taken from a patient with a polypeptide according to anyone of claims 1 to 8, under conditions enabling an in vitro immunological reaction between said polypeptide and the antibodies which may be present in the biological sample and
- the in vitro detection of the antigen/antibody complex which may be formed.

30. Method for the in vitro diagnosis of tuberculosis in a patient liable to be infected by M. tuberculosis, comprising the following steps :
- contacting the biological sample with an appropriate antibody according to claim 25, under conditions enabling an in vitro immunological reaction between said antibody and the antigens of M. tuberculosis which are possibly present in the biological sample and
- the in vitro detection of the antigen/antibody complex which may be formed.

31. Kit for an in vitro diagnostic method of tuberculosis in a patient liable to be infected by Mycobacterium tuberculosis according to claim 28, said kit comprising a determined amount of a nucleotide probe according to claim 26.

32. Kit for an in vitro diagnostic method of tuberculosis in a patient liable to be infected by Mycobacterium tuberculosis according to claim 29, comprising
- a polypeptide according to anyone of claims 1 to 8,
- reagents for making a medium appropriate for the immunological reaction to occur,
- reagents enabling to detect the antigen/antibody complex which has been produced by the immunological reaction, said reagents possibly having a label, or being liable to be recognized by a labelled reagent, more particularly in the case where the above mentioned polypeptide is not labelled.

33. Kit for an in vitro diagnostic method of tuberculosis in a patient liable to be infected by Mycobacterium tuberculosis according to claim 30, comprising
- an antibody according to claim 25,
- reagents for making a medium appropriate for the immunological reaction to occur,
- reagents enabling to detect the antigen/antibody complexes which have been produced by the immunological reaction, said reagents possibly having a label or being liable to be recognized by a labelled reagent, more particularly in the case where the above mentioned antibody is not labelled.

34. Bioligically pure recombinant polypeptide according to anyone of claims 1 to 8 for use in an immunogenic composition in association with a pharmaceutically acceptable vehicle.

35. Biologically pure recombinant polypeptide according to anyone of claims 1 to 8, for use in a vaccine composition

36. Process for the enzymatical amplification of a nucleotide sequence according to any of claims 13 to 15, and detection of the amplified nucleotide sequence, wherein the amplification is achieved by the PCR technique by means of a primer set and the detection of the PCR amplified product is achieved by a hybridization reaction with a detection probe, with said primer set and detection probe being chosen from among the following elements : 
Primer set
P1 GAGTACCTGCAGGTGCCGTCGCCGTCGATGGGCCG
 
P2 compl.    GTACCACTCGAACGCCGGGGTGTTGAT
Probe B
TCGCCCGCCCTGTACCTG

Primer set
P1    GAGTACCTGCAGGTGCCGTCGCCGTCGATGGGCCG
P3 compl.    TCCCACTTGTAAGTCTGGCA
Probe B
TCGCCCGCCCTGTACCTG

Primer set
P1    GAGTACCTGCAGGTGCCGTCGCCGTCGATGGGCCG
P4 compl.    CGGCAGCTCGCTGGTCAGGA
Probe B
TCGCCCGCCCTGTACCTG

Primer set
P1    GAGTACCTGCAGGTGCCGTCGCCGTCGATGGGCCG
P5 compl.    GCGTCACCCATCGCCAGGCCGATCAGG
Probe B
TCGCCCGCCCTGTACCTG or
Probe C
GCGCTGACGCTGGCGATCTATC

Primer set
P1    GAGTACCTGCAGGTGCCGTCGCCGTCGATGGGCCG
P6 compl.    GCGCCCCAGTACTCCCAGCTGTGCGT
Probe B
TCGCCCGCCCTGTACCTG or
Probe C
GCGCTGACGCTGGCGATCTATC or
Probe D
 
CCGCTGTTGAACGTCGGGAAG or
Probe E
AAGCCGTCGGATCTGGGTGGCAAC

Primer set
P2 ATCAACACCCCGGCGTTCGAGTGGTAC
P5 compl. GCGTCACCCATCGCCAGGCCGATCAGG
Probe C
CGCGTGACGCTGGCGATCTATC

Primer set
P2 ATCAACACCCCGGCGTTCGAGTGGTAC
P6 compl. GCGCCCCAGTACTCCCAGCTGTGCGT
Probe C
GCGCTGACGCTGGCGATCTATC or
Probe D
CCGCTGTTGAACGTCGGGAAG or
Probe E
AAGCCGTCGGATCTGGGTGGCAAC

Primer set
P3 TGCCAGACTTACAAGTGGGA
P5 compl. GCGTCACCCATCGCCAGGCCGATCAGG
Probe C
GCGCTGACGCTGGCGATCTATC

Primer set
P3 TGCCAGACTTACAAGTGGGA
P6 compl. GCGCCCCAGTACTCCCAGCTGTGCGT
Probe C
GCGCTGACGCTGGCGATCTATC or
Probe D
 
CCGCTGTTGAACGTCGGGAAG or
Probe E
AAGCCGTCGGATCTGGGTGGCAAC

Primer set
P4 TCCTGACCAGCGAGCTGCCG
P5 compl. GCGTCACCCATCGCCAGGCCGATCAGG
Probe C
GCGCTGACGCTGGCGATCTATC

Primer set
P4 TCCTGACCAGCGAGCTGCCG
P6 compl. GCGCCCCAGTACTCCCAGCTGTGCGT
Probe C
GCGCTGACGCTGGCGATCTATC or
Probe D
CCGCTGTTGAACGTCGGGAAG or
Probe E
AAGCCGTCGGATCTGGGTGGCAAC

Primer set
P5 CCTGATCGGCCTGGCGATGGGTGACGC
P6 compl. GCGCCCCAGTACTCCCAGCTGTGCGT
Probe D
CCGCTGTTGAACGTCGGGAAG or
Probe E
AAGCCGTCGGATCTGGGTGGCAAC
 or with said primer set being chosen from among the primer sets according to claim 19, and the detection probe being constituted by any oligonucleotide sequence of at least 10 nucleotides, said sequence being located between the two PCR primers constituting the primer set which has been used for amplifying said nucleotide sequence.

37. A recombinant vector according to claim 20, with the vector sequence having either the nucleic acid sequence represented in fig. 10b, or the nucleic acid sequence represented in fig. 11b, or the nucleic acid sequence represented in fig. 12b.

38. Peptides according to claim 1, advantageously used to produce antibodies, particularly monoclonal antibodies and which have the following amino acid sequences :
| Amino acid position (NH₂- terminal) | | Amino acid position (COOH-terminal) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |

39. A process for preparing a biologically pure recombinant polypeptide containing in its polypeptide chain one at least of the following amino acid sequences :
- the one extending from the extremity constituted by amino acid at position (-29) to the extremity constituted by amino acid at position (-1) represented on fig. 3a and fig. 3b, or
- the one extending from the extremity constituted by amino acid at position (12) to the extremity constituted by amino acid at position (31) represented on fig. 3a and fig. 3b, or
- the one extending from the extremity constituted by amino acid at position (36) to the extremity constituted by amino acid at position (55) represented on fig. 3a and fig. 3b, or
- the one extending from the extremity constituted by amino acid at position (77) to the extremity constituted by amino acid at position (96) represented on fig. 3a and fig. 3b, or
- the one extending from the extremity constituted by amino acid at position (101) to the extremity constituted by amino acid at position (120) represented on fig. 3a and fig. 3b, or
- the one extending from the extremity constituted by amino acid at position (175) to the extremity constituted by amino acid at position (194) represented on fig. 3a and fig. 3b, or
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b, or
- the one extending from the extremity constituted by amino acid at position (1) to the extremity constituted by amino acid at position (294) represented on fig. 3a and fig. 3b, or
- the one extending from the extremity constituted by amino acid at position (-42) to the extremity constituted by amino acid at position (-1) represented on fig. 3a and fig. 3b, or
- the one extending from the extremity constituted by amino acid at position (-47) to the extremity constituted by amino acid at position (-1) represented on fig. 3a and fig. 3b, or
- the one extending from the extremity constituted by amino acid at position (-49) to the extremity constituted by amino acid at position (-1) represented on fig. 3a and fig. 3b, or
- the one extending from the extremity constituted by amino acid at position (-55) to the extremity constituted by amino acid at position (-1) represented on fig. 3a and fig. 3b, or
- the one extending from the extremity constituted by amino acid at position (-59) to the extremity constituted by amino acid at position (-1) represented on fig. 3a and fig. 3b, or
- the one extending from the extremity constituted by amino acid at position (-29) to the extremity constituted by amino acid at position (294) represented on fig. 3a and fig. 3b, or
- the one extending from the extremity constituted by amino acid at position (-42) to the extremity constituted by amino acid at position (294) represented on fig. 3a and fig. 3b, or
- the one extending from the extremity constituted by amino acid at position (-47) to the extremity constituted by amino acid at position (294) represented on fig. 3a and fig. 3b, or
- the one extending from the extremity constituted by amino acid at position (-49) to the extremity constituted by amino acid at position (294) represented on fig. 3a and fig. 3b, or
- the one extending from the extremity constituted by amino acid at position (-55) to the extremity constituted by amino acid at position (294) represented on fig. 3a and fig. 3b, or
- the one extending from the extremity constituted by amino acid at position (-59) to the extremity constituted by amino acid at position (294) represented on fig. 3a and fig. 3b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells, said process comprising the steps of
- producing the polypeptide by genetic engineering, i.e. by expression in a cellular host of the nucleic acids encoding said polypeptides or by classical chemical synthesis, and
- purifying the polypeptide produced.

40. A process for preparing a biologically pure recombinant polypeptide according to claim 39, said polypeptide containing in its polypeptide chain one at least of the following amino acid sequences :
- the one extending from the extremity constituted by amino acid at position (-29) to the extremity constituted by amino acid at position (-1) represented on fig. 4a and fig. 4b, or
- the one extending from the extremity constituted by amino acid at position (12) to the extremity constituted by amino acid at position (31) represented on fig. 4a and fig. 4b, or
- the one extending from the extremity constituted by amino acid at position (36) to the extremity constituted by amino acid at position (55) represented on fig. 4a and fig. 4b, or
- the one extending from the extremity constituted by amino acid at position (77) to the extremity constituted by amino acid at position (96) represented on fig. 4a and fig. 4b, or
- the one extending from the extremity constituted by amino acid at position (101) to the extremity constituted by amino acid at position (120) represented on fig. 4a and fig. 4b, or
- the one extending from the extremity constituted by amino acid at position (175) to the extremity constituted by amino acid at position (194) represented on fig. 4a and fig. 4b, or
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b, or
- the one extending from the extremity constituted by amino, acid at position (1) to the extremity constituted by amino acid at position (294) represented on fig. 4a and fig. 4b, or
- the one extending from the extremity constituted by amino acid at position (-42) to the extremity constituted by amino acid at position (-1) represented on fig. 4a and fig. 4b, or
- the one extending from the extremity constituted by amino acid at position (-47) to the extremity constituted by amino acid at position (-1) represented on fig. 4a and fig. 4b, or
- the one extending from the extremity constituted by amino acid at position (-49) to the extremity constituted by amino acid at position (-1) represented on fig. 4a and fig. 4b, or
- the one extending from the extremity constituted by amino acid at position (-55) to the extremity constituted by amino acid at position (-1) represented on fig. 4a and fig. 4b, or
- the one extending from the extremity constituted by amino acid at position (-59) to the extremity constituted by amino acid at position (-1) represented on fig. 4a and fig. 4b, or
- the one extending from the extremity constituted by amino acid at position (-29) to the extremity constituted by amino acid at position (294) represented on fig. 4a and fig. 4b, or
- the one extending from the extremity constituted by amino acid at position (-42) to the extremity constituted by amino acid at position (294) represented on fig. 4a and fig. 4b, or
- the one extending from the extremity constituted by amino acid at position (-47) to the extremity constituted by amino acid at position (294) represented on fig. 4a and fig. 4b, or
- the one extending from the extremity constituted by amino acid at position (-49) to the extremity constituted by amino acid at position (294) represented on fig. 4a and fig. 4b, or
- the one extending from the extremity constituted by amino acid at position (-55) to the extremity constituted by amino acid at position (294) represented on fig. 4a and fig. 4b, or
- the one extending from the extremity constituted by amino acid at position (-59) to the extremity constituted by amino acid at position (294) represented on fig. 4a and fig. 4b, or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

41. A process for preparing a biologically pure recombinant polypeptide according to claim 39, said polypeptide containing in its polypeptide chain one at least of the following amino acid sequences :
- the one extending from the extremity constituted by amino acid at position (-30) to the extremity constituted by amino acid at position (-1) represented on fig. 5, or
- the one extending from the extremity constituted by amino acid at position (12) to the extremity constituted by amino acid at position (31) represented on fig. 5, or
- the one extending from the extremity constituted by amino acid at position (36) to the extremity constituted by amino acid at position (55) represented on fig. 5, or
- the one extending from the extremity constituted by amino acid at position (77) to the extremity constituted by amino acid at position (96) represented on fig. 5, or
- the one extending from the extremity constituted by amino acid at position (101) to the extremity constituted by amino acid at position (120) represented on fig. 5, or
- the one extending from the extremity constituted by amino acid at position (175) to the extremity constituted by amino acid at position (194) represented on fig. 5, or
- the one extending from the extremity constituted by amino acid at position (211) to the extremity constituted by amino acid at position (230) represented on fig. 5, or
- the one extending from the extremity constituted by amino acid at position (275) to the extremity constituted by amino acid at position (295) represented on fig. 5, or
- the one extending from the extremity constituted by amino acid at position (1) to the extremity constituted by amino acid at position (295) represented on fig. 5, or
- the one extending from the extremity constituted by amino acid at position (-43) to the extremity constituted by amino acid at position (-1) represented on fig. 5, or
- the one extending from the extremity constituted by amino acid at position (-30) to the extremity constituted by amino acid at position (295) represented on fig. 5, or
- the one extending from the extremity constituted by amino acid at position (-43) to the extremity constituted by amino acid at position (295) represented on fig. 5,
or the peptidic sequences resulting from the modification of the free carboxyl and/or amino groups in the polypeptide sequence and/or resulting from the glycosylation of the above-mentioned polypeptides, in so far as this modification does not alter the following properties:
- the polypeptides react with rabbit polyclonal antiserum raised against the protein of 32-kDa of M. bovis BCG culture filtrate, and/or
- the polypeptides react selectively with human sera from tuberculosis patients and particularly patients developing an evolutive tuberculosis at an early stage, and/or
- a conjugate of said polypeptides is able to induce in vivo the production of antibodies neutralizing M. tuberculosis or induce in vivo a cellular immune response by activating M. tuberculosis responsive T-cells.

42. A process for preparing a recombinant polypeptide according to claim 39, said polypeptide consisting of one of the following amino acid sequences :
- the one extending from the extremity constituted by amino acid at position (-59) to the extremity constituted by amino acid at position (294) represented on fig. 3a and fig. 3b,
- the one extending from the extremity constituted by amino acid at position (-59) to the extremity constituted by amino acid at position (-1) represented on fig. 3a and fig. 3b,
- the one extending from the extremity constituted by amino acid at position (-55) to the extremity constituted by amino acid at position (294) represented on fig. 3a and fig. 3b,
- the one extending from the extremity constituted by amino acid at position (-55) to the extremity constituted by amino acid at position (-1) represented on fig. 3a and fig. 3b,
- the one extending from the extremity constituted by amino acid at position (-49) to the extremity constituted by amino acid at position (294) represented on fig. 3a and fig. 3b,
- the one extending from the extremity constituted by amino acid at position (-49) to the extremity constituted by amino acid at position (-1) represented on fig. 3a and fig. 3b,
- the one extending from the extremity constituted by amino acid at position (-47) to the extremity constituted by amino acid at position (294) represented on fig. 3a and fig. 3b,
- the one extending from the extremity constituted by amino acid at position (-47) to the extremity constituted by amino acid at position (-1) represented on fig. 3a and fig. 3b,
- the one extending from the extremity constituted by amino acid at position (-42) to the extremity constituted by amino acid at position (294) represented on fig. 3a and fig. 3b,
- the one extending from the extremity constituted by amino acid at position (-42) to the extremity constituted by amino acid at position (-1) represented on fig. 3a and fig. 3b,
- the one extending from the extremity constituted by amino acid at position (-29) to the extremity constituted by amino acid at position (294) represented on fig. 3a and fig. 3b,
- the one extending from the extremity constituted by amino acid at position (-29) to the extremity constituted by amino acid at position (-1) represented on fig. 3a and fig. 3b,
- the one extending from the extremity constituted by amino acid at position (1) to the extremity constituted by amino acid at position (294) represented on fig. 3a and fig. 3b,
- the one extending from the extremity constituted by amino acid at position (12) to the extremity constituted by amino acid at position (31) represented on fig. 3a and fig. 3b,
- the one extending from the extremity constituted by amino acid at position (36) to the extremity constituted by amino acid at position (55) represented on fig. 3a and fig. 3b,
- the one extending from the extremity constituted by amino acid at position (77) to the extremity constituted by amino acid at position (96) represented on fig. 3a and fig. 3b,
- the one extending from the extremity constituted by amino acid at position (101) to the extremity constituted by amino acid at position (120) represented on fig. 3a and fig. 3b,
- the one extending from the extremity constituted by amino acid at position (175) to the extremity constituted by amino acid at position (194) represented on fig. 3a and fig. 3b,
- the one extending from the extremity constituted by amino acid at position (211) to the extremity constituted by amino acid at position (230) represented on fig. 3a and fig. 3b, or
- the one extending from the extremity constituted by amino acid at position (275) to the extremity constituted by amino acid at position (294) represented on fig. 3a and fig. 3b.

43. A process for preparing a recombinant polypeptide according to claim 39, said polypeptide consisting of one of the following amino acid sequences :
- the one extending from the extremity constituted by amino acid at position (-59) to the extremity constituted by amino acid at position (294) represented on fig. 4a and fig. 4b,
- the one extending from the extremity constituted by amino acid at position (-59) to the extremity constituted by amino acid at position (-1) represented on fig. 4a and fig. 4b,
- the one extending from the extremity constituted by amino acid at position (-55) to the extremity constituted by amino acid at position (294) represented on fig. 4a and fig. 4b,
- the one extending from the extremity constituted by amino acid at position (-55) to the extremity constituted by amino acid at position (-1) represented on fig. 4a and fig. 4b,
- the one extending from the extremity constituted by amino acid at position (-49) to the extremity constituted by amino acid at position (294) represented on fig. 4a and fig. 4b,
- the one extending from the extremity constituted by amino acid at position (-49) to the extremity constituted by amino acid at position (-1) represented on fig. 4a and fig. 4b,
- the one extending from the extremity constituted by amino acid at position (-47) to the extremity constituted by amino acid at position (294) represented on fig. 4a and fig. 4b,
- the one extending from the extremity constituted by amino acid at position (-47) to the extremity constituted by amino acid at position (-1) represented on fig. 4a and fig. 4b,
- the one extending from the extremity constituted by amino acid at position (-42) to the extremity constituted by amino acid at position (294) represented on fig. 4a and fig. 4b,
- the one extending from the extremity constituted by amino acid at position (-42) to the extremity constituted by amino acid at position (-1) represented on fig. 4a and fig. 4b,
- the one extending from the extremity constituted by amino acid at position (-29) to the extremity constituted by amino acid at position (294) represented on fig. 4a and fig. 4b,
- the one extending from the extremity constituted by amino acid at position (-29) to the extremity constituted by amino acid at position (-1) represented on fig. 4a and fig. 4b,
- the one extending from the extremity constituted by amino acid at position (1) to the extremity constituted by amino acid at position (294) represented on fig. 4a and fig. 4b,
- the one extending from the extremity constituted by amino acid at position (12) to the extremity constituted by amino acid at position (31) represented on fig. 4a and fig. 4b,
- the one extending from the extremity constituted by amino acid at position (36) to the extremity constituted by amino acid at position (55) represented on fig. 4a and fig. 4b,
- the one extending from the extremity constituted by amino acid at position (77) to the extremity constituted by amino acid at position (96) represented on fig. 4a and fig. 4b,
- the one extending from the extremity constituted by amino acid at position (101) to the extremity constituted by amino acid at position (120) represented on fig. 4a and fig. 4b,
- the one extending from the extremity constituted by amino acid at position (175) to the extremity constituted by amino acid at position (194) represented on fig. 4a and fig. 4b,
- the one extending from the extremity constituted by amino acid at position (211) to the extremity constituted by amino acid at position (230) represented on fig. 4a and fig. 4b, or
- the one extending from the extremity constituted by amino acid at position (275) to the extremity constituted by amino acid at position (294) represented on fig. 4a and fig. 4b.

44. A process for preparing a recombinant polypeptide according to claim 39, said polypeptide consisting of one of the following amino acid sequences :
- the one extending from the extremity constituted by amino acid at position (-43) to the extremity constituted by amino acid at position (295) represented on fig. 5,
- the one extending from the extremity constituted by amino acid at position (-43) to the extremity constituted by amino acid at position (-1) represented on fig. 5,
- the one extending from the extremity constituted by amino acid at position (-30) to the extremity constituted by amino acid at position (295) represented on fig. 5,
- the one extending from the extremity constituted by amino acid at position (-30) to the extremity constituted by amino acid at position (-1) represented on fig. 5,
- the one extending from the extremity constituted by amino acid at position (1) to the extremity constituted by amino acid at position (295) represented on fig. 5,
- the one extending from the extremity constituted by amino acid at position (12) to the extremity constituted by amino acid at position (31) represented on fig. 5,
- the one extending from the extremity constituted by amino acid at position (36) to the extremity constituted by amino acid at position (55) represented on fig. 5,
- the one extending from the extremity constituted by amino acid at position (77) to the extremity constituted by amino acid at position (96) represented on fig. 5,
- the one extending from the extremity constituted by amino acid at position (101) to the extremity constituted by amino acid at position (120) represented on fig. 5,
- the one extending from the extremity constituted by amino acid at position (175) to the extremity constituted by amino acid at position (194) represented on fig. 5,
- the one extending from the extremity constituted by amino acid at position (211) to the extremity constituted by amino acid at position (230) represented on fig. 5, or - the one extending from the extremity constituted by amino acid at position (275) to the extremity constituted by amino acid at position (295) represented on fig. 5.

45. A process for preparing a biologically pure recombinant polypeptide according to claim 39, said polypeptide constituted by a polypeptide according to any of claims 1 to 6, and a protein having a heterologous sequence with respect to said polypeptide, said heterologous sequence comprising from about 1 to about 1000 amino acids.

46. A process for preparing a recombinant polypeptide according to claim 45, wherein said heterologous protein is by β-galactosidase.

47. A process for preparing a nucleic acid comprising
(a) a nucleotide sequence coding for a polypeptide according to any of claims 1 to 6, or
(b) a nucleotide sequence which is complementary to (a), or
(c) the nucleotide sequences of (a) or (b) wherein T can be replaced by U, said process being a chemical synthesis process, and comprising the steps of
- synthesis of single stranded nucleic acids using the automatic beta-cyanoethyl phosphoramidite method, and
- in case of double stranded nucleic acids, combining the single stranded sense and antisense oligonucleotides by hybridization in order to form a DNA duplex, cloning the DNA duplex obtained into a suitable plasmid vector and recovery of the DNA according to classical methods, such as restriction enzyme digestion and agarose gel electrophoresis, and
- in case of nucleic acids greater than 100 nucleotides, assembling chemically synthesized oligonucleotides which are provided at their ends with different restriction sites, the sequences of which are compatible with the succession of the amino acids in the natural peptide, cloning the DNA thereby obtained into a suitable plasmid vector and recovery of the desired nucleic acid according to classical methods, such as restriction enzyme digestion and agarose gel electrophoresis.

48. A process for preparing a nucleic acid according to claim 47, said nucleic acid comprising one at least of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (1242) to the extremity constituted by nucleotide at position (1358), wherein N represents one of the five A, T, C, G or I nucleotides, represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1358) represented in fig. 3a and fig. 3b, or the complementary sequences thereof,
or the above said nucleotide sequences wherein T is replaced by U.

49. A process for preparing a nucleic acid according to claim 47, said nucleic acid comprising one at least of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (1242) to the extremity constituted by nucleotide at position (1358), wherein N represents one of the five A, T, C, G or I nucleotides, represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (1) to the extremity' constituted by nucleotide at position (1241) represented in fig. 4a and fig. 4b,
- the one extending from the extremity constituted by nucleotide at position (183) to the extremity constituted by nucleotide at position (1358) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1358) represented in fig. 4a and fig. 4b, or the complementary sequences thereof,
or the above said nucleotide sequences wherein T is replaced by U.

50. A process for preparing a nucleic acid according to claim 47, said nucleic acid comprising one at least of the following nucleotide sequences :
- the one extending from the extremity constituted by nucleotide at position (130) to the extremity constituted by nucleotide at position (219) represented in fig. 5,
- the one extending from the extremity constituted by nucleotide at position (220) to the extremity constituted by nucleotide at position (1104) represented in fig. 5,
- the one extending from the extremity constituted by nucleotide at position (1105) to the extremity constituted by nucleotide at position (1299),
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1299) represented in fig. 5,
- the one extending from the extremity constituted by nucleotide at position (90) to the extremity constituted by nucleotide at position (1104) represented in fig. 5,
- the one extending from the extremity constituted by nucleotide at position (130) to the extremity constituted by nucleotide at position (1104) represented in fig. 5,
- the one extending from the extremity constituted by nucleotide at position (130) to the extremity constituted by nucleotide at position (1299) represented in fig. 5,
- the one extending from the extremity constituted by nucleotide at position (220) to the extremity constituted by nucleotide at position (1299) represented in fig. 5, or the complementary sequences thereof,
or the above said nucleotide sequences wherein T is replaced by U.

51. A process for preparing a nucleic acid according to claim 47, said nucleic acid consisting of one of the following nucleotide sequences :
- the one extending from the extremity constituted by nucleotide at position (1) to the extremity constituted by nucleotide at position (182) represented in fig. 3a and fig. 3b,
- the one extending from the extremity constituted by nucleotide at position (1) to the extremity constituted by nucleotide at position (194) represented in fig. 3a and fig. 3b,
- the one extending from the extremity constituted by nucleotide at position (1) to the extremity constituted by nucleotide at position (212) represented in fig. 3a and fig. 3b,
- the one extending from the extremity constituted by nucleotide at position (1) to the extremity constituted by nucleotide at position (218) represented in fig. 3a and fig. 3b,
- the one extending from the extremity constituted by nucleotide at position (1) to the extremity constituted by nucleotide at position (272) represented in fig. 3a and fig. 3b,
- the one extending from the extremity constituted by nucleotide at position (1) to the extremity constituted by nucleotide at position (359) represented in fig. 3a and fig. 3b,
- the one extending from the extremity constituted by nucleotide at position (1) to the extremity constituted by nucleotide at position (1241) represented in fig. 3a and fig. 3b,
- the one extending from the extremity constituted by nucleotide at position (1) to the extremity constituted by nucleotide at position (1358) represented in fig. 3a and fig. 3b,
- the one extending from the extremity constituted by nucleotide at position (183) to the extremity constituted by nucleotide at position (359) represented in fig. 3a and fig. 3b,
- the one extending from the extremity constituted by nucleotide at position (183) to the extremity constituted by nucleotide at position (1241) represented in fig. 3a and fig. 3b,
- the one extending from the extremity constituted by nucleotide at position (183) to the extremity constituted by nucleotide at position (1358) represented in fig. 3a and fig. 3b,
- the one extending from the extremity constituted by nucleotide at position (195) to the extremity constituted by nucleotide at position (359) represented in fig. 3a and fig. 3b,
- the one extending from the extremity constituted by nucleotide at position (195) to the extremity constituted by nucleotide at position (1241) represented in fig. 3a and fig. 3b,
- the one extending from the extremity constituted by nucleotide at position (195) to the extremity constituted by nucleotide at position (1358) represented in fig. 3a and fig. 3b,
- the one extending from the extremity constituted by nucleotide at position (213) to the extremity constituted by nucleotide at position (359) represented in fig. 3a and fig. 3b,
- the one extending from the extremity constituted by nucleotide at position (213) to the extremity constituted by nucleotide at position (1241) represented in fig. 3a and fig. 3b,
- the one extending from the extremity constituted by nucleotide at position (213) to the extremity constituted by nucleotide at position (1358) represented in fig. 3a and fig. 3b,
- the one extending from the extremity constituted by nucleotide at position (219) to the extremity constituted by nucleotide at position (359) represented in fig. 3a and fig. 3b,
- the one extending from the extremity constituted by nucleotide at position (219) to the extremity constituted by nucleotide at position (1241) represented in fig. 3a and fig. 3b,
- the one extending from the extremity constituted by nucleotide at position (219) to the extremity constituted by nucleotide at position (1358) represented in fig. 3a and fig. 3b,
- the one extending from the extremity constituted by nucleotide at position (234) to the extremity constituted by nucleotide at position (359) represented in fig. 3a and fig. 3b,
- the one extending from the extremity constituted by nucleotide at position (234) to the extremity constituted by nucleotide at position (1241) represented in fig. 3a and fig. 3b,
- the one extending from the extremity constituted by nucleotide at position (234) to the extremity constituted by nucleotide at position (1358) represented in fig. 3a and fig. 3b,
- the one extending from the extremity constituted by nucleotide at position (273) to the extremity constituted by nucleotide at position (359) represented in fig. 3a and fig. 3b,
- the one extending from the extremity constituted by nucleotide at position (273) to the extremity constituted by nucleotide at position (1241) represented in fig. 3a and fig. 3b,
- the one extending from the extremity constituted by nucleotide at position (273) to the extremity constituted by nucleotide at position (1358) represented in fig. 3a and fig. 3b,
- the one extending from the extremity constituted by nucleotide at position (360) to the extremity constituted by nucleotide at position (1241) represented in fig. 3a and fig. 3b,
- the one extending from the extremity constituted by nucleotide at position (360) to the extremity constituted by nucleotide at position (1358) represented in fig. 3a and fig. 3b,
- the one extending from the extremity constituted by nucleotide at position (1242) to the extremity constituted by nucleotide at position (1358) represented in fig. 3a and fig. 3b, or the complementary sequences thereof,
or the above said nucleotide sequences wherein T is replaced by U.

52. A process for preparing a nucleic acid according to claim 47, said nucleic acid consisting of one of the following nucleotide sequences :
- the one extending from the extremity constituted by nucleotide at position (1) to the extremity constituted by nucleotide at position (182) represented in fig. 4a and fig. 4b,
- the one extending from the extremity constituted by nucleotide at position (1) to the extremity constituted by nucleotide at position (194) represented in fig. 4a and fig. 4b,
- the one extending from the extremity constituted by nucleotide at position (1) to the extremity constituted by nucleotide at position (212) represented in fig. 4a and fig. 4b,
- the one extending from the extremity constituted by nucleotide at position (1) to the extremity constituted by nucleotide at position (218) represented in fig. 4a and fig. 4b,
- the one extending from the extremity constituted by nucleotide at position (1) to the extremity constituted by nucleotide at position (272) represented in fig. 4a and fig. 4b,
- the one extending from the extremity constituted by nucleotide at position (1) to the extremity constituted by nucleotide at position (359) represented in fig. 4a and fig. 4b,
- the one extending from the extremity constituted by nucleotide at position (1) to the extremity constituted by nucleotide at position (1241) represented in fig. 4a and fig. 4b,
- the one extending from the extremity constituted by nucleotide at position (1) to the extremity constituted by nucleotide at position (1358) represented in fig. 4a and fig. 4b,
- the one extending from the extremity constituted by nucleotide at position (183) to the extremity constituted by nucleotide at position (359) represented in fig. 4a and fig. 4b,
- the one extending from the extremity constituted by nucleotide at position (183) to the extremity constituted by nucleotide at position (1241) represented in fig. 4a and fig. 4b,
- the one extending from the extremity' constituted by nucleotide at position (183) to the extremity constituted by nucleotide at position(1358) represented in fig. 4a and fig. 4b,
- the one extending from the extremity constituted by nucleotide at position (195) to the extremity constituted by nucleotide at position (359) represented in fig. 4a and fig. 4b,
- the one extending from the extremity constituted by nucleotide at position (195) to the extremity constituted by nucleotide at position (1241) represented in fig. 4a and fig. 4b,
- the one extending from the extremity constituted by nucleotide at position (195) to the extremity constituted by nucleotide at position (1358) represented in fig. 4a and fig. 4b,
- the one extending from the extremity constituted by nucleotide at position (213) to the extremity constituted by nucleotide at position (359) represented in fig. 4a and fig. 4b,
- the one extending from the extremity constituted by nucleotide at position (213) to the extremity constituted by nucleotide at position (1241) represented in fig. 4a and fig. 4b,
- the one extending from the extremity constituted by nucleotide at position (213) to the extremity constituted by nucleotide at position (1358) represented in fig. 4a and fig. 4b,
- the one extending from the extremity constituted by nucleotide at position (219) to the extremity constituted by nucleotide at position (359) represented in fig. 4a and fig. 4b,
- the one extending from the extremity constituted by nucleotide at position (219) to the extremity constituted by nucleotide at position (1241) represented in fig. 4a and fig. 4b,
- the one extending from the extremity constituted by nucleotide at position (219) to the extremity constituted by nucleotide at position (1358) represented in fig. 4a and fig. 4b,
- the one extending from the extremity constituted by nucleotide at position (234) to the extremity constituted by nucleotide at position (359) represented in fig. 4a and fig. 4b,
- the one extending from the extremity constituted by nucleotide at position (234) to the extremity constituted by nucleotide at position (1241) represented in fig. 4a and fig. 4b,
- the one extending from the extremity constituted by nucleotide at position (234) to the extremity constituted by nucleotide at position (1358) represented in fig. 4a and fig. 4b,
- the one extending from the extremity constituted by nucleotide at position (273) to the extremity constituted by nucleotide at position (359) represented in fig. 4a and fig. 4b,
- the one extending from the extremity constituted by nucleotide at position (273) to the extremity constituted by nucleotide at position (1241) represented in fig. 4a and fig. 4b,
- the one extending from the extremity constituted by nucleotide at position (273) to the extremity constituted by nucleotide at position (1358) represented in fig. 4a and fig. 4b,
- the one extending from the extremity constituted by nucleotide at position (360) to the extremity constituted by nucleotide at position (1241) represented in fig. 4a and fig. 4b,
- the one extending from the extremity constituted by nucleotide at position (360) to the extremity constituted by nucleotide at position (1358) represented in fig. 4a and fig. 4b,
- the one extending from the extremity constituted by nucleotide at position (1242) to the extremity constituted by nucleotide at position (1358) represented in fig. 4a and fig. 4b, or the complementary sequences thereof,
or the above said nucleotide sequences wherein T is replaced by U.

53. A process for preparing a nucleic acid according to claim 47, said nucleic acid consisting of one of the following nucleotide sequences :
- the one extending from the extremity constituted by nucleotide at position (1) to the extremity constituted by nucleotide at position (129) represented in fig. 5,
- the one extending from the extremity constituted by nucleotide at position (1) to the extremity constituted by nucleotide at position (219) represented in fig. 5,
- the one extending from the extremity constituted by nucleotide at position (1) to the extremity constituted by nucleotide at position (1104) represented in fig. 5,
- the one extending from the extremity constituted by nucleotide at position (1) to the extremity constituted by nucleotide at position (1299) represented in fig. 5,
- the one extending from the extremity constituted by nucleotide at position (90) to the extremity constituted by nucleotide at position (219) represented in fig. 5,
- the one extending from the extremity constituted by nucleotide at position (90) to the extremity constituted by nucleotide at position (1104) represented in fig. 5,
- the one extending from the extremity constituted by nucleotide at position (90) to the extremity constituted by nucleotide at position (1299) represented in fig. 5,
- the one extending from the extremity constituted by nucleotide at position (130) to the extremity constituted by nucleotide at position (219) represented in fig. 5,
- the one extending from the extremity constituted by nucleotide at position (130) to the extremity constituted by nucleotide at position (1104) represented in fig. 5,
- the one extending from the extremity' constituted by nucleotide at position (130) to the extremity constituted by nucleotide at position (1299) represented in fig. 5,
- the one extending from the extremity constituted by nucleotide at position (220) to the extremity constituted by nucleotide at position (1104) represented in fig. 5,
- the one extending from the extremity constituted by nucleotide at position (220) to the extremity constituted by nucleotide at position (1299) represented in fig. 5,
- the one extending from the extremity constituted by nucleotide at position (1105) to the extremity constituted by nucleotide at position (1299) represented in fig. 5, or the complementary sequences thereof,
or the above said nucleotide sequences wherein T is replaced by U.

54. A process for preparing a recombinant nucleic acid, by inserting at least one of the nucleotide sequences according to claims 9 to 15, in a heterologous nucleic acid.

55. A process for preparing a DNA or RNA primer wherein said primer is constituted by 15 to 25 nucleotides of a nucleotide sequence according to any one of claims 13 to 15, and can be used as a primer to specifically amplify any of said nucleotide sequences, and wherein said process is carried out by chemical synthesis using the automatic beta-cyanoethyl phosphoramidite method.

56. A process for preparing a DNA or RNA primer according to claim 55, with said primer being chosen from the following group of nucleotide sequences:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
or the complementary sequences thereof or the corresponding ribonucleotide sequences.

57. A process for preparing a DNA or RNA primer set for the enzymatic amplification of a nucleotide sequence according to any one of claims 13 to 15, said process comprising the step of combining in said primer set
- a A nucleotide sequence with the complementary nucleotide sequence of either B, or C, or D, or E, or F; or
- the B nucleotide sequence with the complementary nucleotide sequence of C, or D, or E, or F; or
- the C nucleotide sequence with the complementary nucleotide sequence of D, or E, or F; or
- the D nucleotide sequence with the complementary nucleotide sequence of E, or F; or
- the E nucleotide sequence with a complementary nucleotide sequence of F, with the nucleotide sequences A, B, C, D, E and F being specified in claim 18, and with A meaning any of the sequences A(i), A(ii), A(iii), A(iv), and A(v) and F meaning any of the sequences F(i), F(ii), F(iii), and F(iv).

58. A process for preparing a recombinant vector, particularly for cloning and/or expression, comprising the step of inserting a nucleic acid according to anyone of claims 9 to 15 into a vector sequence of the type plasmid, cosmid or phage, in one of the non essential sites for its replication.

59. A process for preparing a recombinant vector according to claim 58, wherein said vector contains in one of the non essential sites for its replication the necessary elements to promote the expression of a polypeptide according to anyone of claims 1 to 6 in a cellular host.

60. A process for preparing a recombinant vector according to claim 58, wherein said vector contains the elements enabling the expression by E. coli of a polypeptide according to anyone of claims 1 to 6.

61. A process for preparing a cellular host containing a recombinant vector according to anyone of claims 20 to 22, and comprising the regulation elements enabling the expression of a polypeptide according to anyone of claims 1 to 6 in this host, said process comprising the step of transforming said cellular host by said recombinant vector.

62. A process for preparing a cellular host according to claim 61, with said cellular host being chosen from among bacteria such as E. coli, transformed by a vector according to claim 20, or chosen from among eukaryotic organisms, transformed by a vector according to claim 20.

63. A process for preparing a monoclonal antibody specifically directed against a recombinant polypeptide according to anyone of claims 1 to 6, said process comprising the production of said monoclonal antibody by a hybridoma which is formed according to classical methods from splenic cells of an animal, particularly of a mouse or rat, immunized against a polypeptide according to any of claims 1 to 6 on the one hand, and of cells of a myeloma cell line on the other hand, and which is selected by its ability to produce a monoclonal antibody recognizing the polypeptide which has initially been used for the immunziation of the animals.

64. A process for preparing a nucleotide probe hybridizing with a nucleic acid according to any of claims 13 to 15, chosen from the following group of nucleotide sequences:
| Probes A(i), A(ii), A(iii), A(iv) and A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Probe B | |
|---|---|
| TCGCCCGCCCTGTACCTG, | |
| Probe C | |
|---|---|
| GCGCTGACGCTGGCGATCTATC, | |
| Probe D | |
|---|---|
| CCGCTGTTGAACGTCGGGAAG, | |
| Probe E | |
|---|---|
| AAGCCGTCGGATCTGGGTGGCAAC, | |
| Probe F(i) and F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| or their complementary nucleotide sequences, | |
wherein said process is carried out by chemical synthesis using the automatic beta-cyanoethyl phosphoramidite method.

65. A process for preparing a kit for an in vitro diagnostic method of tuberculosis in a patient liable to be infected by Mycobacterium tuberculosis according to claim 28, said process comprising the step of gathering at least the following elements
- a determined amount of a nucleotide probe according to claim 26,
- an appropiate medium for creating a hybridization reaction between the sequence to be detected and said probe,
- reagents enabling the detection of the hybridization complex formed between the nucleotide sequence and the probe.

66. A process for preparing a kit for an in vitro diagnostic method of tuberculosis in a patient liable to be infected by Mycobacterium tuberculosis according to claim 29, said process comprising the step of gathering at least the following elements
- a polypeptide according to anyone of claims 1 to 8,
- reagents for malting a medium appropriate for the immunological reaction to occur,
- reagents enabling to detect the antigen/antibody complex which has been produced by the immunological reaction, said reagents possibly having a label, or being liable to be recognized by a labelled reagent, more particularly in the case where the above mentioned polypeptide is not labelled.

67. A process for preparing a kit for an in vitro diagnostic method of tuberculosis in a patient liable to be infected by Mycobacterium tuberculosis according to claim 30, said process comprising the step of gathering at least the following elements
- an antibody according to claim 25,
- reagents for making a medium appropriate for the immunological reaction to occur,
- reagents enabling to detect the antigen/antibody complexes which have been produced by the immunological reaction, said reagents possibly having a label or being liable to be recognized by a labelled reagent, more particularly in the case where the above mentioned antibody is not labelled.

68. A process for preparing a biologically pure recombinant polypeptide according to anyone of claims 1 to 8, for use in an immunogenic composition in association with a pharmaceutically acceptable vehicle, with said process comprising the steps of
- producing the polypeptide by genetic engineering, i.e. by expression in a cellular host of the nucleic acids encoding said polypeptides or by classical chemical synthesis, and
- purifying the polypeptide produced.

69. A process for preparing a biologically pure recombinant polypeptide according to anyone of claims 1 to 8, for use in a vaccine composition, with said process comprising the steps of
- producing the polypeptide by genetic engineering, i.e. by expression in a cellular host of the nucleic acids encoding said polypeptides or by classical chemical synthesis, and
- purifying the polypeptide produced.

70. A process for preparing a peptide according to claim 1, said peptide advantageously used to produce antibodies, particularly monoclonal antibodies, and having at least one of the following amino acid sequences:
| Amino acid position (NH₂- terminal) | | Amino acid position (COOH-terminal) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |
said process comprising the steps of
- producing the peptide by genetic engineering, i.e. by expression in a cellular host of the nucleic acid encoding said peptide or by classical chemical synthesis, and
- purifying the peptide produced.

## Patentansprüche (Patentansprüche für folgende(n) Vertragsstaat(en): AT, BE, CH, LI, DE, DK, FR, GB, IT, LU, MC, NL, SE)

1. Biologisch reines rekombinantes Polypeptid, das in seiner Polypeptidkette mindestens eine der folgenden Aminosäuresequenzen enthält:
- diejenige, die sich von dem durch die Aminosäure in Position (-29) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 3a und Fig. 3b dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 3a und Fig. 3b dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 3a und Fig. 3b dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 3a und Fig. 3b dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 3a und Fig. 3b dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 3a und Fig. 3b dargestellt erstreckt, oder
- diejenige, die sich von dem durch die Aminosäure in Position (275) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (1) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-42) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-47) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-49) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-55) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-59) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-29) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-42) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-47) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-49) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-55) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-59) gebildeten äußersten Ende zu dem durch die Aminosäure in Position (294) gebildeten äußersten Ende wie in Fig. 3a und Fig. 3b dargestellt erstreckt, oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

2. Biologisch reines rekombinantes Polypeptid nach Anspruch 1, das in seiner Polypeptidkette mindestens eine der folgenden Aminosäuresequenzen enthält:
- diejenige, die sich von dem durch die Aminosäure in Position (-29) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 4a und Fig. 4b dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 4a und Fig. 4b dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 4a und Fig. 4b dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 4a und Fig. 4b dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 4a und Fig. 4b dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 4a und Fig. 4b dargestellt erstreckt, oder
- diejenige, die sich von dem durch die Aminosäure in Position (275) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (1) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-42) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-47) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-49) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-55) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-59) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-29) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-42) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-47) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-49) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-55) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-59) gebildeten äußersten Ende zu dem durch die Aminosäure in Position (294) gebildeten äußersten Ende wie in Fig. 4a und Fig. 4b dargestellt erstreckt, oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

3. Biologisch reines rekombinantes Polypeptid nach Anspruch 1, das in seiner Polypeptidkette mindestens eine der folgenden Aminosäuresequenzen enthält:
- diejenige, die sich von dem durch die Aminosäure in Position (-30) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 5 dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 5 dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 5 dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 5 dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 5 dargestellt erstreckt, oder
- diejenige, die sich von dem durch die Aminosäure in Position (-30) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (275) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (1) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (-43) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (-30) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (-43) gebildeten äußersten Ende zu dem durch die Aminosäure in Position (295) gebildeten äußersten Ende wie in Fig. 5 dargestellt erstreckt,
oder die durch Modifikation der freien Carboxyl- und/oder Aminogruppen in der Polypeptidsequenz entstandenen Peptidsequenzen und/oder die durch die Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

4. Rekombinantes Polypeptid nach Anspruch 1, bestehend aus einer der folgenden Aminosäuresequenzen:
- derjenigen, die sich von dem durch die Aminosäure in Position (-59) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-59) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-55) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-55) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-49) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-49) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-47) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-47) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-42) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-42) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-29) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-29) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (1) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 3a und Fig. 3b dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 3a und Fig. 3b dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 3a und Fig. 3b dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 3a und Fig. 3b dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 3a und Fig. 3b dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 3a und Fig. 3b dargestellt erstreckt,
- derjenigen, die sich von dem durch die Aminosäure in Position (275) gebildeten äußersten Ende zu dem durch die Aminosäure in Position (294) gebildeten äußersten Ende wie in Fig. 3a und Fig. 3b dargestellt erstreckt.

5. Rekombinantes Polypeptid nach Anspruch 2, das aus einer der folgenden Aminosäuresequenzen besteht:
- derjenigen, die sich von dem durch die Aminosäure in Position (-59) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-59) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-55) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-55) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-49) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-49) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-47) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-47) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-42) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-42) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-29) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-29) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (1) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 4a und Fig. 4b dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 4a und Fig. 4b dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 4a und Fig. 4b dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 4a und Fig. 4b dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 4a und Fig. 4b dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 5 dargestellt erstreckt,
- derjenigen, die sich von dem durch die Aminosäure in Position (275) gebildeten äußersten Ende zu dem durch die Aminosäure in Position (294) gebildeten äußersten Ende wie in Fig. 5 dargestellt erstreckt.

6. Rekombinantes Polypeptid nach Anspruch 3, das aus einer der folgenden Aminosäuresequenzen besteht:
- derjenigen, die sich von dem durch die Aminosäure in Position (-43) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (-43) gebildeten äußersten Ende zu dem durch die Aminosäure in Position (-1) gebildeten äußersten Ende wie in Fig. 5 dargestellt erstreckt,
- derjenigen, die sich von dem durch die Aminosäure in Position (-30) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (-30) gebildeten äußersten Ende zu dem durch die Aminosäure in Position (-1) gebildeten äußersten Ende wie in Fig. 5 dargestellt erstreckt,
- derjenigen, die sich von dem durch die Aminosäure in Position (1) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 5 dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 5 dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 5 dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 5 dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 5 dargestellt erstreckt.
- derjenigen, die sich von dem durch die Aminosäure in Position (211) gebildeten äußersten Ende zu dem durch die Aminosäure in Position (230) gebildeten äußersten Ende wie in Fig. 5 dargestellt erstreckt.
- derjenigen, die sich von dem durch die Aminosäure in Position (275) gebildeten äußersten Ende zu dem durch die Aminosäure in Position (295) gebildeten äußersten Ende wie in Fig. 5 dargestellt erstreckt.

7. Biologisch reines rekombinantes Polypeptid, dargestellt durch ein Polypeptid nach einem der Ansprüche 1 bis 6 und einem Protein mit einer heterologen Sequenz in Bezug auf dieses Polypeptid, wobei die heterologe Sequenz ungefähr 1 bis ungefähr 1000 Aminosäuren umfaßt.

8. Rekombinantes Polypeptid nach Anspruch 7, wobei es sich bei dem heterologen Protein um β-Galactosidase handelt.

9. Nucleinsäure mit
(a) einer Nucleotidsequenz, die für ein Polypeptid nach einem der Ansprüche 1 bis 6 kodiert oder
(b) einer Nucleotidsequenz, die zu (a) komplementär ist, oder
(c) den Nucleotidsequenzen von (a) oder (b), wobei T durch U ersetzt sein kann.

10. Nucleinsäure mit mindestens einer der folgenden Nucleotidsequenzen:
- derjenigen, die sich von dem durch das Nucleotid in Position (1) gebildeten äußersten Ende zu dem durch das Nucleotid in Position (182) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 3a und Fig. 3b dargestellt erstreckt, wobei N eines der fünf Nucleotide A, T, C, G oder I darstellt,
- derjenigen, die sich von dem durch das Nucleotid in Position (1) gebildeten äußersten Ende zu dem durch das Nucleotid in Position (194) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 3a und Fig. 3b dargestellt erstreckt, oder deren komplementären Sequenzen,
oder den oben genannten Nucleotidsequenzen, wobei T durch U ersetzt ist.

11. Nucleinsäure nach Anspruch 10 mit mindestens einer der folgenden Nucleotidsequenzen:
- derjenigen, die sich von dem durch das Nucleotid in Position (1) gebildeten äußersten Ende zu dem durch das Nucleotid in Position (182) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 4a und Fig. 4b dargestellt erstreckt, wobei N eines der fünf Nucleotide A, T, C, G oder I darstellt,
- derjenigen, die sich von dem durch das Nucleotid in Position (1) gebildeten äußersten Ende zu dem durch das Nucleotid in Position (194) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 4a und Fig. 4b dargestellt erstreckt, oder deren komplementären Sequenzen,
oder den oben genannten Nucleotidsequenzen, wobei T durch U ersetzt ist.

12. Nucleinsäure nach Anspruch 10 mit mindestens einer der folgenden Nucleotidsequenzen:
- derjenigen, die sich von dem durch das Nucleotid in Position (130) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1105) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (129) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1299) gebildeten äußersten Ende wie in Fig. 5 dargestellt erstreckt,
oder deren komplementären Sequenzen,
oder den oben genannten Nucleotidsequenzen, wobei T durch U ersetzt ist.

13. Nucleinsäure nach Anspruch 10, das aus einer der folgenden Nucleotidsequenzen besteht:
- derjenigen, die sich von dem durch das Nucleotid in Position (1) gebildeten äußersten Ende zu dem durch das Nucleotid in Position (182) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (194) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 3a und Fig. 3b dargestellt erstreckt, oder deren komplementären Sequenzen,
oder den oben genannten Nucleotidsequenzen, wobei T durch U ersetzt ist.

14. Nucleinsäure nach Anspruch 11 bestehend aus einer der folgenden Nucleotidsequenzen:
- derjenigen, die sich von dem durch das Nucleotid in Position (1) gebildeten äußersten Ende zu dem durch das Nucleotid in Position (182) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (194) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 4a und Fig. 4b dargestellt erstreckt, oder deren komplementären Sequenzen,
oder den oben genannten Nucleotidsequenzen, wobei T durch U ersetzt ist.

15. Nucleinsäure nach Anspruch 12 bestehend aus einer der folgenden Nucleotidsequenzen:
- derjenigen, die sich von dem durch das Nucleotid in Position (1) gebildeten äußersten Ende zu dem durch das Nucleotid in Position (129) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (1105) gebildeten äußersten Ende zu dem durch das Nucleotid in Position (1299) gebildeten äußersten Ende wie in Fig. 5 dargestellt erstreckt,
oder deren komplementären Sequenzen,
oder den oben genannten Nucleotidsequenzen, wobei T durch U ersetzt ist.

16. Rekombinante Nucleinsäure mit mindestens einer der Nucleotidsequenzen nach Anspruch 9 bis 15 als Insertion in eine heterologe Nucleinsäure.

17. DNA- oder RNA-Primer aus 15 bis 25 Nucleotiden einer Nucleotidsequenz nach einem der Ansprüche 13 bis 15, der als Primer für die spezifische Amplifikation einer der beliebigen Nucleotidsequenzen verwendet werden kann.

18. DNA- oder RNA-Primer nach Anspruch 17, wobei dieser Primer aus der folgenden Gruppe von Nucleotidsequenzen ausgewählt ist:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCAACACCGGGCCCGCGCCCCA,
oder deren komplementären Sequenzen oder den entsprechenden Ribonucleotidsequenzen.

19. DNA- oder RNA-Primer-Satz für die enzymatische Amplifizierung einer Nucleotidsequenz nach einem der Ansprüche 13 bis 15, wobei dieser Primer-Satz folgendes umfaßt:
- eine Nucleotidsequenz A in Kombination mit der komplementären Nucleotidsequenz von entweder B oder C oder D oder E oder F; oder
- die Nucleotidsequenz B in Kombination mit der komplementären Nucleotidsequenz von C oder D oder E oder F; oder
- die Nucleotidsequenz C in Kombination mit der komplementären Nucleotidsequenz von D oder E oder F; oder
- die Nucleotidsequenz D in Kombination mit der komplementären Nucleotidsequenz von E oder F; oder
- die Nucleotidsequenz E in Kombination mit einer komplementären Nucleotidsequenz von F,
wobei die Nucleotidsequenzen A, B, C, D, E und F in Anspruch 18 beschrieben sind und wobei A eine beliebige der Sequenzen A(i), A(ii), A(iii), A(iv) und A(v) und F eine beliebige der Sequenzen F(i), F(ii), F(iii) und F(iv) bedeutet.

20. Rekombinanter Vektor, insbesondere für das Klonieren und/oder die Expression, mit einer Vektorsequenz vom Plasmid-, Cosmid- oder Phagentyp und einer Nucleinsäure nach einem der Ansprüche 9 bis 15 in einer der für seine Replikation unwesentlichen Stellen.

21. Rekombinanter Vektor nach Anspruch 20, enthaltend in einer der für seine Replikation unwesentlichen Stellen die zur Promotion der Expression eines Polypeptids nach einem der Ansprüche 1 bis 6 in einer Wirtszelle erforderlichen Elemente.

22. Rekombinanter Vektor nach Anspruch 21, enthaltend die Elemente, die die Expression eines Polypeptids nach einem der Ansprüche 1 bis 6 durch E. coli gestatten.

23. Wirtszelle enthaltend einen rekombinanten Vektor nach einem der Ansprüche 20 bis 22, und mit den die Expression eines Polypeptids nach einem der Ansprüche 1 bis 6 in diesem Wirt gestattenden Regulationselementen.

24. Wirtszelle nach Anspruch 23, ausgewählt aus der Gruppe der Bakterien, wie E. coli, die mit einem Vektor nach Anspruch 20 transformiert sind, oder aus der Gruppe der Eukaryonten, die mit einem Vektor nach Anspruch 20 transformiert sind.

25. Monoklonaler Antikörper, der spezifisch gegen ein rekombinantes Polypeptid nach einem der Ansprüche 1 bis 6 gerichtet ist.

26. Nucleotidsonde, die mit einer Nucleinsäure nach einem der Ansprüche 13 bis 15 hybridisiert, ausgewählt aus der folgenden Gruppe von Nucleotidsequenzen:
| Sonden A(i), A(ii), A(iii), A(iv) und A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Sonde B | |
|---|---|
| TCGCCCGCCCTGTACCTG, | |
| Sonde C | |
|---|---|
| GCGCTGACGCTGGCGATCTATC, | |
| Sonde D | |
|---|---|
| CCGCTGTTGAACGTCGGGAAG, | |
| Sonde E | |
|---|---|
| AAGCCGTCGGATCTGGGTGGCAAC, | |
| Sonde F(i) und F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| oder ihren komplementären Nucleotidsequenzen. | |

27. Verfahren zur Herstellung eines rekombinanten Polypeptids nach einem der Ansprüche 1 bis 8 mit den folgenden Schritten:
- Kultur in einem geeigneten Medium einer Wirtszelle, die zuvor mit einem geeigneten Vektor, der eine Nucleinsäure nach einem der Ansprüche 9 bis 15 enthält, transformiert worden ist, sowie
- Gewinnung des von der genannten transformierten Wirtszelle produzierten Polypeptids aus dem genannten Kulturmedium.

28. Verfahren für die in-vitro-Diagnose von Tuberkulose bei einem Patienten, der eventuell durch Mycobacterium tuberculosis infiziert ist, mit den folgenden Schritten:
- Amplifizierung einer Nucleinsäure nach einem der Ansprüche 9 bis 15, die sich gegebenenfalls in einer von diesem Patienten stammenden biologischen Probe befindet mittels eines Primers nach einem der Ansprüche 17 bis 18, und/oder
- Kontaktieren der genannten biologischen Probe mit einer Nucleotidsonde nach Anspruch 26 unter Bedingungen, die die Bildung eines Hybridisierungskomplexes zwischen der Sonde und der Nucleinsäure der Probe gestattet, sowie
- Nachweisen des genannten Hybridisierungskomplexes.

29. Verfahren für die in-vitro-Diagnose von Tuberkulose bei einem Patienten, der eventuell durch Mycobacterium tuberculosis infiziert ist, mit den Schritten:
- Kontaktieren einer von einem Patienten stammenden biologischen Probe mit einem Polypeptid nach einem der Ansprüche 1 bis 8 unter Bedingungen, die eine in-vitro-Immunreaktion zwischen dem Polypeptid und den Antikörpern, die eventuell in dieser biologischen Probe vorhanden sind, gestatten, sowie
- in-vitro-Nachweis des eventuell gebildeten Antigen-Antikörper-Komplexes.

30. Verfahren für die in-vitro-Diagnose von Tuberkulose bei einem Patienten, der eventuell durch M. tuberculosis infiziert ist, mit den folgenden Schritten:
- Kontaktieren der biologischen Probe mit einem geeigneten Antikörper nach Anspruch 25 unter Bedingungen, die eine in-vitro-Immunreaktion zwischen dem Antikörper und den möglicherweise in der biologischen Probe vorhandenen M. tuberculosis-Antigenen gestattet, sowie
- in-vitro-Nachweis des eventuell gebildeten Antigen-Antikörper-Komplexes.

31. Kit für ein in-vitro-Diagnoseverfahren für Tuberkulose bei einem Patienten, der eventuell durch Mycobacterium tuberculosis infiziert ist, nach Anspruch 28, wobei dieses Kit eine bestimmte Menge einer Nucleotidsonde nach Anspruch 26 umfaßt.

32. Kit für ein in-vitro-Diagnoseverfahren für Tuberkulose bei einem Patienten, der eventuell durch Mycobacterium tuberculosis infiziert ist, nach Anspruch 29 mit
- einem Polypeptid nach einem der Ansprüche 1 bis 8,
- Reagentien zur Herstellung eines Mediums, das sich für das Ablaufen der Immunreaktion eignet,
- Reagentien, die den Nachweis des durch die Immunreaktion produzierten Antigen-Antikörper-Komplexes gestattet, wobei diese Reagentien gegebenenfalls markiert sind oder mit einem markierten Reagens erkannt werden können, insbesondere dann, wenn das genannte Polypeptid nicht markiert ist.

33. Kit für ein in-vitro-Diagnoseverfahren für Tuberkulose bei einem Patienten, der eventuell durch Mycobacterium tuberculosis infiziert ist, nach Anspruch 30 mit
- einem Antikörper nach Anspruch 25,
- Reagentien zur Herstellung eines Mediums, das sich für das Ablaufen der Immunreaktion eignet,
- Reagentien, die den Nachweis des durch die Immunreaktion produzierten Antigen-Antikörper-Komplexes gestattet, wobei diese Reagentien gegebenenfalls markiert sind oder mit einem markierten Reagens erkannt werden können, insbesondere dann, wenn das genannte Antikörper nicht markiert ist.

34. Biologisch reines rekombinantes Polypeptid nach einem der Ansprüche 1 bis 8 zur Verwendung in einer immunogenen Zusammensetzung in Kombination mit einem pharmazeutisch unbedenklichen Konstituens.

35. Biologisch reines rekombinantes Polypeptid nach einem der Ansprüche 1 bis 8 zur Verwendung in einer Impfstoffzusammensetzung.

36. Verfahren zur enzymatischen Amplifizierung einer Nucleotidsequenz nach einem der Ansprüche 13 bis 15 und den Nachweis der amplifizierten Nucleotidsequenz, wobei mittels PCR-Technik mit einem Primer-Satz amplifiziert wird und das PCR-amplifizierte Produkt durch eine Hybridisierungsreaktion mit einer Nachweissonde nachgewiesen wird, wobei der Primer-Satz und die Nachweisprobe aus den folgenden Elementen ausgewählt sind: 
Primer-Satz
P1 GAGTACCTGCAGGTGCCGTCGCCGTCGATGGGCCG
P2 kompl. GTACCACTCGAACGCCGGGTGTTGAT
Sonde B
TCGCCCGCCCTGTACCTG

Primer-Satz
P1 GAGTACCTGCAGGTGCCGTCGCCGTCGATGGGCCG
P3 kompl. TCCCACTTGTAAGTCTGGCA
Sonde B
TCGCCCGCCCTGTACCTG

Primer-Satz
P1 GAGTACCTGCAGGTGCCGTCGCCGTCGATGGGCCG
P4 kompl. CGGCAGCTCGCTGGTCAGGA
Sonde B
TCGCCCGCCCTGTACCTG

Primer-Satz
P1 GAGTACCTGCAGGTGCCGTCGCCGTCGATGGGCCG
P5 kompl. GCGTCACCCATCGCCAGGCCGATCAGG
Sonde B
TCGCCCGCCCTGTACCTG oder
Sonde C
GCGCTGACGCTGGCGATCTATC
 
Primer-Satz
P1        GAGTACCTGCAGGTGCCGTCGCCGTCGATGGGCCG
P6 kompl. GCGCCCCAGTACTCCCAGCTGTGCGT
Sonde B
TCGCCCGCCCTGTACCTG oder
Sonde C
GCGCTGACGCTGGCGATCTATC oder
Sonde D
CCGCTGTTGAACGTCGGGAAG oder
Sonde E
AAGCCGTCGGATCTGGGTGGCAAC

Primer-Satz
P2        ATCAACACCCCGGCGTTCGAGTGGTAC
P5 kompl. GCGTCACCCATCGCCAGGCCGATCAGG
Sonde C
CGCGTGACGCTGGCGATCTATC

Primer-Satz
P2        ATCAACACCCCGGCGTTCGAGTGGTAC
P6 kompl. GCGCCCCAGTACTCCCAGCTGTGCGT
Sonde C
GCGCTGACGCTGGCGATCTATC oder
Sonde D
CCGCTGTTGAACGTCGGGAAG oder
Sonde E
AAGCCGTCGGATCTGGGTGGCAAC

Primer-Satz
P3        TGCCAGACTTACAAGTGGGA
P5 kompl. GCGTCACCCATCGCCAGGCCGATCAGG
Sonde C
GCGCTGACGCTGGCGATCTATC

Primer-Satz
P3        TGCCAGACTTACAAGTGGGA
P6 kompl. GCGCCCCAGTACTCCCAGCTGTGCGT
Sonde C
GCGCTGACGCTGGCGATCTATC oder
 
Sonde D
CCGCTGTTGAACGTCGGGAAG oder
Sonde E
AAGCCGTCGGATCTGGGTGGCAAC

Primer-Satz
P4 TCCTGACCAGCGAGCTGCCG
P5 kompl. GCGTCACCCATCGCCAGGCCGATCAGG
Sonde C
GCGCTGACGCTGGCGATCTATC

Primer-Satz
P4 TCCTGACCAGCGAGCTGCCG
P6 kompl. GCGCCCCAGTACTCCCAGCTGTGCGT
Sonde C
GCGCTGACGCTGGCGATCTATC oder
Sonde D
CCGCTGTTGAACGTCGGGAAG oder
Sonde E
AAGCCGTCGGATCTGGGTGGCAAC

Primer-Satz
P5 CCTGATCGGCCTGGCGATGGGTGACGC
P6 kompl. GCGCCCCAGTACTCCCAGCTGTGCGT
Sonde D
CCGCTGTTGAACGTCGGGAAG oder
Sonde E
AAGCCGTCGGATCTGGGTGGCAAC
 oder wobei dieser Primer-Satz aus den Primer-Sätzen nach Anspruch 19 ausgewählt ist und die Nachweissonde aus einer beliebigen Oligonucleotidsequenz mit mindestens 10 Nucleotiden besteht, wobei sich diese Sequenz zwischen den beiden PCR-Primern befindet, die den für die Amplifizierung der Nucleotidsequenz verwendeten Primer-Satz darstellen.

37. Rekombinanter Vektor nach Anspruch 20, wobei die Vektorsequenz entweder die in Fig. 10b dargestellte Nucleinsäuresequenz oder die in Fig. 11b dargestellte Nucleinsäuresequenz oder die in Fig. 12b dargestellte Nucleinsäuresequenz aufweist.

38. Peptide nach Anspruch 1, die vorteilhaft zur Produktion von Antikörpern, insbesondere monoklonalen Antikörpern verwendet werden und die folgenden Aminosäuresequenzen aufweisen:
| Aminosäure in position (NH₂-Ende) | | Aminosäure in position (COOH-Ende) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |

## Patentansprüche (Patentansprüche für folgende(n) Vertragsstaat(en): ES, GR)

1. Biologisch reines rekombinantes Polypeptid, das in seiner Polypeptidkette mindestens eine der folgenden Aminosäuresequenzen enthält:
- diejenige, die sich von dem durch die Aminosäure in Position (-29) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 3a und Fig. 3b dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 3a und Fig. 3b dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 3a und Fig. 3b dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 3a und Fig. 3b dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 3a und Fig. 3b dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 3a und Fig. 3b dargestellt erstreckt, oder
- diejenige, die sich von dem durch die Aminosäure in Position (275) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (1) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-42) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-47) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-49) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-55) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-59) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (-29) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-42) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-47) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-49) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-55) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (-59) gebildeten äußersten Ende zu dem durch die Aminosäure in Position (294) gebildeten äußersten Ende wie in Fig. 3a und Fig. 3b dargestellt erstreckt, oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

2. Biologisch reines rekombinantes Polypeptid nach Anspruch 1, das in seiner Polypeptidkette mindestens eine der folgenden Aminosäuresequenzen enthält:
- diejenige, die sich von dem durch die Aminosäure in Position (-29) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 4a und Fig. 4b dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 4a und Fig. 4b dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 4a und Fig. 4b dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 4a und Fig. 4b dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 4a und Fig. 4b dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 4a und Fig. 4b dargestellt erstreckt, oder
- diejenige, die sich von dem durch die Aminosäure in Position (275) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (1) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-42) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-47) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-49) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-55) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-59) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (-29) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-42) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-47) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-49) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-55) gebildeten äußersten Ende zu dem durch die Aminosäure in Position (294) gebildeten äußersten Ende wie in Fig. 4a und Fig. 4b dargestellt erstreckt, oder
- diejenige, die sich von dem durch die Aminosäure in Position (-59) gebildeten äußersten Ende zu dem durch die Aminosäure in Position (294) gebildeten äußersten Ende wie in Fig. 4a und Fig. 4b dargestellt erstreckt, oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

3. Biologisch reines rekombinantes Polypeptid nach Anspruch 1, das in seiner Polypeptidkette mindestens eine der folgenden Aminosäuresequenzen enthält:
- diejenige, die sich von dem durch die Aminosäure in Position (-30) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 5 dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 5 dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 5 dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 5 dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 5 dargestellt erstreckt, oder
- diejenige, die sich von dem durch die Aminosäure in Position (-30) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (275) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (1) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (-43) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (-30) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (-43) gebildeten äußersten Ende zu dem durch die Aminosäure in Position (295) gebildeten äußersten Ende wie in Fig. 5 dargestellt erstreckt,
oder die durch Modifikation der freien Carboxyl- und/oder Aminogruppen in der Polypeptidsequenz entstandenen Peptidsequenzen und/oder die durch die Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

4. Rekombinantes Polypeptid nach Anspruch 1, bestehend aus einer der folgenden Aminosäuresequenzen:
- derjenigen, die sich von dem durch die Aminosäure in Position (-59) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-59) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-55) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-55) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-49) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-49) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-47) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-47) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-42) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-42) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-29) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-29) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (1) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 3a und Fig. 3b dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 3a und Fig. 3b dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 3a und Fig. 3b dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 3a und Fig. 3b dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 3a und Fig. 3b dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 3a und Fig. 3b dargestellt erstreckt,
- derjenigen, die sich von dem durch die Aminosäure in Position (275) gebildeten äußersten Ende zu dem durch die Aminosäure in Position (294) gebildeten äußersten Ende wie in Fig. 3a und Fig. 3b dargestellt erstreckt.

5. Rekombinantes Polypeptid nach Anspruch 2, das aus einer der folgenden Aminosäuresequenzen besteht:
- derjenigen, die sich von dem durch die Aminosäure in Position (-59) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-59) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-55) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-55) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-49) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-49) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-47) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-47) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-42) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-42) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-29) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-29) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (1) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 4a und Fig. 4b dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 4a und Fig. 4b dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 4a und Fig. 4b dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 4a und Fig. 4b dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 4a und Fig. 4b dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 5 dargestellt erstreckt,
- derjenigen, die sich von dem durch die Aminosäure in Position (275) gebildeten äußersten Ende zu dem durch die Aminosäure in Position (294) gebildeten äußersten Ende wie in Fig. 5 dargestellt erstreckt.

6. Rekombinantes Polypeptid nach Anspruch 3, das aus einer der folgenden Aminosäuresequenzen besteht:
- derjenigen, die sich von dem durch die Aminosäure in Position (-43) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (-43) gebildeten äußersten Ende zu dem durch die Aminosäure in Position (-1) gebildeten äußersten Ende wie in Fig. 5 dargestellt erstreckt,
- derjenigen, die sich von dem durch die Aminosäure in Position (-30) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (-30) gebildeten äußersten Ende zu dem durch die Aminosäure in Position (-1) gebildeten äußersten Ende wie in Fig. 5 dargestellt erstreckt,
- derjenigen, die sich von dem durch die Aminosäure in Position (1) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 5 dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 5 dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 5 dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 5 dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 5 dargestellt erstreckt.
- derjenigen, die sich von dem durch die Aminosäure in Position (211) gebildeten äußersten Ende zu dem durch die Aminosäure in Position (230) gebildeten äußersten Ende wie in Fig. 5 dargestellt erstreckt.
- derjenigen, die sich von dem durch die Aminosäure in Position (275) gebildeten äußersten Ende zu dem durch die Aminosäure in Position (295) gebildeten äußersten Ende wie in Fig. 5 dargestellt erstreckt.

7. Biologisch reines rekombinantes Polypeptid, dargestellt durch ein Polypeptid nach einem der Ansprüche 1 bis 6 und einem Protein mit einer heterologen Sequenz in Bezug auf dieses Polypeptid, wobei die heterologe Sequenz ungefähr 1 bis ungefähr 1000 Aminosäuren umfaßt.

8. Rekombinantes Polypeptid nach Anspruch 7, wobei es sich bei dem heterologen Protein um β-Galactosidase handelt.

9. Nucleinsäure mit
(a) einer Nucleotidsequenz, die für ein Polypeptid nach einem der Ansprüche 1 bis 6 kodiert oder
(b) einer Nucleotidsequenz, die zu (a) komplementär ist, oder
(c) den Nucleotidsequenzen von (a) oder (b), wobei T durch U ersetzt sein kann.

10. Nucleinsäure mit mindestens einer der folgenden Nucleotidsequenzen:
- derjenigen, die sich von dem durch das Nucleotid in Position (1) gebildeten äußersten Ende zu dem durch das Nucleotid in Position (182) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 3a und Fig. 3b dargestellt erstreckt, wobei N eines der fünf Nucleotide A, T, C, G oder I darstellt,
- derjenigen, die sich von dem durch das Nucleotid in Position (1) gebildeten äußersten Ende zu dem durch das Nucleotid in Position (194) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 3a und Fig. 3b dargestellt erstreckt, oder deren komplementären Sequenzen,
oder den oben genannten Nucleotidsequenzen, wobei T durch U ersetzt ist.

11. Nucleinsäure nach Anspruch 10 mit mindestens einer der folgenden Nucleotidsequenzen:
- derjenigen, die sich von dem durch das Nucleotid in Position (1) gebildeten äußersten Ende zu dem durch das Nucleotid in Position (182) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 4a und Fig. 4b dargestellt erstreckt, wobei N eines der fünf Nucleotide A, T, C, G oder I darstellt,
- derjenigen, die sich von dem durch das Nucleotid in Position (1) gebildeten äußersten Ende zu dem durch das Nucleotid in Position (194) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 4a und Fig. 4b dargestellt erstreckt, oder deren komplementären Sequenzen,
oder den oben genannten Nucleotidsequenzen, wobei T durch U ersetzt ist.

12. Nucleinsäure nach Anspruch 10 mit mindestens einer der folgenden Nucleotidsequenzen:
- derjenigen, die sich von dem durch das Nucleotid in Position (130) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1105) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (129) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1299) gebildeten äußersten Ende wie in Fig. 5 dargestellt erstreckt,
oder deren komplementären Sequenzen,
oder den oben genannten Nucleotidsequenzen, wobei T durch U ersetzt ist.

13. Nucleinsäure nach Anspruch 10, das aus einer der folgenden Nucleotidsequenzen besteht:
- derjenigen, die sich von dem durch das Nucleotid in Position (1) gebildeten äußersten Ende zu dem durch das Nucleotid in Position (182) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (194) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 3a und Fig. 3b dargestellt erstreckt, oder deren komplementären Sequenzen,
oder den oben genannten Nucleotidsequenzen, wobei T durch U ersetzt ist.

14. Nucleinsäure nach Anspruch 11 bestehend aus einer der folgenden Nucleotidsequenzen:
- derjenigen, die sich von dem durch das Nucleotid in Position (1) gebildeten äußersten Ende zu dem durch das Nucleotid in Position (182) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (194) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 4a und Fig. 4b dargestellt erstreckt, oder deren komplementären Sequenzen,
oder den oben genannten Nucleotidsequenzen, wobei T durch U ersetzt ist.

15. Nucleinsäure nach Anspruch 12 bestehend aus einer der folgenden Nucleotidsequenzen:
- derjenigen, die sich von dem durch das Nucleotid in Position (1) gebildeten äußersten Ende zu dem durch das Nucleotid in Position (129) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (1105) gebildeten äußersten Ende zu dem durch das Nucleotid in Position (1299) gebildeten äußersten Ende wie in Fig. 5 dargestellt erstreckt,
oder deren komplementären Sequenzen,
oder den oben genannten Nucleotidsequenzen, wobei T durch U ersetzt ist.

16. Rekombinante Nucleinsäure mit mindestens einer der Nucleotidsequenzen nach Anspruch 9 bis 15 als Insertion in eine heterologe Nucleinsäure.

17. DNA- oder RNA-Primer aus 15 bis 25 Nucleotiden einer Nucleotidsequenz nach einem der Ansprüche 13 bis 15, der als Primer für die spezifische Amplifikation einer der beliebigen Nucleotidsequenzen verwendet werden kann.

18. DNA- oder RNA-Primer nach Anspruch 17, wobei dieser Primer aus der folgenden Gruppe von Nucleotidsequenzen ausgewählt ist:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCAACACCGGGCCCGCGCCCCA,
oder deren komplementären Sequenzen oder den entsprechenden Ribonucleotidsequenzen.

19. DNA- oder RNA-Primer-Satz für die enzymatische Amplifizierung einer Nucleotidsequenz nach einem der Ansprüche 13 bis 15, wobei dieser Primer-Satz folgendes umfaßt:
- eine Nucleotidsequenz A in Kombination mit der komplementären Nucleotidsequenz von entweder B oder C oder D oder E oder F; oder
- die Nucleotidsequenz B in Kombination mit der komplementären Nucleotidsequenz von C oder D oder E oder F; oder
- die Nucleotidsequenz C in Kombination mit der komplementären Nucleotidsequenz von D oder E oder F; oder
- die Nucleotidsequenz D in Kombination mit der komplementären Nucleotidsequenz von E oder F; oder
- die Nucleotidsequenz E in Kombination mit einer komplementären Nucleotidsequenz von F,
wobei die Nucleotidsequenzen A, B, C, D, E und F in Anspruch 18 beschrieben sind und wobei A eine beliebige der Sequenzen A(i), A(ii), A(iii), A(iv) und A(v) und F eine beliebige der Sequenzen F(i), F(ii), F(iii) und F(iv) bedeutet.

20. Rekombinanter Vektor, insbesondere für das Klonieren und/oder die Expression, mit einer Vektorsequenz vom Plasmid-, Cosmid- oder Phagentyp und einer Nucleinsäure nach einem der Ansprüche 9 bis 15 in einer der für seine Replikation unwesentlichen Stellen.

21. Rekombinanter Vektor nach Anspruch 20, enthaltend in einer der für seine Replikation unwesentlichen Stellen die zur Promotion der Expression eines Polypeptids nach einem der Ansprüche 1 bis 6 in einer Wirtszelle erforderlichen Elemente.

22. Rekombinanter Vektor nach Anspruch 21, enthaltend die Elemente, die die Expression eines Polypeptids nach einem der Ansprüche 1 bis 6 durch E. coli gestatten.

23. Wirtszelle enthaltend einen rekombinanten Vektor nach einem der Ansprüche 20 bis 22, und mit den die Expression eines Polypeptids nach einem der Ansprüche 1 bis 6 in diesem Wirt gestattenden Regulationselementen.

24. Wirtszelle nach Anspruch 23, ausgewählt aus der Gruppe der Bakterien, wie E. coli, die mit einem Vektor nach Anspruch 20 transformiert sind, oder aus der Gruppe der Eukaryonten, die mit einem Vektor nach Anspruch 20 transformiert sind.

25. Monoklonaler Antikörper, der spezifisch gegen ein rekombinantes Polypeptid nach einem der Ansprüche 1 bis 6 gerichtet ist.

26. Nucleotidsonde, die mit einer Nucleinsäure nach einem der Ansprüche 13 bis 15 hybridisiert, ausgewählt aus der folgenden Gruppe von Nucleotidsequenzen:
| Sonden A(i), A(ii), A(iii), A(iv) und A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Sonde B | |
|---|---|
| TCGCCCGCCCTGTACCTG, | |
| Sonde C | |
|---|---|
| GCGCTGACGCTGGCGATCTATC, | |
| Sonde D | |
|---|---|
| CCGCTGTTGAACGTCGGGAAG, | |
| Sonde E | |
|---|---|
| AAGCCGTCGGATCTGGGTGGCAAC, | |
| Sonde F(i) und F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| oder ihren komplementären Nucleotidsequenzen. | |

27. Verfahren zur Herstellung eines rekombinanten Polypeptids nach einem der Ansprüche 1 bis 8 mit den folgenden Schritten:
- Kultur in einem geeigneten Medium einer Wirtszelle, die zuvor mit einem geeigneten Vektor, der eine Nucleinsäure nach einem der Ansprüche 9 bis 15 enthält, transformiert worden ist, sowie
- Gewinnung des von der genannten transformierten Wirtszelle produzierten Polypeptids aus dem genannten Kulturmedium.

28. Verfahren für die in-vitro-Diagnose von Tuberkulose bei einem Patienten, der eventuell durch Mycobacterium tuberculosis infiziert ist, mit den folgenden Schritten:
- Amplifizierung einer Nucleinsäure nach einem der Ansprüche 9 bis 15, die sich gegebenenfalls in einer von diesem Patienten stammenden biologischen Probe befindet mittels eines Primers nach einem der Ansprüche 17 bis 18, und/oder
- Kontaktieren der genannten biologischen Probe mit einer Nucleotidsonde nach Anspruch 26 unter Bedingungen, die die Bildung eines Hybridisierungskomplexes zwischen der Sonde und der Nucleinsäure der Probe gestattet, sowie
- Nachweisen des genannten Hybridisierungskomplexes.

29. Verfahren für die in-vitro-Diagnose von Tuberkulose bei einem Patienten, der eventuell durch Mycobacterium tuberculosis infiziert ist, mit den Schritten:
- Kontaktieren einer von einem Patienten stammenden biologischen Probe mit einem Polypeptid nach einem der Ansprüche 1 bis 8 unter Bedingungen, die eine in-vitro-Immunreaktion zwischen dem Polypeptid und den Antikörpern, die eventuell in dieser biologischen Probe vorhanden sind, gestatten, sowie
- in-vitro-Nachweis des eventuell gebildeten Antigen-Antikörper-Komplexes.

30. Verfahren für die in-vitro-Diagnose von Tuberkulose bei einem Patienten, der eventuell durch M. tuberculosis infiziert ist, mit den folgenden Schritten:
- Kontaktieren der biologischen Probe mit einem geeigneten Antikörper nach Anspruch 25 unter Bedingungen, die eine in-vitro-Immunreaktion zwischen dem Antikörper und den möglicherweise in der biologischen Probe vorhandenen M. tuberculosis-Antigenen gestattet, sowie
- in-vitro-Nachweis des eventuell gebildeten Antigen-Antikörper-Komplexes.

31. Kit für ein in-vitro-Diagnoseverfahren für Tuberkulose bei einem Patienten, der eventuell durch Mycobacterium tuberculosis infiziert ist, nach Anspruch 28, wobei dieses Kit eine bestimmte Menge einer Nucleotidsonde nach Anspruch 26 umfaßt.

32. Kit für ein in-vitro-Diagnoseverfahren für Tuberkulose bei einem Patienten, der eventuell durch Mycobacterium tuberculosis infiziert ist, nach Anspruch 29 mit
- einem Polypeptid nach einem der Ansprüche 1 bis 8,
- Reagentien zur Herstellung eines Mediums, das sich für das Ablaufen der Immunreaktion eignet,
- Reagentien, die den Nachweis des durch die Immunreaktion produzierten Antigen-Antikörper-Komplexes gestattet, wobei diese Reagentien gegebenenfalls markiert sind oder mit einem markierten Reagens erkannt werden können, insbesondere dann, wenn das genannte Polypeptid nicht markiert ist.

33. Kit für ein in-vitro-Diagnoseverfahren für Tuberkulose bei einem Patienten, der eventuell durch Mycobacterium tuberculosis infiziert ist, nach Anspruch 30 mit
- einem Antikörper nach Anspruch 25,
- Reagentien zur Herstellung eines Mediums, das sich für das Ablaufen der Immunreaktion eignet,
- Reagentien, die den Nachweis des durch die Immunreaktion produzierten Antigen-Antikörper-Komplexes gestattet, wobei diese Reagentien gegebenenfalls markiert sind oder mit einem markierten Reagens erkannt werden können, insbesondere dann, wenn das genannte Antikörper nicht markiert ist.

34. Biologisch reines rekombinantes Polypeptid nach einem der Ansprüche 1 bis 8 zur Verwendung in einer immunogenen Zusammensetzung in Kombination mit einem pharmazeutisch unbedenklichen Konstituens.

35. Biologisch reines rekombinantes Polypeptid nach einem der Ansprüche 1 bis 8 zur Verwendung in einer Impfstoffzusammensetzung.

36. Verfahren zur enzymatischen Amplifizierung einer Nucleotidsequenz nach einem der Ansprüche 13 bis 15 und den Nachweis der amplifizierten Nucleotidsequenz, wobei mittels PCR-Technik mit einem Primer-Satz amplifiziert wird und das PCR-amplifizierte Produkt durch eine Hybridisierungsreaktion mit einer Nachweissonde nachgewiesen wird, wobei der Primer-Satz und die Nachweisprobe aus den folgenden Elementen ausgewählt sind: 
Primer-Satz
P1 GAGTACCTGCAGGTGCCGTCGCCGTCGATGGGCCG
P2 kompl. GTACCACTCGAACGCCGGGTGTTGAT
Sonde B
TCGCCCGCCCTGTACCTG

Primer-Satz
P1 GAGTACCTGCAGGTGCCGTCGCCGTCGATGGGCCG
P3 kompl. TCCCACTTGTAAGTCTGGCA
Sonde B
TCGCCCGCCCTGTACCTG

Primer-Satz
P1 GAGTACCTGCAGGTGCCGTCGCCGTCGATGGGCCG
P4 kompl. CGGCAGCTCGCTGGTCAGGA
Sonde B
TCGCCCGCCCTGTACCTG

Primer-Satz
P1 GAGTACCTGCAGGTGCCGTCGCCGTCGATGGGCCG
P5 kompl. GCGTCACCCATCGCCAGGCCGATCAGG
Sonde B
TCGCCCGCCCTGTACCTG oder
Sonde C
GCGCTGACGCTGGCGATCTATC
 
Primer-Satz
P1        GAGTACCTGCAGGTGCCGTCGCCGTCGATGGGCCG
P6 kompl. GCGCCCCAGTACTCCCAGCTGTGCGT
Sonde B
TCGCCCGCCCTGTACCTG oder
Sonde C
GCGCTGACGCTGGCGATCTATC oder
Sonde D
CCGCTGTTGAACGTCGGGAAG oder
Sonde E
AAGCCGTCGGATCTGGGTGGCAAC

Primer-Satz
P2        ATCAACACCCCGGCGTTCGAGTGGTAC
P5 kompl. GCGTCACCCATCGCCAGGCCGATCAGG
Sonde C
CGCGTGACGCTGGCGATCTATC

Primer-Satz
P2        ATCAACACCCCGGCGTTCGAGTGGTAC
P6 kompl. GCGCCCCAGTACTCCCAGCTGTGCGT
Sonde C
GCGCTGACGCTGGCGATCTATC oder
Sonde D
CCGCTGTTGAACGTCGGGAAG oder
Sonde E
AAGCCGTCGGATCTGGGTGGCAAC

Primer-Satz
P3        TGCCAGACTTACAAGTGGGA
P5 kompl. GCGTCACCCATCGCCAGGCCGATCAGG
Sonde C
GCGCTGACGCTGGCGATCTATC

Primer-Satz
P3        TGCCAGACTTACAAGTGGGA
P6 kompl. GCGCCCCAGTACTCCCAGCTGTGCGT
Sonde C
GCGCTGACGCTGGCGATCTATC oder
 
Sonde D
CCGCTGTTGAACGTCGGGAAG oder
Sonde E
AAGCCGTCGGATCTGGGTGGCAAC

Primer-Satz
P4 TCCTGACCAGCGAGCTGCCG
P5 kompl. GCGTCACCCATCGCCAGGCCGATCAGG
Sonde C
GCGCTGACGCTGGCGATCTATC

Primer-Satz
P4 TCCTGACCAGCGAGCTGCCG
P6 kompl. GCGCCCCAGTACTCCCAGCTGTGCGT
Sonde C
GCGCTGACGCTGGCGATCTATC oder
Sonde D
CCGCTGTTGAACGTCGGGAAG oder
Sonde E
AAGCCGTCGGATCTGGGTGGCAAC

Primer-Satz
P5 CCTGATCGGCCTGGCGATGGGTGACGC
P6 kompl. GCGCCCCAGTACTCCCAGCTGTGCGT
Sonde D
CCGCTGTTGAACGTCGGGAAG oder
Sonde E
AAGCCGTCGGATCTGGGTGGCAAC
 oder wobei dieser Primer-Satz aus den Primer-Sätzen nach Anspruch 19 ausgewählt ist und die Nachweissonde aus einer beliebigen Oligonucleotidsequenz mit mindestens 10 Nucleotiden besteht, wobei sich diese Sequenz zwischen den beiden PCR-Primern befindet, die den für die Amplifizierung der Nucleotidsequenz verwendeten Primer-Satz darstellen.

37. Rekombinanter Vektor nach Anspruch 20, wobei die Vektorsequenz entweder die in Fig. 10b dargestellte Nucleinsäuresequenz oder die in Fig. 11b dargestellte Nucleinsäuresequenz oder die in Fig. 12b dargestellte Nucleinsäuresequenz aufweist.

38. Peptide nach Anspruch 1, die vorteilhaft zur Produktion von Antikörpern, insbesondere monoklonalen Antikörpern verwendet werden und die folgenden Aminosäuresequenzen aufweisen:
| Aminosäure in position (NH₂-Ende) | | Aminosäure in position (COOH-Ende) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |

39. Verfahren zur Herstellung eines biologisch reinen rekombinanten Polypeptids, das in seiner Polypeptidkette mindestens eine der folgenden Aminosäuresequenzen enthält:
- diejenige, die sich von dem durch die Aminosäure in Position (-29) gebildeten äußersten Ende zu dem durch die Aminosäure in Position (-1) gebildeten äußersten Ende wie in Fig. 3a und Fig. 3b dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 3a und Fig. 3b dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 3a und Fig. 3b dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 3a und Fig. 3b dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 3a und Fig. 3b dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 3a und Fig. 3b dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 3a und Fig. 3b dargestellt erstreckt, oder
- diejenige, die sich von dem durch die Aminosäure in Position (275) gebildeten äußersten Ende zu dem durch die Aminosäure in Position (294) gebildeten äußersten Ende wie in Fig. 3a und Fig. 3b dargestellt erstreckt, oder
- diejenige, die sich von dem durch die Aminosäure in Position (1) gebildeten äußersten Ende zu dem durch die Aminosäure in Position (294) gebildeten äußersten Ende wie in Fig. 3a und Fig. 3b dargestellt erstreckt,
- diejenige, die sich von dem durch die Aminosäure in Position (-42) gebildeten äußersten Ende zu dem durch die Aminosäure in Position (-1) gebildeten äußersten Ende wie in Fig. 3a und Fig. 3b dargestellt erstreckt,
- diejenige, die sich von dem durch die Aminosäure in Position (-47) gebildeten äußersten Ende zu dem durch die Aminosäure in Position (-1) gebildeten äußersten Ende wie in Fig. 3a und Fig. 3b dargestellt erstreckt,
- diejenige, die sich von dem durch die Aminosäure in Position (-49) gebildeten äußersten Ende zu dem durch die Aminosäure in Position (-1) gebildeten äußersten Ende wie in Fig. 3a und Fig. 3b dargestellt erstreckt,
- diejenige, die sich von dem durch die Aminosäure in Position (-55) gebildeten äußersten Ende zu dem durch die Aminosäure in Position (-1) gebildeten äußersten Ende wie in Fig. 3a und Fig. 3b dargestellt erstreckt,
- diejenige, die sich von dem durch die Aminosäure in Position (-59) gebildeten äußersten Ende zu dem durch die Aminosäure in Position (-1) gebildeten äußersten Ende wie in Fig. 3a und Fig. 3b dargestellt erstreckt,
- diejenige, die sich von dem durch die Aminosäure in Position (-29) gebildeten äußersten Ende zu dem durch die Aminosäure in Position (294) gebildeten äußersten Ende wie in Fig. 3a und Fig. 3b dargestellt erstreckt,
- diejenige, die sich von dem durch die Aminosäure in Position (-42) gebildeten äußersten Ende zu dem durch die Aminosäure in Position (294) gebildeten äußersten Ende wie in Fig. 3a und Fig. 3b dargestellt erstreckt,
- diejenige, die sich von dem durch die Aminosäure in Position (-47) gebildeten äußersten Ende zu dem durch die Aminosäure in Position (294) gebildeten äußersten Ende wie in Fig. 3a und Fig. 3b dargestellt erstreckt,
- diejenige, die sich von dem durch die Aminosäure in Position (-49) gebildeten äußersten Ende zu dem durch die Aminosäure in Position (294) gebildeten äußersten Ende wie in Fig. 3a und Fig. 3b dargestellt erstreckt,
- diejenige, die sich von dem durch die Aminosäure in Position (-55) gebildeten äußersten Ende zu dem durch die Aminosäure in Position (294) gebildeten äußersten Ende wie in Fig. 3a und Fig. 3b dargestellt erstreckt,
- diejenige, die sich von dem durch die Aminosäure in Position (-59) gebildeten äußersten Ende zu dem durch die Aminosäure in Position (294) gebildeten äußersten Ende wie in Fig. 3a und Fig. 3b dargestellt erstreckt, oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren, wobei dieses Verfahren folgende Schritte umfaßt:
- Herstellung des Polypeptids auf gentechnischem Weg, d.h. durch Expression der diese Polypeptide kodierenden Nucleinsäuren in einer Wirtszelle, oder durch klassische chemische Synthese, sowie
- Reinigung der hergestellten Polypeptide.

40. Verfahren zur Herstellung eines biologisch reinen rekombinanten Polypeptids nach Anspruch 39, wobei dieses Polypeptid in seiner Polypeptidkette mindestens eine der folgenden Aminosäuresequenzen enthält:
- diejenige, die sich von dem durch die Aminosäure in Position (-29) gebildeten äußersten Ende zu dem durch die Aminosäure in Position (-1) gebildeten äußersten Ende wie in Fig. 4a und Fig. 4b dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 4a und Fig. 4b dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 4a und Fig. 4b dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 4a und Fig. 4b dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 4a und Fig. 4b dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 4a und Fig. 4b dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 4a und Fig. 4b dargestellt erstreckt, oder
- diejenige, die sich von dem durch die Aminosäure in Position (275) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (1) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (-42) gebildeten äußersten Ende zu dem durch die Aminosäure in Position (-1) gebildeten äußersten Ende wie in Fig. 4a und Fig. 4b dargestellt erstreckt,
- diejenige, die sich von dem durch die Aminosäure in Position (-47) gebildeten äußersten Ende zu dem durch die Aminosäure in Position (-1) gebildeten äußersten Ende wie in Fig. 4a und Fig. 4b dargestellt erstreckt,
- diejenige, die sich von dem durch die Aminosäure in Position (-49) gebildeten äußersten Ende zu dem durch die Aminosäure in Position (-1) gebildeten äußersten Ende wie in Fig. 4a und Fig. 4b dargestellt erstreckt,
- diejenige, die sich von dem durch die Aminosäure in Position (-55) gebildeten äußersten Ende zu dem durch die Aminosäure in Position (-1) gebildeten äußersten Ende wie in Fig. 4a und Fig. 4b dargestellt erstreckt,
- diejenige, die sich von dem durch die Aminosäure in Position (-59) gebildeten äußersten Ende zu dem durch die Aminosäure in Position (-1) gebildeten äußersten Ende wie in Fig. 4a und Fig. 4b dargestellt erstreckt,
- diejenige, die sich von dem durch die Aminosäure in Position (-29) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (-42) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (-47) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (-49) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (-55) gebildeten äußersten Ende zu dem durch die Aminosäure in Position (294) gebildeten äußersten Ende wie in Fig. 4a und Fig. 4b dargestellt erstreckt,
- diejenige, die sich von dem durch die Aminosäure in Position (-59) gebildeten äußersten Ende zu dem durch die Aminosäure in Position (294) gebildeten äußersten Ende wie in Fig. 4a und Fig. 4b dargestellt erstreckt, oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

41. Verfahren zur Herstellung eines biologisch reinen rekombinanten Polypeptids nach Anspruch 39, wobei dieses Polypeptid in seiner Polypeptidkette mindestens eine der folgenden Aminosäuresequenzen enthält:
- diejenige, die sich von dem durch die Aminosäure in Position (-30) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (12) gebildeten äußersten Ende zu dem durch die Aminosäure in Position (31) gebildeten äußersten Ende wie in Fig. 5 dargestellt erstreckt, oder
- diejenige, die sich von dem durch die Aminosäure in Position (36) gebildeten äußersten Ende zu dem durch die Aminosäure in Position (55) gebildeten äußersten Ende wie in Fig. 5 dargestellt erstreckt, oder
- diejenige, die sich von dem durch die Aminosäure in Position (77) gebildeten äußersten Ende zu dem durch die Aminosäure in Position (96) gebildeten äußersten Ende wie in Fig. 5 dargestellt erstreckt, oder
- diejenige, die sich von dem durch die Aminosäure in Position (101) gebildeten äußersten Ende zu dem durch die Aminosäure in Position (120) gebildeten äußersten Ende wie in Fig. 5 dargestellt erstreckt, oder
- diejenige, die sich von dem durch die Aminosäure in Position (175) gebildeten äußersten Ende zu dem durch die Aminosäure in Position (194) gebildeten äußersten Ende wie in Fig. 5 dargestellt erstreckt, oder
- diejenige, die sich von dem durch die Aminosäure in Position (211) gebildeten äußersten Ende zu dem durch die Aminosäure in Position (230) gebildeten äußersten Ende wie in Fig. 5 dargestellt erstreckt, oder
- diejenige, die sich von dem durch die Aminosäure in Position (275) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (1) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (-43) gebildeten äußersten Ende zu dem durch die Aminosäure in Position (-1) gebildeten äußersten Ende wie in Fig. 5 dargestellt erstreckt, oder
- diejenige, die sich von dem durch die Aminosäure in Position (-30) gebildeten äußersten Ende zu dem durch die Aminosäure in Position (295) gebildeten äußersten Ende wie in Fig. 5 dargestellt erstreckt, oder
- diejenige, die sich von dem durch die Aminosäure in Position (-43) gebildeten äußersten Ende zu dem durch die Aminosäure in Position (295) gebildeten äußersten Ende wie in Fig. 5 dargestellt erstreckt,
oder die durch Modifikation der freien Carboxyl- und/oder Aminogruppen in der Polypeptidsequenz entstandenen Peptidsequenzen und/oder die durch Glycosylierung der genannten Polypeptide entstandenen Peptidsequenzen, soweit diese Modifikation nicht die folgenden Eigenschaften ändert:
- die Polypeptide reagieren mit polyklonalem Kaninchen-Antiserum gegen das 32-kDa-Protein eines M. bovis-BCG-Kulturfiltrats und/oder
- die Polypeptide reagieren selektiv mit Humanseren von Tuberkulosepatienten, insbesondere Patienten, die im Frühstadium eine evolutive Tuberkulose entwickeln, und/oder
- ein Konjugat dieser Polypeptide ist fähig, in vivo die Produktion von M. tuberculosis-neutralisierenden Antikörpern zu induzieren, oder in vivo durch Aktivierung von T-Zellen, die auf M. tuberculosis reagieren, eine Immunantwort in der Zelle zu induzieren.

42. Verfahren zur Herstellung eines rekombinanten Polypeptids nach Anspruch 39, wobei dieses Polypeptid aus einer der folgenden Aminosäuresequenzen besteht:
- derjenigen, die sich von dem durch die Aminosäure in Position (-59) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-59) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-55) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-55) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-49) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-49) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-47) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-47) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-42) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-42) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (-29) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (-29) gebildeten äußersten Ende zu dem durch die Aminosäure in Position (-1) gebildeten äußersten Ende wie in Fig. 3a und Fig. 3b dargestellt erstreckt,
- derjenigen, die sich von dem durch die Aminosäure in Position (1) gebildeten äußersten Ende zu dem durch die Aminosäure in Position (294) gebildeten äußersten Ende wie in Fig. 3a und Fig. 3b dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 3a und Fig. 3b dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 3a und Fig. 3b dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 3a und Fig. 3b dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 3a und Fig. 3b dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 3a und Fig. 3b dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 3a und Fig. 3b dargestellt erstreckt,
- derjenigen, die sich von dem durch die Aminosäure in Position (275) gebildeten äußersten Ende zu dem durch die Aminosäure in Position (294) gebildeten äußersten Ende wie in Fig. 3a und Fig. 3b dargestellt erstreckt.

43. Verfahren zur Herstellung eines rekombinanten Polypeptids nach Anspruch 39, wobei dieses Polypeptid aus einer der folgenden Aminosäuresequenzen besteht:
- derjenigen, die sich von dem durch die Aminosäure in Position (-59) gebildeten äußusten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-59) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-55) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-55) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-49) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-49) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-47) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-47) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-42) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-42) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (-29) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (-29) gebildeten äußersten Ende zu dem durch die Aminosäure in Position (-1) gebildeten äußersten Ende wie in Fig. 4a und Fig. 4b dargestellt erstreckt,
- derjenigen, die sich von dem durch die Aminosäure in Position (1) gebildeten äußersten Ende zu dem durch die Aminosäure in Position (294) gebildeten äußersten Ende wie in Fig. 4a und Fig. 4b dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 4a und Fig. 4b dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 4a und Fig. 4b dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 4a und Fig. 4b dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 4a und Fig. 4b dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 4a und Fig. 4b dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 4a und Fig. 4b dargestellt erstreckt,
- derjenigen, die sich von dem durch die Aminosäure in Position (275) gebildeten äußersten Ende zu dem durch die Aminosäure in Position (294) gebildeten äußersten Ende wie in Fig. 4a und Fig. 4b dargestellt erstreckt.

44. Verfahren zur Herstellung eines rekombinanten Polypeptids nach Anspruch 39, wobei dieses Polypeptid aus einer der folgenden Aminosäuresequenzen besteht:
- derjenigen, die sich von dem durch die Aminosäure in Position (-43) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (-43) gebildeten äußersten Ende zu dem durch die Aminosäure in Position (-1) gebildeten äußersten Ende wie in Fig. 5 dargestellt erstreckt,
- derjenigen, die sich von dem durch die Aminosäure in Position (-30) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (30) gebildeten äußersten Ende zu dem durch die Aminosäure in Position (-1) gebildeten äußersten Ende wie in Fig. 5 dargestellt erstreckt,
- derjenigen, die sich von dem durch die Aminosäure in Position (1) gebildeten äußersten Ende zu dem durch die Aminosäure in Position (295) gebildeten äußersten Ende wie in Fig. 5 dargestellt erstreckt,
- derjenigen, die sich von dem durch die Aminosäure in Position (12) gebildeten äußersten Ende zu dem durch die Aminosäure in Position (31) gebildeten äußersten Ende wie in Fig. 5 dargestellt erstreckt,
- derjenigen, die sich von dem durch die Aminosäure in Position (36) gebildeten äußersten Ende zu dem durch die Aminosäure in Position (55) gebildeten äußersten Ende wie in Fig. 5 dargestellt erstreckt,
- derjenigen, die sich von dem durch die Aminosäure in Position (77) gebildeten äußersten Ende zu dem durch die Aminosäure in Position (96) gebildeten äußersten Ende wie in Fig. 5 dargestellt erstreckt,
- derjenigen, die sich von dem durch die Aminosäure in Position (101) gebildeten äußersten Ende zu dem durch die Aminosäure in Position (120) gebildeten äußersten Ende wie in Fig. 5 dargestellt erstreckt,
- derjenigen, die sich von dem durch die Aminosäure in Position (175) gebildeten äußersten Ende zu dem durch die Aminosäure in Position (194) gebildeten äußersten Ende wie in Fig. 5 dargestellt erstreckt,
- derjenigen, die sich von dem durch die Aminosäure in Position (211) gebildeten äußersten Ende zu dem durch die Aminosäure in Position (230) gebildeten äußersten Ende wie in Fig. 5 dargestellt erstreckt,
- derjenigen, die sich von dem durch die Aminosäure in Position (275) gebildeten äußersten Ende zu dem durch die Aminosäure in Position (295) gebildeten äußersten Ende wie in Fig. 5 dargestellt erstreckt.

45. Verfahren zur Herstellung eines biologisch reinen rekombinanten Polypeptids nach Anspruch 39, wobei dieses Polypeptid aus einem Polypeptid nach einem der Ansprüche 1 bis 6 besteht, und einem Protein mit einer heterologen Sequenz in bezug auf dieses Polypeptid, wobei die heterologe Sequenz ungefähr 1 bis ungefähr 100 Aminosäuren umfaßt.

46. Verfahren zur Herstellung eines rekombinanten Polypeptids nach Anspruch 45, wobei es sich bei dem heterologen Protein um β-Galactosidase handelt.

47. Verfahren zur Herstellung einer Nucleinsäure mit
(a) einer Nucleotidsequenz, die für ein Polypeptid nach einem der Ansprüche 1 bis 6 kodiert, oder
(b) einer Nucleotidsequenz, die zu (a) komplementär ist, oder
(c) den Nucleotidsequenzen von (a) oder (b), wobei T durch U ersetzt sein kann, wobei es sich bei diesem Verfahren um ein chemisches Syntheseverfahren mit den folgenden Schritten handelt:
- Synthese von einzelsträngigen Nucleinsäuren mit einem automatisierten beta-Cyanethylphosphoramiditverfahren, sowie
- bei doppelsträngigen Nucleinsäuren Kombinieren der einzelsträngigen sense- und antisense Oligonucleotide mittels Hybridisierung unter Bildung einer Doppelstrang-DNA Klonieren der erhaltenen Doppelstrang-DNA in einen geeigneten Plasmidvektor und Gewinnung der DNA nach klassischen Verfahren, wie Verdauung mit Restriktionsenzymen und Agarosegel-Elektrophorese, sowie
- bei Nucleinsäuren mit über 100 Nucleotiden Zusammenfügen von chemisch synthetisierten Oligonucleotiden, die an ihren Enden unterschiedliche Restriktionsstellen aufweisen, deren Sequenzen mit der Aufeinanderfolge der Aminosäuren in dem natürlichen Peptid kompatibel sind, Klonieren der so erhaltenen DNA in einen geeigneten Plasmidvektor und Gewinnung der erwünschten Nucleinsäure nach klassischen Verfahren wie Verdauung mit Restriktionsenzymen und Agarosegel-Elektrophorese.

48. Verfahren zur Herstellung einer Nucleinsäure nach Anspruch 47, wobei diese Nucleinsäure mindestens eine der folgenden Nucleotidsequenzen umfaßt:
- derjenigen, die sich von dem durch das Nucleotid in Position (1) gebildeten äußersten Ende zu dem durch das Nucleotid in Position (182) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 3a und Fig. 3b dargestellt erstreckt, wobei N eines der fünf Nucleotide A, T, C, G oder I darstellt,
- derjenigen, die sich von dem durch das Nucleotid in Position (1) gebildeten äußersten Ende zu dem durch das Nucleotid in Position (194) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 3a und Fig. 3b dargestellt erstreckt, oder deren komplementären Sequenzen,
oder den oben genannten Nucleotidsequenzen, wobei T durch U ersetzt ist.

49. Verfahren zur Herstellung einer Nucleinsäure nach Anspruch 47, wobei diese Nucleinsäure mindestens eine der folgenden Nucleotidsequenzen umfaßt:
- diejenige, die sich von dem durch das Nucleotid in Position (1) gebildeten äußersten Ende zu dem durch das Nucleotid in Position (182) gebildeten äußersten Ende wie in Fig. 4a und Fig. 4b dargestellt erstreckt,
- diejenige, die sich von dem durch das Nucleotid in Position (273) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (360) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (1242) gebildeten äußersten Ende zu dem durch das Nucleotid in Position (1358) gebildeten äußersten Ende wie in Fig. 4a und Fig. 4b dargestellt erstreckt, wobei N eines der fünf Nucleotide A, T, C, G oder I darstellt,
- diejenige, die sich von dem durch das Nucleotid in Position (1) gebildeten äußersten Ende zu dem durch das Nucleotid in Position (194) gebildeten äußersten Ende wie in Fig. 4a und Fig. 4b dargestellt erstreckt,
- diejenige, die sich von dem durch das Nucleotid in Position (195) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (1) gebildeten äußersten Ende zu dem durch das Nucleotid in Position (212) gebildeten äußersten Ende wie in Fig. 4a und Fig. 4b dargestellt erstreckt,
- diejenige, die sich von dem durch das Nucleotid in Position (213) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (1) gebildeten äußersten Ende zu dem durch das Nucleotid in Position (218) gebildeten äußersten Ende wie in Fig. 4a und Fig. 4b dargestellt erstreckt,
- diejenige, die sich von dem durch das Nucleotid in Position (219) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (1) gebildeten äußersten Ende zu dem durch das Nucleotid in Position (272) gebildeten äußersten Ende wie in Fig. 4a und Fig. 4b dargestellt erstreckt,
- diejenige, die sich von dem durch das Nucleotid in Position (1) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (183) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (1) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (1) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (183) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (183) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (195) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (195) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (213) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (213) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (219) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (219) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (234) gebildeten äußersten Ende zu dem durch das Nucleotid in Position (359) gebildeten äußersten Ende wie in Fig. 4a und Fig. 4b dargestellt erstreckt,
- diejenige, die sich von dem durch das Nucleotid in Position (234) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (234) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (273) gebildeten äußersten Ende zu dem durch das Nucleotid in Position (1241) gebildeten äußersten Ende wie in Fig. 4a und Fig. 4b dargestellt erstreckt,
- diejenige, die sich von dem durch das Nucleotid in Position (273) gebildeten äußersten Ende zu dem durch das Nucleotid in Position (1358) gebildeten äußersten Ende wie in Fig. 4a und Fig. 4b dargestellt erstreckt,
- diejenige, die sich von dem durch das Nucleotid in Position (360) gebildeten äußersten Ende zu dem durch das Nucleotid in Position (1358) gebildeten äußersten Ende wie in Fig. 4a und Fig. 4b dargestellt erstreckt, oder deren komplementären Sequenzen,
oder den oben genannten Nucleotidsequenzen, wobei T durch U ersetzt ist.

50. Verfahren zur Herstellung einer Nucleinsäure nach Anspruch 47, wobei diese Nucleinsäure mindestens über eine der folgenden Nucleotidsequenzen verfügt:
- diejenige, die sich von dem durch das Nucleotid in Position (130) gebildeten äußersten Ende zu dem durch das Nucleotid in Position (219) gebildeten äußersten Ende wie in Fig. 5 dargestellt erstreckt,
- diejenige, die sich von dem durch das Nucleotid in Position (220) gebildeten äußersten Ende zu dem durch das Nucleotid in Position (1104) gebildeten äußersten Ende wie in Fig. 5 dargestellt erstreckt,
- diejenige, die sich von dem durch das Nucleotid in Position (1105) gebildeten äußersten Ende zu dem durch das Nucleotid in Position (1299) gebildeten äußersten Ende wie in Fig. 5 dargestellt erstreckt,
- diejenige, die sich von dem durch das Nucleotid in Position (1) gebildeten äußersten Ende zu dem durch das Nucleotid in Position (129) gebildeten äußersten Ende wie in Fig. 5 dargestellt erstreckt,
- diejenige, die sich von dem durch das Nucleotid in Position (1) gebildeten äußersten Ende zu dem durch das Nucleotid in Position (219) gebildeten äußersten Ende wie in Fig. 5 dargestellt erstreckt,
- diejenige, die sich von dem durch das Nucleotid in Position (1) gebildeten äußersten Ende zu dem durch das Nucleotid in Position (1104) gebildeten äußersten Ende wie in Fig. 5 dargestellt erstreckt,
- diejenige, die sich von dem durch das Nucleotid in Position (1) gebildeten äußersten Ende zu dem durch das Nucleotid in Position (1299) gebildeten äußersten Ende wie in Fig. 5 dargestellt erstreckt,
- diejenige, die sich von dem durch das Nucleotid in Position (90) gebildeten äußersten Ende zu dem durch das Nucleotid in Position (219) gebildeten äußersten Ende wie in Fig. 5 dargestellt erstreckt,
- diejenige, die sich von dem durch das Nucleotid in Position (90) gebildeten äußersten Ende zu dem durch das Nucleotid in Position (1299) gebildeten äußersten Ende wie in Fig. 5 dargestellt erstreckt,
- diejenige, die sich von dem durch das Nucleotid in Position (90) gebildeten äußersten Ende zu dem durch das Nucleotid in Position (1104) gebildeten äußersten Ende wie in Fig. 5 dargestellt erstreckt,
- diejenige, die sich von dem durch das Nucleotid in Position (130) gebildeten äußersten Ende zu dem durch das Nucleotid in Position (1104) gebildeten äußersten Ende wie in Fig. 5 dargestellt erstreckt,
- diejenige, die sich von dem durch das Nucleotid in Position (130) gebildeten äußersten Ende zu dem durch das Nucleotid in Position (1299) gebildeten äußersten Ende wie in Fig. 5 dargestellt erstreckt,
- diejenige, die sich von dem durch das Nucleotid in Position (220) gebildeten äußersten Ende zu dem durch das Nucleotid in Position (1299) gebildeten äußersten Ende wie in Fig. 5 dargestellt erstreckt,
oder deren komplementären Sequenzen,
oder den oben genannten Nucleotidsequenzen, wobei T durch U ersetzt ist.

51. Verfahren zur Herstellung einer Nucleinsäure nach Anspruch 47, wobei diese Nucleinsäure aus einer der folgenden Nucleotidsequenzen besteht:
- derjenigen, die sich von dem durch das Nucleotid in Position (1) gebildeten äußersten Ende zu dem durch das Nucleotid in Position (182) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (194) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 3a und Fig. 3b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 3a und Fig. 3b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 3a und Fig. 3b dargestellt erstreckt, oder deren komplementären Sequenzen,
oder den oben genannten Nucleotidsequenzen, wobei T durch U ersetzt ist.

52. Verfahren zur Herstellung einer Nucleinsäure nach Anspruch 47, wobei diese Nucleinsäure aus einer der folgenden Nucleotidsequenzen besteht:
- derjenigen, die sich von dem durch das Nucleotid in Position (1) gebildeten äußersten Ende zu dem durch das Nucleotid in Position (182) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (194) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (212) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (218) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (272) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (1) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (183) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (195) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (213) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (219) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (234) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (359) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (273) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1241) gebildeten äußersten Ende wie in Fig. 4a und Fig. 4b dargestellt erstreckt,
- derjenigen, die sich von dem durch das Nucleotid in Position (360) gebildeten äußersten Ende zu dem durch das Nucleotid in Position (1358) gebildeten äußersten Ende wie in Fig. 4a und Fig. 4b dargestellt erstreckt,
- derjenigen, die sich von dem durch das Nucleotid in Position (1242) gebildeten äußersten Ende zu dem durch das Nucleotid in Position (1358) gebildeten äußersten Ende wie in Fig. 4a und Fig. 4b dargestellt erstreckt, oder deren komplementären Sequenzen,
oder den oben genannten Nucleotidsequenzen, wobei T durch U ersetzt ist.

53. Verfahren zur Herstellung einer Nucleinsäure nach Anspruch 47, wobei diese Nucleinsäure aus einer der folgenden Nucleotidsequenzen besteht:
- derjenigen, die sich von dem durch das Nucleotid in Position (1) gebildeten äußersten Ende zu dem durch das Nucleotid in Position (129) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (1) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (90) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (219) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (130) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1104) gebildeten äußersten Ende wie in Fig. 5 dargestellt erstreckt,
- derjenigen, die sich von dem durch das Nucleotid in Position (220) gebildeten äußersten Ende zu dem durch das Nucleotid in Position (1299) gebildeten äußersten Ende wie in Fig. 5 dargestellt erstreckt,
- derjenigen, die sich von dem durch das Nucleotid in Position (1105) gebildeten äußersten Ende zu dem durch das Nucleotid in Position (1299) gebildeten äußersten Ende wie in Fig. 5 dargestellt erstreckt,
oder deren komplementären Sequenzen,
oder den oben genannten Nucleotidsequenzen, wobei T durch U ersetzt ist.

54. Verfahren zur Herstellung einer rekombinanten Nucleinsäure durch Insertieren mindestens einer der Nucleotidsequenzen nach Anspruch 9 bis 15 in eine heterologe Nucleinsäure.

55. Verfahren zur Herstellung eines DNA- oder RNA-Primers, wobei dieser Primer aus 15 bis 25 Nucleotiden einer Nucleotidsequenz nach einem der Ansprüche 13 bis 15 gebildet wird und als Primer für die spezifische Amplifizierung einer dieser Nucleotidsequenzen verwendet werden kann, wobei dieses Verfahren auf dem Weg der chemischen Synthese mit dem automatischen β-Cyanethylphosphoramiditverfahren durchgeführt wird.

56. Verfahren zur Herstellung eines DNA- oder RNA-Primers nach Anspruch 55, wobei dieser Primer aus der folgenden Gruppe von Nucleotidsequenzen ausgewählt wird:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
oder deren komplementären Sequenzen oder den entsprechenden Ribonucleotidsequenzen.

57. Verfahren zur Herstellung eines DNA- oder RNA-Primer-Satzes für die enzymatische Amplifizierung einer Nucleotidsequenz nach einem der Ansprüche 13 bis 15, wobei das Verfahren den Schritt umfaßt, daß man in dem Primer-Satz
- eine Nucleotidsequenz A mit der komplementären Nucleotidsequenz von entweder B oder C oder D oder E oder F kombiniert; oder
- die Nucleotidsequenz B mit der komplementären Nucleotidsequenz von C oder D oder E oder F kombiniert; oder
- die Nucleotidsequenz C mit der komplementären Nucleotidsequenz von D oder E oder F kombiniert; oder
- die Nucleotidsequenz D mit der komplementären Nucleotidsequenz von E oder F kombiniert; oder
- die Nucleotidsequenz E mit einer komplementären Nucleotidsequenz von F kombiniert, wobei die Nucleotidsequenzen A, B, C, D, E und F wie in Anspruch 18 definiert sind und wobei A eine beliebige der Sequenzen A(i), A(ii), A(iii), A(iv) und A(v) bedeutet und F eine beliebige der Sequenzen F(i), F(ii), F(iii) und F(iv) bedeutet.

58. Verfahren zur Herstellung eines rekombinanten Vektors, insbesondere zum Klonieren und/oder für die Expression, umfassend den Schritt, daß man eine Nucleinsäure nach einem der Ansprüche 9 bis 15 in eine Vektorsequenz des Plasmid-, Cosmid- oder Phagentyps in eine der für seine Replikation unwesentlichen Stellen insertiert.

59. Verfahren zur Herstellung eines rekombinanten Vektors nach Anspruch 58, wobei dieser Vektor in einer der für seine Replikation unwesentlichen Stellen die notwendigen Elemente zur Promotion der Expression eines Polypeptids nach einem der Ansprüche 1 bis 6 in einer Wirtszelle enthält.

60. Verfahren zur Herstellung eines rekombinanten Vektors nach Anspruch 58, wobei dieser Vektor die Elemente enthält, die die Expression eines Polypeptids nach einem der Ansprüche 1 bis 6 durch E. coli gestattet.

61. Verfahren zur Herstellung einer Wirtszelle enthaltend einen rekombinanten Vektor nach einem der Ansprüche 20 bis 22, und mit den die Expression eines Polypeptids nach einem der Ansprüche 1 bis 6 in diesem Wirt gestattenden Regulationselementen, wobei dieses Verfahren den Schritt umfaßt, daß man diese Wirtszelle mit dem rekombinanten Vektor transformiert.

62. Verfahren zur Herstellung einer Wirtszelle nach Anspruch 61, wobei diese Wirtszelle aus der Gruppe der mit einem Vektor nach Anspruch 20 transformierten Bakterien, wie E. coli, oder aus der Gruppe der mit einem Vektor nach Anspruch 20 transformierten Eukaryonten ausgewählt ist.

63. Verfahren zur Herstellung eines monoklonalen Antikörpers, der spezifisch gegen ein rekombinantes Polypeptid nach einem der Ansprüche 1 bis 6 gerichtet ist, wobei dieses Verfahren umfaßt, daß man diesen monoklonalen Antikörper mit einem Hybridom produziert, der nach klassischen Verfahren aus einerseits Milzzellen eines gegen ein Polypeptid nach einem der Ansprüche 1 bis 6 immunisierten Tiers, insbesondere einer Maus oder Ratte, und andererseits Zellen einer Myelomzellinie gebildet wird und aufgrund seiner Fähigkeit, einen monoklonalen Antikörper, der das Polypeptid, das ursprünglich für die Immunisierung der Tiere verwendet wurde, erkennt, zu bilden, selektiert wird.

64. Verfahren zur Herstellung einer Nucleotidsonde, die mit einer Nucleinsäure nach einem der Ansprüche 13 bis 15 hybridisiert und ausgewählt ist aus der folgenden Gruppe von Nucleotidsequenzen:
| Sonde A(i), A(ii), A(iii), A(iv) und A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Sonde B | |
|---|---|
| TCGCCCGCCCTGTACCTG, | |
| Sonde C | |
|---|---|
| GCGCTGACGCTGGCGATCTATC, | |
| Sonde D | |
|---|---|
| CCGCTGTTGAACGTCGGGAAG, | |
| Sonde E | |
|---|---|
| AAGCCGTCGGATCTGGGTGGCAAC, | |
| Sonde F(i) und F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA |
| oder ihren komplementären Nucleotidsequenzen, | |
wobei dieses Verfahren auf dem Weg der chemischen Synthese mit dem automatischen β-Cyanethylphosphoramiditverfahren durchgeführt wird.

65. Verfahren zur Herstellung eines Kits für ein in-vitro-Diagnoseverfahren für Tuberkulose bei einem Patienten, der eventuell mit Mycobacterium tuberculosis infiziert ist, nach Anspruch 28, wobei dieses Verfahren den Schritt umfaßt, daß man zumindest die folgenden Elemente kombiniert:
- eine bestimmte Menge an Nucleotidsonde nach Anspruch 26,
- ein geeignetes Medium, damit zwischen der nachzuweisenden Sequenz und der Sonde eine Hybridisierungsreaktion eintreten kann,
- Reagentien, die den Nachweis des zwischen der Nucleotidsequenz und der Sonde gebildeten Hybridisierungskomplexes gestatten.

66. Verfahren zur Herstellung eines Kits für ein in-vitro-Diagnoseverfahren für Tuberkulose bei einem Patienten, der eventuell mit Mycobacterium tuberculosis infiziert ist, nach Anspruch 29, wobei dieses Verfahren den Schritt umfaßt, daß man zumindest die folgenden Elemente kombiniert:
- ein Polypeptid nach einem der Ansprüche 1 bis 8,
- Reagentien zur Herstellung eines Mediums, das sich für das Ablaufen der Immunreaktion eignet,
- Reagentien, die den Nachweis des durch die Immunreaktion produzierten Antigen-Antikörper-Komplexes gestattet, wobei diese Reagentien gegebenenfalls markiert sind oder mit einem markierten Reagens erkannt werden können, insbesondere dann, wenn das genannte Polypeptid nicht markiert ist.

67. Verfahren zur Herstellung eines Kits für ein in-vitro-Diagnoseverfahren für Tuberkulose bei einem Patienten, der eventuell mit Mycobacterium tuberculosis infiziert ist, nach Anspruch 30, wobei dieses Verfahren den Schritt umfaßt, daß man mindestens die folgenden Elemente kombiniert:
- einen Antikörper nach Anspruch 25,
- Reagentien zur Herstellung eines Mediums, das sich für das Ablaufen der Immunreaktion eignet,
- Reagentien, die den Nachweis der durch die Immunreaktion produzierten Antigen-Antikörper-Komplexe gestattet, wobei diese Reagentien gegebenenfalls markiert sind oder mit einem markierten Reagens erkannt werden können, insbesondere dann, wenn der genannte Antikörper nicht markiert ist.

68. Verfahren zur Herstellung eines biologisch reinen rekombinanten Polypeptids nach einem der Ansprüche 1 bis 8 zur Verwendung in einer immunogenen Zusammensetzung in Kombination mit einem pharmazeutisch unbedenklichen Konstituens, wobei dieses Verfahren die Schritte umfaßt, daß man
- das Polypeptid mittels Gentechnik, d.h. durch Expression der für diese Polypeptide kodierenden Nucleinsäuren in einer Wirtszelle, oder auf dem Weg der klassischen chemischen Synthese herstellt, und
- das hergestellte Polypeptid reinigt.

69. Verfahren zur Herstellung eines biologisch reinen rekombinanten Polypeptids nach einem der Ansprüche 1 bis 8 zur Verwendung in einer Impfstoffzusammensetzung, wobei dieses Verfahren die Schritte umfaßt, daß man
- das Polypeptid mittels Gentechnik, d.h. durch Expression der für diese Polypeptide kodierenden Nucleinsäuren in einer Wirtszelle, oder auf dem Weg der klassischen chemischen Synthese herstellt, und
- das hergestellte Polypeptid reinigt.

70. Verfahren zur Herstellung eines Peptids nach Anspruch 1, wobei dieses Peptid vorteilhaft zur Produktion von Antikörpern, insbesondere monoklonalen Antikörpern, verwendet wird und mindestens eine der folgenden Aminosäuresequenzen aufweist:
| Aminosäure in position (NH₂-Ende) | | Aminosäure in position (COOH-Ende) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295, |
wobei dieses Verfahren die Schritte umfaßt, daß man
- das Polypeptid mittels Gentechnik, d.h. durch Expression der für dieses Peptid kodierenden Nucleinsäure in einer Wirtszelle, oder auf dem Weg der klassischen chemischen Synthese herstellt, und
- das hergestellte Peptid reinigt.

## Revendications (Revendications pour l'(les) Etat(s) contractant(s) suivant(s): AT, BE, CH, LI, DE, DK, FR, GB, IT, LU, MC, NL, SE)

1. Polypeptide recombinant biologiquement pur contenant, dans sa chaîne polypeptidique, au moins une des séquences d'acides aminés suivantes:
- celle s'étendant de l'extrémité constituée par l'acide aminé en position (-29) à l'extrémité constituée par l'acide aminé en position (-1) représentée sur la figure 3a et la figure 3b, ou
- celle s'étendant de l'extrémité constituée par l'acide aminé en position (12) à l'extrémité constituée par l'acide aminé en position (31) représentée sur la figure 3a et la figure 3b, ou
- celle s'étendant de l'extrémité constituée par l'acide aminé en position (36) à l'extrémité constituée par l'acide aminé en position (55) représentée sur la figure 3a et la figure 3b, ou
- celle s'étendant de l'extrémité constituée par l'acide aminé en position (77) à l'extrémité constituée par l'acide aminé en position (96) représentée sur la figure 3a et la figure 3b, ou
- celle s'étendant de l'extrémité constituée par l'acide aminé en position (101) à l'extrémité constituée par l'acide aminé en position (120) représentée sur la figure 3a et la figure 3b, ou
- celle s'étendant de l'extrémité constituée par l'acide aminé en position (175) à l'extrémité constituée par l'acide aminé en position (194) représentée sur la figure 3a et la figure 3b, ou
- celle s'étendant de l'extrémité constituée par l'acide aminé en position (211) à l'extrémité constituée par l'acide aminé en position (230) représentée sur la figure 3a et la figure 3b, ou
- celle s'étendant de l'extrémité constituée par l'acide aminé en position (275) à l'extrémité constituée par l'acide aminé en position (294) représentée sur la figure 3a et la figure 3b, ou
- celle s'étendant de l'extrémité constituée par l'acide aminé en position (1) à l'extrémité constituée par l'acide aminé en position (294) représentée sur la figure 3a et la figure 3b, ou
- celle s'étendant de l'extrémité constituée par l'acide aminé en position (-42) à l'extrémité constituée par l'acide aminé en position (-1) représentée sur la figure 3a et la figure 3b, ou
- celle s'étendant de l'extrémité constituée par l'acide aminé en position (-47) à l'extrémité constituée par l'acide aminé en position (-1) représentée sur la figure 3a et la figure 3b, ou
- celle s'étendant de l'extrémité constituée par l'acide aminé en position (-49) à l'extrémité constituée par l'acide aminé en position (-1) représentée sur la figure 3a et la figure 3b, ou
- celle s'étendant de l'extrémité constituée par l'acide aminé en position (-55) à l'extrémité constituée par l'acide aminé en position (-1) représentée sur la figure 3a et la figure 3b, ou
- celle s'étendant de l'extrémité constituée par l'acide aminé en position (-59) à l'extrémité constituée par l'acide aminé en position (-1) représentée sur la figure 3a et la figure 3b, ou
- celle s'étendant de l'extrémité constituée par l'acide aminé en position (-29) à l'extrémité constituée par l'acide aminé en position (294) représentée sur la figure 3a et la figure 3b, ou
- celle s'étendant de l'extrémité constituée par l'acide aminé en position (-42) à l'extrémité constituée par l'acide aminé en position (294) représentée sur la figure 3a et la figure 3b, ou
- celle s'étendant de l'extrémité constituée par l'acide aminé en position (-47) à l'extrémité constituée par l'acide aminé en position (294) représentée sur la figure 3a et la figure 3b, ou
- celle s'étendant de l'extrémité constituée par l'acide aminé en position (-49) à l'extrémité constituée par l'acide aminé en position (294) représentée sur la figure 3a et la figure 3b, ou
- celle s'étendant de l'extrémité constituée par l'acide aminé en position (-55) à l'extrémité constituée par l'acide aminé en position (294) représentée sur la figure 3a et la figure 3b, ou
- celle s'étendant de l'extrémité constituée par l'acide aminé en position (-59) à l'extrémité constituée par l'acide aminé en position (294) représentée sur la figure 3a et la figure 3b,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

2. Polypeptide recombinant biologiquement pur selon la revendication 1 contenant, dans sa chaîne polypeptidique, au moins une des séquences d'acides aminés suivantes:
- celle s'étendant de l'extrémité constituée par l'acide aminé en position (-29) à l'extrémité constituée par l'acide aminé en position (-1) représentée sur la figure 4a et la figure 4b, ou
- celle s'étendant de l'extrémité constituée par l'acide aminé en position (12) à l'extrémité constituée par l'acide aminé en position (31) représentée sur la figure 4a et la figure 4b, ou
- celle s'étendant de l'extrémité constituée par l'acide aminé en position (36) à l'extrémité constituée par l'acide aminé en position (55) représentée sur la figure 4a et la figure 4b, ou
- celle s'étendant de l'extrémité constituée par l'acide aminé en position (77) à l'extrémité constituée par l'acide aminé en position (96) représentée sur la figure 4a et la figure 4b, ou
- celle s'étendant de l'extrémité constituée par l'acide aminé en position (101) à l'extrémité constituée par l'acide aminé en position (120) représentée sur la figure 4a et la figure 4b, ou
- celle s'étendant de l'extrémité constituée par l'acide aminé en position (175) à l'extrémité constituée par l'acide aminé en position (194) représentée sur la figure 4a et la figure 4b, ou
- celle s'étendant de l'extrémité constituée par l'acide aminé en position (211) à l'extrémité constituée par l'acide aminé en position (230) représentée sur la figure 4a et la figure 4b, ou
- celle s'étendant de l'extrémité constituée par l'acide aminé en position (275) à l'extrémité constituée par l'acide aminé en position (294) représentée sur la figure 4a et la figure 4b, ou
- celle s'étendant de l'extrémité constituée par l'acide aminé en position (1) à l'extrémité constituée par l'acide aminé en position (294) représentée sur la figure 4a et la figure 4b, ou
- celle s'étendant de l'extrémité constituée par l'acide aminé en position (-42) à l'extrémité constituée par l'acide aminé en position (-1) représentée sur la figure 4a et la figure 4b, ou
- celle s'étendant de l'extrémité constituée par l'acide aminé en position (-47) à l'extrémité constituée par l'acide aminé en position (-1) représentée sur la figure 4a et la figure 4b, ou
- celle s'étendant de l'extrémité constituée par l'acide aminé en position (-49) à l'extrémité constituée par l'acide aminé en position (-1) représentée sur la figure 4a et la figure 4b, ou
- celle s'étendant de l'extrémité constituée par l'acide aminé en position (-55) à l'extrémité constituée par l'acide aminé en position (-1) représentée sur la figure 4a et la figure 4b, ou
- celle s'étendant de l'extrémité constituée par l'acide aminé en position (-59) à l'extrémité constituée par l'acide aminé en position (-1) représentée sur la figure 4a et la figure 4b, ou
- celle s'étendant de l'extrémité constituée par l'acide aminé en position (-29) à l'extrémité constituée par l'acide aminé en position (294) représentée sur la figure 4a et la figure 4b, ou
- celle s'étendant de l'extrémité constituée par l'acide aminé en position (-42) à l'extrémité constituée par l'acide aminé en position (294) représentée sur la figure 4a et la figure 4b, ou
- celle s'étendant de l'extrémité constituée par l'acide aminé en position (-47) à l'extrémité constituée par l'acide aminé en position (294) représentée sur la figure 4a et la figure 4b, ou
- celle s'étendant de l'extrémité constituée par l'acide aminé en position (-49) à l'extrémité constituée par l'acide aminé en position (294) représentée sur la figure 4a et la figure 4b, ou
- celle s'étendant de l'extrémité constituée par l'acide aminé en position (-55) à l'extrémité constituée par l'acide aminé en position (294) représentée sur la figure 4a et la figure 4b, ou
- celle s'étendant de l'extrémité constituée par l'acide aminé en position (-59) à l'extrémité constituée par l'acide aminé en position (294) représentée sur la figure 4a et la figure 4b,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

3. Polypeptide recombinant biologiquement pur selon la revendication 1 contenant, dans sa chaîne polypeptidique, au moins une des séquences d'acides aminés suivantes:
- celle s'étendant de l'extrémité constituée par l'acide aminé en position (-30) à l'extrémité constituée par l'acide aminé en position (-1) représentée sur la figure 5, ou
- celle s'étendant de l'extrémité constituée par l'acide aminé en position (12) à l'extrémité constituée par l'acide aminé en position (31) représentée sur la figure 5, ou
- celle s'étendant de l'extrémité constituée par l'acide aminé en position (36) à l'extrémité constituée par l'acide aminé en position (55) représentée sur la figure 5, ou
- celle s'étendant de l'extrémité constituée par l'acide aminé en position (77) à l'extrémité constituée par l'acide aminé en position (96) représentée sur la figure 5, ou
- celle s'étendant de l'extrémité constituée par l'acide aminé en position (101) à l'extrémité constituée par l'acide aminé en position (120) représentée sur la figure 5, ou
- celle s'étendant de l'extrémité constituée par l'acide aminé en position (175) à l'extrémité constituée par l'acide aminé en position (194) représentée sur la figure 5, ou
- celle s'étendant de l'extrémité constituée par l'acide aminé en position (211) à l'extrémité constituée par l'acide aminé en position (230) représentée sur la figure 5, ou
- celle s'étendant de l'extrémité constituée par l'acide aminé en position (275) à l'extrémité constituée par l'acide aminé en position (295) représentée sur la figure 5,
- celle s'étendant de l'extrémité constituée par l'acide aminé en position (1) à l'extrémité constituée par l'acide aminé en position (295) représentée sur la figure 5, ou
- celle s'étendant de l'extrémité constituée par l'acide aminé en position (-43) à l'extrémité constituée par l'acide aminé en position (-1) représentée sur la figure 5, ou
- celle s'étendant de l'extrémité constituée par l'acide aminé en position (-30) à l'extrémité constituée par l'acide aminé en position (295) représentée sur la figure 5, ou
- celle s'étendant de l'extrémité constituée par l'acide aminé en position (-43) à l'extrémité constituée par l'acide aminé en position (295) représentée sur la figure 5,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

4. Polypeptide recombinant selon la revendication 1 constitué d'une des séquences d'acides aminés suivantes:
- celle s'étendant de l'extrémité constituée par l'acide aminé en position (-59) à l'extrémité constituée par l'acide aminé en position (294) représentée sur la figure 3a et la figure 3b,
- celle s'étendant de l'extrémité constituée par l'acide aminé en position (-59) à l'extrémité constituée par l'acide aminé en position (-1) représentée sur la figure 3a et la figure 3b,
- celle s'étendant de l'extrémité constituée par l'acide aminé en position (-55) à l'extrémité constituée par l'acide aminé en position (294) représentée sur la figure 3a et la figure 3b,
- celle s'étendant de l'extrémité constituée par l'acide aminé en position (-55) à l'extrémité constituée par l'acide aminé en position (-1) représentée sur la figure 3a et la figure 3b,
- celle s'étendant de l'extrémité constituée par l'acide aminé en position (-49) à l'extrémité constituée par l'acide aminé en position (294) représentée sur la figure 3a et la figure 3b,
- celle s'étendant de l'extrémité constituée par l'acide aminé en position (-49) à l'extrémité constituée par l'acide aminé en position (-1) représentée sur la figure 3a et la figure 3b,
- celle s'étendant de l'extrémité constituée par l'acide aminé en position (-47) à l'extrémité constituée par l'acide aminé en position (294) représentée sur la figure 3a et la figure 3b,
- celle s'étendant de l'extrémité constituée par l'acide aminé en position (-47) à l'extrémité constituée par l'acide aminé en position (-1) représentée sur la figure 3a et la figure 3b,
- celle s'étendant de l'extrémité constituée par l'acide aminé en position (-42) à l'extrémité constituée par l'acide aminé en position (294) représentée sur la figure 3a et la figure 3b,
- celle s'étendant de l'extrémité constituée par l'acide aminé en position (-42) à l'extrémité constituée par l'acide aminé en position (-1) représentée sur la figure 3a et la figure 3b,
- celle s'étendant de l'extrémité constituée par l'acide aminé en position (-29) à l'extrémité constituée par l'acide aminé en position (294) représentée sur la figure 3a et la figure 3b,
- celle s'étendant de l'extrémité constituée par l'acide aminé en position (-29) à l'extrémité constituée par l'acide aminé en position (-1) représentée sur la figure 3a et la figure 3b,
- celle s'étendant de l'extrémité constituée par l'acide aminé en position (1) à l'extrémité constituée par l'acide aminé en position (294) représentée sur la figure 3a et la figure 3b,
- celle s'étendant de l'extrémité constituée par l'acide aminé en position (12) à l'extrémité constituée par l'acide aminé en position (31) représentée sur la figure 3a et la figure 3b,
- celle s'étendant de l'extrémité constituée par l'acide aminé en position (36) à l'extrémité constituée par l'acide aminé en position (55) représentée sur la figure 3a et la figure 3b,
- celle s'étendant de l'extrémité constituée par l'acide aminé en position (77) à l'extrémité constituée par l'acide aminé en position (96) représentée sur la figure 3a et la figure 3b,
- celle s'étendant de l'extrémité constituée par l'acide aminé en position (101) à l'extrémité constituée par l'acide aminé en position (120) représentée sur la figure 3a et la figure 3b,
- celle s'étendant de l'extrémité constituée par l'acide aminé en position (175) à l'extrémité constituée par l'acide aminé en position (194) représentée sur la figure 3a et la figure 3b,
- celle s'étendant de l'extrémité constituée par l'acide aminé en position (211) à l'extrémité constituée par l'acide aminé en position (230) représentée sur la figure 3a et la figure 3b,
- celle s'étendant de l'extrémité constituée par l'acide aminé en position (275) à l'extrémité constituée par l'acide aminé en position (294) représentée sur la figure 3a et la figure 3b.

5. Polypeptide recombinant selon la revendication 2, constitué d'une des séquences d'acides aminés suivantes:
- celle s'étendant de l'extrémité constituée par l'acide aminé en position (-59) à l'extrémité constituée par l'acide aminé en position (294) représentée sur la figure 4a et la figure 4b,
- celle s'étendant de l'extrémité constituée par l'acide aminé en position (-59) à l'extrémité constituée par l'acide aminé en position (-1) représentée sur la figure 4a et la figure 4b,
- celle s'étendant de l'extrémité constituée par l'acide aminé en position (-55) à l'extrémité constituée par l'acide aminé en position (294) représentée sur la figure 4a et la figure 4b,
- celle s'étendant de l'extrémité constituée par l'acide aminé en position (-55) à l'extrémité constituée par l'acide aminé en position (-1) représentée sur la figure 4a et la figure 4b,
- celle s'étendant de l'extrémité constituée par l'acide aminé en position (-49) à l'extrémité constituée par l'acide aminé en position (294) représentée sur la figure 4a et la figure 4b,
- celle s'étendant de l'extrémité constituée par l'acide aminé en position (-49) à l'extrémité constituée par l'acide aminé en position (-1) représentée sur la figure 4a et la figure 4b,
- celle s'étendant de l'extrémité constituée par l'acide aminé en position (-47) à l'extrémité constituée par l'acide aminé en position (294) représentée sur la figure 4a et la figure 4b,
- celle s'étendant de l'extrémité constituée par l'acide aminé en position (-47) à l'extrémité constituée par l'acide aminé en position (-1) représentée sur la figure 4a et la figure 4b,
- celle s'étendant de l'extrémité constituée par l'acide aminé en position (-42) à l'extrémité constituée par l'acide aminé en position (294) représentée sur la figure 4a et la figure 4b,
- celle s'étendant de l'extrémité constituée par l'acide aminé en position (-42) à l'extrémité constituée par l'acide aminé en position (-1) représentée sur la figure 4a et la figure 4b,
- celle s'étendant de l'extrémité constituée par l'acide aminé en position (-29) à l'extrémité constituée par l'acide aminé en position (294) représentée sur la figure 4a et la figure 4b,
- celle s'étendant de l'extrémité constituée par l'acide aminé en position (-29) à l'extrémité constituée par l'acide aminé en position (-1) représentée sur la figure 4a et la figure 4b,
- celle s'étendant de l'extrémité constituée par l'acide aminé en position (1) à l'extrémité constituée par l'acide aminé en position (294) représentée sur la figure 4a et la figure 4b,
- celle s'étendant de l'extrémité constituée par l'acide aminé en position (12) à l'extrémité constituée par l'acide aminé en position (31) représentée sur la figure 4a et la figure 4b,
- celle s'étendant de l'extrémité constituée par l'acide aminé en position (36) à l'extrémité constituée par l'acide aminé en position (55) représentée sur la figure 4a et la figure 4b,
- celle s'étendant de l'extrémité constituée par l'acide aminé en position (77) à l'extrémité constituée par l'acide aminé en position (96) représentée sur la figure 4a et la figure 4b,
- celle s'étendant de l'extrémité constituée par l'acide aminé en position (101) à l'extrémité constituée par l'acide aminé en position (120) représentée sur la figure 4a et la figure 4b,
- celle s'étendant de l'extrémité constituée par l'acide aminé en position (175) à l'extrémité constituée par l'acide aminé en position (194) représentée sur la figure 4a et la figure 4b,
- celle s'étendant de l'extrémité constituée par l'acide aminé en position (211) à l'extrémité constituée par l'acide aminé en position (230) représentée sur la figure 4a et la figure 4b,
- celle s'étendant de l'extrémité constituée par l'acide aminé en position (275) à l'extrémité constituée par l'acide aminé en position (294) représentée sur la figure 4a et la figure 4b.

6. Polypeptide recombinant selon la revendication 3, constitué d'une des séquences d'acides aminés suivantes:
- celle s'étendant de l'extrémité constituée par l'acide aminé en position (-43) à l'extrémité constituée par l'acide aminé en position (295) représentée sur la figure 5,
- celle s'étendant de l'extrémité constituée par l'acide aminé en position (-43) à l'extrémité constituée par l'acide aminé en position (-1) représentée sur la figure 5,
- celle s'étendant de l'extrémité constituée par l'acide aminé en position (-30) à l'extrémité constituée par l'acide aminé en position (295) représentée sur la figure 5,
- celle s'étendant de l'extrémité constituée par l'acide aminé en position (-30) à l'extrémité constituée par l'acide aminé en position (-1) représentée sur la figure 5,
- celle s'étendant de l'extrémité constituée par l'acide aminé en position (1) à l'extrémité constituée par l'acide aminé en position (295) représentée sur la figure 5,
- celle s'étendant de l'extrémité constituée par l'acide aminé en position (12) à l'extrémité constituée par l'acide aminé en position (31) représentée sur la figure 5,
- celle s'étendant de l'extrémité constituée par l'acide aminé en position (36) à l'extrémité constituée par l'acide aminé en position (55) représentée sur la figure 5,
- celle s'étendant de l'extrémité constituée par l'acide aminé en position (77) à l'extrémité constituée par l'acide aminé en position (96) représentée sur la figure 5,
- celle s'étendant de l'extrémité constituée par l'acide aminé en position (101) à l'extrémité constituée par l'acide aminé en position (120) représentée sur la figure 5,
- celle s'étendant de l'extrémité constituée par l'acide aminé en position (175) à l'extrémité constituée par l'acide aminé en position (194) représentée sur la figure 5,
- celle s'étendant de l'extrémité constituée par l'acide aminé en position (211) à l'extrémité constituée par l'acide aminé en position (230) représentée sur la figure 5,
- celle s'étendant de l'extrémité constituée par l'acide aminé en position (275) à l'extrémité constituée par l'acide aminé en position (295) représentée sur la figure 5.

7. Polypeptide recombinant biologiquement pur constitué d'un polypeptide selon l'une quelconque des revendications 1 à 6 et d'une protéine possédant une séquence hétérologue vis-à-vis dudit polypeptide, ladite séquence hétérologue comprenant d'environ 1 à environ 1 000 acides aminés.

8. Polypeptide recombinant selon la revendication 7, dans lequel la protéine hétérologue est la β-galactosidase.

9. Acide nucléique comprenant
(a) une séquence nucléotidique codant pour un polypeptide selon l'une quelconque des revendications 1 à 6, ou
(b) une séquence nucléotidique qui est complémentaire de (a), ou
(c) les séquences nucléotidiques de (a) ou de (b) dans lesquelles T peut être remplacé par U.

10. Acide nucléique comprenant au moins une des séquences nucléotidiques suivantes:
- celle s'étendant de l'extrémité constituée par le nucléotide en position (1) à l'extrémité constituée par le nucléotide en position (182) représentée sur la figure 3a et la figure 3b,
- celle s'étendant de l'extrémité constituée par le nucléotide en position (273) à l'extrémité constituée par le nucléotide en position (359) représentée sur la figure 3a et la figure 3b,
- celle s'étendant de l'extrémité constituée par le nucléotide en position (360) à l'extrémité constituée par le nucléotide en position (1241) représentée sur la figure 3a et la figure 3b,
- celle s'étendant de l'extrémité constituée par le nucléotide en position (1242) à l'extrémité constituée par le nucléotide en position (1358), dans laquelle N représente un des cinq nucléotides A, T, C, G ou I, représentée sur la figure 3a et la figure 3b,
- celle s'étendant de l'extrémité constituée par le nucléotide en position (1) à l'extrémité constituée par le nucléotide en position (194) représentée sur la figure 3a et la figure 3b,
- celle s'étendant de l'extrémité constituée par le nucléotide en position (195) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (212) représentée sur la figure 3a et la figure 3b,
- celle s'étendant de l'extrémité constituée par le nucléotide en position (213) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (218) représentée sur la figure 3a et la figure 3b,
- celle s'étendant de l'extrémité constituée par le nucléotide en position (219) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (272) représentée sur la figure 3a et la figure 3b,
- celle s'étendant de l'extrémité constituée par le nucléotide en position (1) à l'extrémité constituée par le nucléotide en position (359) représentée sur la figure 3a et la figure 3b,
- celle s'étendant de l'extrémité constituée par le nucléotide en position (183) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (1358) représentée sur la figure 3a et la figure 3b,
- celle s'étendant de l'extrémité constituée par le nucléotide en position (1) à l'extrémité constituée par le nucléotide en position (1241) représentée sur la figure 3a et la figure 3b,
- celle s'étendant de l'extrémité constituée par le nucléotide en position (183) à l'extrémité constituée par le nucléotide en position (1358) représentée sur la figure 3a et la figure 3b,
- celle s'étendant de l'extrémité constituée par le nucléotide en position (183) à l'extrémité constituée par le nucléotide en position (1241) représentée sur la figure 3a et la figure 3b,
- celle s'étendant de l'extrémité constituée par le nucléotide en position (195) à l'extrémité constituée par le nucléotide en position (1358) représentée sur la figure 3a et la figure 3b,
- celle s'étendant de l'extrémité constituée par le nucléotide en position (195) à l'extrémité constituée par le nucléotide en position (1241) représentée sur la figure 3a et la figure 3b,
- celle s'étendant de l'extrémité constituée par le nucléotide en position (213) à l'extrémité constituée par le nucléotide en position (1358) représentée sur la figure 3a et la figure 3b,
- celle s'étendant de l'extrémité constituée par le nucléotide en position (213) à l'extrémité constituée par le nucléotide en position (1241) représentée sur la figure 3a et la figure 3b,
- celle s'étendant de l'extrémité constituée par le nucléotide en position (219) à l'extrémité constituée par le nucléotide en position (1358) représentée sur la figure 3a et la figure 3b,
- celle s'étendant de l'extrémité constituée par le nucléotide en position (219) à l'extrémité constituée par le nucléotide en position (1241) représentée sur la figure 3a et la figure 3b,
- celle s'étendant de l'extrémité constituée par le nucléotide en position (234) à l'extrémité constituée par le nucléotide en position (359) représentée sur la figure 3a et la figure 3b,
- celle s'étendant de l'extrémité constituée par le nucléotide en position (234) à l'extrémité constituée par le nucléotide en position (1358) représentée sur la figure 3a et la figure 3b,
- celle s'étendant de l'extrémité constituée par le nucléotide en position (234) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1358) représentée sur la figure 3a et la figure 3b,
- celle s'étendant de l'extrémité constituée par le nucléotide en position (360) à l'extrémité constituée par le nucléotide en position (1358) représentée sur la figure 3a et la figure 3b,
ou leurs séquences complémentaires,
ou lesdites séquences nucléotidiques ci-dessus dans lesquelles T est remplacé par U.

11. Acide nucléique selon la revendication 10 comprenant au moins une des séquences nucléotidiques suivantes:
- celle s'étendant de l'extrémité constituée par le nucléotide en position (1) à l'extrémité constituée par le nucléotide en position (182) représentée sur la figure 4a et la figure 4b,
- celle s'étendant de l'extrémité constituée par le nucléotide en position (273) à l'extrémité constituée par le nucléotide en position (359) représentée sur la figure 4a et la figure 4b,
- celle s'étendant de l'extrémité constituée par le nucléotide en position (360) à l'extrémité constituée par le nucléotide en position (1241) représentée sur la figure 4a et la figure 4b,
- celle s'étendant de l'extrémité constituée par le nucléotide en position (1242) à l'extrémité constituée par le nucléotide en position (1358), dans laquelle N représente un des cinq nucléotides A, T, C, G ou I, représentée sur la figure 4a et la figure 4b,
- celle s'étendant de l'extrémité constituée par le nucléotide en position (1) à l'extrémité constituée par le nucléotide en position (194) représentée sur la figure 4a et la figure 4b,
- celle s'étendant de l'extrémité constituée par le nucléotide en position (195) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (212) représentée sur la figure 4a et la figure 4b,
- celle s'étendant de l'extrémité constituée par le nucléotide en position (213) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (218) représentée sur la figure 4a et la figure 4b,
- celle s'étendant de l'extrémité constituée par le nucléotide en position (219) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (272) représentée sur la figure 4a et la figure 4b,
- celle s'étendant de l'extrémité constituée par le nucléotide en position (1) à l'extrémité constituée par le nucléotide en position (359) représentée sur la figure 4a et la figure 4b,
- celle s'étendant de l'extrémité constituée par le nucléotide en position (183) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (1358) représentée sur la figure 4a et la figure 4b,
- celle s'étendant de l'extrémité constituée par le nucléotide en position (1) à l'extrémité constituée par le nucléotide en position (1241) représentée sur la figure 4a et la figure 4b,
- celle s'étendant de l'extrémité constituée par le nucléotide en position (183) à l'extrémité constituée par le nucléotide en position (1358) représentée sur la figure 4a et la figure 4b,
- celle s'étendant de l'extrémité constituée par le nucléotide en position (183) à l'extrémité constituée par le nucléotide en position (1241) représentée sur la figure 4a et la figure 4b,
- celle s'étendant de l'extrémité constituée par le nucléotide en position (195) à l'extrémité constituée par le nucléotide en position (1358) représentée sur la figure 4a et la figure 4b,
- celle s'étendant de l'extrémité constituée par le nucléotide en position (195) à l'extrémité constituée par le nucléotide en position (1241) représentée sur la figure 4a et la figure 4b,
- celle s'étendant de l'extrémité constituée par le nucléotide en position (213) à l'extrémité constituée par le nucléotide en position (1358) représentée sur la figure 4a et la figure 4b,
- celle s'étendant de l'extrémité constituée par le nucléotide en position (213) à l'extrémité constituée par le nucléotide en position (1241) représentée sur la figure 4a et la figure 4b,
- celle s'étendant de l'extrémité constituée par le nucléotide en position (219) à l'extrémité constituée par le nucléotide en position (1358) représentée sur la figure 4a et la figure 4b,
- celle s'étendant de l'extrémité constituée par le nucléotide en position (219) à l'extrémité constituée par le nucléotide en position (1241) représentée sur la figure 4a et la figure 4b,
- celle s'étendant de l'extrémité constituée par le nucléotide en position (234) à l'extrémité constituée par le nucléotide en position (359) représentée sur la figure 4a et la figure 4b,
- celle s'étendant de l'extrémité constituée par le nucléotide en position (234) à l'extrémité constituée par le nucléotide en position (1358) représentée sur la figure 4a et la figure 4b,
- celle s'étendant de l'extrémité constituée par le nucléotide en position (234) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1358) représentée sur la figure 4a et la figure 4b,
- celle s'étendant de l'extrémité constituée par le nucléotide en position (360) à l'extrémité constituée par le nucléotide en position (1358) représentée sur la figure 4a et la figure 4b,
ou leurs séquences complémentaires,
ou lesdites séquences nucléotidiques ci-dessus dans lesquelles T est remplacé par U.

12. Acide nucléique selon la revendication 10 comprenant au moins une des séquences nucléotidiques suivantes:
- celle s'étendant de l'extrémité constituée par le nucléotide en position (130) à l'extrémité constituée par le nucléotide en position (219) représentée sur la figure 5,
- celle s'étendant de l'extrémité constituée par le nucléotide en position (220) à l'extrémité constituée par le nucléotide en position (1104) représentée sur la figure 5,
- celle s'étendant de l'extrémité constituée par le nucléotide en position (1105) à l'extrémité constituée par le nucléotide en position (1299) représentée sur la figure 5,
- celle s'étendant de l'extrémité constituée par le nucléotide en position (1) à l'extrémité constituée par le nucléotide en position (129) représentée sur la figure 5,
- celle s'étendant de l'extrémité constituée par le nucléotide en position (1) à l'extrémité constituée par le nucléotide en position (219) représentée sur la figure 5,
- celle s'étendant de l'extrémité constituée par le nucléotide en position (1) à l'extrémité constituée par le nucléotide en position (1104) représentée sur la figure 5,
- celle s'étendant de l'extrémité constituée par le nucléotide en position (1) à l'extrémité constituée par le nucléotide en position (1299) représentée sur la figure 5,
- celle s'étendant de l'extrémité constituée par le nucléotide en position (90) à l'extrémité constituée par le nucléotide en position (219) représentée sur la figure 5,
- celle s'étendant de l'extrémité constituée par le nucléotide en position (90) à l'extrémité constituée par le nucléotide en position (1299) représentée sur la figure 5,
- celle s'étendant de l'extrémité constituée par le nucléotide en position (90) à l'extrémité constituée par le nucléotide en position (1104) représentée sur la figure 5,
- celle s'étendant de l'extrémité constituée par le nucléotide en position (130) à l'extrémité constituée par le nucléotide en position (1104) représentée sur la figure 5,
- celle s'étendant de l'extrémité constituée par le nucléotide en position (130) à l'extrémité constituée par le nucléotide en position (1299) représentée sur la figure 5,
- celle s'étendant de l'extrémité constituée par le nucléotide en position (220) à l'extrémité constituée par le nucléotide en position (1299) représentée sur la figure 5,
ou leurs séquences complémentaires,
ou lesdites séquences nucléotidiques ci-dessus dans lesquelles T est remplacé par U.

13. Acide nucléique selon la revendication 10 constitué d'une des séquences nucléotidiques suivantes:
- celle s'étendant de l'extrémité constituée par le nucléotide en position (1) à l'extrémité constituée par le nucléotide en position (182) représentée sur la figure 3a et la figure 3b,
- celle s'étendant de l'extrémité constituée par le nucléotide en position (1) à l'extrémité constituée par le nucléotide en position (194) représentée sur la figure 3a et la figure 3b,
- celle s'étendant de l'extrémité constituée par le nucléotide en position (1) à l'extrémité constituée par le nucléotide en position (212) représentée sur la figure 3a et la figure 3b,
- celle s'étendant de l'extrémité constituée par le nucléotide en position (1) à l'extrémité constituée par le nucléotide en position (218) représentée sur la figure 3a et la figure 3b,
- celle s'étendant de l'extrémité constituée par le nucléotide en position (1) à l'extrémité constituée par le nucléotide en position (272) représentée sur la figure 3a et la figure 3b,
- celle s'étendant de l'extrémité constituée par le nucléotide en position (1) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (1241) représentée sur la figure 3a et la figure 3b,
- celle s'étendant de l'extrémité constituée par le nucléotide en position (1) à l'extrémité constituée par le nucléotide en position (1358) représentée sur la figure 3a et la figure 3b,
- celle s'étendant de l'extrémité constituée par le nucléotide en position (183) à l'extrémité constituée par le nucléotide en position (359) représentée sur la figure 3a et la figure 3b,
- celle s'étendant de l'extrémité constituée par le nucléotide en position (183) à l'extrémité constituée par le nucléotide en position (1241) représentée sur la figure 3a et la figure 3b,
- celle s'étendant de l'extrémité constituée par le nucléotide en position (183) à l'extrémité constituée par le nucléotide en position (1358), représentée sur la figure 3a et la figure 3b,
- celle s'étendant de l'extrémité constituée par le nucléotide en position (195) à l'extrémité constituée par le nucléotide en position (359) représentée sur la figure 3a et la figure 3b,
- celle s'étendant de l'extrémité constituée par le nucléotide en position (195) à l'extrémité constituée par le nucléotide en position (1241) représentée sur la figure 3a et la figure 3b,
- celle s'étendant de l'extrémité constituée par le nucléotide en position (195) à l'extrémité constituée par le nucléotide en position (1358) représentée sur la figure 3a et la figure 3b,
- celle s'étendant de l'extrémité constituée par le nucléotide en position (213) à l'extrémité constituée par le nucléotide en position (359) représentée sur la figure 3a et la figure 3b,
- celle s'étendant de l'extrémité constituée par le nucléotide en position (213) à l'extrémité constituée par le nucléotide en position (1241) représentée sur la figure 3a et la figure 3b,
- celle s'étendant de l'extrémité constituée par le nucléotide en position (213) à l'extrémité constituée par le nucléotide en position (1358) représentée sur la figure 3a et la figure 3b,
- celle s'étendant de l'extrémité constituée par le nucléotide en position (219) à l'extrémité constituée par le nucléotide en position (359) représentée sur la figure 3a et la figure 3b,
- celle s'étendant de l'extrémité constituée par le nucléotide en position (219) à l'extrémité constituée par le nucléotide en position (1241) représentée sur la figure 3a et la figure 3b,
- celle s'étendant de l'extrémité constituée par le nucléotide en position (219) à l'extrémité constituée par le nucléotide en position (1358) représentée sur la figure 3a et la figure 3b,
- celle s'étendant de l'extrémité constituée par le nucléotide en position (234) à l'extrémité constituée par le nucléotide en position (359) représentée sur la figure 3a et la figure 3b,
- celle s'étendant de l'extrémité constituée par le nucléotide en position (234) à l'extrémité constituée par le nucléotide en position (1241) représentée sur la figure 3a et la figure 3b,
- celle s'étendant de l'extrémité constituée par le nucléotide en position (234) à l'extrémité constituée par le nucléotide en position (1358) représentée sur la figure 3a et la figure 3b,
- celle s'étendant de l'extrémité constituée par le nucléotide en position (273) à l'extrémité constituée par le nucléotide en position (359) représentée sur la figure 3a et la figure 3b,
- celle s'étendant de l'extrémité constituée par le nucléotide en position (273) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1358) représentée sur la figure 3a et la figure 3b, - celle s'étendant de l'extrémité constituée par le nucléotide en position (360) à l'extrémité constituée par le nucléotide en position (1241) représentée sur la figure 3a et la figure 3b,
- celle s'étendant de l'extrémité constituée par le nucléotide en position (360) à l'extrémité constituée par le nucléotide en position (1358) représentée sur la figure 3a et la figure 3b,
- celle s'étendant de l'extrémité constituée par le nucléotide en position (1242) à l'extrémité constituée par le nucléotide en position (1358) représentée sur la figure 3a et la figure 3b,
ou leurs séquences complémentaires,
ou lesdites séquences nucléotidiques ci-dessus dans lesquelles T est remplacé par U.

14. Acide nucléique selon la revendication 11 constitué d'une des séquences nucléotidiques suivantes:
- celle s'étendant de l'extrémité constituée par le nucléotide en position (1) à l'extrémité constituée par le nucléotide en position (182) représentée sur la figure 4a et la figure 4b,
- celle s'étendant de l'extrémité constituée par le nucléotide en position (1) à l'extrémité constituée par le nucléotide en position (194) représentée sur la figure 4a et la figure 4b,
- celle s'étendant de l'extrémité constituée par le nucléotide en position (1) à l'extrémité constituée par le nucléotide en position (212) représentée sur la figure 4a et la figure 4b,
- celle s'étendant de l'extrémité constituée par le nucléotide en position (1) à l'extrémité constituée par le nucléotide en position (218) représentée sur la figure 4a et la figure 4b,
- celle s'étendant de l'extrémité constituée par le nucléotide en position (1) à l'extrémité constituée par le nucléotide en position (272) représentée sur la figure 4a et la figure 4b,
- celle s'étendant de l'extrémité constituée par le nucléotide en position (1) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (1241) représentée sur la figure 4a et la figure 4b,
- celle s'étendant de l'extrémité constituée par le nucléotide en position (1) à l'extrémité constituée par le nucléotide en position (1358) représentée sur la figure 4a et la figure 4b,
- celle s'étendant de l'extrémité constituée par le nucléotide en position (183) à l'extrémité constituée par le nucléotide en position (359) représentée sur la figure 4a et la figure 4b,
- celle s'étendant de l'extrémité constituée par le nucléotide en position (183) à l'extrémité constituée par le nucléotide en position (1241) représentée sur la figure 4a et la figure 4b,
- celle s'étendant de l'extrémité constituée par le nucléotide en position (183) à l'extrémité constituée par le nucléotide en position (1358), représentée sur la figure 4a et la figure 4b,
- celle s'étendant de l'extrémité constituée par le nucléotide en position (195) à l'extrémité constituée par le nucléotide en position (359) représentée sur la figure 4a et la figure 4b,
- celle s'étendant de l'extrémité constituée par le nucléotide en position (195) à l'extrémité constituée par le nucléotide en position (1241) représentée sur la figure 4a et la figure 4b,
- celle s'étendant de l'extrémité constituée par le nucléotide en position (195) à l'extrémité constituée par le nucléotide en position (1358) représentée sur la figure 4a et la figure 4b,
- celle s'étendant de l'extrémité constituée par le nucléotide en position (213) à l'extrémité constituée par le nucléotide en position (359) représentée sur la figure 4a et la figure 4b,
- celle s'étendant de l'extrémité constituée par le nucléotide en position (213) à l'extrémité constituée par le nucléotide en position (1241) représentée sur la figure 4a et la figure 4b,
- celle s'étendant de l'extrémité constituée par le nucléotide en position (213) à l'extrémité constituée par le nucléotide en position (1358) représentée sur la figure 4a et la figure 4b,
- celle s'étendant de l'extrémité constituée par le nucléotide en position (219) à l'extrémité constituée par le nucléotide en position (359) représentée sur la figure 4a et la figure 4b,
- celle s'étendant de l'extrémité constituée par le nucléotide en position (219) à l'extrémité constituée par le nucléotide en position (1241) représentée sur la figure 4a et la figure 4b,
- celle s'étendant de l'extrémité constituée par le nucléotide en position (219) à l'extrémité constituée par le nucléotide en position (1358) représentée sur la figure 4a et la figure 4b,
- celle s'étendant de l'extrémité constituée par le nucléotide en position (234) à l'extrémité constituée par le nucléotide en position (359) représentée sur la figure 4a et la figure 4b,
- celle s'étendant de l'extrémité constituée par le nucléotide en position (234) à l'extrémité constituée par le nucléotide en position (1241) représentée sur la figure 4a et la figure 4b,
- celle s'étendant de l'extrémité constituée par le nucléotide en position (234) à l'extrémité constituée par le nucléotide en position (1358) représentée sur la figure 4a et la figure 4b,
- celle s'étendant de l'extrémité constituée par le nucléotide en position (273) à l'extrémité constituée par le nucléotide en position (359) représentée sur la figure 4a et la figure 4b,
- celle s'étendant de l'extrémité constituée par le nucléotide en position (273) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1358) représentée sur la figure 4a et la figure 4b,
- celle s'étendant de l'extrémité constituée par le nucléotide en position (360) à l'extrémité constituée par le nucléotide en position (1241) représentée sur la figure 4a et la figure 4b,
- celle s'étendant de l'extrémité constituée par le nucléotide en position (360) à l'extrémité constituée par le nucléotide en position (1358) représentée sur la figure 4a et la figure 4b,
- celle s'étendant de l'extrémité constituée par le nucléotide en position (1242) à l'extrémité constituée par le nucléotide en position (1358) représentée sur la figure 4a et la figure 4b,
ou leurs séquences complémentaires,
ou lesdites séquences nucléotidiques ci-dessus dans lesquelles T est remplacé par U.

15. Acide nucléique selon la revendication 12 constitué d'une des séquences nucléotidiques suivantes:
- celle s'étendant de l'extrémité constituée par le nucléotide en position (1) à l'extrémité constituée par le nucléotide en position (129) représentée sur la figure 5,
- celle s'étendant de l'extrémité constituée par le nucléotide en position (1) à l'extrémité constituée par le nucléotide en position (219) représentée sur la figure 5,
- celle s'étendant de l'extrémité constituée par le nucléotide en position (1) à l'extrémité constituée par le nucléotide en position (1104) représentée sur la figure 5,
- celle s'étendant de l'extrémité constituée par le nucléotide en position (1) à l'extrémité constituée par le nucléotide en position (1299) représentée sur la figure 5,
- celle s'étendant de l'extrémité constituée par le nucléotide en position (90) à l'extrémité constituée par le nucléotide en position (219) représentée sur la figure 5,
- celle s'étendant de l'extrémité constituée par le nucléotide en position (90) à l'extrémité constituée par le nucléotide en position (1104) représentée sur la figure 5,
- celle s'étendant de l'extrémité constituée par le nucléotide en position (90) à l'extrémité constituée par le nucléotide en position (1299) représentée sur la figure 5,
- celle s'étendant de l'extrémité constituée par le nucléotide en position (130) à l'extrémité constituée par le nucléotide en position (219) représentée sur la figure 5,
- celle s'étendant de l'extrémité constituée par le nucléotide en position (130) à l'extrémité constituée par le nucléotide en position (1104) représentée sur la figure 5,
- celle s'étendant de l'extrémité constituée par le nucléotide en position (130) à l'extrémité constituée par le nucléotide en position (1299) représentée sur la figure 5,
- celle s'étendant de l'extrémité constituée par le nucléotide en position (220) à l'extrémité constituée par le nucléotide en position (1104) représentée sur la figure 5,
- celle s'étendant de l'extrémité constituée par le nucléotide en position (220) à l'extrémité constituée par le nucléotide en position (1299) représentée sur la figure 5,
- celle s'étendant de l'extrémité constituée par le nucléotide en position (1105) à l'extrémité constituée par le nucléotide en position (1299) représentée sur la figure 5,
ou leurs séquences complémentaires,
ou lesdites séquences nucléotidiques ci-dessus dans lesquelles T est remplacé par U.

16. Acide nucléique recombinant contenant au moins une des séquences nucléotidiques selon les revendications 9 à 15, insérées dans un acide nucléique hétérologue.

17. Amorce d'ADN ou d'ARN constituée de 15 à 25 nucléotides d'une séquence nucléotidique selon l'une quelconque des revendications 13 à 15, qui peut être utilisée comme amorce pour amplifier spécifiquement l'une quelconque desdites séquences nucléotidiques.

18. Amorce d'ADN ou d'ARN selon la revendication 17, ladite amorce étant choisie dans le groupe suivant des séquences nucléotidiques:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
ou leurs séquences complémentaires ou les séquences ribonucléotidiques correspondantes.

19. Jeu d'amorces d'ADN ou d'ARN pour l'amplification enzymatique d'une séquence nucléotidique selon l'une quelconque des revendications 13 à 15, ledit jeu d'amorces comprenant:
- une séquence nucléotidique A combinée avec la séquence nucléotidique complémentaire de B, de C, de D, de E ou de F; ou
- la séquence nucléotidique B combinée avec la séquence nucléotidique complémentaire de C, de D, de E ou de F; ou
- la séquence nucléotidique C combinée avec la séquence nucléotidique complémentaire de D, de E ou de F; ou
- la séquence nucléotidique D combinée avec la séquence nucléotidique complémentaire de E ou de F; ou
- la séquence nucléotidique E combinée avec une séquence nucléotidique complémentaire de F;
les séquences nucléotidiques A, B, C, D, E et F étant précisées dans la revendication 18, et A signifiant l'une quelconque des séquences A(i), A(ii), A(iii), A(iv) et A(v) et F signifiant l'une quelconque des séquences F(i), F(ii), F(iii) et F(iv).

20. Vecteur recombinant, en particulier pour le clonage et/ou l'expression, comprenant une séquence de vecteur de type plasmide, cosmide ou phage, et un acide nucléique selon l'une quelconque des revendications 9 à 15, dans un des sites non essentiels pour sa réplication.

21. Vecteur recombinant selon la revendication 20, contenant, dans un des sites non essentiels pour sa réplication, les éléments nécessaires pour promouvoir l'expression d'un polypeptide selon l'une quelconque des revendications 1 à 6 dans un hôte cellulaire.

22. Vecteur recombinant selon la revendication 21, contenant les éléments permettant l'expression par E. coli d'un polypeptide selon l'une quelconque des revendications 1 à 6.

23. Hôte cellulaire contenant un vecteur recombinant selon l'une quelconque des revendications 20 à 22 et comprenant les éléments de régulation permettant l'expression d'un polypeptide selon l'une quelconque des revendications 1 à 6 dans cet hôte.

24. Hôte cellulaire selon la revendication 23, choisi parmi les bactéries telles que E. coli transformées par un vecteur selon la revendication 20, ou choisi parmi les organismes eucaryotes transformés par un vecteur selon la revendication 20.

25. Anticorps monoclonal spécifiquement dirigé contre un polypeptide recombinant selon l'une quelconque des revendications 1 à 6.

26. Sonde nucléotidique s'hybridant avec un acide nucléique selon l'une quelconque des revendications 13 à 15, choisie parmi le groupe suivant de séquences nucléotidiques:
| Sondes A(i), A(ii), A(iii), A(iv) et A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Sonde B | |
|---|---|
| TCGCCCGCCCTGTACCTG, | |
| Sonde C | |
|---|---|
| GCGCTGACGCTGGCGATCTATC, | |
| Sonde D | |
|---|---|
| CCGCTGTTGAACGTCGGGAAG, | |
| Sonde E | |
|---|---|
| AAGCCGTCGGATCTGGGTGGCAAC, | |
| Sondes F(i) et F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| ou leurs séquences nucléotidiques complémentaires. | |

27. Procédé pour préparer un polypeptide recombinant selon l'une quelconque des revendications 1 à 8, comprenant les étapes suivantes:
- la culture, dans un milieu approprié, d'un hôte cellulaire ayant été préalablement transformé par un vecteur approprié contenant un acide nucléique selon l'une quelconque des revendications 9 à 15, et
- la récupération du polypeptide produit par ledit hôte cellulaire transformé ci-dessus dans ledit milieu de culture ci-dessus.

28. Procédé de diagnostic in vitro de la tuberculose chez un patient susceptible d'être infecté par Mycobacterium tuberculosis, comprenant les étapes suivantes:
- l'amplification d'un acide nucléique selon l'une quelconque des revendications 9 à 15, susceptible d'être contenu dans un échantillon biologique prélevé chez ledit patient, au moyen d'une amorce selon l'une quelconque des revendications 17 à 18, et/ou
- la mise en contact de l'échantillon biologique mentionné ci-dessus avec une sonde nucléotidique selon la revendication 26, dans des conditions permettant la formation d'un complexe d'hybridation entre ladite sonde et ledit acide nucléique de l'échantillon, et
- la détection dudit complexe d'hybridation ci-dessus.

29. Procédé de diagnostic in vitro de la tuberculose chez un patient susceptible d'être infecté par Mycobacterium tuberculosis, comprenant:
- la mise en contact d'un échantillon biologique prélevé chez un patient avec un polypeptide selon l'une quelconque des revendications 1 à 8, dans des conditions permettant une réaction immunologique in vitro entre ledit polypeptide et les anticorps éventuellement présents dans l'échantillon biologique, et
- la détection in vitro du complexe antigène/anticorps éventuellement formé.

30. Procédé de diagnostic in vitro de la tuberculose chez un patient susceptible d'être infecté par M. tuberculosis, comprenant les étapes suivantes:
- la mise en contact de l'échantillon biologique avec un anticorps selon la revendication 25, dans des conditions permettant une réaction immunologique in vitro entre ledit anticorps et les antigènes de M. tuberculosis éventuellement présents dans l'échantillon biologique, et
- la détection in vitro du complexe antigène/anticorps éventuellement formé.

31. Trousse pour un procédé diagnostique in vitro de la tuberculose chez un patient susceptible d'être infecté par Mycobacterium tuberculosis selon la revendication 28, ladite trousse comprenant une quantité déterminée d'une sonde nucléotidique selon la revendication 26.

32. Trousse pour un procédé diagnostique in vitro de la tuberculose chez un patient susceptible d'être infecté par Mycobacterium tuberculosis selon la revendication 29, comprenant:
- un polypeptide selon l'une quelconque des revendications 1 à 8,
- des réactifs pour préparer un milieu approprié pour qu'ait lieu la réaction immunologique,
- des réactifs permettant de détecter le complexe antigène/anticorps qui a été produit par la réaction immunologique, lesdits réactifs comportant éventuellement un marqueur, ou étant susceptibles d'être reconnus par un réactif marqué, plus particulièrement dans le cas où le polypeptide mentionné ci-dessus n'est pas marqué.

33. Trousse pour un procédé diagnostique in vitro de la tuberculose chez un patient susceptible d'être infecté par Mycobacterium tuberculosis selon la revendication 30, comprenant:
- un anticorps selon la revendication 25,
- des réactifs pour préparer un milieu approprié pour qu'ait lieu la réaction immunologique,
- des réactifs permettant de détecter le complexe antigène/anticorps qui a été produit par la réaction immunologique, lesdits réactifs comportant éventuellement un marqueur, ou étant susceptibles d'être reconnus par un réactif marqué, plus particulièrement dans le cas où le polypeptide
mentionné ci-dessus n'est pas marqué.

34. Polypeptide recombinant biologiquement pur selon l'une quelconque des revendications 1 à 8 à utiliser dans une composition immunogène en association avec un véhicule pharmaceutiquement acceptable.

35. Polypeptide recombinant biologiquement pur selon l'une quelconque des revendications 1 à 8 à utiliser dans une composition de vaccin.

36. Procédé pour l'amplification enzymatique d'une séquence nucléotidique selon l'une quelconque des revendications 13 à 15 et la détection de la séquence nucléotidique amplifiée, dans lequel l'amplification est réalisée par la technique ACP au moyen d'un jeu d'amorces et la détection du produit amplifié par ACP est réalisée par une réaction d'hybridation avec une sonde de détection, ledit jeu d'amorces et ladite sonde de détection étant choisis parmi les éléments suivants: 
Jeu d'amorces
P1 GAGTACCTGCAGGTGCCGTCGCCGTCGATGGGCCG
P2 compl. GTACCACTCGAACGCCGGGTGTTGAT
Sonde B
TCGCCCGCCCTGTACCTG
Jeu d'amorces
P1 GAGTACCTGCAGGTGCCGTCGCCGTCGATGGGCCG
P3 compl. TCCCACTTGTAAGTCTGGCA
Sonde B
TCGCCCGCCCTGTACCTG
Jeu d'amorces
P1 GAGTACCTGCAGGTGCCGTCGCCGTCGATGGGCCG
P4 compl. CGGCAGCTCGCTGGTCAGGA
Sonde B
TCGCCCGCCCTGTACCTG
Jeu d'amorces
P1 GAGTACCTGCAGGTGCCGTCGCCGTCGATGGGCCG
P5 compl. GCGTCACCCATCGCCAGGCCGATCAGG
Sonde B
TCGCCCGCCCTGTACCTG
Sonde C
GCGCTGACGCTGGCGATCTATC
 
Jeu d'amorces
P1 GAGTACCTGCAGGTGCCGTCGCCGTCGATGGGCCG
P6 compl. GCGCCCCAGTACTCCCAGCTGTGCGT
Sonde B
TCGCCCGCCCTGTACCTG
Sonde C
GCGCTGACGCTGGCGATCTATC
Sonde D
CCGCTGTTGAACGTCGGGAAG
Sonde E
AAGCCGTCGGATCTGGGTGGCAAC
Jeu d'amorces
P2 ATCAACACCCCGGCGTTCGAGTGGTAC
P5 compl. GCGTCACCCATCGCCAGGCCGATCAGG
Sonde C
CGCGTGACGCTGGCGATCTATC
Jeu d'amorces
P2 ATCAACACCCCGGCGTTCGAGTGGTAC
P6 compl. GCGCCCCAGTACTCCCAGCTGTGCGT
Sonde C
GCGCTGACGCTGGCGATCTATC
Sonde D
CCGCTGTTGAACGTCGGGAAG
Sonde E
AAGCCGTCGGATCTGGGTGGCAAC
Jeu d'amorces
P3 TGCCAGACTTACAAGTGGGA
P5 compl. GCGTCACCCATCGCCAGGCCGATCAGG
Sonde C
GCGCTGACGCTGGCGATCTATC
Jeu d'amorces
P3 TGCCAGACTTACAAGTGGGA
P6 compl. GCGCCCCAGTACTCCCAGCTGTGCGT
Sonde C
GCGCTGACGCTGGCGATCTATC
Sonde D
CCGCTGTTGAACGTCGGGAAG
Sonde E
 
AAGCCGTCGGATCTGGGTGGCAAC
Jeu d'amorces
P4 TCCTGACCAGCGAGCTGCCG
P5 compl. GCGTCACCCATCGCCAGGCCGATCAGG
Sonde C
GCGCTGACGCTGGCGATCTATC
Jeu d'amorces
P4 TCCTGACCAGCGAGCTGCCG
P6 compl. GCGCCCCAGTACTCCCAGCTGTGCGT
Sonde C
GCGCTGACGCTGGCGATCTATC
Sonde D
CCGCTGTTGAACGTCGGGAAG
Sonde E
AAGCCGTCGGATCTGGGTGGCAAC
Jeu d'amorces
P5 CCTGATCGGCCTGGCGATGGGTGACGC
P6 compl. GCGCCCCAGTACTCCCAGCTGTGCGT
Sonde D
CCGCTGTTGAACGTCGGGAAG
Sonde E
AAGCCGTCGGATCTGGGTGGCAAC
 ou ledit jeu d'amorces étant choisi parmi les jeux d'amorces selon la revendication 19, et la sonde de détection étant constituée d'une séquence oligonucléotidique d'au moins 10 nucléotides, ladite séquence étant située entre les deux amorces APC constituant le jeu d'amorces ayant été utilisé pour amplifier ladite séquence nucléotidique.

37. Vecteur recombinant selon la revendication 20, la séquence du vecteur possédant soit la séquence d'acides nucléiques représentée sur la figure 10b, soit la séquence d'acides nucléiques représentée sur la figure 11b, ou encore la séquence d'acides nucléiques représentée sur la figure 12b.

38. Peptides selon la revendication 1, avantageusement utilisés pour produire des anticorps, en particulier des anticorps monoclonaux et qui possèdent les séquences d'acides aminés suivantes:
| Position de l'acide aminé (extrémité NH₂) | | Position de l'acide aminé (extrémité COOH) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |

## Revendications (Revendications pour l'(les) Etat(s) contractant(s) suivant(s): ES, GR)

1. Polypeptide recombinant biologiquement pur contenant, dans sa chaîne polypeptidique, au moins une des séquences d'acides aminés suivantes:
- celle s'étendant de l'extrémité constituée par l'acide aminé en position (-29) à l'extrémité constituée par l'acide aminé en position (-1) représentée sur la figure 3a et la figure 3b, ou
- celle s'étendant de l'extrémité constituée par l'acide aminé en position (12) à l'extrémité constituée par l'acide aminé en position (31) représentée sur la figure 3a et la figure 3b, ou
- celle s'étendant de l'extrémité constituée par l'acide aminé en position (36) à l'extrémité constituée par l'acide aminé en position (55) représentée sur la figure 3a et la figure 3b, ou
- celle s'étendant de l'extrémité constituée par l'acide aminé en position (77) à l'extrémité constituée par l'acide aminé en position (96) représentée sur la figure 3a et la figure 3b, ou
- celle s'étendant de l'extrémité constituée par l'acide aminé en position (101) à l'extrémité constituée par l'acide aminé en position (120) représentée sur la figure 3a et la figure 3b, ou
- celle s'étendant de l'extrémité constituée par l'acide aminé en position (175) à l'extrémité constituée par l'acide aminé en position (194) représentée sur la figure 3a et la figure 3b, ou
- celle s'étendant de l'extrémité constituée par l'acide aminé en position (211) à l'extrémité constituée par l'acide aminé en position (230) représentée sur la figure 3a et la figure 3b, ou
- celle s'étendant de l'extrémité constituée par l'acide aminé en position (275) à l'extrémité constituée par l'acide aminé en position (294) représentée sur la figure 3a et la figure 3b, ou
- celle s'étendant de l'extrémité constituée par l'acide aminé en position (1) à l'extrémité constituée par l'acide aminé en position (294) représentée sur la figure 3a et la figure 3b, ou
- celle s'étendant de l'extrémité constituée par l'acide aminé en position (-42) à l'extrémité constituée par l'acide aminé en position (-1) représentée sur la figure 3a et la figure 3b, ou
- celle s'étendant de l'extrémité constituée par l'acide aminé en position (-47) à l'extrémité constituée par l'acide aminé en position (-1) représentée sur la figure 3a et la figure 3b, ou
- celle s'étendant de l'extrémité constituée par l'acide aminé en position (-49) à l'extrémité constituée par l'acide aminé en position (-1) représentée sur la figure 3a et la figure 3b, ou
- celle s'étendant de l'extrémité constituée par l'acide aminé en position (-55) à l'extrémité constituée par l'acide aminé en position (-1) représentée sur la figure 3a et la figure 3b, ou
- celle s'étendant de l'extrémité constituée par l'acide aminé en position (-59) à l'extrémité constituée par l'acide aminé en position (-1) représentée sur la figure 3a et la figure 3b, ou
- celle s'étendant de l'extrémité constituée par l'acide aminé en position (-29) à l'extrémité constituée par l'acide aminé en position (294) représentée sur la figure 3a et la figure 3b, ou
- celle s'étendant de l'extrémité constituée par l'acide aminé en position (-42) à l'extrémité constituée par l'acide aminé en position (294) représentée sur la figure 3a et la figure 3b, ou
- celle s'étendant de l'extrémité constituée par l'acide aminé en position (-47) à l'extrémité constituée par l'acide aminé en position (294) représentée sur la figure 3a et la figure 3b, ou
- celle s'étendant de l'extrémité constituée par l'acide aminé en position (-49) à l'extrémité constituée par l'acide aminé en position (294) représentée sur la figure 3a et la figure 3b, ou
- celle s'étendant de l'extrémité constituée par l'acide aminé en position (-55) à l'extrémité constituée par l'acide aminé en position (294) représentée sur la figure 3a et la figure 3b, ou
- celle s'étendant de l'extrémité constituée par l'acide aminé en position (-59) à l'extrémité constituée par l'acide aminé en position (294) représentée sur la figure 3a et la figure 3b,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

2. Polypeptide recombinant biologiquement pur selon la revendication 1 contenant, dans sa chaîne polypeptidique, au moins une des séquences d'acides aminés suivantes:
- celle s'étendant de l'extrémité constituée par l'acide aminé en position (-29) à l'extrémité constituée par l'acide aminé en position (-1) représentée sur la figure 4a et la figure 4b, ou
- celle s'étendant de l'extrémité constituée par l'acide aminé en position (12) à l'extrémité constituée par l'acide aminé en position (31) représentée sur la figure 4a et la figure 4b, ou
- celle s'étendant de l'extrémité constituée par l'acide aminé en position (36) à l'extrémité constituée par l'acide aminé en position (55) représentée sur la figure 4a et la figure 4b, ou
- celle s'étendant de l'extrémité constituée par l'acide aminé en position (77) à l'extrémité constituée par l'acide aminé en position (96) représentée sur la figure 4a et la figure 4b, ou
- celle s'étendant de l'extrémité constituée par l'acide aminé en position (101) à l'extrémité constituée par l'acide aminé en position (120) représentée sur la figure 4a et la figure 4b, ou
- celle s'étendant de l'extrémité constituée par l'acide aminé en position (175) à l'extrémité constituée par l'acide aminé en position (194) représentée sur la figure 4a et la figure 4b, ou
- celle s'étendant de l'extrémité constituée par l'acide aminé en position (211) à l'extrémité constituée par l'acide aminé en position (230) représentée sur la figure 4a et la figure 4b, ou
- celle s'étendant de l'extrémité constituée par l'acide aminé en position (275) à l'extrémité constituée par l'acide aminé en position (294) représentée sur la figure 4a et la figure 4b, ou
- celle s'étendant de l'extrémité constituée par l'acide aminé en position (1) à l'extrémité constituée par l'acide aminé en position (294) représentée sur la figure 4a et la figure 4b, ou
- celle s'étendant de l'extrémité constituée par l'acide aminé en position (-42) à l'extrémité constituée par l'acide aminé en position (-1) représentée sur la figure 4a et la figure 4b, ou
- celle s'étendant de l'extrémité constituée par l'acide aminé en position (-47) à l'extrémité constituée par l'acide aminé en position (-1) représentée sur la figure 4a et la figure 4b, ou
- celle s'étendant de l'extrémité constituée par l'acide aminé en position (-49) à l'extrémité constituée par l'acide aminé en position (-1) représentée sur la figure 4a et la figure 4b, ou
- celle s'étendant de l'extrémité constituée par l'acide aminé en position (-55) à l'extrémité constituée par l'acide aminé en position (-1) représentée sur la figure 4a et la figure 4b, ou
- celle s'étendant de l'extrémité constituée par l'acide aminé en position (-59) à l'extrémité constituée par l'acide aminé en position (-1) représentée sur la figure 4a et la figure 4b, ou
- celle s'étendant de l'extrémité constituée par l'acide aminé en position (-29) à l'extrémité constituée par l'acide aminé en position (294) représentée sur la figure 4a et la figure 4b, ou
- celle s'étendant de l'extrémité constituée par l'acide aminé en position (-42) à l'extrémité constituée par l'acide aminé en position (294) représentée sur la figure 4a et la figure 4b, ou
- celle s'étendant de l'extrémité constituée par l'acide aminé en position (-47) à l'extrémité constituée par l'acide aminé en position (294) représentée sur la figure 4a et la figure 4b, ou
- celle s'étendant de l'extrémité constituée par l'acide aminé en position (-49) à l'extrémité constituée par l'acide aminé en position (294) représentée sur la figure 4a et la figure 4b, ou
- celle s'étendant de l'extrémité constituée par l'acide aminé en position (-55) à l'extrémité constituée par l'acide aminé en position (294) représentée sur la figure 4a et la figure 4b, ou
- celle s'étendant de l'extrémité constituée par l'acide aminé en position (-59) à l'extrémité constituée par l'acide aminé en position (294) représentée sur la figure 4a et la figure 4b,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

3. Polypeptide recombinant biologiquement pur selon la revendication 1 contenant, dans sa chaîne polypeptidique, au moins une des séquences d'acides aminés suivantes:
- celle s'étendant de l'extrémité constituée par l'acide aminé en position (-30) à l'extrémité constituée par l'acide aminé en position (-1) représentée sur la figure 5, ou
- celle s'étendant de l'extrémité constituée par l'acide aminé en position (12) à l'extrémité constituée par l'acide aminé en position (31) représentée sur la figure 5, ou
- celle s'étendant de l'extrémité constituée par l'acide aminé en position (36) à l'extrémité constituée par l'acide aminé en position (55) représentée sur la figure 5, ou
- celle s'étendant de l'extrémité constituée par l'acide aminé en position (77) à l'extrémité constituée par l'acide aminé en position (96) représentée sur la figure 5, ou
- celle s'étendant de l'extrémité constituée par l'acide aminé en position (101) à l'extrémité constituée par l'acide aminé en position (120) représentée sur la figure 5, ou
- celle s'étendant de l'extrémité constituée par l'acide aminé en position (175) à l'extrémité constituée par l'acide aminé en position (194) représentée sur la figure 5, ou
- celle s'étendant de l'extrémité constituée par l'acide aminé en position (211) à l'extrémité constituée par l'acide aminé en position (230) représentée sur la figure 5, ou
- celle s'étendant de l'extrémité constituée par l'acide aminé en position (275) à l'extrémité constituée par l'acide aminé en position (295) représentée sur la figure 5,
- celle s'étendant de l'extrémité constituée par l'acide aminé en position (1) à l'extrémité constituée par l'acide aminé en position (295) représentée sur la figure 5, ou
- celle s'étendant de l'extrémité constituée par l'acide aminé en position (-43) à l'extrémité constituée par l'acide aminé en position (-1) représentée sur la figure 5, ou
- celle s'étendant de l'extrémité constituée par l'acide aminé en position (-30) à l'extrémité constituée par l'acide aminé en position (295) représentée sur la figure 5, ou
- celle s'étendant de l'extrémité constituée par l'acide aminé en position (-43) à l'extrémité constituée par l'acide aminé en position (295) représentée sur la figure 5,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

4. Polypeptide recombinant selon la revendication 1 constitué d'une des séquences d'acides aminés suivantes:
- celle s'étendant de l'extrémité constituée par l'acide aminé en position (-59) à l'extrémité constituée par l'acide aminé en position (294) représentée sur la figure 3a et la figure 3b,
- celle s'étendant de l'extrémité constituée par l'acide aminé en position (-59) à l'extrémité constituée par l'acide aminé en position (-1) représentée sur la figure 3a et la figure 3b,
- celle s'étendant de l'extrémité constituée par l'acide aminé en position (-55) à l'extrémité constituée par l'acide aminé en position (294) représentée sur la figure 3a et la figure 3b,
- celle s'étendant de l'extrémité constituée par l'acide aminé en position (-55) à l'extrémité constituée par l'acide aminé en position (-1) représentée sur la figure 3a et la figure 3b,
- celle s'étendant de l'extrémité constituée par l'acide aminé en position (-49) à l'extrémité constituée par l'acide aminé en position (294) représentée sur la figure 3a et la figure 3b,
- celle s'étendant de l'extrémité constituée par l'acide aminé en position (-49) à l'extrémité constituée par l'acide aminé en position (-1) représentée sur la figure 3a et la figure 3b,
- celle s'étendant de l'extrémité constituée par l'acide aminé en position (-47) à l'extrémité constituée par l'acide aminé en position (294) représentée sur la figure 3a et la figure 3b,
- celle s'étendant de l'extrémité constituée par l'acide aminé en position (-47) à l'extrémité constituée par l'acide aminé en position (-1) représentée sur la figure 3a et la figure 3b,
- celle s'étendant de l'extrémité constituée par l'acide aminé en position (-42) à l'extrémité constituée par l'acide aminé en position (294) représentée sur la figure 3a et la figure 3b,
- celle s'étendant de l'extrémité constituée par l'acide aminé en position (-42) à l'extrémité constituée par l'acide aminé en position (-1) représentée sur la figure 3a et la figure 3b,
- celle s'étendant de l'extrémité constituée par l'acide aminé en position (-29) à l'extrémité constituée par l'acide aminé en position (294) représentée sur la figure 3a et la figure 3b,
- celle s'étendant de l'extrémité constituée par l'acide aminé en position (-29) à l'extrémité constituée par l'acide aminé en position (-1) représentée sur la figure 3a et la figure 3b,
- celle s'étendant de l'extrémité constituée par l'acide aminé en position (1) à l'extrémité constituée par l'acide aminé en position (294) représentée sur la figure 3a et la figure 3b,
- celle s'étendant de l'extrémité constituée par l'acide aminé en position (12) à l'extrémité constituée par l'acide aminé en position (31) représentée sur la figure 3a et la figure 3b,
- celle s'étendant de l'extrémité constituée par l'acide aminé en position (36) à l'extrémité constituée par l'acide aminé en position (55) représentée sur la figure 3a et la figure 3b,
- celle s'étendant de l'extrémité constituée par l'acide aminé en position (77) à l'extrémité constituée par l'acide aminé en position (96) représentée sur la figure 3a et la figure 3b,
- celle s'étendant de l'extrémité constituée par l'acide aminé en position (101) à l'extrémité constituée par l'acide aminé en position (120) représentée sur la figure 3a et la figure 3b,
- celle s'étendant de l'extrémité constituée par l'acide aminé en position (175) à l'extrémité constituée par l'acide aminé en position (194) représentée sur la figure 3a et la figure 3b,
- celle s'étendant de l'extrémité constituée par l'acide aminé en position (211) à l'extrémité constituée par l'acide aminé en position (230) représentée sur la figure 3a et la figure 3b,
- celle s'étendant de l'extrémité constituée par l'acide aminé en position (275) à l'extrémité constituée par l'acide aminé en position (294) représentée sur la figure 3a et la figure 3b.

5. Polypeptide recombinant selon la revendication 2, constitué d'une des séquences d'acides aminés suivantes:
- celle s'étendant de l'extrémité constituée par l'acide aminé en position (-59) à l'extrémité constituée par l'acide aminé en position (294) représentée sur la figure 4a et la figure 4b,
- celle s'étendant de l'extrémité constituée par l'acide aminé en position (-59) à l'extrémité constituée par l'acide aminé en position (-1) représentée sur la figure 4a et la figure 4b,
- celle s'étendant de l'extrémité constituée par l'acide aminé en position (-55) à l'extrémité constituée par l'acide aminé en position (294) représentée sur la figure 4a et la figure 4b,
- celle s'étendant de l'extrémité constituée par l'acide aminé en position (-55) à l'extrémité constituée par l'acide aminé en position (-1) représentée sur la figure 4a et la figure 4b,
- celle s'étendant de l'extrémité constituée par l'acide aminé en position (-49) à l'extrémité constituée par l'acide aminé en position (294) représentée sur la figure 4a et la figure 4b,
- celle s'étendant de l'extrémité constituée par l'acide aminé en position (-49) à l'extrémité constituée par l'acide aminé en position (-1) représentée sur la figure 4a et la figure 4b,
- celle s'étendant de l'extrémité constituée par l'acide aminé en position (-47) à l'extrémité constituée par l'acide aminé en position (294) représentée sur la figure 4a et la figure 4b,
- celle s'étendant de l'extrémité constituée par l'acide aminé en position (-47) à l'extrémité constituée par l'acide aminé en position (-1) représentée sur la figure 4a et la figure 4b,
- celle s'étendant de l'extrémité constituée par l'acide aminé en position (-42) à l'extrémité constituée par l'acide aminé en position (294) représentée sur la figure 4a et la figure 4b,
- celle s'étendant de l'extrémité constituée par l'acide aminé en position (-42) à l'extrémité constituée par l'acide aminé en position (-1) représentée sur la figure 4a et la figure 4b,
- celle s'étendant de l'extrémité constituée par l'acide aminé en position (-29) à l'extrémité constituée par l'acide aminé en position (294) représentée sur la figure 4a et la figure 4b,
- celle s'étendant de l'extrémité constituée par l'acide aminé en position (-29) à l'extrémité constituée par l'acide aminé en position (-1) représentée sur la figure 4a et la figure 4b,
- celle s'étendant de l'extrémité constituée par l'acide aminé en position (1) à l'extrémité constituée par l'acide aminé en position (294) représentée sur la figure 4a et la figure 4b,
- celle s'étendant de l'extrémité constituée par l'acide aminé en position (12) à l'extrémité constituée par l'acide aminé en position (31) représentée sur la figure 4a et la figure 4b,
- celle s'étendant de l'extrémité constituée par l'acide aminé en position (36) à l'extrémité constituée par l'acide aminé en position (55) représentée sur la figure 4a et la figure 4b,
- celle s'étendant de l'extrémité constituée par l'acide aminé en position (77) à l'extrémité constituée par l'acide aminé en position (96) représentée sur la figure 4a et la figure 4b,
- celle s'étendant de l'extrémité constituée par l'acide aminé en position (101) à l'extrémité constituée par l'acide aminé en position (120) représentée sur la figure 4a et la figure 4b,
- celle s'étendant de l'extrémité constituée par l'acide aminé en position (175) à l'extrémité constituée par l'acide aminé en position (194) représentée sur la figure 4a et la figure 4b,
- celle s'étendant de l'extrémité constituée par l'acide aminé en position (211) à l'extrémité constituée par l'acide aminé en position (230) représentée sur la figure 4a et la figure 4b,
- celle s'étendant de l'extrémité constituée par l'acide aminé en position (275) à l'extrémité constituée par l'acide aminé en position (294) représentée sur la figure 4a et la figure 4b.

6. Polypeptide recombinant selon la revendication 3, constitué d'une des séquences d'acides aminés suivantes:
- celle s'étendant de l'extrémité constituée par l'acide aminé en position (-43) à l'extrémité constituée par l'acide aminé en position (295) représentée sur la figure 5,
- celle s'étendant de l'extrémité constituée par l'acide aminé en position (-43) à l'extrémité constituée par l'acide aminé en position (-1) représentée sur la figure 5,
- celle s'étendant de l'extrémité constituée par l'acide aminé en position (-30) à l'extrémité constituée par l'acide aminé en position (295) représentée sur la figure 5,
- celle s'étendant de l'extrémité constituée par l'acide aminé en position (-30) à l'extrémité constituée par l'acide aminé en position (-1) représentée sur la figure 5,
- celle s'étendant de l'extrémité constituée par l'acide aminé en position (1) à l'extrémité constituée par l'acide aminé en position (295) représentée sur la figure 5,
- celle s'étendant de l'extrémité constituée par l'acide aminé en position (12) à l'extrémité constituée par l'acide aminé en position (31) représentée sur la figure 5,
- celle s'étendant de l'extrémité constituée par l'acide aminé en position (36) à l'extrémité constituée par l'acide aminé en position (55) représentée sur la figure 5,
- celle s'étendant de l'extrémité constituée par l'acide aminé en position (77) à l'extrémité constituée par l'acide aminé en position (96) représentée sur la figure 5,
- celle s'étendant de l'extrémité constituée par l'acide aminé en position (101) à l'extrémité constituée par l'acide aminé en position (120) représentée sur la figure 5,
- celle s'étendant de l'extrémité constituée par l'acide aminé en position (175) à l'extrémité constituée par l'acide aminé en position (194) représentée sur la figure 5,
- celle s'étendant de l'extrémité constituée par l'acide aminé en position (211) à l'extrémité constituée par l'acide aminé en position (230) représentée sur la figure 5,
- celle s'étendant de l'extrémité constituée par l'acide aminé en position (275) à l'extrémité constituée par l'acide aminé en position (295) représentée sur la figure 5.

7. Polypeptide recombinant biologiquement pur constitué d'un polypeptide selon l'une quelconque des revendications 1 à 6 et d'une protéine possédant une séquence hétérologue vis-à-vis dudit polypeptide, ladite séquence hétérologue comprenant d'environ 1 à environ 1 000 acides aminés.

8. Polypeptide recombinant selon la revendication 7, dans lequel la protéine hétérologue est la β-galactosidase.

9. Acide nucléique comprenant
(a) une séquence nucléotidique codant pour un polypeptide selon l'une quelconque des revendications 1 à 6, ou
(b) une séquence nucléotidique qui est complémentaire de (a), ou
(c) les séquences nucléotidiques de (a) ou de (b) dans lesquelles T peut être remplacé par U.

10. Acide nucléique comprenant au moins une des séquences nucléotidiques suivantes:
- celle s'étendant de l'extrémité constituée par le nucléotide en position (1) à l'extrémité constituée par le nucléotide en position (182) représentée sur la figure 3a et la figure 3b,
- celle s'étendant de l'extrémité constituée par le nucléotide en position (273) à l'extrémité constituée par le nucléotide en position (359) représentée sur la figure 3a et la figure 3b,
- celle s'étendant de l'extrémité constituée par le nucléotide en position (360) à l'extrémité constituée par le nucléotide en position (1241) représentée sur la figure 3a et la figure 3b,
- celle s'étendant de l'extrémité constituée par le nucléotide en position (1242) à l'extrémité constituée par le nucléotide en position (1358), dans laquelle N représente un des cinq nucléotides A, T, C, G ou I, représentée sur la figure 3a et la figure 3b,
- celle s'étendant de l'extrémité constituée par le nucléotide en position (1) à l'extrémité constituée par le nucléotide en position (194) représentée sur la figure 3a et la figure 3b,
- celle s'étendant de l'extrémité constituée par le nucléotide en position (195) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (212) représentée sur la figure 3a et la figure 3b,
- celle s'étendant de l'extrémité constituée par le nucléotide en position (213) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (218) représentée sur la figure 3a et la figure 3b,
- celle s'étendant de l'extrémité constituée par le nucléotide en position (219) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (272) représentée sur la figure 3a et la figure 3b,
- celle s'étendant de l'extrémité constituée par le nucléotide en position (1) à l'extrémité constituée par le nucléotide en position (359) représentée sur la figure 3a et la figure 3b,
- celle s'étendant de l'extrémité constituée par le nucléotide en position (183) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (1358) représentée sur la figure 3a et la figure 3b,
- celle s'étendant de l'extrémité constituée par le nucléotide en position (1) à l'extrémité constituée par le nucléotide en position (1241) représentée sur la figure 3a et la figure 3b,
- celle s'étendant de l'extrémité constituée par le nucléotide en position (183) à l'extrémité constituée par le nucléotide en position (1358) représentée sur la figure 3a et la figure 3b,
- celle s'étendant de l'extrémité constituée par le nucléotide en position (183) à l'extrémité constituée par le nucléotide en position (1241) représentée sur la figure 3a et la figure 3b,
- celle s'étendant de l'extrémité constituée par le nucléotide en position (195) à l'extrémité constituée par le nucléotide en position (1358) représentée sur la figure 3a et la figure 3b,
- celle s'étendant de l'extrémité constituée par le nucléotide en position (195) à l'extrémité constituée par le nucléotide en position (1241) représentée sur la figure 3a et la figure 3b,
- celle s'étendant de l'extrémité constituée par le nucléotide en position (213) à l'extrémité constituée par le nucléotide en position (1358) représentée sur la figure 3a et la figure 3b,
- celle s'étendant de l'extrémité constituée par le nucléotide en position (213) à l'extrémité constituée par le nucléotide en position (1241) représentée sur la figure 3a et la figure 3b,
- celle s'étendant de l'extrémité constituée par le nucléotide en position (219) à l'extrémité constituée par le nucléotide en position (1358) représentée sur la figure 3a et la figure 3b,
- celle s'étendant de l'extrémité constituée par le nucléotide en position (219) à l'extrémité constituée par le nucléotide en position (1241) représentée sur la figure 3a et la figure 3b,
- celle s'étendant de l'extrémité constituée par le nucléotide en position (234) à l'extrémité constituée par le nucléotide en position (359) représentée sur la figure 3a et la figure 3b,
- celle s'étendant de l'extrémité constituée par le nucléotide en position (234) à l'extrémité constituée par le nucléotide en position (1358) représentée sur la figure 3a et la figure 3b,
- celle s'étendant de l'extrémité constituée par le nucléotide en position (234) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1358) représentée sur la figure 3a et la figure 3b,
- celle s'étendant de l'extrémité constituée par le nucléotide en position (360) à l'extrémité constituée par le nucléotide en position (1358) représentée sur la figure 3a et la figure 3b,
ou leurs séquences complémentaires,
ou lesdites séquences nucléotidiques ci-dessus dans lesquelles T est remplacé par U.

11. Acide nucléique selon la revendication 10 comprenant au moins une des séquences nucléotidiques suivantes:
- celle s'étendant de l'extrémité constituée par le nucléotide en position (1) à l'extrémité constituée par le nucléotide en position (182) représentée sur la figure 4a et la figure 4b,
- celle s'étendant de l'extrémité constituée par le nucléotide en position (273) à l'extrémité constituée par le nucléotide en position (359) représentée sur la figure 4a et la figure 4b,
- celle s'étendant de l'extrémité constituée par le nucléotide en position (360) à l'extrémité constituée par le nucléotide en position (1241) représentée sur la figure 4a et la figure 4b,
- celle s'étendant de l'extrémité constituée par le nucléotide en position (1242) à l'extrémité constituée par le nucléotide en position (1358), dans laquelle N représente un des cinq nucléotides A, T, C, G ou I, représentée sur la figure 4a et la figure 4b,
- celle s'étendant de l'extrémité constituée par le nucléotide en position (1) à l'extrémité constituée par le nucléotide en position (194) représentée sur la figure 4a et la figure 4b,
- celle s'étendant de l'extrémité constituée par le nucléotide en position (195) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (212) représentée sur la figure 4a et la figure 4b,
- celle s'étendant de l'extrémité constituée par le nucléotide en position (213) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (218) représentée sur la figure 4a et la figure 4b,
- celle s'étendant de l'extrémité constituée par le nucléotide en position (219) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (272) représentée sur la figure 4a et la figure 4b,
- celle s'étendant de l'extrémité constituée par le nucléotide en position (1) à l'extrémité constituée par le nucléotide en position (359) représentée sur la figure 4a et la figure 4b,
- celle s'étendant de l'extrémité constituée par le nucléotide en position (183) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (1358) représentée sur la figure 4a et la figure 4b,
- celle s'étendant de l'extrémité constituée par le nucléotide en position (1) à l'extrémité constituée par le nucléotide en position (1241) représentée sur la figure 4a et la figure 4b,
- celle s'étendant de l'extrémité constituée par le nucléotide en position (183) à l'extrémité constituée par le nucléotide en position (1358) représentée sur la figure 4a et la figure 4b,
- celle s'étendant de l'extrémité constituée par le nucléotide en position (183) à l'extrémité constituée par le nucléotide en position (1241) représentée sur la figure 4a et la figure 4b,
- celle s'étendant de l'extrémité constituée par le nucléotide en position (195) à l'extrémité constituée par le nucléotide en position (1358) représentée sur la figure 4a et la figure 4b,
- celle s'étendant de l'extrémité constituée par le nucléotide en position (195) à l'extrémité constituée par le nucléotide en position (1241) représentée sur la figure 4a et la figure 4b,
- celle s'étendant de l'extrémité constituée par le nucléotide en position (213) à l'extrémité constituée par le nucléotide en position (1358) représentée sur la figure 4a et la figure 4b,
- celle s'étendant de l'extrémité constituée par le nucléotide en position (213) à l'extrémité constituée par le nucléotide en position (1241) représentée sur la figure 4a et la figure 4b,
- celle s'étendant de l'extrémité constituée par le nucléotide en position (219) à l'extrémité constituée par le nucléotide en position (1358) représentée sur la figure 4a et la figure 4b,
- celle s'étendant de l'extrémité constituée par le nucléotide en position (219) à l'extrémité constituée par le nucléotide en position (1241) représentée sur la figure 4a et la figure 4b,
- celle s'étendant de l'extrémité constituée par le nucléotide en position (234) à l'extrémité constituée par le nucléotide en position (359) représentée sur la figure 4a et la figure 4b,
- celle s'étendant de l'extrémité constituée par le nucléotide en position (234) à l'extrémité constituée par le nucléotide en position (1358) représentée sur la figure 4a et la figure 4b,
- celle s'étendant de l'extrémité constituée par le nucléotide en position (234) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1358) représentée sur la figure 4a et la figure 4b,
- celle s'étendant de l'extrémité constituée par le nucléotide en position (360) à l'extrémité constituée par le nucléotide en position (1358) représentée sur la figure 4a et la figure 4b,
ou leurs séquences complémentaires,
ou lesdites séquences nucléotidiques ci-dessus dans lesquelles T est remplacé par U.

12. Acide nucléique selon la revendication 10 comprenant au moins une des séquences nucléotidiques suivantes:
- celle s'étendant de l'extrémité constituée par le nucléotide en position (130) à l'extrémité constituée par le nucléotide en position (219) représentée sur la figure 5,
- celle s'étendant de l'extrémité constituée par le nucléotide en position (220) à l'extrémité constituée par le nucléotide en position (1104) représentée sur la figure 5,
- celle s'étendant de l'extrémité constituée par le nucléotide en position (1105) à l'extrémité constituée par le nucléotide en position (1299) représentée sur la figure 5,
- celle s'étendant de l'extrémité constituée par le nucléotide en position (1) à l'extrémité constituée par le nucléotide en position (129) représentée sur la figure 5,
- celle s'étendant de l'extrémité constituée par le nucléotide en position (1) à l'extrémité constituée par le nucléotide en position (219) représentée sur la figure 5,
- celle s'étendant de l'extrémité constituée par le nucléotide en position (1) à l'extrémité constituée par le nucléotide en position (1104) représentée sur la figure 5,
- celle s'étendant de l'extrémité constituée par le nucléotide en position (1) à l'extrémité constituée par le nucléotide en position (1299) représentée sur la figure 5,
- celle s'étendant de l'extrémité constituée par le nucléotide en position (90) à l'extrémité constituée par le nucléotide en position (219) représentée sur la figure 5,
- celle s'étendant de l'extrémité constituée par le nucléotide en position (90) à l'extrémité constituée par le nucléotide en position (1299) représentée sur la figure 5,
- celle s'étendant de l'extrémité constituée par le nucléotide en position (90) à l'extrémité constituée par le nucléotide en position (1104) représentée sur la figure 5,
- celle s'étendant de l'extrémité constituée par le nucléotide en position (130) à l'extrémité constituée par le nucléotide en position (1104) représentée sur la figure 5,
- celle s'étendant de l'extrémité constituée par le nucléotide en position (130) à l'extrémité constituée par le nucléotide en position (1299) représentée sur la figure 5,
- celle s'étendant de l'extrémité constituée par le nucléotide en position (220) à l'extrémité constituée par le nucléotide en position (1299) représentée sur la figure 5,
ou leurs séquences complémentaires,
ou lesdites séquences nucléotidiques ci-dessus dans lesquelles T est remplacé par U.

13. Acide nucléique selon la revendication 10 constitué d'une des séquences nucléotidiques suivantes:
- celle s'étendant de l'extrémité constituée par le nucléotide en position (1) à l'extrémité constituée par le nucléotide en position (182) représentée sur la figure 3a et la figure 3b,
- celle s'étendant de l'extrémité constituée par le nucléotide en position (1) à l'extrémité constituée par le nucléotide en position (194) représentée sur la figure 3a et la figure 3b,
- celle s'étendant de l'extrémité constituée par le nucléotide en position (1) à l'extrémité constituée par le nucléotide en position (212) représentée sur la figure 3a et la figure 3b,
- celle s'étendant de l'extrémité constituée par le nucléotide en position (1) à l'extrémité constituée par le nucléotide en position (218) représentée sur la figure 3a et la figure 3b,
- celle s'étendant de l'extrémité constituée par le nucléotide en position (1) à l'extrémité constituée par le nucléotide en position (272) représentée sur la figure 3a et la figure 3b,
- celle s'étendant de l'extrémité constituée par le nucléotide en position (1) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (1241) représentée sur la figure 3a et la figure 3b,
- celle s'étendant de l'extrémité constituée par le nucléotide en position (1) à l'extrémité constituée par le nucléotide en position (1358) représentée sur la figure 3a et la figure 3b,
- celle s'étendant de l'extrémité constituée par le nucléotide en position (183) à l'extrémité constituée par le nucléotide en position (359) représentée sur la figure 3a et la figure 3b,
- celle s'étendant de l'extrémité constituée par le nucléotide en position (183) à l'extrémité constituée par le nucléotide en position (1241) représentée sur la figure 3a et la figure 3b,
- celle s'étendant de l'extrémité constituée par le nucléotide en position (183) à l'extrémité constituée par le nucléotide en position (1358), représentée sur la figure 3a et la figure 3b,
- celle s'étendant de l'extrémité constituée par le nucléotide en position (195) à l'extrémité constituée par le nucléotide en position (359) représentée sur la figure 3a et la figure 3b,
- celle s'étendant de l'extrémité constituée par le nucléotide en position (195) à l'extrémité constituée par le nucléotide en position (1241) représentée sur la figure 3a et la figure 3b,
- celle s'étendant de l'extrémité constituée par le nucléotide en position (195) à l'extrémité constituée par le nucléotide en position (1358) représentée sur la figure 3a et la figure 3b,
- celle s'étendant de l'extrémité constituée par le nucléotide en position (213) à l'extrémité constituée par le nucléotide en position (359) représentée sur la figure 3a et la figure 3b,
- celle s'étendant de l'extrémité constituée par le nucléotide en position (213) à l'extrémité constituée par le nucléotide en position (1241) représentée sur la figure 3a et la figure 3b,
- celle s'étendant de l'extrémité constituée par le nucléotide en position (213) à l'extrémité constituée par le nucléotide en position (1358) représentée sur la figure 3a et la figure 3b,
- celle s'étendant de l'extrémité constituée par le nucléotide en position (219) à l'extrémité constituée par le nucléotide en position (359) représentée sur la figure 3a et la figure 3b,
- celle s'étendant de l'extrémité constituée par le nucléotide en position (219) à l'extrémité constituée par le nucléotide en position (1241) représentée sur la figure 3a et la figure 3b,
- celle s'étendant de l'extrémité constituée par le nucléotide en position (219) à l'extrémité constituée par le nucléotide en position (1358) représentée sur la figure 3a et la figure 3b,
- celle s'étendant de l'extrémité constituée par le nucléotide en position (234) à l'extrémité constituée par le nucléotide en position (359) représentée sur la figure 3a et la figure 3b,
- celle s'étendant de l'extrémité constituée par le nucléotide en position (234) à l'extrémité constituée par le nucléotide en position (1241) représentée sur la figure 3a et la figure 3b,
- celle s'étendant de l'extrémité constituée par le nucléotide en position (234) à l'extrémité constituée par le nucléotide en position (1358) représentée sur la figure 3a et la figure 3b,
- celle s'étendant de l'extrémité constituée par le nucléotide en position (273) à l'extrémité constituée par le nucléotide en position (359) représentée sur la figure 3a et la figure 3b,
- celle s'étendant de l'extrémité constituée par le nucléotide en position (273) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1358) représentée sur la figure 3a et la figure 3b,
- celle s'étendant de l'extrémité constituée par le nucléotide en position (360) à l'extrémité constituée par le nucléotide en position (1241) représentée sur la figure 3a et la figure 3b,
- celle s'étendant de l'extrémité constituée par le nucléotide en position (360) à l'extrémité constituée par le nucléotide en position (1358) représentée sur la figure 3a et la figure 3b,
- celle s'étendant de l'extrémité constituée par le nucléotide en position (1242) à l'extrémité constituée par le nucléotide en position (1358) représentée sur la figure 3a et la figure 3b,
ou leurs séquences complémentaires,
ou lesdites séquences nucléotidiques ci-dessus dans lesquelles T est remplacé par U.

14. Acide nucléique selon la revendication 11 constitué d'une des séquences nucléotidiques suivantes:
- celle s'étendant de l'extrémité constituée par le nucléotide en position (1) à l'extrémité constituée par le nucléotide en position (182) représentée sur la figure 4a et la figure 4b,
- celle s'étendant de l'extrémité constituée par le nucléotide en position (1) à l'extrémité constituée par le nucléotide en position (194) représentée sur la figure 4a et la figure 4b,
- celle s'étendant de l'extrémité constituée par le nucléotide en position (1) à l'extrémité constituée par le nucléotide en position (212) représentée sur la figure 4a et la figure 4b,
- celle s'étendant de l'extrémité constituée par le nucléotide en position (1) à l'extrémité constituée par le nucléotide en position (218) représentée sur la figure 4a et la figure 4b,
- celle s'étendant de l'extrémité constituée par le nucléotide en position (1) à l'extrémité constituée par le nucléotide en position (272) représentée sur la figure 4a et la figure 4b,
- celle s'étendant de l'extrémité constituée par le nucléotide en position (1) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (1241) représentée sur la figure 4a et la figure 4b,
- celle s'étendant de l'extrémité constituée par le nucléotide en position (1) à l'extrémité constituée par le nucléotide en position (1358) représentée sur la figure 4a et la figure 4b,
- celle s'étendant de l'extrémité constituée par le nucléotide en position (183) à l'extrémité constituée par le nucléotide en position (359) représentée sur la figure 4a et la figure 4b,
- celle s'étendant de l'extrémité constituée par le nucléotide en position (183) à l'extrémité constituée par le nucléotide en position (1241) représentée sur la figure 4a et la figure 4b,
- celle s'étendant de l'extrémité constituée par le nucléotide en position (183) à l'extrémité constituée par le nucléotide en position (1358), représentée sur la figure 4a et la figure 4b,
- celle s'étendant de l'extrémité constituée par le nucléotide en position (195) à l'extrémité constituée par le nucléotide en position (359) représentée sur la figure 4a et la figure 4b,
- celle s'étendant de l'extrémité constituée par le nucléotide en position (195) à l'extrémité constituée par le nucléotide en position (1241) représentée sur la figure 4a et la figure 4b,
- celle s'étendant de l'extrémité constituée par le nucléotide en position (195) à l'extrémité constituée par le nucléotide en position (1358) représentée sur la figure 4a et la figure 4b,
- celle s'étendant de l'extrémité constituée par le nucléotide en position (213) à l'extrémité constituée par le nucléotide en position (359) représentée sur la figure 4a et la figure 4b,
- celle s'étendant de l'extrémité constituée par le nucléotide en position (213) à l'extrémité constituée par le nucléotide en position (1241) représentée sur la figure 4a et la figure 4b,
- celle s'étendant de l'extrémité constituée par le nucléotide en position (213) à l'extrémité constituée par le nucléotide en position (1358) représentée sur la figure 4a et la figure 4b,
- celle s'étendant de l'extrémité constituée par le nucléotide en position (219) à l'extrémité constituée par le nucléotide en position (359) représentée sur la figure 4a et la figure 4b,
- celle s'étendant de l'extrémité constituée par le nucléotide en position (219) à l'extrémité constituée par le nucléotide en position (1241) représentée sur la figure 4a et la figure 4b,
- celle s'étendant de l'extrémité constituée par le nucléotide en position (219) à l'extrémité constituée par le nucléotide en position (1358) représentée sur la figure 4a et la figure 4b,
- celle s'étendant de l'extrémité constituée par le nucléotide en position (234) à l'extrémité constituée par le nucléotide en position (359) représentée sur la figure 4a et la figure 4b,
- celle s'étendant de l'extrémité constituée par le nucléotide en position (234) à l'extrémité constituée par le nucléotide en position (1241) représentée sur la figure 4a et la figure 4b,
- celle s'étendant de l'extrémité constituée par le nucléotide en position (234) à l'extrémité constituée par le nucléotide en position (1358) représentée sur la figure 4a et la figure 4b,
- celle s'étendant de l'extrémité constituée par le nucléotide en position (273) à l'extrémité constituée par le nucléotide en position (359) représentée sur la figure 4a et la figure 4b,
- celle s'étendant de l'extrémité constituée par le nucléotide en position (273) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1358) représentée sur la figure 4a et la figure 4b,
- celle s'étendant de l'extrémité constituée par le nucléotide en position (360) à l'extrémité constituée par le nucléotide en position (1241) représentée sur la figure 4a et la figure 4b,
- celle s'étendant de l'extrémité constituée par le nucléotide en position (360) à l'extrémité constituée par le nucléotide en position (1358) représentée sur la figure 4a et la figure 4b,
- celle s'étendant de l'extrémité constituée par le nucléotide en position (1242) à l'extrémité constituée par le nucléotide en position (1358) représentée sur la figure 4a et la figure 4b,
ou leurs séquences complémentaires,
ou lesdites séquences nucléotidiques ci-dessus dans lesquelles T est remplacé par U.

15. Acide nucléique selon la revendication 12 constitué d'une des séquences nucléotidiques suivantes:
- celle s'étendant de l'extrémité constituée par le nucléotide en position (1) à l'extrémité constituée par le nucléotide en position (129) représentée sur la figure 5,
- celle s'étendant de l'extrémité constituée par le nucléotide en position (1) à l'extrémité constituée par le nucléotide en position (219) représentée sur la figure 5,
- celle s'étendant de l'extrémité constituée par le nucléotide en position (1) à l'extrémité constituée par le nucléotide en position (1104) représentée sur la figure 5,
- celle s'étendant de l'extrémité constituée par le nucléotide en position (1) à l'extrémité constituée par le nucléotide en position (1299) représentée sur la figure 5,
- celle s'étendant de l'extrémité constituée par le nucléotide en position (90) à l'extrémité constituée par le nucléotide en position (219) représentée sur la figure 5,
- celle s'étendant de l'extrémité constituée par le nucléotide en position (90) à l'extrémité constituée par le nucléotide en position (1104) représentée sur la figure 5,
- celle s'étendant de l'extrémité constituée par le nucléotide en position (90) à l'extrémité constituée par le nucléotide en position (1299) représentée sur la figure 5,
- celle s'étendant de l'extrémité constituée par le nucléotide en position (130) à l'extrémité constituée par le nucléotide en position (219) représentée sur la figure 5,
- celle s'étendant de l'extrémité constituée par le nucléotide en position (130) à l'extrémité constituée par le nucléotide en position (1104) représentée sur la figure 5,
- celle s'étendant de l'extrémité constituée par le nucléotide en position (130) à l'extrémité constituée par le nucléotide en position (1299) représentée sur la figure 5,
- celle s'étendant de l'extrémité constituée par le nucléotide en position (220) à l'extrémité constituée par le nucléotide en position (1104) représentée sur la figure 5,
- celle s'étendant de l'extrémité constituée par le nucléotide en position (220) à l'extrémité constituée par le nucléotide en position (1299) représentée sur la figure 5,
- celle s'étendant de l'extrémité constituée par le nucléotide en position (1105) à l'extrémité constituée par le nucléotide en position (1299) représentée sur la figure 5,
ou leurs séquences complémentaires,
ou lesdites séquences nucléotidiques ci-dessus dans lesquelles T est remplacé par U.

16. Acide nucléique recombinant contenant au moins une des séquences nucléotidiques selon les revendications 9 à 15, insérées dans un acide nucléique hétérologue.

17. Amorce d'ADN ou d'ARN constituée de 15 à 25 nucléotides d'une séquence nucléotidique selon l'une quelconque des revendications 13 à 15, qui peut être utilisée comme amorce pour amplifier spécifiquement l'une quelconque desdites séquences nucléotidiques.

18. Amorce d'ADN ou d'ARN selon la revendication 17, ladite amorce étant choisie dans le groupe suivant des séquences nucléotidiques:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
ou leurs séquences complémentaires ou les séquences ribonucléotidiques correspondantes.

19. Jeu d'amorces d'ADN ou d'ARN pour l'amplification enzymatique d'une séquence nucléotidique selon l'une quelconque des revendications 13 à 15, ledit jeu d'amorces comprenant:
- une séquence nucléotidique A combinée avec la séquence nucléotidique complémentaire de B, de C, de D, de E ou de F; ou
- la séquence nucléotidique B combinée avec la séquence nucléotidique complémentaire de C, de D, de E ou de F; ou
- la séquence nucléotidique C combinée avec la séquence nucléotidique complémentaire de D, de E ou de F; ou
- la séquence nucléotidique D combinée avec la séquence nucléotidique complémentaire de E ou de F; ou
- la séquence nucléotidique E combinée avec une séquence nucléotidique complémentaire de F; les séquences nucléotidiques A, B, C, D, E et F étant précisées dans la revendication 18, et A signifiant l'une quelconque des séquences A(i), A(ii), A(iii), A(iv) et A(v) et F signifiant l'une quelconque des séquences F(i), F(ii), F(iii) et F(iv).

20. Vecteur recombinant, en particulier pour le clonage et/ou l'expression, comprenant une séquence de vecteur de type plasmide, cosmide ou phage, et un acide nucléique selon l'une quelconque des revendications 9 à 15, dans un des sites non essentiels pour sa réplication.

21. Vecteur recombinant selon la revendication 20, contenant, dans un des sites non essentiels pour sa réplication, les éléments nécessaires pour promouvoir l'expression d'un polypeptide selon l'une quelconque des revendications 1 à 6 dans un hôte cellulaire.

22. Vecteur recombinant selon la revendication 21, contenant les éléments permettant l'expression par E. coli d'un polypeptide selon l'une quelconque des revendications 1 à 6.

23. Hôte cellulaire contenant un vecteur recombinant selon l'une quelconque des revendications 20 à 22 et comprenant les éléments de régulation permettant l'expression d'un polypeptide selon l'une quelconque des revendications 1 à 6 dans cet hôte.

24. Hôte cellulaire selon la revendication 23, choisi parmi les bactéries telles que E. coli transformées par un vecteur selon la revendication 20, ou choisi parmi les organismes eucaryotes transformés par un vecteur selon la revendication 20.

25. Anticorps monoclonal spécifiquement dirigé contre un polypeptide recombinant selon l'une quelconque des revendications 1 à 6.

26. Sonde nucléotidique s'hybridant avec un acide nucléique selon l'une quelconque des revendications 13 à 15, choisie parmi le groupe suivant de séquences nucléotidiques:
| Sondes A(i), A(ii), A(iii), A(iv) et A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Sonde B | |
|---|---|
| TCGCCCGCCCTGTACCTG, | |
| Sonde C | |
|---|---|
| GCGCTGACGCTGGCGATCTATC, | |
| Sonde D | |
|---|---|
| CCGCTGTTGAACGTCGGGAAG, | |
| Sonde E | |
|---|---|
| AAGCCGTCGGATCTGGGTGGCAAC, | |
| Sondes F(i) et F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| ou leurs séquences nucléotidiques complémentaires. | |

27. Procédé pour préparer un polypeptide recombinant selon l'une quelconque des revendications 1 à 8, comprenant les étapes suivantes:
- la culture, dans un milieu approprié, d'un hôte cellulaire ayant été préalablement transformé par un vecteur approprié contenant un acide nucléique selon l'une quelconque des revendications 9 à 15, et
- la récupération du polypeptide produit par ledit hôte cellulaire transformé ci-dessus dans ledit milieu de culture ci-dessus.

28. Procédé de diagnostic in vitro de la tuberculose chez un patient susceptible d'être infecté par Mycobacterium tuberculosis, comprenant les étapes suivantes:
- l'amplification d'un acide nucléique selon l'une quelconque des revendications 9 à 15, susceptible d'être contenu dans un échantillon biologique prélevé chez ledit patient, au moyen d'une amorce selon l'une quelconque des revendications 17 à 18, et/ou
- la mise en contact de l'échantillon biologique mentionné ci-dessus avec une sonde nucléotidique selon la revendication 26, dans des conditions permettant la formation d'un complexe d'hybridation entre ladite sonde et ledit acide nucléique de l'échantillon, et
- la détection dudit complexe d'hybridation ci-dessus.

29. Procédé de diagnostic in vitro de la tuberculose chez un patient susceptible d'être infecté par Mycobacterium tuberculosis, comprenant:
- la mise en contact d'un échantillon biologique prélevé chez un patient avec un polypeptide selon l'une quelconque des revendications 1 à 8, dans des conditions permettant une réaction immunologique in vitro entre ledit polypeptide et les anticorps éventuellement présents dans l'échantillon biologique, et
- la détection in vitro du complexe antigène/anticorps éventuellement formé.

30. Procédé de diagnostic in vitro de la tuberculose chez un patient susceptible d'être infecté par M. tuberculosis, comprenant les étapes suivantes:
- la mise en contact de l'échantillon biologique avec un anticorps selon la revendication 25, dans des conditions permettant une réaction immunologique in vitro entre ledit anticorps et les antigènes de M. tuberculosis éventuellement présents dans l'échantillon biologique, et
- la détection in vitro du complexe antigène/anticorps éventuellement formé.

31. Trousse pour un procédé diagnostique in vitro de la tuberculose chez un patient susceptible d'être infecté par Mycobacterium tuberculosis selon la revendication 28, ladite trousse comprenant une quantité déterminée d'une sonde nucléotidique selon la revendication 26.

32. Trousse pour un procédé diagnostique in vitro de la tuberculose chez un patient susceptible d'être infecté par Mycobacterium tuberculosis selon la revendication 29, comprenant:
- un polypeptide selon l'une quelconque des revendications 1 à 8,
- des réactifs pour préparer un milieu approprié pour qu'ait lieu la réaction immunologique,
- des réactifs permettant de détecter le complexe antigène/anticorps qui a été produit par la réaction immunologique, lesdits réactifs comportant éventuellement un marqueur, ou étant susceptibles d'être reconnus par un réactif marqué, plus particulièrement dans le cas où le polypeptide mentionné ci-dessus n'est pas marqué.

33. Trousse pour un procédé diagnostique in vitro de la tuberculose chez un patient susceptible d'être infecté par Mycobacterium tuberculosis selon la revendication 30, comprenant:
- un anticorps selon la revendication 25,
- des réactifs pour préparer un milieu approprié pour qu'ait lieu la réaction immunologique,
- des réactifs permettant de détecter le complexe antigène/anticorps qui a été produit par la réaction immunologique, lesdits réactifs comportant éventuellement un marqueur, ou étant susceptibles d'être reconnus par un réactif marqué, plus particulièrement dans le cas où le polypeptide mentionné ci-dessus n'est pas marqué.

34. Polypeptide recombinant biologiquement pur selon l'une quelconque des revendications 1 à 8 à utiliser dans une composition immunogène en association avec un véhicule pharmaceutiquement acceptable.

35. Polypeptide recombinant biologiquement pur selon l'une quelconque des revendications 1 à 8 à utiliser dans une composition de vaccin.

36. Procédé pour l'amplification enzymatique d'une séquence nucléotidique selon l'une quelconque des revendications 13 à 15 et la détection de la séquence nucléotidique amplifiée, dans lequel l'amplification est réalisée par la technique ACP au moyen d'un jeu d'amorces et la détection du produit amplifié par ACP est réalisée par une réaction d'hybridation avec une sonde de détection, ledit jeu d'amorces et ladite sonde de détection étant choisis parmi les éléments suivants: 
Jeu d'amorces
P1 GAGTACCTGCAGGTGCCGTCGCCGTCGATGGGCCG

P2 compl. GTACCACTCGAACGCCGGGTGTTGAT

Sonde B
TCGCCCGCCCTGTACCTG

Jeu d'amorces
P1 GAGTACCTGCAGGTGCCGTCGCCGTCGATGGGCCG
P3 compl. TCCCACTTGTAAGTCTGGCA

Sonde B
TCGCCCGCCCTGTACCTG

Jeu d'amorces
P1 GAGTACCTGCAGGTGCCGTCGCCGTCGATGGGCCG
P4 compl. CGGCAGCTCGCTGGTCAGGA

Sonde B
TCGCCCGCCCTGTACCTG

Jeu d'amorces
P1 GAGTACCTGCAGGTGCCGTCGCCGTCGATGGGCCG
P5 compl. GCGTCACCCATCGCCAGGCCGATCAGG

Sonde B
TCGCCCGCCCTGTACCTG

Sonde C
GCGCTGACGCTGGCGATCTATC
 
Jeu d'amorces
P1 GAGTACCTGCAGGTGCCGTCGCCGTCGATGGGCCG.
P6 compl. GCGCCCCAGTACTCCCAGCTGTGCGT
Sonde B
TCGCCCGCCCTGTACCTG
Sonde C
GCGCTGACGCTGGCGATCTATC
Sonde D
CCGCTGTTGAACGTCGGGAAG
Sonde E
AAGCCGTCGGATCTGGGTGGCAAC
Jeu d'amorces
P2 ATCAACACCCCGGCGTTCGAGTGGTAC
P5 compl. GCGTCACCCATCGCCAGGCCGATCAGG
Sonde C
CGCGTGACGCTGGCGATCTATC
Jeu d'amorces
P2 ATCAACACCCCGGCGTTCGAGTGGTAC
P6 compl. GCGCCCCAGTACTCCCAGCTGTGCGT
Sonde C
GCGCTGACGCTGGCGATCTATC
Sonde D
CCGCTGTTGAACGTCGGGAAG
Sonde E
AAGCCGTCGGATCTGGGTGGCAAC
Jeu d'amorces
P3 TGCCAGACTTACAAGTGGGA
P5 compl. GCGTCACCCATCGCCAGGCCGATCAGG
Sonde C
GCGCTGACGCTGGCGATCTATC
Jeu d'amorces
P3 TGCCAGACTTACAAGTGGGA
P6 compl. GCGCCCCAGTACTCCCAGCTGTGCGT
Sonde C
GCGCTGACGCTGGCGATCTATC
Sonde D
CCGCTGTTGAACGTCGGGAAG
Sonde E
 
AAGCCGTCGGATCTGGGTGGCAAC
Jeu d'amorces
P4 TCCTGACCAGCGAGCTGCCG
P5 compl. GCGTCACCCATCGCCAGGCCGATCAGG
Sonde C
GCGCTGACGCTGGCGATCTATC
Jeu d'amorces
P4 TCCTGACCAGCGAGCTGCCG
P6 compl. GCGCCCCAGTACTCCCAGCTGTGCGT
Sonde C
GCGCTGACGCTGGCGATCTATC
Sonde D
CCGCTGTTGAACGTCGGGAAG
Sonde E
AAGCCGTCGGATCTGGGTGGCAAC
Jeu d'amorces
P5 CCTGATCGGCCTGGCGATGGGTGACGC
P6 compl. GCGCCCCAGTACTCCCAGCTGTGCGT
Sonde D
CCGCTGTTGAACGTCGGGAAG
Sonde E
AAGCCGTCGGATCTGGGTGGCAAC
 ou ledit jeu d'amorces étant choisi parmi les jeux d'amorces selon la revendication 19, et la sonde de détection étant constituée d'une séquence oligonucléotidique d'au moins 10 nucléotides, ladite séquence étant située entre les deux amorces APC constituant le jeu d'amorces ayant été utilisé pour amplifier ladite séquence nucléotidique.

37. Vecteur recombinant selon la revendication 20, la séquence du vecteur possédant soit la séquence d'acides nucléiques représentée sur la figure 10b, soit la séquence d'acides nucléiques représentée sur la figure 11b, ou encore la séquence d'acides nucléiques représentée sur la figure 12b.

38. Peptides selon la revendication 1, avantageusement utilisés pour produire des anticorps, en particulier des anticorps monoclonaux et qui possèdent les séquences d'acides aminés suivantes:
| Position de l'acide aminé (extrémité NH₂) | | Position de l'acide aminé (extrémité COOH) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |

39. Procédé pour préparer un polypeptide recombinant biologiquement pur contenant, dans sa chaîne polypeptidique, au moins une des séquences d'acides aminés suivantes:
- celle s'étendant de l'extrémité constituée par l'acide aminé en position (-29) à l'extrémité constituée par l'acide aminé en position (-1) représentée sur la figure 3a et la figure 3b, ou
- celle s'étendant de l'extrémité constituée par l'acide aminé en position (12) à l'extrémité constituée par l'acide aminé en position (31) représentée sur la figure 3a et la figure 3b, ou
- celle s'étendant de l'extrémité constituée par l'acide aminé en position (36) à l'extrémité constituée par l'acide aminé en position (55) représentée sur la figure 3a et la figure 3b, ou
- celle s'étendant de l'extrémité constituée par l'acide aminé en position (77) à l'extrémité constituée par l'acide aminé en position (96) représentée sur la figure 3a et la figure 3b, ou
- celle s'étendant de l'extrémité constituée par l'acide aminé en position (101) à l'extrémité constituée par l'acide aminé en position (120) représentée sur la figure 3a et la figure 3b, ou
- celle s'étendant de l'extrémité constituée par l'acide aminé en position (175) à l'extrémité constituée par l'acide aminé en position (194) représentée sur la figure 3a et la figure 3b, ou
- celle s'étendant de l'extrémité constituée par l'acide aminé en position (211) à l'extrémité constituée par l'acide aminé en position (230) représentée sur la figure 3a et la figure 3b, ou
- celle s'étendant de l'extrémité constituée par l'acide aminé en position (275) à l'extrémité constituée par l'acide aminé en position (294) représentée sur la figure 3a et la figure 3b, ou
- celle s'étendant de l'extrémité constituée par l'acide aminé en position (1) à l'extrémité constituée par l'acide aminé en position (294) représentée sur la figure 3a et la figure 3b,
- celle s'étendant de l'extrémité constituée par l'acide aminé en position (-42) à l'extrémité constituée par l'acide aminé en position (-1) représentée sur la figure 3a et la figure 3b,
- celle s'étendant de l'extrémité constituée par l'acide aminé en position (-47) à l'extrémité constituée par l'acide aminé en position (-1) représentée sur la figure 3a et la figure 3b,
- celle s'étendant de l'extrémité constituée par l'acide aminé en position (-49) à l'extrémité constituée par l'acide aminé en position (-1) représentée sur la figure 3a et la figure 3b,
- celle s'étendant de l'extrémité constituée par l'acide aminé en position (-55) à l'extrémité constituée par l'acide aminé en position (-1) représentée sur la figure 3a et la figure 3b,
- celle s'étendant de l'extrémité constituée par l'acide aminé en position (-59) à l'extrémité constituée par l'acide aminé en position (-1) représentée sur la figure 3a et la figure 3b,
- celle s'étendant de l'extrémité constituée par l'acide aminé en position (-29) à l'extrémité constituée par l'acide aminé en position (294) représentée sur la figure 3a et la figure 3b,
- celle s'étendant de l'extrémité constituée par l'acide aminé en position (-42) à l'extrémité constituée par l'acide aminé en position (294) représentée sur la figure 3a et la figure 3b,
- celle s'étendant de l'extrémité constituée par l'acide aminé en position (-47) à l'extrémité constituée par l'acide aminé en position (294) représentée sur la figure 3a et la figure 3b,
- celle s'étendant de l'extrémité constituée par l'acide aminé en position (-49) à l'extrémité constituée par l'acide aminé en position (294) représentée sur la figure 3a et la figure 3b,
- celle s'étendant de l'extrémité constituée par l'acide aminé en position (-55) à l'extrémité constituée par l'acide aminé en position (294) représentée sur la figure 3a et la figure 3b,
- celle s'étendant de l'extrémité constituée par l'acide aminé en position (-59) à l'extrémité constituée par l'acide aminé en position (294) représentée sur la figure 3a et la figure 3b,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis, ledit procédé comprenant les étapes de
- production du polypeptide par manipulation génétique, à savoir par expression dans un hôte cellulaire des acides nucléiques codant pour lesdits polypeptides, ou par synthèse chimique classique, et de
- purification du polypeptide produit.

40. Procédé pour préparer un polypeptide recombinant biologiquement pur selon la revendication 39, ledit polypeptide contenant, dans sa chaîne polypeptidique, au moins une des séquences d'acides aminés suivantes:
- celle s'étendant de l'extrémité constituée par l'acide aminé en position (-29) à l'extrémité constituée par l'acide aminé en position (-1) représentée sur la figure 4a et la figure 4b, ou
- celle s'étendant de l'extrémité constituée par l'acide aminé en position (12) à l'extrémité constituée par l'acide aminé en position (31) représentée sur la figure 4a et la figure 4b, ou
- celle s'étendant de l'extrémité constituée par l'acide aminé en position (36) à l'extrémité constituée par l'acide aminé en position (55) représentée sur la figure 4a et la figure 4b, ou
- celle s'étendant de l'extrémité constituée par l'acide aminé en position (77) à l'extrémité constituée par l'acide aminé en position (96) représentée sur la figure 4a et la figure 4b, ou
- celle s'étendant de l'extrémité constituée par l'acide aminé en position (101) à l'extrémité constituée par l'acide aminé en position (120) représentée sur la figure 4a et la figure 4b, ou
- celle s'étendant de l'extrémité constituée par l'acide aminé en position (175) à l'extrémité constituée par l'acide aminé en position (194) représentée sur la figure 4a et la figure 4b, ou
- celle s'étendant de l'extrémité constituée par l'acide aminé en position (211) à l'extrémité constituée par l'acide aminé en position (230) représentée sur la figure 4a et la figure 4b, ou
- celle s'étendant de l'extrémité constituée par l'acide aminé en position (275) à l'extrémité constituée par l'acide aminé en position (294) représentée sur la figure 4a et la figure 4b,
- celle s'étendant de l'extrémité constituée par l'acide aminé en position (1) à l'extrémité constituée par l'acide aminé en position (294) représentée sur la figure 4a et la figure 4b,
- celle s'étendant de l'extrémité constituée par l'acide aminé en position (-42) à l'extrémité constituée par l'acide aminé en position (-1) représentée sur la figure 4a et la figure 4b,
- celle s'étendant de l'extrémité constituée par l'acide aminé en position (-47) à l'extrémité constituée par l'acide aminé en position (-1) représentée sur la figure 4a et la figure 4b,
- celle s'étendant de l'extrémité constituée par l'acide aminé en position (-49) à l'extrémité constituée par l'acide aminé en position (-1) représentée sur la figure 4a et la figure 4b,
- celle s'étendant de l'extrémité constituée par l'acide aminé en position (-55) à l'extrémité constituée par l'acide aminé en position (-1) représentée sur la figure 4a et la figure 4b,
- celle s'étendant de l'extrémité constituée par l'acide aminé en position (-59) à l'extrémité constituée par l'acide aminé en position (-1) représentée sur la figure 4a et la figure 4b,
- celle s'étendant de l'extrémité constituée par l'acide aminé en position (-29) à l'extrémité constituée par l'acide aminé en position (294) représentée sur la figure 4a et la figure 4b,
- celle s'étendant de l'extrémité constituée par l'acide aminé en position (-42) à l'extrémité constituée par l'acide aminé en position (294) représentée sur la figure 4a et la figure 4b,
- celle s'étendant de l'extrémité constituée par l'acide aminé en position (-47) à l'extrémité constituée par l'acide aminé en position (294) représentée sur la figure 4a et la figure 4b,
- celle s'étendant de l'extrémité constituée par l'acide aminé en position (-49) à l'extrémité constituée par l'acide aminé en position (294) représentée sur la figure 4a et la figure 4b,
- celle s'étendant de l'extrémité constituée par l'acide aminé en position (-55) à l'extrémité constituée par l'acide aminé en position (294) représentée sur la figure 4a et la figure 4b,
- celle s'étendant de l'extrémité constituée par l'acide aminé en position (-59) à l'extrémité constituée par l'acide aminé en position (294) représentée sur la figure 4a et la figure 4b,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

41. Procédé pour préparer un polypeptide recombinant biologiquement pur selon la revendication 39, ledit polypeptide contenant, dans sa chaîne polypeptidique, au moins une des séquences d'acides aminés suivantes:
- celle s'étendant de l'extrémité constituée par l'acide aminé en position (-30) à l'extrémité constituée par l'acide aminé en position (-1) représentée sur la figure 5, ou
- celle s'étendant de l'extrémité constituée par l'acide aminé en position (12) à l'extrémité constituée par l'acide aminé en position (31) représentée sur la figure 5, ou
- celle s'étendant de l'extrémité constituée par l'acide aminé en position (36) à l'extrémité constituée par l'acide aminé en position (55) représentée sur la figure 5, ou
- celle s'étendant de l'extrémité constituée par l'acide aminé en position (77) à l'extrémité constituée par l'acide aminé en position (96) représentée sur la figure 5, ou
- celle s'étendant de l'extrémité constituée par l'acide aminé en position (101) à l'extrémité constituée par l'acide aminé en position (120) représentée sur la figure 5, ou
- celle s'étendant de l'extrémité constituée par l'acide aminé en position (175) à l'extrémité constituée par l'acide aminé en position (194) représentée sur la figure 5, ou
- celle s'étendant de l'extrémité constituée par l'acide aminé en position (211) à l'extrémité constituée par l'acide aminé en position (230) représentée sur la figure 5, ou
- celle s'étendant de l'extrémité constituée par l'acide aminé en position (275) à l'extrémité constituée par l'acide aminé en position (295) représentée sur la figure 5, ou
- celle s'étendant de l'extrémité constituée par l'acide aminé en position (1) à l'extrémité constituée par l'acide aminé en position (295) représentée sur la figure 5, ou
- celle s'étendant de l'extrémité constituée par l'acide aminé en position (-43) à l'extrémité constituée par l'acide aminé en position (-1) représentée sur la figure 5, ou
- celle s'étendant de l'extrémité constituée par l'acide aminé en position (-30) à l'extrémité constituée par l'acide aminé en position (295) représentée sur la figure 5,
- celle s'étendant de l'extrémité constituée par l'acide aminé en position (-43) à l'extrémité constituée par l'acide aminé en position (295) représentée sur la figure 5,
ou les séquences peptidiques résultant de la modification des groupes carboxyle et/ou amino libres dans la séquence polypeptidique et/ou résultant de la glycosylation des polypeptides mentionnés ci-dessus, dans la mesure où cette modification n'altère pas les propriétés suivantes:
- les polypeptides réagissent avec l'antisérum polyclonal de lapin élevé contre la protéine de 32 kDa du filtrat de culture de BCG de M. bovis, et/ou
- les polypeptides réagissent sélectivement avec les sérums humains de patients tuberculeux et en particulier de patients développant une tuberculose évolutive à un stade précoce, et/ou
- un conjugué desdits polypeptides est capable de déclencher in vivo la production d'anticorps neutralisant M. tuberculosis ou de déclencher in vivo une réponse immunitaire cellulaire en activant les cellules T sensibles à M. tuberculosis.

42. Procédé pour préparer un polypeptide recombinant selon la revendication 39, ledit polypeptide étant constitué d'une des séquences d'acides aminés suivantes:
- celle s'étendant de l'extrémité constituée par l'acide aminé en position (-59) à l'extrémité constituée par l'acide aminé en position (294) représentée sur la figure 3a et la figure 3b,
- celle s'étendant de l'extrémité constituée par l'acide aminé en position (-59) à l'extrémité constituée par l'acide aminé en position (-1) représentée sur la figure 3a et la figure 3b,
- celle s'étendant de l'extrémité constituée par l'acide aminé en position (-55) à l'extrémité constituée par l'acide aminé en position (294) représentée sur la figure 3a et la figure 3b,
- celle s'étendant de l'extrémité constituée par l'acide aminé en position (-55) à l'extrémité constituée par l'acide aminé en position (-1) représentée sur la figure 3a et la figure 3b,
- celle s'étendant de l'extrémité constituée par l'acide aminé en position (-49) à l'extrémité constituée par l'acide aminé en position (294) représentée sur la figure 3a et la figure 3b,
- celle s'étendant de l'extrémité constituée par l'acide aminé en position (-49) à l'extrémité constituée par l'acide aminé en position (-1) représentée sur la figure 3a et la figure 3b,
- celle s'étendant de l'extrémité constituée par l'acide aminé en position (-47) à l'extrémité constituée par l'acide aminé en position (294) représentée sur la figure 3a et la figure 3b,
- celle s'étendant de l'extrémité constituée par l'acide aminé en position (-47) à l'extrémité constituée par l'acide aminé en position (-1) représentée sur la figure 3a et la figure 3b,
- celle s'étendant de l'extrémité constituée par l'acide aminé en position (-42) à l'extrémité constituée par l'acide aminé en position (294) représentée sur la figure 3a et la figure 3b,
- celle s'étendant de l'extrémité constituée par l'acide aminé en position (-42) à l'extrémité constituée par l'acide aminé en position (-1) représentée sur la figure 3a et la figure 3b,
- celle s'étendant de l'extrémité constituée par l'acide aminé en position (-29) à l'extrémité constituée par l'acide aminé en position (294) représentée sur la figure 3a et la figure 3b,
- celle s'étendant de l'extrémité constituée par l'acide aminé en position (-29) à l'extrémité constituée par l'acide aminé en position (-1) représentée sur la figure 3a et la figure 3b,
- celle s'étendant de l'extrémité constituée par l'acide aminé en position (1) à l'extrémité constituée par l'acide aminé en position (294) représentée sur la figure 3a et la figure 3b,
- celle s'étendant de l'extrémité constituée par l'acide aminé en position (12) à l'extrémité constituée par l'acide aminé en position (31) représentée sur la figure 3a et la figure 3b,
- celle s'étendant de l'extrémité constituée par l'acide aminé en position (36) à l'extrémité constituée par l'acide aminé en position (55) représentée sur la figure 3a et la figure 3b,
- celle s'étendant de l'extrémité constituée par l'acide aminé en position (77) à l'extrémité constituée par l'acide aminé en position (96) représentée sur la figure 3a et la figure 3b,
- celle s'étendant de l'extrémité constituée par l'acide aminé en position (101) à l'extrémité constituée par l'acide aminé en position (120) représentée sur la figure 3a et la figure 3b,
- celle s'étendant de l'extrémité constituée par l'acide aminé en position (175) à l'extrémité constituée par l'acide aminé en position (194) représentée sur la figure 3a et la figure 3b,
- celle s'étendant de l'extrémité constituée par l'acide aminé en position (211) à l'extrémité constituée par l'acide aminé en position (230) représentée sur la figure 3a et la figure 3b,
- celle s'étendant de l'extrémité constituée par l'acide aminé en position (275) à l'extrémité constituée par l'acide aminé en position (294) représentée sur la figure 3a et la figure 3b.

43. Procédé pour préparer un polypeptide recombinant selon la revendication 39, ledit polypeptide étant constitué d'une des séquences d'acides aminés suivantes:
- celle s'étendant de l'extrémité constituée par l'acide aminé en position (-59) à l'extrémité constituée par l'acide aminé en position (294) représentée sur la figure 4a et la figure 4b,
- celle s'étendant de l'extrémité constituée par l'acide aminé en position (-59) à l'extrémité constituée par l'acide aminé en position (-1) représentée sur la figure 4a et la figure 4b,
- celle s'étendant de l'extrémité constituée par l'acide aminé en position (-55) à l'extrémité constituée par l'acide aminé en position (294) représentée sur la figure 4a et la figure 4b,
- celle s'étendant de l'extrémité constituée par l'acide aminé en position (-55) à l'extrémité constituée par l'acide aminé en position (-1) représentée sur la figure 4a et la figure 4b,
- celle s'étendant de l'extrémité constituée par l'acide aminé en position (-49) à l'extrémité constituée par l'acide aminé en position (294) représentée sur la figure 4a et la figure 4b,
- celle s'étendant de l'extrémité constituée par l'acide aminé en position (-49) à l'extrémité constituée par l'acide aminé en position (-1) représentée sur la figure 4a et la figure 4b,
- celle s'étendant de l'extrémité constituée par l'acide aminé en position (-47) à l'extrémité constituée par l'acide aminé en position (294) représentée sur la figure 4a et la figure 4b,
- celle s'étendant de l'extrémité constituée par l'acide aminé en position (-47) à l'extrémité constituée par l'acide aminé en position (-1) représentée sur la figure 4a et la figure 4b,
- celle s'étendant de l'extrémité constituée par l'acide aminé en position (-42) à l'extrémité constituée par l'acide aminé en position (294) représentée sur la figure 4a et la figure 4b,
- celle s'étendant de l'extrémité constituée par l'acide aminé en position (-42) à l'extrémité constituée par l'acide aminé en position (-1) représentée sur la figure 4a et la figure 4b,
- celle s'étendant de l'extrémité constituée par l'acide aminé en position (-29) à l'extrémité constituée par l'acide aminé en position (294) représentée sur la figure 4a et la figure 4b,
- celle s'étendant de l'extrémité constituée par l'acide aminé en position (-29) à l'extrémité constituée par l'acide aminé en position (-1) représentée sur la figure 4a et la figure 4b,
- celle s'étendant de l'extrémité constituée par l'acide aminé en position (1) à l'extrémité constituée par l'acide aminé en position (294) représentée sur la figure 4a et la figure 4b,
- celle s'étendant de l'extrémité constituée par l'acide aminé en position (12) à l'extrémité constituée par l'acide aminé en position (31) représentée sur la figure 4a et la figure 4b,
- celle s'étendant de l'extrémité constituée par l'acide aminé en position (36) à l'extrémité constituée par l'acide aminé en position (55) représentée sur la figure 4a et la figure 4b,
- celle s'étendant de l'extrémité constituée par l'acide aminé en position (77) à l'extrémité constituée par l'acide aminé en position (96) représentée sur la figure 4a et la figure 4b,
- celle s'étendant de l'extrémité constituée par l'acide aminé en position (101) à l'extrémité constituée par l'acide aminé en position (120) représentée sur la figure 4a et la figure 4b,
- celle s'étendant de l'extrémité constituée par l'acide aminé en position (175) à l'extrémité constituée par l'acide aminé en position (194) représentée sur la figure 4a et la figure 4b,
- celle s'étendant de l'extrémité constituée par l'acide aminé en position (211) à l'extrémité constituée par l'acide aminé en position (230) représentée sur la figure 4a et la figure 4b,
- celle s'étendant de l'extrémité constituée par l'acide aminé en position (275) à l'extrémité constituée par l'acide aminé en position (294) représentée sur la figure 4a et la figure 4b.

44. Procédé pour préparer un polypeptide recombinant selon la revendication 39, ledit polypeptide étant constitué d'une des séquences d'acides aminés suivantes:
- celle s'étendant de l'extrémité constituée par l'acide aminé en position (-43) à l'extrémité constituée par l'acide aminé en position (295) représentée sur la figure 5,
- celle s'étendant de l'extrémité constituée par l'acide aminé en position (-43) à l'extrémité constituée par l'acide aminé en position (-1) représentée sur la figure 5,
- celle s'étendant de l'extrémité constituée par l'acide aminé en position (-30) à l'extrémité constituée par l'acide aminé en position (295) représentée sur la figure 5,
- celle s'étendant de l'extrémité constituée par l'acide aminé en position (-30) à l'extrémité constituée par l'acide aminé en position (-1) représentée sur la figure 5,
- celle s'étendant de l'extrémité constituée par l'acide aminé en position (1) à l'extrémité constituée par l'acide aminé en position (295) représentée sur la figure 5,
- celle s'étendant de l'extrémité constituée par l'acide aminé en position (12) à l'extrémité constituée par l'acide aminé en position (31) représentée sur la figure 5,
- celle s'étendant de l'extrémité constituée par l'acide aminé en position (36) à l'extrémité constituée par l'acide aminé en position (55) représentée sur la figure 5,
- celle s'étendant de l'extrémité constituée par l'acide aminé en position (77) à l'extrémité constituée par l'acide aminé en position (96) représentée sur la figure 5,
- celle s'étendant de l'extrémité constituée par l'acide aminé en position (101) à l'extrémité constituée par l'acide aminé en position (120) représentée sur la figure 5,
- celle s'étendant de l'extrémité constituée par l'acide aminé en position (175) à l'extrémité constituée par l'acide aminé en position (194) représentée sur la figure 5,
- celle s'étendant de l'extrémité constituée par l'acide aminé en position (211) à l'extrémité constituée par l'acide aminé en position (230) représentée sur la figure 5,
- celle s'étendant de l'extrémité constituée par l'acide aminé en position (275) à l'extrémité constituée par l'acide aminé en position (295) représentée sur la figure 5.

45. Procédé pour préparer un polypeptide recombinant biologiquement pur selon la revendication 39, ledit polypeptide étant constitué d'un polypeptide selon l'une quelconque des revendications 1 à 6 et d'une protéine possédant une séquence hétérologue vis-à-vis dudit polypeptide, ladite séquence hétérologue comprenant d'environ 1 à environ 1 000 acides aminés.

46. Procédé pour préparer un polypeptide recombinant selon la revendication 45, dans lequel ladite protéine hétérologue est la β-galactosidase.

47. Procédé pour préparer un acide nucléique comprenant
(a) une séquence nucléotidique codant pour un polypeptide selon l'une quelconque des revendications 1 à 6, ou
(b) une séquence nucléotidique qui est complémentaire à (a), ou
(c) les séquences nucléotidiques de (a) ou de (b) dans lesquelles T peut être remplacé par U, ledit procédé étant un procédé de synthèse chimique et comprenant les étapes suivantes:
- synthèse d'acides nucléiques à un seul brin à l'aide d'un procédé automatique au phosphoramidite de β-cyanoéthyle, et
- dans le cas d'acides nucléiques à deux brins, combinaison des oligonucléotides sens et anti-sens à un seul brin par hybridation afin de former un duplex d'ADN, clonage du duplex d'ADN obtenu dans un vecteur plasmide approprié et récupération de l'ADN selon des procédés classiques, tels que digestion par enzymes de restriction et électrophorèse sur gel d'agarose, et
- dans le cas d'acides nucléiques plus grands que 100 nucléotides, assemblage des oligonucléotides synthétisés par voie chimique qui sont munis à leurs extrémités de sites de restriction différents, dont les séquences sont compatibles avec la succession des acides aminés dans le peptide naturel, clonage de l'ADN ainsi obtenu dans un vecteur plasmide approprié et récupération de l'acide nucléique recherché selon des procédés classiques, tels que digestion par enzymes de restriction et électrophorèse sur gel d'agarose.

48. Procédé pour préparer un acide nucléique selon la revendication 47, ledit acide nucléique comprenant au moins une des séquences nucléotidiques suivantes:
- celle s'étendant de l'extrémité constituée par le nucléotide en position (1) à l'extrémité constituée par le nucléotide en position (182) représentée sur la figure 3a et la figure 3b,
- celle s'étendant de l'extrémité constituée par le nucléotide en position (273) à l'extrémité constituée par le nucléotide en position (259) représentée sur la figure 3a et la figure 3b,
- celle s'étendant de l'extrémité constituée par le nucléotide en position (360) à l'extrémité constituée par le nucléotide en position (1241) représentée sur la figure 3a et la figure 3b,
- celle s'étendant de l'extrémité constituée par le nucléotide en position (1242) à l'extrémité constituée par le nucléotide en position (1358), dans laquelle N représente un des cinq nucléotides A, T, C, G ou I, représentée sur la figure 3a et la figure 3b,
- celle s'étendant de l'extrémité constituée par le nucléotide en position (1) à l'extrémité constituée par le nucléotide en position (194) représentée sur la figure 3a et la figure 3b,
- celle s'étendant de l'extrémité constituée par le nucléotide en position (195) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (212) représentée sur la figure 3a et la figure 3b,
- celle s'étendant de l'extrémité constituée par le nucléotide en position (213) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (218) représentée sur la figure 3a et la figure 3b,
- celle s'étendant de l'extrémité constituée par le nucléotide en position (219) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (272) représentée sur la figure 3a et la figure 3b,
- celle s'étendant de l'extrémité constituée par le nucléotide en position (1) à l'extrémité constituée par le nucléotide en position (359) représentée sur la figure 3a et la figure 3b,
- celle s'étendant de l'extrémité constituée par le nucléotide en position (183) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (1358) représentée sur la figure 3a et la figure 3b,
- celle s'étendant de l'extrémité constituée par le nucléotide en position (1) à l'extrémité constituée par le nucléotide en position (1241) représentée sur la figure 3a et la figure 3b,
- celle s'étendant de l'extrémité constituée par le nucléotide en position (183) à l'extrémité constituée par le nucléotide en position (1358) représentée sur la figure 3a et la figure 3b,
- celle s'étendant de l'extrémité constituée par le nucléotide en position (183) à l'extrémité constituée par le nucléotide en position (1241) représentée sur la figure 3a et la figure 3b,
- celle s'étendant de l'extrémité constituée par le nucléotide en position (195) à l'extrémité constituée par le nucléotide en position (1358) représentée sur la figure 3a et la figure 3b,
- celle s'étendant de l'extrémité constituée par le nucléotide en position (195) à l'extrémité constituée par le nucléotide en position (1241) représentée sur la figure 3a et la figure 3b,
- celle s'étendant de l'extrémité constituée par le nucléotide en position (213) à l'extrémité constituée par le nucléotide en position (1358) représentée sur la figure 3a et la figure 3b,
- celle s'étendant de l'extrémité constituée par le nucléotide en position (213) à l'extrémité constituée par le nucléotide en position (1241) représentée sur la figure 3a et la figure 3b,
- celle s'étendant de l'extrémité constituée par le nucléotide en position (219) à l'extrémité constituée par le nucléotide en position (1358) représentée sur la figure 3a et la figure 3b,
- celle s'étendant de l'extrémité constituée par le nucléotide en position (219) à l'extrémité constituée par le nucléotide en position (1241) représentée sur la figure 3a et la figure 3b,
- celle s'étendant de l'extrémité constituée par le nucléotide en position (234) à l'extrémité constituée par le nucléotide en position (359) représentée sur la figure 3a et la figure 3b,
- celle s'étendant de l'extrémité constituée par le nucléotide en position (234) à l'extrémité constituée par le nucléotide en position (1358) représentée sur la figure 3a et la figure 3b,
- celle s'étendant de l'extrémité constituée par le nucléotide en position (234) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1358) représentée sur la figure 3a et la figure 3b,
- celle s'étendant de l'extrémité constituée par le nucléotide en position (360) à l'extrémité constituée par le nucléotide en position (1358) représentée sur la figure 3a et la figure 3b,
ou leurs séquences complémentaires,
ou lesdites séquences nucléotidiques ci-dessus dans lesquelles T est remplacé par U.

49. Procédé pour préparer un acide nucléique selon la revendication 47, ledit acide nucléique comprenant au moins une des séquences nucléotidiques suivantes:
- celle s'étendant de l'extrémité constituée par le nucléotide en position (1) à l'extrémité constituée par le nucléotide en position (182) représentée sur la figure 4a et la figure 4b,
- celle s'étendant de l'extrémité constituée par le nucléotide en position (273) à l'extrémité constituée par le nucléotide en position (359) représentée sur la figure 4a et la figure 4b,
- celle s'étendant de l'extrémité constituée par le nucléotide en position (360) à l'extrémité constituée par le nucléotide en position (1241) représentée sur la figure 4a et la figure 4b,
- celle s'étendant de l'extrémité constituée par le nucléotide en position (1242) à l'extrémité constituée par le nucléotide en position (1358), dans laquelle N représente un des cinq nucléotides A, T, C, G ou I, représentée sur la figure 4a et la figure 4b,
- celle s'étendant de l'extrémité constituée par le nucléotide en position (1) à l'extrémité constituée par le nucléotide en position (194) représentée sur la figure 4a et la figure 4b,
- celle s'étendant de l'extrémité constituée par le nucléotide en position (195) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (212) représentée sur la figure 4a et la figure 4b,
- celle s'étendant de l'extrémité constituée par le nucléotide en position (213) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (218) représentée sur la figure 4a et la figure 4b,
- celle s'étendant de l'extrémité constituée par le nucléotide en position (219) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (272) représentée sur la figure 4a et la figure 4b,
- celle s'étendant de l'extrémité constituée par le nucléotide en position (1) à l'extrémité constituée par le nucléotide en position (359) représentée sur la figure 4a et la figure 4b,
- celle s'étendant de l'extrémité constituée par le nucléotide en position (183) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (1358) représentée sur la figure 4a et la figure 4b,
- celle s'étendant de l'extrémité constituée par le nucléotide en position (1) à l'extrémité constituée par le nucléotide en position (1241) représentée sur la figure 4a et la figure 4b,
- celle s'étendant de l'extrémité constituée par le nucléotide en position (183) à l'extrémité constituée par le nucléotide en position (1358) représentée sur la figure 4a et la figure 4b,
- celle s'étendant de l'extrémité constituée par le nucléotide en position (183) à l'extrémité constituée par le nucléotide en position (1241) représentée sur la figure 4a et la figure 4b,
- celle s'étendant de l'extrémité constituée par le nucléotide en position (195) à l'extrémité constituée par le nucléotide en position (1358) représentée sur la figure 4a et la figure 4b,
- celle s'étendant de l'extrémité constituée par le nucléotide en position (195) à l'extrémité constituée par le nucléotide en position (1241) représentée sur la figure 4a et la figure 4b,
- celle s'étendant de l'extrémité constituée par le nucléotide en position (213) à l'extrémité constituée par le nucléotide en position (1358) représentée sur la figure 4a et la figure 4b,
- celle s'étendant de l'extrémité constituée par le nucléotide en position (213) à l'extrémité constituée par le nucléotide en position (1241) représentée sur la figure 4a et la figure 4b,
- celle s'étendant de l'extrémité constituée par le nucléotide en position (219) à l'extrémité constituée par le nucléotide en position (1358) représentée sur la figure 4a et la figure 4b,
- celle s'étendant de l'extrémité constituée par le nucléotide en position (219) à l'extrémité constituée par le nucléotide en position (1241) représentée sur la figure 4a et la figure 4b,
- celle s'étendant de l'extrémité constituée par le nucléotide en position (234) à l'extrémité constituée par le nucléotide en position (359) représentée sur la figure 4a et la figure 4b,
- celle s'étendant de l'extrémité constituée par le nucléotide en position (234) à l'extrémité constituée par le nucléotide en position (1358) représentée sur la figure 4a et la figure 4b,
- celle s'étendant de l'extrémité constituée par le nucléotide en position (234) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1358) représentée sur la figure 4a et la figure 4b,
- celle s'étendant de l'extrémité constituée par le nucléotide en position (360) à l'extrémité constituée par le nucléotide en position (1358) représentée sur la figure 4a et la figure 4b,
ou leurs séquences complémentaires,
ou lesdites séquences nucléotidiques ci-dessus dans lesquelles T est remplacé par U.

50. Procédé pour préparer un acide nucléique selon la revendication 47, ledit acide nucléique comprenant au moins une des séquences nucléotidiques suivantes:
- celle s'étendant de l'extrémité constituée par le nucléotide en position (130) à l'extrémité constituée par le nucléotide en position (219) représentée sur la figure 5,
- celle s'étendant de l'extrémité constituée par le nucléotide en position (220) à l'extrémité constituée par le nucléotide en position (1104) représentée sur la figure 5,
- celle s'étendant de l'extrémité constituée par le nucléotide en position (1105) à l'extrémité constituée par le nucléotide en position (1299) représentée sur la figure 5,
- celle s'étendant de l'extrémité constituée par le nucléotide en position (1) à l'extrémité constituée par le nucléotide en position (129) représentée sur la figure 5,
- celle s'étendant de l'extrémité constituée par le nucléotide en position (1) à l'extrémité constituée par le nucléotide en position (219) représentée sur la figure 5,
- celle s'étendant de l'extrémité constituée par le nucléotide en position (1) à l'extrémité constituée par le nucléotide en position (1104) représentée sur la figure 5,
- celle s'étendant de l'extrémité constituée par le nucléotide en position (1) à l'extrémité constituée par le nucléotide en position (1299) représentée sur la figure 5,
- celle s'étendant de l'extrémité constituée par le nucléotide en position (90) à l'extrémité constituée par le nucléotide en position (219) représentée sur la figure 5,
- celle s'étendant de l'extrémité constituée par le nucléotide en position (90) à l'extrémité constituée par le nucléotide en position (1299) représentée sur la figure 5,
- celle s'étendant de l'extrémité constituée par le nucléotide en position (90) à l'extrémité constituée par le nucléotide en position (1104) représentée sur la figure 5,
- celle s'étendant de l'extrémité constituée par le nucléotide en position (130) à l'extrémité constituée par le nucléotide en position (1104) représentée sur la figure 5,
- celle s'étendant de l'extrémité constituée par le nucléotide en position (130) à l'extrémité constituée par le nucléotide en position (1299) représentée sur la figure 5,
- celle s'étendant de l'extrémité constituée par le nucléotide en position (220) à l'extrémité constituée par le nucléotide en position (1299) représentée sur la figure 5,
ou leurs séquences complémentaires,
ou lesdites séquences nucléotidiques ci-dessus dans lesquelles T est remplacé par U.

51. Procédé pour préparer un acide nucléique selon la revendication 47, ledit acide nucléique étant constitué d'une des séquences nucléotidiques suivantes:
- celle s'étendant de l'extrémité constituée par le nucléotide en position (1) à l'extrémité constituée par le nucléotide en position (182) représentée sur la figure 3a et la figure 3b,
- celle s'étendant de l'extrémité constituée par le nucléotide en position (1) à l'extrémité constituée par le nucléotide en position (194) représentée sur la figure 3a et la figure 3b,
- celle s'étendant de l'extrémité constituée par le nucléotide en position (1) à l'extrémité constituée par le nucléotide en position (212) représentée sur la figure 3a et la figure 3b,
- celle s'étendant de l'extrémité constituée par le nucléotide en position (1) à l'extrémité constituée par le nucléotide en position (218) représentée sur la figure 3a et la figure 3b,
- celle s'étendant de l'extrémité constituée par le nucléotide en position (1) à l'extrémité constituée par le nucléotide en position (272) représentée sur la figure 3a et la figure 3b,
- celle s'étendant de l'extrémité constituée par le nucléotide en position (1) à l'extrémité constituée par le nucléotide en position (359) représentée sur la figure 3a et la figure 3b,
- celle s'étendant de l'extrémité constituée par le nucléotide en position (1) à l'extrémité constituée par le nucléotide en position (1241) représentée sur la figure 3a et la figure 3b,
- celle s'étendant de l'extrémité constituée par le nucléotide en position (1) à l'extrémité constituée par le nucléotide en position (1358) représentée sur la figure 3a et la figure 3b,
- celle s'étendant de l'extrémité constituée par le nucléotide en position (183) à l'extrémité constituée par le nucléotide en position (359) représentée sur la figure 3a et la figure 3b,
- celle s'étendant de l'extrémité constituée par le nucléotide en position (183) à l'extrémité constituée par le nucléotide en position (1241) représentée sur la figure 3a et la figure 3b,
- celle s'étendant de l'extrémité constituée par le nucléotide en position (183) à l'extrémité constituée par le nucléotide en position (1358), représentée sur la figure 3a et la figure 3b,
- celle s'étendant de l'extrémité constituée par le nucléotide en position (195) à l'extrémité constituée par le nucléotide en position (359) représentée sur la figure 3a et la figure 3b,
- celle s'étendant de l'extrémité constituée par le nucléotide en position (195) à l'extrémité constituée par le nucléotide en position (1241) représentée sur la figure 3a et la figure 3b,
- celle s'étendant de l'extrémité constituée par le nucléotide en position (195) à l'extrémité constituée par le nucléotide en position (1358) représentée sur la figure 3a et la figure 3b,
- celle s'étendant de l'extrémité constituée par le nucléotide en position (213) à l'extrémité constituée par le nucléotide en position (359) représentée sur la figure 3a et la figure 3b,
- celle s'étendant de l'extrémité constituée par le nucléotide en position (213) à l'extrémité constituée par le nucléotide en position (1241) représentée sur la figure 3a et la figure 3b,
- celle s'étendant de l'extrémité constituée par le nucléotide en position (213) à l'extrémité constituée par le nucléotide en position (1358) représentée sur la figure 3a et la figure 3b,
- celle s'étendant de l'extrémité constituée par le nucléotide en position (219) à l'extrémité constituée par le nucléotide en position (359) représentée sur la figure 3a et la figure 3b,
- celle s'étendant de l'extrémité constituée par le nucléotide en position (219) à l'extrémité constituée par le nucléotide en position (1241) représentée sur la figure 3a et la figure 3b,
- celle s'étendant de l'extrémité constituée par le nucléotide en position (219) à l'extrémité constituée par le nucléotide en position (1358) représentée sur la figure 3a et la figure 3b,
- celle s'étendant de l'extrémité constituée par le nucléotide en position (234) à l'extrémité constituée par le nucléotide en position (359) représentée sur la figure 3a et la figure 3b,
- celle s'étendant de l'extrémité constituée par le nucléotide en position (234) à l'extrémité constituée par le nucléotide en position (1241) représentée sur la figure 3a et la figure 3b,
- celle s'étendant de l'extrémité constituée par le nucléotide en position (234) à l'extrémité constituée par le nucléotide en position (1358) représentée sur la figure 3a et la figure 3b,
- celle s'étendant de l'extrémité constituée par le nucléotide en position (273) à l'extrémité constituée par le nucléotide en position (359) représentée sur la figure 3a et la figure 3b,
- celle s'étendant de l'extrémité constituée par le nucléotide en position (273) à l'extrémité constituée par le nucléotide en position (1241) représentée sur la figure 3a et la figure 3b,
- celle s'étendant de l'extrémité constituée par le nucléotide en position (273) à l'extrémité constituée par le nucléotide en position (1358) représentée sur la figure 3a et la figure 3b,
- celle s'étendant de l'extrémité constituée par le nucléotide en position (360) à l'extrémité constituée par le nucléotide en position (1241) représentée sur la figure 3a et la figure 3b,
- celle s'étendant de l'extrémité constituée par le nucléotide en position (360) à l'extrémité constituée par le nucléotide en position (1358) représentée sur la figure 3a et la figure 3b,
- celle s'étendant de l'extrémité constituée par le nucléotide en position (1242) à l'extrémité constituée par le nucléotide en position (1358) représentée sur la figure 3a et la figure 3b,
ou leurs séquences complémentaires,
ou lesdites séquences nucléotidiques ci-dessus dans lesquelles T est remplacé par U.

52. Procédé pour préparer un acide nucléique selon la revendication 47, ledit acide nucléique étant constitué d'une des séquences nucléotidiques suivantes:
- celle s'étendant de l'extrémité constituée par le nucléotide en position (1) à l'extrémité constituée par le nucléotide en position (182) représentée sur la figure 4a et la figure 4b,
- celle s'étendant de l'extrémité constituée par le nucléotide en position (1) à l'extrémité constituée par le nucléotide en position (194) représentée sur la figure 4a et la figure 4b,
- celle s'étendant de l'extrémité constituée par le nucléotide en position (1) à l'extrémité constituée par le nucléotide en position (212) représentée sur la figure 4a et la figure 4b,
- celle s'étendant de l'extrémité constituée par le nucléotide en position (1) à l'extrémité constituée par le nucléotide en position (218) représentée sur la figure 4a et la figure 4b,
- celle s'étendant de l'extrémité constituée par le nucléotide en position (1) à l'extrémité constituée par le nucléotide en position (272) représentée sur la figure 4a et la figure 4b,
- celle s'étendant de l'extrémité constituée par le nucléotide en position (1) à l'extrémité constituée par le nucléotide en position (359) représentée sur la figure 4a et la figure 4b,
- celle s'étendant de l'extrémité constituée par le nucléotide en position (1) à l'extrémité constituée par le nucléotide en position (1241) représentée sur la figure 4a et la figure 4b,
- celle s'étendant de l'extrémité constituée par le nucléotide en position (1) à l'extrémité constituée par le nucléotide en position (1358) représentée sur la figure 4a et la figure 4b,
- celle s'étendant de l'extrémité constituée par le nucléotide en position (183) à l'extrémité constituée par le nucléotide en position (359) représentée sur la figure 4a et la figure 4b,
- celle s'étendant de l'extrémité constituée par le nucléotide en position (183) à l'extrémité constituée par le nucléotide en position (1241) représentée sur la figure 4a et la figure 4b,
- celle s'étendant de l'extrémité constituée par le nucléotide en position (183) à l'extrémité constituée par le nucléotide en position (1358), représentée sur la figure 4a et la figure 4b,
- celle s'étendant de l'extrémité constituée par le nucléotide en position (195) à l'extrémité constituée par le nucléotide en position (359) représentée sur la figure 4a et la figure 4b,
- celle s'étendant de l'extrémité constituée par le nucléotide en position (195) à l'extrémité constituée par le nucléotide en position (1241) représentée sur la figure 4a et la figure 4b,
- celle s'étendant de l'extrémité constituée par le nucléotide en position (195) à l'extrémité constituée par le nucléotide en position (1358) représentée sur la figure 4a et la figure 4b,
- celle s'étendant de l'extrémité constituée par le nucléotide en position (213) à l'extrémité constituée par le nucléotide en position (359) représentée sur la figure 4a et la figure 4b,
- celle s'étendant de l'extrémité constituée par le nucléotide en position (213) à l'extrémité constituée par le nucléotide en position (1241) représentée sur la figure 4a et la figure 4b,
- celle s'étendant de l'extrémité constituée par le nucléotide en position (213) à l'extrémité constituée par le nucléotide en position (1358) représentée sur la figure 4a et la figure 4b,
- celle s'étendant de l'extrémité constituée par le nucléotide en position (219) à l'extrémité constituée par le nucléotide en position (359) représentée sur la figure 4a et la figure 4b,
- celle s'étendant de l'extrémité constituée par le nucléotide en position (219) à l'extrémité constituée par le nucléotide en position (1241) représentée sur la figure 4a et la figure 4b,
- celle s'étendant de l'extrémité constituée par le nucléotide en position (219) à l'extrémité constituée par le nucléotide en position (1358) représentée sur la figure 4a et la figure 4b,
- celle s'étendant de l'extrémité constituée par le nucléotide en position (234) à l'extrémité constituée par le nucléotide en position (359) représentée sur la figure 4a et la figure 4b,
- celle s'étendant de l'extrémité constituée par le nucléotide en position (234) à l'extrémité constituée par le nucléotide en position (1241) représentée sur la figure 4a et la figure 4b,
- celle s'étendant de l'extrémité constituée par le nucléotide en position (234) à l'extrémité constituée par le nucléotide en position (1358) représentée sur la figure 4a et la figure 4b,
- celle s'étendant de l'extrémité constituée par le nucléotide en position (273) à l'extrémité constituée par le nucléotide en position (359) représentée sur la figure 4a et la figure 4b,
- celle s'étendant de l'extrémité constituée par le nucléotide en position (273) à l'extrémité constituée par le nucléotide en position (1241) représentée sur la figure 4a et la figure 4b,
- celle s'étendant de l'extrémité constituée par le nucléotide en position (273) à l'extrémité constituée par le nucléotide en position (1358) représentée sur la figure 4a et la figure 4b,
- celle s'étendant de l'extrémité constituée par le nucléotide en position (360) à l'extrémité constituée par le nucléotide en position (1241) représentée sur la figure 4a et la figure 4b,
- celle s'étendant de l'extrémité constituée par le nucléotide en position (360) à l'extrémité constituée par le nucléotide en position (1358) représentée sur la figure 4a et la figure 4b,
- celle s'étendant de l'extrémité constituée par le nucléotide en position (1242) à l'extrémité constituée par le nucléotide en position (1358) représentée sur la figure 4a et la figure 4b,
ou leurs séquences complémentaires,
ou lesdites séquences nucléotidiques ci-dessus dans lesquelles T est remplacé par U.

53. Procédé pour préparer un acide nucléique selon la revendication 47, ledit acide nucléique étant constitué d'une des séquences nucléotidiques suivantes:
- celle s'étendant de l'extrémité constituée par le nucléotide en position (1) à l'extrémité constituée par le nucléotide en position (129) représentée sur la figure 5,
- celle s'étendant de l'extrémité constituée par le nucléotide en position (1) à l'extrémité constituée par le nucléotide en position (219) représentée sur la figure 5,
- celle s'étendant de l'extrémité constituée par le nucléotide en position (1) à l'extrémité constituée par le nucléotide en position (1104) représentée sur la figure 5,
- celle s'étendant de l'extrémité constituée par le nucléotide en position (1) à l'extrémité constituée par le nucléotide en position (1299) représentée sur la figure 5,
- celle s'étendant de l'extrémité constituée par le nucléotide en position (90) à l'extrémité constituée par le nucléotide en position (219) représentée sur la figure 5,
- celle s'étendant de l'extrémité constituée par le nucléotide en position (90) à l'extrémité constituée par le nucléotide en position (1104) représentée sur la figure 5,
- celle s'étendant de l'extrémité constituée par le nucléotide en position (90) à l'extrémité constituée par le nucléotide en position (1299) représentée sur la figure 5,
- celle s'étendant de l'extrémité constituée par le nucléotide en position (130) à l'extrémité constituée par le nucléotide en position (219) représentée sur la figure 5,
- celle s'étendant de l'extrémité constituée par le nucléotide en position (130) à l'extrémité constituée par le nucléotide en position (1104) représentée sur la figure 5,
- celle s'étendant de l'extrémité constituée par le nucléotide en position (130) à l'extrémité constituée par le nucléotide en position (1299) représentée sur la figure 5,
- celle s'étendant de l'extrémité constituée par le nucléotide en position (220) à l'extrémité constituée par le nucléotide en position (1104) représentée sur la figure 5,
- celle s'étendant de l'extrémité constituée par le nucléotide en position (220) à l'extrémité constituée par le nucléotide en position (1299) représentée sur la figure 5,
- celle s'étendant de l'extrémité constituée par le nucléotide en position (1105) à l'extrémité constituée par le nucléotide en position (1299) représentée sur la figure 5,
ou leurs séquences complémentaires,
ou lesdites séquences nucléotidiques ci-dessus dans lesquelles T est remplacé par U.

54. Procédé pour préparer un acide nucléique recombinant, consistant à insérer au moins une des séquences nucléotidiques selon les revendications 9 à 15 dans un acide nucléique hétérologue.

55. Procédé pour préparer une amorce d'ADN ou d'ARN, dans lequel ladite amorce est constituée de 15 à 25 nucléotides d'une séquence nucléotidique selon l'une quelconque des revendications 13 à 15 et peut être utilisée comme amorce pour amplifier spécifiquement l'une quelconque desdites séquences nucléotidiques, et dans lequel ledit procédé est réalisé par synthèse chimique par le procédé automatique au phosphoramidite de β-cyanoéthyle.

56. Procédé pour préparer une amorce d'ADN ou d'ARN selon la revendication 55, ladite amorce étant choisie dans le groupe suivant des séquences nucléotidiques:
A(i) CAGCTTGTTGACAGGGTTCGTGGC,
A(ii) GGTTCGTGGCGCCGTCACG,
A(iii) CGTCGCGCGCCTAGTGTCGG,
A(iv) CGGCGCCGTCGGTGGCACGGCGA,
A(v) CGTCGGCGCGGCCCTAGTGTCGG,
B TCGCCCGCCCTGTACCTG,
C GCGCTGACGCTGGCGATCTATC,
D CCGCTGTTGAACGTCGGGAAG,
E AAGCCGTCGGATCTGGGTGGCAAC,
F(i) ACGGCACTGGGTGCCACGCCCAAC,
F(ii) ACGCCCAACACCGGGCCCGCCGCA,
F(iii) ACGGGCACTGGGTGCCACGCCCAAC,
F(iv) ACGCCCCAACACCGGGCCCGCGCCCCA,
ou leurs séquences complémentaires ou les séquences ribonucléotidiques correspondantes.

57. Procédé pour préparer un jeu d'amorces d'ADN ou d'ARN pour l'amplification enzymatique d'une séquence nucléotidique selon l'une quelconque des revendications 13 à 15, ledit procédé comprenant l'étape de combinaison dans ledit jeu d'amorces:
- d'une séquence nucléotidique A avec la séquence nucléotidique complémentaire de B, de C, de D, de E ou de F; ou
- de la séquence nucléotidique B avec la séquence nucléotidique complémentaire de C, de D, de E ou de F; ou
- de la séquence nucléotidique C avec la séquence nucléotidique complémentaire de D, de E ou de F; ou
- de la séquence nucléotidique D avec la séquence nucléotidique complémentaire de E ou de F; ou
- de la séquence nucléotidique E avec une séquence nucléotidique complémentaire de F;
les séquences nucléotidiques A, B, C, D, E et F étant précisées dans la revendication 18, et A signifiant l'une quelconque des séquences A(i), A(ii), A(iii), A(iv) et A(v) et F signifiant l'une quelconque des séquences F(i), F (ii), F(iii) et F(iv).

58. Procédé pour préparer un vecteur recombinant, en particulier pour le clonage et/ou l'expression, comprenant l'étape d'insertion d'un acide nucléique selon l'une quelconque des revendications 9 à 15 dans une séquence de vecteur de type plasmide, cosmide ou phage, dans un des sites non essentiels pour sa réplication.

59. Procédé pour préparer un vecteur recombinant selon la revendication 58, dans lequel ledit vecteur contient, dans un des sites non essentiels pour sa réplication, les éléments nécessaires pour promouvoir l'expression d'un polypeptide selon l'une quelconque des revendications 1 à 6 dans un hôte cellulaire.

60. Procédé pour préparer un vecteur recombinant selon la revendication 58, dans lequel ledit vecteur contient les éléments permettant l'expression par E. coli d'un polypeptide selon l'une quelconque des revendications 1 à 6.

61. Procédé pour préparer un hôte cellulaire contenant un vecteur recombinant selon l'une quelconque des revendications 20 à 22 et comprenant les éléments de régulation permettant l'expression d'un polypeptide selon l'une quelconque des revendications 1 à 6 dans cet hôte, ledit procédé comprenant l'étape de transformation dudit hôte cellulaire par ledit vecteur recombinant.

62. Procédé pour préparer un hôte cellulaire selon la revendication 61, ledit hôte cellulaire étant choisi parmi les bactéries telles que E. coli transformées par un vecteur selon la revendication 20, ou choisi parmi les organismes eucaryotes transformés par un vecteur selon la revendication 20.

63. Procédé pour préparer un anticorps monoclonal spécifiquement dirigé contre un polypeptide recombinant selon l'une quelconque des revendications 1 à 6, ledit procédé comprenant la production dudit anticorps monoclonal par un hybridome qui est formé selon des procédés classiques à partir de splénocytes d'un animal, en particulier d'une souris ou d'un rat, immunisé contre un polypeptide selon l'une quelconque des revendications 1 à 6 d'une part, et de cellules d'une lignée cellulaire de myélome d'autre part, et qui est choisi en fonction de sa capacité à produire un anticorps monoclonal reconnaissant le polypeptide qui a été initialement utilisé pour l'immunisation des animaux.

64. Procédé pour préparer un sonde nucléotidique s'hybridant avec un acide nucléique selon l'une quelconque des revendications 13 à 15, ladite sonde nucléotidique étant choisie parmi le groupe suivant de séquences nucléotidiques:
| Sondes A(i), A(ii), A(iii), A(iv) et A(v) | |
|---|---|
| A(i) | CAGCTTGTTGACAGGGTTCGTGGC, |
| A(ii) | GGTTCGTGGCGCCGTCACG, |
| A(iii) | CGTCGCGCGCCTAGTGTCGG, |
| A(iv) | CGGCGCCGTCGGTGGCACGGCGA, |
| A(v) | CGTCGGCGCGGCCCTAGTGTCGG, |
| Sonde B | |
|---|---|
| TCGCCCGCCCTGTACCTG, | |
| Sonde C | |
|---|---|
| GCGCTGACGCTGGCGATCTATC, | |
| Sonde D | |
|---|---|
| CCGCTGTTGAACGTCGGGAAG, | |
| Sonde E | |
|---|---|
| AAGCCGTCGGATCTGGGTGGCAAC, | |
| Sondes F(i) et F(ii) | |
|---|---|
| F(i) | ACGGCACTGGGTGCCACGCCCAAC, |
| F(ii) | ACGCCCAACACCGGGCCCGCCGCA, |
| F(iii) | ACGGGCACTGGGTGCCACGCCCAAC, |
| F(iv) | ACGCCCCAACACCGGGCCCGCGCCCCA, |
| ou leurs séquences nucléotidiques complémentaires, | |
ledit procédé étant réalisé par synthèse chimique avec le procédé automatique au phosphoramidite de β-cyanoéthyle.

65. Procédé pour préparer une trousse pour un procédé diagnostique in vitro de la tuberculose chez un patient susceptible d'être infecté par Mycobacterium tuberculosis selon la revendication 28, ledit procédé comprenant l'étape consistant à regrouper au moins les éléments suivants :
- une quantité déterminée d'une sonde nucléotidique selon la revendication 26,
- un milieu approprié pour créer une réaction d'hybridation entre la séquence à détecter et ladite sonde,
- des réactifs permettant la détection du complexe d'hybridation formé entre la séquence nucléotidique et la sonde.

66. Procédé pour préparer une trousse pour un procédé diagnostique in vitro de la tuberculose chez un patient susceptible d'être infecté par Mycobacterium tuberculosis selon la revendication 29, ledit procédé comprenant l'étape consistant à regrouper au moins les éléments suivants:
- un polypeptide selon l'une quelconque des revendications 1 à 8,
- des réactifs pour préparer un milieu approprié pour qu'ait lieu la réaction immunologique,
- des réactifs permettant de détecter le complexe antigène/anticorps qui a été produit par la réaction immunologique, lesdits réactifs comportant éventuellement un marqueur, ou étant susceptibles d'être reconnus par un réactif marqué, plus particulièrement dans le cas où le polypeptide mentionné ci-dessus n'est pas marqué.

67. Procédé pour préparer une trousse pour un procédé diagnostique in vitro de la tuberculose chez un patient susceptible d'être infecté par Mycobacterium tuberculosis selon la revendication 30, ledit procédé comprenant l'étape consistant à regrouper au moins les éléments suivants:
- un anticorps selon la revendication 25,
- des réactifs pour préparer un milieu approprié pour qu'ait lieu la réaction immunologique,
- des réactifs permettant de détecter les complexes antigène/anticorps qui ont été produits par la réaction immunologique, lesdits réactifs comportant éventuellement un marqueur, ou étant susceptibles d'être reconnus par un réactif marqué, plus particulièrement dans le cas où le polypeptide mentionné ci-dessus n'est pas marqué.

68. Procédé pour préparer un polypeptide recombinant biologiquement pur selon l'une quelconque des revendications 1 à 8 à utiliser dans une composition immunogène en association avec un véhicule pharmaceutiquement acceptable, ledit procédé comprenant les étapes de
- production du polypeptide par manipulation génétique, à savoir par expression dans un hôte cellulaire des acides nucléiques codant pour lesdits polypeptides ou par synthèse chimique classique, et de
- purification du polypeptide produit.

69. Procédé pour préparer un polypeptide recombinant biologiquement pur selon l'une quelconque des revendications 1 à 8 à utiliser dans une composition de vaccin, ledit procédé comprenant les étapes de
- production du polypeptide par manipulation génétique, à savoir par expression dans un hôte cellulaire des acides nucléiques codant pour lesdits polypeptides ou par synthèse chimique classique, et de
- purification du polypeptide produit.

70. Procédé pour préparer un peptide selon la revendication 1, ledit peptide étant avantageusement utilisé pour produire des anticorps, en particulier des anticorps monoclonaux, et comportant au moins une des séquences d'acides aminés suivantes:
| Position de l'acide aminé (extrémité NH₂) | | Position de l'acide aminé (extrémité COOH) |
|---|---|---|
| 12 | QVPSPSMGRDIKVQFQSGGA | 31 |
| 36 | LYLLDGLRAQDDFSGWDINT | 55 |
| 77 | SFYSDWYQPACRKAGCATYK | 96 |
| 101 | LTSELPGWLQANRHVKPTGS | 120 |
| 175 | KASDMWGPKEDPAWQRNDPL | 194 |
| 211 | CGNGKPSDLGGNNLPAKFLE | 230 |
| 275 | KPDLQRHWVPRPTPGPPQGA | 294 |
| 77 | SFYSDWYQPACGKAGCQTYK | 96 |
| 276 | PDLQRALGATPNTGPAPQGA | 295 |
ledit procédé comprenant les étapes de
- production du peptide par manipulation génétique, à savoir par expression dans un hôte cellulaire de l'acide nucléique codant pour ledit peptide ou par synthèse chimique classique, et de
- purification du peptide produit.
